(19) Europäisches Patentamt
European Patent Office
Office européen des brevets

(11) EP 4 421 074 A1

(12) **EUROPEAN PATENT APPLICATION**
published in accordance with Art. 153(4) EPC

(43) Date of publication:
**28.08.2024 Bulletin 2024/35**

(21) Application number: **22882968.5**

(22) Date of filing: **21.10.2022**

(51) International Patent Classification (IPC):
***C07D 471/04*** (2006.01) ***C07D 401/12*** (2006.01)
***A61K 31/498*** (2006.01) ***A61P 35/00*** (2006.01)

(52) Cooperative Patent Classification (CPC):
**A61K 31/498; A61P 35/00; C07D 401/12; C07D 471/04**

(86) International application number:
**PCT/CN2022/126611**

(87) International publication number:
**WO 2023/066363 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.10.2021 CN 202111232944**
**19.11.2021 CN 202111374462**
**28.01.2022 CN 202210103868**
**11.04.2022 CN 202210375573**
**18.05.2022 CN 202210546650**
**07.06.2022 CN 202210636634**
**13.10.2022 CN 202211252554**

(71) Applicant: **Xizang Haisco Pharmaceutical Co., Ltd.**
**Lhoka, Tibet 856099 (CN)**

(72) Inventors:
- **LI, Yao**
**Shannan City Tibet 856000 (CN)**
- **CHEN, Lei**
**Shannan City Tibet 856000 (CN)**
- **HE, Tiancheng**
**Shannan City Tibet 856000 (CN)**
- **GENG, Pengxin**
**Shannan City Tibet 856000 (CN)**
- **YAO, Hao**
**Shannan City Tibet 856000 (CN)**
- **WANG, Haodong**
**Shannan City Tibet 856000 (CN)**
- **FANG, Linyong**
**Shannan City Tibet 856000 (CN)**
- **HU, Gang**
**Shannan City Tibet 856000 (CN)**
- **TANG, Pingming**
**Shannan City Tibet 856000 (CN)**
- **YU, Yan**
**Shannan City Tibet 856000 (CN)**
- **ZHANG, Chen**
**Shannan City Tibet 856000 (CN)**
- **YAN, Pangke**
**Shannan City Tibet 856000 (CN)**

(74) Representative: **Sagittarius IP**
**Marlow International**
**Parkway**
**Marlow SL7 1YL (GB)**

# (54) PARP-1 DEGRADATION AGENT AND USE THEREOF

(57) A compound represented by formula (I), a stereoisomer, deuterated compound, solvate, pharmaceutically acceptable salt or co-crystal thereof, or a pharmaceutical composition containing them, and use of the compound as a PARP-1 inhibitor in the preparation of a drug for treating related diseases, each group in the formula (I) being as defined in the description.

PTM-L-ULM (I)

EP 4 421 074 A1

## Description

### Technical Field

The present invention belongs to the field of drugs, and in particular relates to a small molecule compound having a PARP-1 degradation activity, or a stereoisomer, pharmaceutically acceptable salt, solvate, co-crystal or deuterated compound thereof, and the use thereof in the preparation of a drug for treating related diseases.

### Background Art

Approximately 5% of breast cancer patients are associated with germline mutations in the BRCA1/2 genes (3% in the BRCA1 gene and 2% in the BRCA2 gene). Most breast cancers caused by BRCA1 mutations are triple-negative breast cancers (70%), while BRCA2 mutations are more likely to cause estrogen receptor-positive breast cancers (70%). The BRCA1/2 genes are tumour suppressor genes and play an important role in DNA damage repair, normal cell growth, etc. Mutations in the genes can inhibit the normal repair ability after DNA damage and cause homologous recombination deficiency (HRD), that is, loss of BRCA function or mutation or loss of function in other homologous recombination-related genes, making repair of DNA double-strand breaks impossible through homologous recombination repair (HRR), eventually leading to cancer.

PARP1 is short for poly(ADP-ribose) polymerase 1. It was first reported more than 50 years ago, and since then, it has been gradually discovered to play an important role in DNA repair, maintenance of gene integrity, and regulation of various metabolic and signalling processes. PARP1 can catalyse the transfer of an ADP-ribose residue from NAD+ to a target substrate, thereby constructing a poly(ADP-ribose) chain (referred to as the PAR chain). PARP plays a key role in the process of DNA single-strand base excision and repair. In HRD tumour cells, DNA double-strand breaks cannot be repaired, and PARP inhibitors block the single-strand repair, resulting in a "synthetic lethal" effect, leading to tumour cell death.

PARP inhibitors have a "trapping" effect on the PARP protein, causing the PARP protein that binds to damaged DNA to be trapped on the DNA, directly causing other DNA repair proteins to be unable to bind, eventually leading to cell death. However, conventional small-molecule PARP inhibitors cannot affect the content of PARP protein. Once the drug is withdrawn, PARP protein can quickly resume activity and drug resistance is prone to occur. In addition, some side effects of PARP inhibitors, including blood toxicity such as thrombocytopenia, may also be related to this "trapping" effect. PARP-PROTAC blocks the catalytic and scaffolding functions of PARP1 without causing PARP1 trapping.

Developing druggable PARP-PROTACs remains an unmet clinical need.

### Summary of the Invention

The present invention develops a novel PARP-PROTAC, which can effectively solve the problems associated with the existing PARP inhibitors, has good efficacy, high safety, less side effects, and relatively high PARP1 inhibitory selectivity and good pharmacokinetic characteristics, and has good prospects for clinical development.

Provided is a PARP-1 degradation agent represented by formula (I), or a stereoisomer, deuterated compound, solvate, pharmaceutically acceptable salt or co-crystal thereof,

PTM-L-ULM       I

wherein ULM is an E3 ubiquitin ligase binding moiety;

L is a bond or chemically linking moiety that covalently couples the PTM and ULM;

the PTM is a PARP-1 target binding moiety;

the L of the present invention forms a covalent bonding, including single covalent bonding or multiple covalent bonding, with PTM and ULM at any site. Single covalent bonding means for example forming a covalent bond by replacing one H on PTM with L. Multiple covalent bonding includes bonding with two covalent bonds or three covalent bonds. As examples of bonding with two covalent bonds, two H or two other groups on the same atom in PTM are replaced with L to form a double bond, or two H or two other groups on different atoms in PTM are replaced with L to form a ring, such as a monocyclic ring, fused rings or spiro rings. As examples of bonding with three covalent bonds, three H on the same atom in PTM are replaced with L to form a triple bond, or three H on different atoms in PTM are replaced with L to form bridged rings;

in some specific embodiments, the PTM has the structure of formula (I-1), the L covalently binds to the PTM by replacing any H in formula (I-1) and thus forming a single bond with the PTM, or L covalently binds to PTM by forming, together with $R_4$ and $R_5$ and the N atom to which they are attached, 4- to 7-membered heterocycloalkyl

containing 1-2 N atoms and 0-2 heteroatoms selected from S and O;

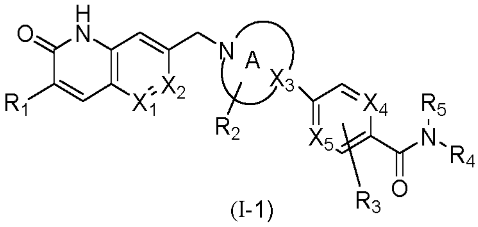

(I-1) ;

in some specific embodiments, the PTM has a structure of formula (I-1) or (I-2), the L covalently binds to the PTM by replacing any H in formula (I-1) or (I-2) and thus forming a single bond with the PTM, or L covalently binds to PTM by forming, together with $R_4$ and $R_5$ and the N atom to which they are attached, 4- to 10-membered heterocycloalkyl containing 1-2 N atoms and 0-2 heteroatoms selected from S and O;

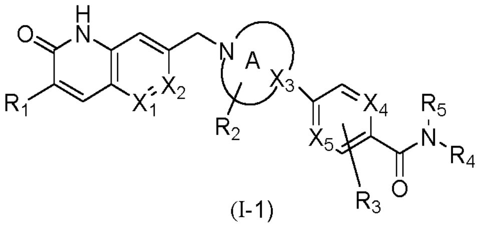

(I-1)

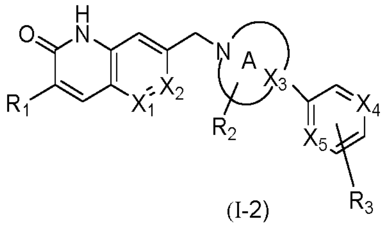

(I-2) ;

In some specific embodiments, the PTM has a structure of formula (I-1), (I-2), (I-3) or (I-4), the L covalently binds to the PTM by replacing any H in formula (I-1), (I-2), (I-3) or (I-4) and thus forming a single bond with the PTM, or L covalently binds to PTM by forming, together with $R_4$ and $R_5$ and the N atom to which they are attached, 4-to 10-membered heterocycloalkyl containing 1-2 N atoms and 0-2 heteroatoms selected from S and O;

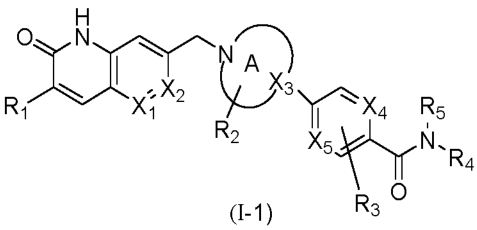

(I-1)

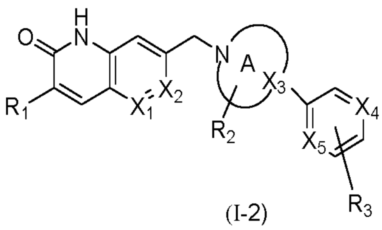

(I-2)

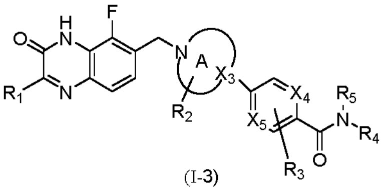

(I-3)

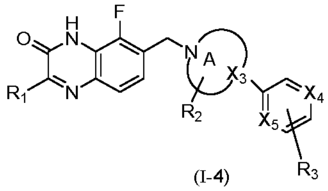

(I-4) ;

in the present invention, the phrase "1-2 N atoms" as described in "L ... by forming, together with $R_4$ and $R_5$ and the N atom to which they are attached, 4- to 10-membered heterocycloalkyl containing 1-2 N atoms and 0-2 heteroatoms selected from S and O" refers to instances where the heterocycloalkyl further comprises 0-1 additional N atom in addition to the N atom to which $R_4$ and $R_5$ are attached;

in some specific embodiments, the PARP-1 degradation agent has a structure of formula (II), (III) or (IV):

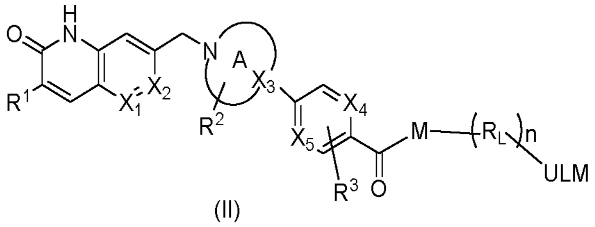

(II)

,

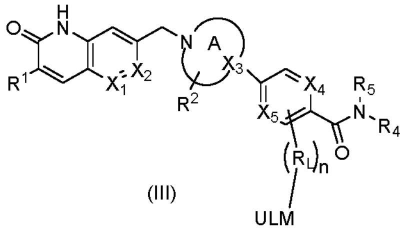

(III)

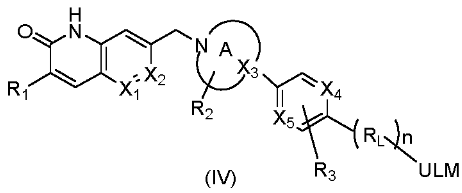

(IV) ;

in some specific embodiments, the PARP-1 degradation agent has a structure of formula (II), (III), (IV), (V), (VI) or (VII):

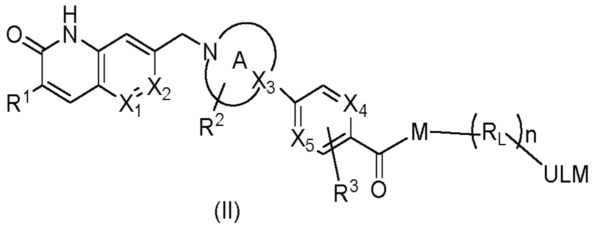

(II)

,

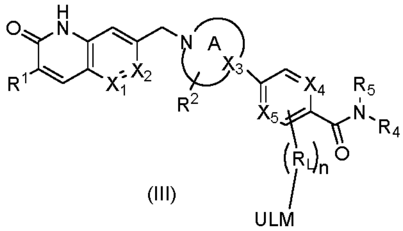

(III)

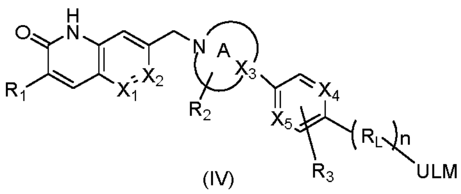

(IV)

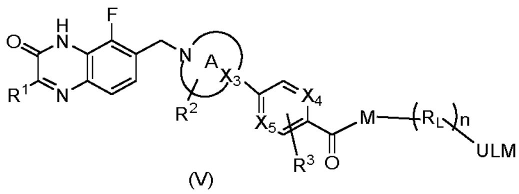

(V)

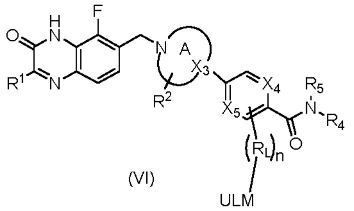

(VI)

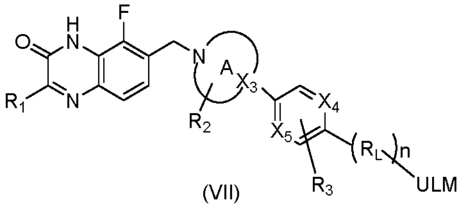

(VII) ;

in some specific embodiments, the PARP-1 degradation agent has a structure of formula (II-1), (III-1), (IV-1), (II-2), (III-2) or (IV-2):

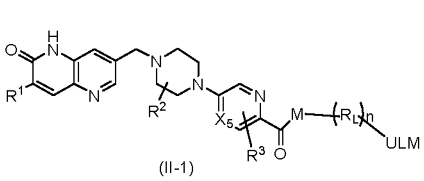

(II-1)

,

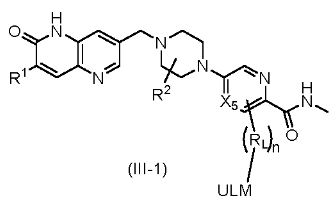

(III-1)

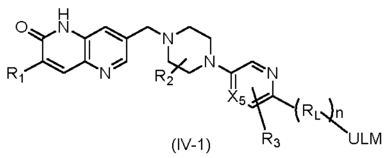
(IV-1)

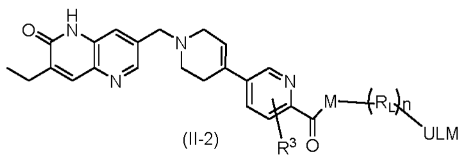
(II-2)

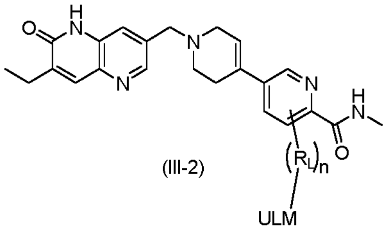
(III-2)

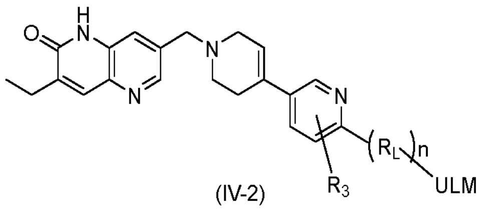
(IV-2) ;

in some specific embodiments, the PARP-1 degradation agent has a structure of formula (II-1), (III-1), (IV-1), (II-2), (III-2), (IV-2), (V-1), (VI-1), (VII-1), (V-2), (VI-2) or (VII-2):

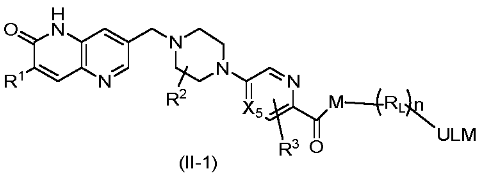
(II-1) ,

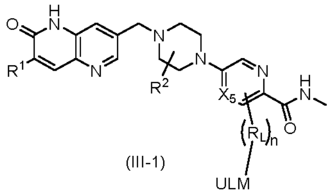
(III-1)

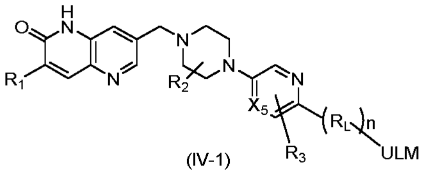
(IV-1) ,

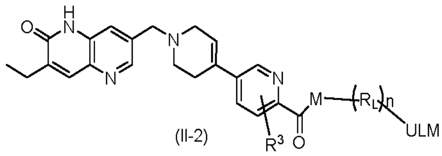
(II-2)

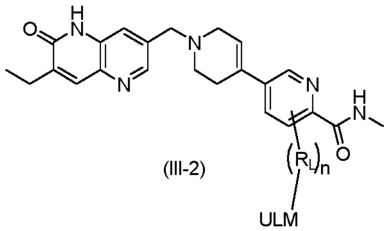
(III-2) ,

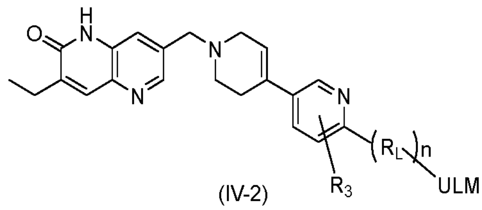
(IV-2)

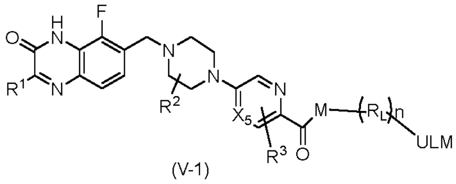
(V-1)

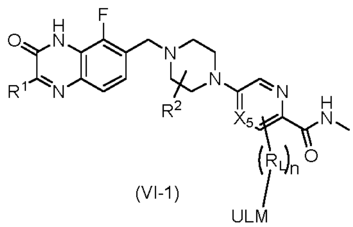
(VI-1)

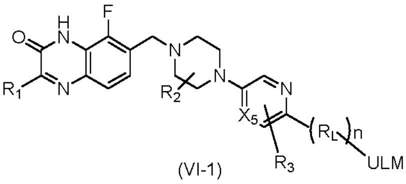
(VI-1)

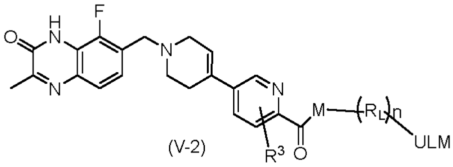
(V-2)

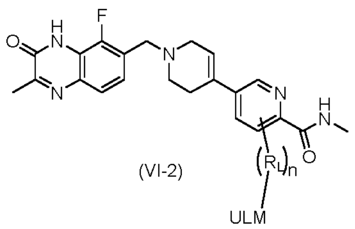
(VI-2)

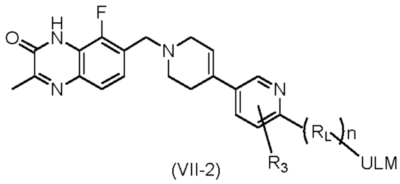
(VII-2)

;

in some specific embodiments, the PARP-1 degradation agent has a structure of formula (I-1), (I-2), (II), (III), (IV), (II-1), (III-1), (IV-1), (II-2), (III-2), (IV-2), (V-1), (VI-1), (VII-1), (V-2), (VI-2) or (VII-2), wherein

$X_1$, $X_2$, $X_3$, $X_4$ and $X_5$ are independently N or $CR_6$; in some specific embodiments, $X_1$, $X_2$, $X_3$, $X_4$ and $X_5$ are independently N or CH; in some specific embodiments, $X_1$, $X_3$ and $X_4$ are N, $X_2$ is CH, and $X_5$ is N or CH;

ring A is 4- to 12-membered heterocycloalkyl containing 1-2 N atoms and 0-2 heteroatoms selected from S and O; in the present invention, the phrase "1-2 N atoms" as described in "ring A is 4- to 12-membered heterocycloalkyl containing 1-2 N atoms and 0-2 heteroatoms selected from S and O" refers to instances where the heterocycloalkyl further comprises 0-1 additional N atom in addition to the N atom already shown in ring A; or refers to instances where $X_3$ is also an N atom in addition to the N atom already shown in formula A, in which case, the heterocycloalkyl further comprises 0 additional N atoms; in some specific embodiments, ring A is 4-to 12-membered monocyclic heterocycloalkyl, spiro heterocycloalkyl, bridged heterocycloalkyl or fused heterocycloalkyl containing 1-2 N atoms and 0-2 heteroatoms selected from S and O; in some specific embodiments, ring A is a piperidine ring, a piperazine ring, a tetrahydropyrrole ring,

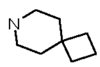, 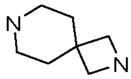,

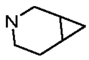 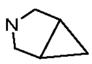, 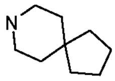, 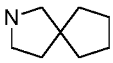, 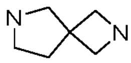, 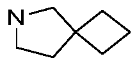, 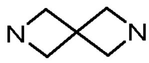,

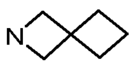, 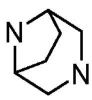, 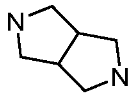, 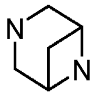, 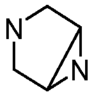, 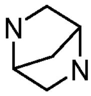, 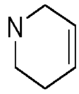,

etc.;

$R_1$ is H, $C_{1-4}$ alkyl, halogen, CN or $C_{3-6}$ cycloalkyl, wherein the alkyl and cycloalkyl are optionally substituted with 1-3 groups selected from halogen, OH, $NH_2$ and COOH; in some specific embodiments, $R_1$ is H or $C_{1-3}$ alkyl, wherein the alkyl is optionally substituted with 1-3 groups selected from F, Cl, Br, I, OH, $NH_2$, $C_{1-4}$ alkoxy and COOH; in some specific embodiments, $C_{1-3}$ alkyl is methyl, ethyl, propyl or isopropyl;

$R_2$ is H, $C_{1-4}$ alkyl, $C_{1-4}$ alkoxy, halogen, OH or $NH_2$, wherein the alkyl and alkoxy are optionally substituted with 1-3 groups selected from halogen, OH, $NH_2$, COOH and $C_{3-6}$ cycloalkyl; in some specific embodiments, $R_2$ is H, $C_{1-3}$ alkyl, $C_{1-3}$ alkoxy, F, Cl, Br or I; in some specific embodiments, $R_2$ is H; in some specific embodiments, $C_{1-3}$ alkyl is methyl, ethyl, propyl or isopropyl; $C_{1-3}$ alkoxy is methoxy, ethoxy, propoxy or isopropoxy;

$R_3$ is H, $C_{1-4}$ alkyl, halogen, $C_{1-4}$ alkoxy or $NH_2$, wherein the alkyl and alkoxy are optionally substituted with 1-3 groups selected from halogen, OH, $NH_2$, COOH and $C_{3-6}$ cycloalkyl; in some specific embodiments, $R_3$ is H, $C_{1-3}$ alkyl, $C_{1-3}$ alkoxy, F, Cl, Br or I; in some specific embodiments, $R_3$ is H; in some specific embodiments, $C_{1-3}$ alkyl is methyl, ethyl, propyl or isopropyl; $C_{1-3}$ alkoxy is methoxy, ethoxy, propoxy or isopropoxy;

$R_4$ is H, $C_{1-4}$ alkyl, $C_{3-6}$ cycloalkyl or 4- to 7-membered heterocycloalkyl containing 1-3 heteroatoms selected from N, S and O, wherein the alkyl, cycloalkyl and heterocycloalkyl are optionally substituted with 1-3 groups selected from halogen, OH, $NH_2$, COOH and $C_{3-6}$ cycloalkyl; in some specific embodiments, $R_4$ is H, methyl, ethyl, propyl, cyclopropyl, cyclobutyl, etc.;

$R_5$ is H, $C_{1-4}$ alkyl or $C_{3-6}$ cycloalkyl, wherein the alkyl and cycloalkyl are optionally substituted with 1-3 groups selected from halogen, OH, $NH_2$ and COOH; in some specific embodiments, $R_5$ is H, methyl, ethyl, propyl, cyclopropyl, cyclobutyl, etc.;

optionally, $R_4$ and $R_5$ together with the N atom to which they are attached form 4- to 7-membered heterocycloalkyl containing 1-3 heteroatoms selected from N, S and O, wherein the heterocycloalkyl is optionally substituted with

1-3 groups selected from halogen, OH, $NH_2$, COOH and $C_{3-6}$ cycloalkyl; or optionally, $R_4$ and $R_5$ together with the N atom to which they are attached form 4- to 10-membered heterocycloalkyl containing 1-3 heteroatoms selected from N, S and O, wherein the heterocycloalkyl is optionally substituted with 1-3 groups selected from halogen, OH, $NH_2$, COOH and $C_{3-6}$ cycloalkyl; in some specific embodiments, $R_4$ and $R_5$ together with the N atom to which they are attached form 4- to 6-membered monocyclic heterocycloalkyl or 7- to 10-membered bicyclic spiro heterocycloalkyl, which contains 1, 2 or 3 heteroatoms selected from N, S and O;

each $R_6$ is independently H, $C_{1-4}$ alkyl, halogen, CN or $C_{3-6}$ cycloalkyl, wherein the alkyl or cycloalkyl is optionally substituted with 1-3 groups selected from halogen, OH, $NH_2$, $C_{1-4}$ alkoxy and COOH; in some specific embodiments, $R_6$ is independently H;

M is $-NR_5-$ or

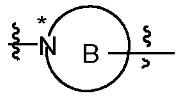,

ring B is 4- to 10-membered heterocycloalkyl containing 1-2 N atoms and 0-2 heteroatoms selected from S and O, and * is the end connected to group on the left side; in the present invention, the phrase "1-2 N atoms" as described in "ring B is 4- to 10-membered heterocycloalkyl containing 1-2 N atoms and 0-2 heteroatoms selected from S and O" refers to instances where the heterocycloalkyl further comprises 0-1 additional N atom in addition to the N atom already shown in ring B; in some specific embodiments, M is -NH- or 4- to 10-membered monocyclic heterocycloalkyl or spiro heterocycloalkyl containing 1-2 N atoms and 0-2 heteroatoms selected from S and O; in some specific embodiments, M is a piperidine ring, a piperazine ring, a tetrahydropyrrole ring, azetidinyl,

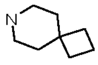,

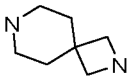, 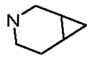 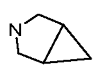, 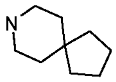, 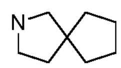, 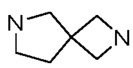, 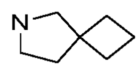,

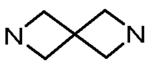, 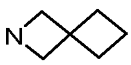, 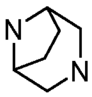, 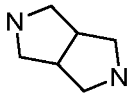, 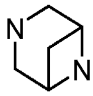, 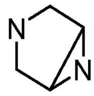, 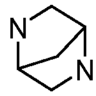, 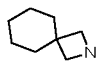,

etc., the ring N atom is the end connected to PTM;

each $R_L$ is independently $-NR_N-$, $C_{1-3}$ alkylene, -CO-, -O-, -S-, $C_{3-6}$ cycloalkylene, phenyl, 5- to 6-membered heteroaryl containing 1-3 heteroatoms selected from N, S and O, 4- to 10-membered heterocycloalkylene containing 1-3 heteroatoms selected from N, S and O, $C_{2-6}$ alkenylene, or $C_{2-6}$ alkynylene, wherein the alkylene, alkenylene, alkynylene, cycloalkylene, phenyl, heteroaryl and heterocycloalkylene are optionally substituted with 1-3 groups selected from $C_{1-4}$ alkyl, halogen, CN, $C_{1-4}$ alkoxy and halo $C_{1-4}$ alkoxy; in some specific embodiments, each $R_L$ is independently -NH-, $C_{1-3}$ alkylene, -CO-, -O-, $C_{3-6}$ cycloalkylene, phenyl, 5- to 6-membered heteroaryl containing 1-3 heteroatoms selected from N, S and O, 4- to 10-membered heterocycloalkylene containing 1-3 heteroatoms selected from N, S and O, $C_{2-6}$ alkenylene, or $C_{2-6}$ alkynylene, wherein the alkylene, alkenylene, alkynylene, phenyl, heteroaryl, cycloalkylene and heterocycloalkylene are optionally substituted with 1-3 groups selected from $C_{1-3}$ alkyl, F, Cl, Br, I, CN, $C_{1-3}$ alkoxy and halo $C_{1-3}$ alkoxy; in some specific embodiments, each $R_L$ is independently $-NR_N-$, $C_{1-3}$ alkylene, -CO-, -O-, -S-, $C_{3-6}$ cycloalkylene, phenyl, or 4- to 7-membered heterocycloalkylene containing 1-3 heteroatoms selected from N, S and O, wherein the alkylene, cycloalkylene, phenyl and heterocycloalkylene are optionally substituted with 1-3 groups selected from $C_{1-4}$ alkyl, halogen, CN, $C_{1-4}$ alkoxy and halo $C_{1-4}$ alkoxy; in some specific embodiments, each $R_L$ is independently -NH-, $C_{1-3}$ alkylene, -O-, $C_{3-6}$ cycloalkylene, phenyl, or 4- to 7-membered heterocycloalkylene containing 1-3 heteroatoms selected from N, S and O, wherein the alkylene, phenyl, cycloalkylene and heterocycloalkylene are optionally substituted with 1-3 groups selected from $C_{1-3}$ alkyl, F, Cl, Br, I, CN, $C_{1-3}$ alkoxy and halo $C_{1-3}$ alkoxy; in some specific embodiments, each $R_L$ is independently methylene, ethylidene, propylene, NH, CO, O, S, piperidylidene, piperazinylene, tetrahydropyrrolidene, azetidinylene,

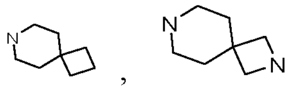,

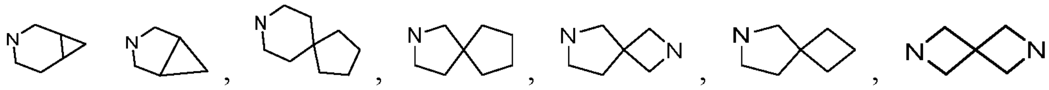

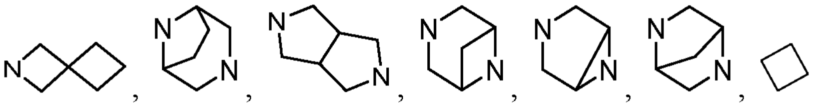, or 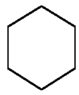,

and the connection site is any two identical or different non-H atoms in the groups; in some specific embodiments, each $R_L$ is independently methylene, ethylidene, propylene, NH, CO, O, piperidylidene, piperazinylene, tetrahydropyrrolidene, azetidinylene,

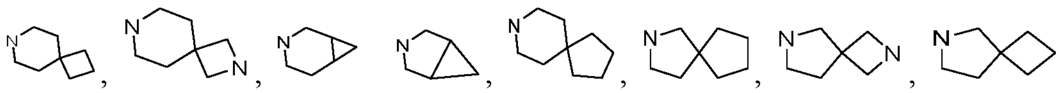

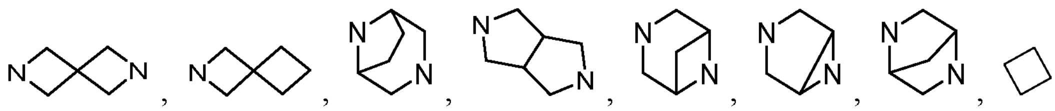

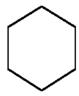,

phenyl, pyrazolyl, imidazolyl, pyridyl, thiazolyl, thienyl, pyridazinyl, pyrazinyl, pyrimidyl, vinylene, propenylene, ethynylene, propynylene, and the connection site is any two identical or different non-H atoms in the groups, provided that in -M-$(R_L)$n-, two identical -$NR_N$-, -O- or -S- are not attached;

$R_N$ is H or $C_{1-4}$ alkyl; in some specific embodiments, $R_N$ is H, methyl, ethyl or propyl;

n is an integer of 0-10; in some specific embodiments, n is an integer of 2-10; in some specific embodiments, n is 0, 1, 2, 3, 4, 5, 6, 7 or 8; in some specific embodiments, n is 1, 2, 3, 4, 5, 6 or 7; in some specific embodiments, n is 2, 3, 4, 5, 6, 7 or 8; in some specific embodiments, n is 2, 3, 4, 5, 6 or 7;

ULM is selected from U1, U2, U3, U4, U5, U6 or U7, in some embodiments, ULM is selected from U1, U2 or U3:

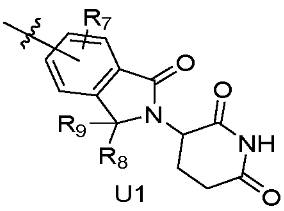
U1 ,

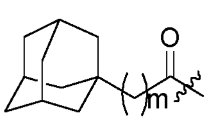
U2 ,

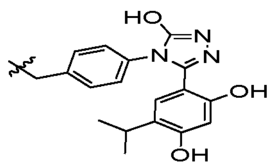
U3

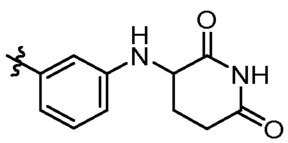
U4 ,

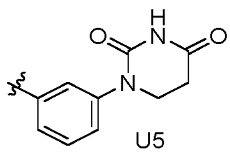
U5 ,

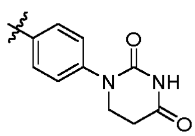
U6 ,

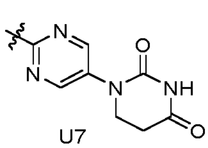
U7 ; in some specific embodiments, ULM is selected from

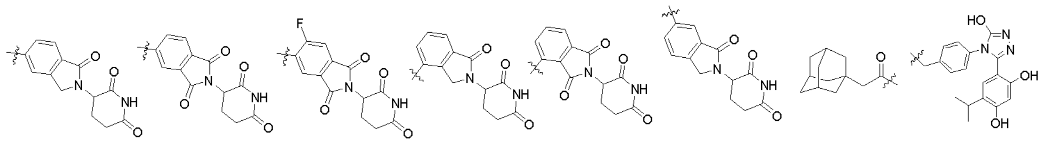

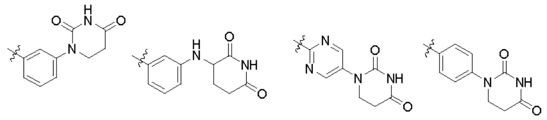

In some specific embodiments, ULM is selected from

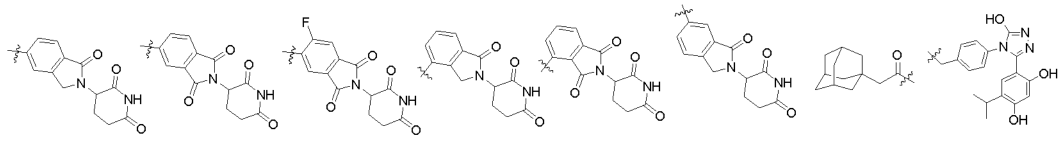

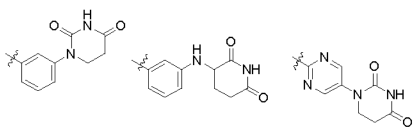

;

m is 1, 2 or 3; in some specific embodiments, m is 1 or 2;

$R_7$ is H, halogen, CN, $NH_2$, OH, $C_{1-4}$ alkyl or $C_{1-4}$ alkoxy, wherein the alkyl or alkoxy is optionally substituted with 1-3 groups selected from halogen, CN, $NH_2$ and OH; in some specific embodiments, $R_7$ is H, F, Cl, Br, I, CN, $NH_2$ or OH;

$R_8$ and $R_9$ are H or together form =O.

Specifically, as a first technical solution of the present invention, provided is a PARP-1 degradation agent represented by formula (I), or a stereoisomer, deuterated compound, solvate, pharmaceutically acceptable salt or co-crystal thereof,

PTM-L-ULM (I)

wherein ULM is an E3 ubiquitin ligase binding moiety;

L is a bond or chemically linking moiety that covalently couples the PTM and ULM;

the PTM has a structure of formula (I-1), (I-2), (I-3) or (I-4), the L covalently binds to the PTM by replacing any H in formula (I-1), (I-2), (I-3) or (I-4) and thus forming a single bond with the PTM, or L covalently binds to PTM by forming, together with $R_4$ and $R_5$ and the N atom to which they are attached, 4- to 10-membered heterocycloalkyl containing 1-2 N atoms and 0-2 heteroatoms selected from S and O;

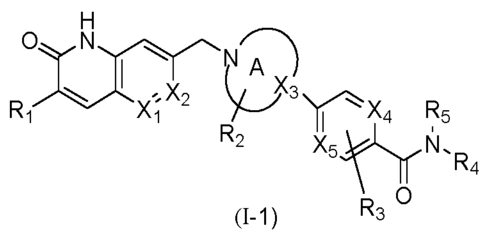

(I-1)

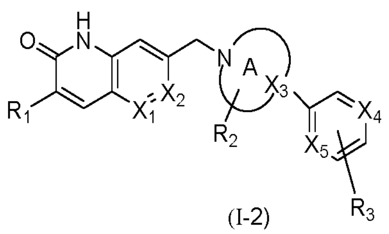

(I-2)

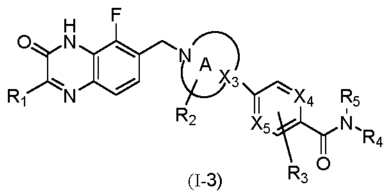

(I-3)

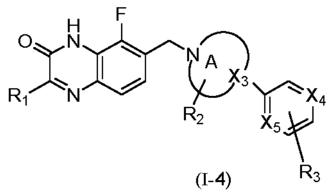

(I-4)

$X_1$, $X_2$, $X_3$, $X_4$ and $X_5$ are independently N or $CR_6$;
ring A is 4- to 12-membered heterocycloalkyl containing 1-2 N atoms and 0-2 heteroatoms selected from S and O;
$R_1$ is H, $C_{1-4}$ alkyl, halogen, CN or $C_{3-6}$ cycloalkyl, wherein the alkyl and cycloalkyl are optionally substituted with 1-3 groups selected from halogen, OH, $NH_2$, $C_{1-4}$ alkoxy and COOH;
$R_2$ is H, $C_{1-4}$ alkyl, $C_{1-4}$ alkoxy, halogen, OH or $NH_2$, wherein the alkyl and alkoxy are optionally substituted with 1-3 groups selected from halogen, OH, $NH_2$, COOH and $C_{3-6}$ cycloalkyl;
$R_3$ is H, $C_{1-4}$ alkyl, halogen, $C_{1-4}$ alkoxy or $NH_2$, wherein the alkyl and alkoxy are optionally substituted with 1-3 groups selected from halogen, OH, $NH_2$, COOH and $C_{3-6}$ cycloalkyl;
$R_4$ is H, $C_{1-4}$ alkyl, $C_{3-6}$ cycloalkyl or 4- to 7-membered heterocycloalkyl containing 1-3 heteroatoms selected from N, S and O, wherein the alkyl, cycloalkyl and heterocycloalkyl are optionally substituted with 1-3 groups selected from halogen, OH, $NH_2$, COOH and $C_{3-6}$ cycloalkyl;
$R_5$ is H, $C_{1-4}$ alkyl or $C_{3-6}$ cycloalkyl, wherein the alkyl and cycloalkyl are optionally substituted with 1-3 groups selected from halogen, OH, $NH_2$ and COOH;
optionally, $R_4$ and $R_5$ together with the N atom to which they are attached form 4- to 10-membered heterocycloalkyl containing 1-3 heteroatoms selected from N, S and O, wherein the heterocycloalkyl is optionally substituted with 1-3 groups selected from halogen, OH, $NH_2$, COOH and $C_{3-6}$ cycloalkyl;
each $R_6$ is independently H, $C_{1-4}$ alkyl, halogen, CN or $C_{3-6}$ cycloalkyl, wherein the alkyl or cycloalkyl is optionally substituted with 1-3 groups selected from halogen, OH, $NH_2$, $C_{1-4}$ alkoxy and COOH.

As a second technical solution of the present invention, provided is a PARP-1 degradation agent, or a stereoisomer, deuterated compound, solvate, pharmaceutically acceptable salt or co-crystal thereof, the PARP-1 degradation agent having a structure of formula (II) or (III):

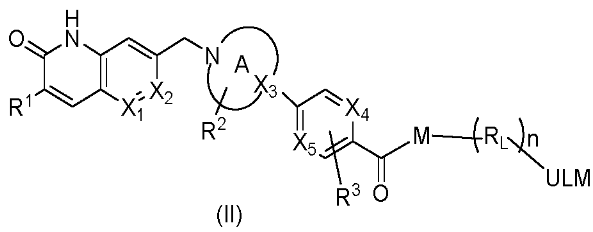

(II)

,

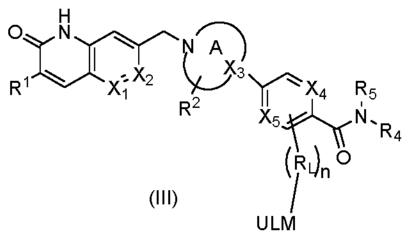

(III)

wherein ring A is 4- to 12-membered monocyclic heterocycloalkyl, spiro heterocycloalkyl, bridged heterocycloalkyl or fused heterocycloalkyl containing 1-2 N atoms and 0-2 heteroatoms selected from S and O;
M is $-NR_5-$ or

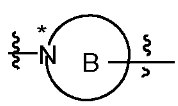

,

ring B is 4- to 10-membered heterocycloalkyl containing 1-2 N atoms and 0-2 heteroatoms selected from S and O, and * is the end connected to group on the left side;
each $R_L$ is independently $-NR_N-$, $C_{1-3}$ alkylene, -CO-, -O-, -S-, $C_{3-6}$ cycloalkylene, phenyl, or 4- to 7-membered heterocycloalkylene containing 1-3 heteroatoms selected from N, S and O, wherein the alkylene, cycloalkylene, phenyl and heterocycloalkylene are optionally substituted with 1-3 groups selected from $C_{1-4}$ alkyl, halogen, CN, $C_{1-4}$ alkoxy and halo $C_{1-4}$ alkoxy;
provided that in $-M-(R_L)n-$, two identical $-NR_N-$, -O- or -S- are not attached;
$R_N$ is H or $C_{1-4}$ alkyl;
n is an integer of 0-10, further an integer of 2-10;

ULM is selected from U1, U2 or U3:

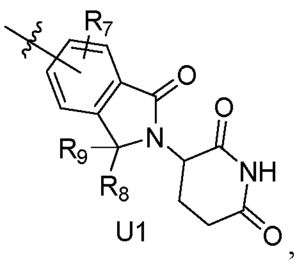
U1,
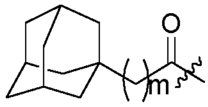
U2,
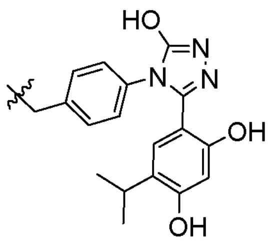
U3;

m is 1, 2 or 3;
$R_7$ is H, halogen, CN, $NH_2$, OH, $C_{1-4}$ alkyl or $C_{1-4}$ alkoxy, wherein the alkyl or alkoxy is optionally substituted with 1-3 groups selected from halogen, CN, $NH_2$ and OH;
$R_8$ and $R_9$ are H or together form =O.

As a third technical solution of the present invention, provided is a PARP-1 degradation agent, or a stereoisomer, deuterated compound, solvate, pharmaceutically acceptable salt or co-crystal thereof, the PARP-1 degradation agent having a structure of formula (II), (III) or (IV):

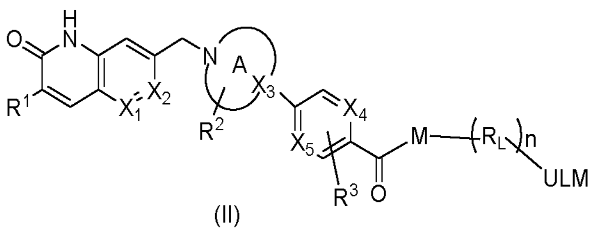
(II),
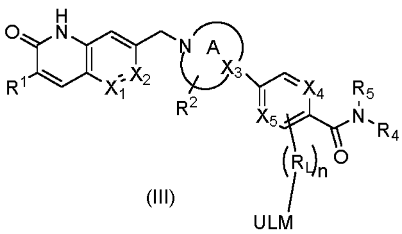
(III)

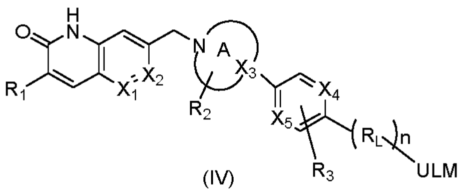
(IV)

wherein ring A is 4- to 12-membered monocyclic heterocycloalkyl, spiro heterocycloalkyl, bridged heterocycloalkyl or fused heterocycloalkyl containing 1-2 N atoms and 0-2 heteroatoms selected from S and O;
M is $-NR_5-$ or

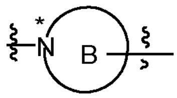,

ring B is 4- to 10-membered heterocycloalkyl containing 1-2 N atoms and 0-2 heteroatoms selected from S and O, and * is the end connected to group on the left side;
each $R_L$ is independently $-NR_N-$, $C_{1-3}$ alkylene, -CO-, -O-, -S-, $C_{3-6}$ cycloalkylene, phenyl, 5- to 6-membered heteroaryl containing 1-3 heteroatoms selected from N, S and O, 4- to 10-membered heterocycloalkylene containing 1-3 heteroatoms selected from N, S and O, $C_{2-6}$ alkenylene, or $C_{2-6}$ alkynylene, wherein the alkylene, alkenylene, alkynylene, cycloalkylene, phenyl, heteroaryl and heterocycloalkylene are optionally substituted with 1-3 groups selected from $C_{1-4}$ alkyl, halogen, CN, $C_{1-4}$ alkoxy and halo $C_{1-4}$ alkoxy,
provided that in $-M-(R_L)n-$, two identical $-NR_N-$, -O- or -S- are not attached;
$R_N$ is H or $C_{1-4}$ alkyl;
n is an integer of 0-10;

ULM is selected from U1, U2 or U3:

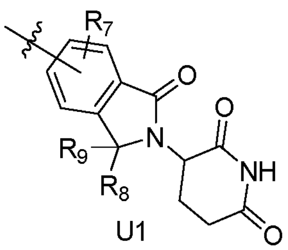
U1,
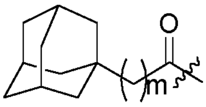
U2,
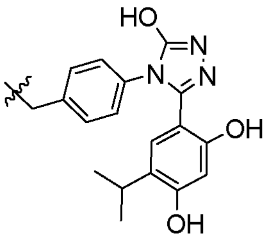
U3;

m is 1, 2 or 3;
$R_7$ is H, halogen, CN, $NH_2$, OH, $C_{1-4}$ alkyl or $C_{1-4}$ alkoxy, wherein the alkyl or alkoxy is optionally substituted with 1-3 groups selected from halogen, CN, $NH_2$ and OH;
$R_8$ and $R_9$ are H or together form =O.

As a fourth technical solution of the present invention, provided is a PARP-1 degradation agent, or a stereoisomer, deuterated compound, solvate, pharmaceutically acceptable salt or co-crystal thereof, the PARP-1 degradation agent having a structure of formula (II), (III), (IV), (V), (VI) or (VII):

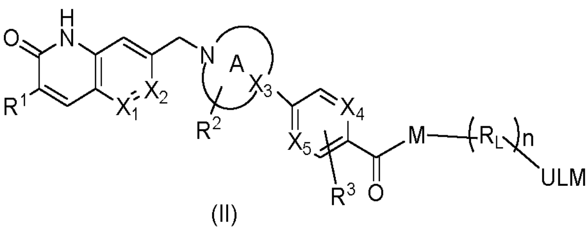
(II),
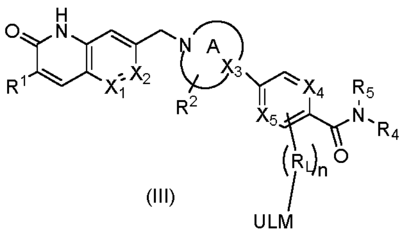
(III)

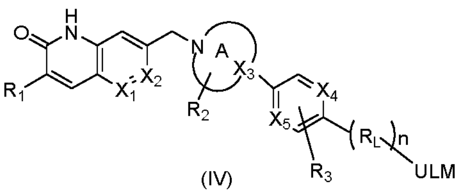
(IV)
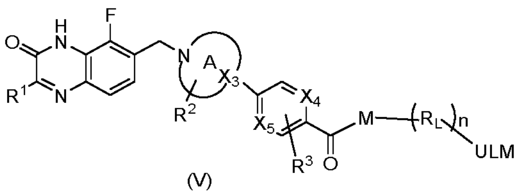
(V)

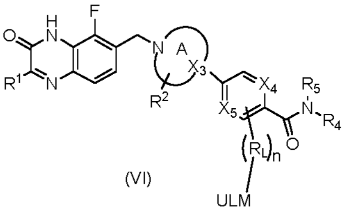
(VI)
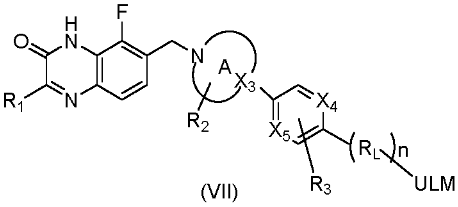
(VII)

wherein ring A is 4- to 12-membered monocyclic heterocycloalkyl, spiro heterocycloalkyl, bridged heterocycloalkyl or fused heterocycloalkyl containing 1-2 N atoms and 0-2 heteroatoms selected from S and O;
M is $-NR_5-$ or

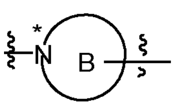,

ring B is 4- to 10-membered heterocycloalkyl containing 1-2 N atoms and 0-2 heteroatoms selected from S and O, and * is the end connected to group on the left side;

each $R_L$ is independently -$NR_N$-, $C_{1-3}$ alkylene, -CO-, -O-, -S-, $C_{3-6}$ cycloalkylene, phenyl, 5- to 6-membered heteroaryl containing 1-3 heteroatoms selected from N, S and O, 4- to 10-membered heterocycloalkylene containing 1-3 heteroatoms selected from N, S and O, $C_{2-6}$ alkenylene, or $C_{2-6}$ alkynylene, wherein the alkylene, alkenylene, alkynylene, cycloalkylene, phenyl, heteroaryl and heterocycloalkylene are optionally substituted with 1-3 groups selected from $C_{1-4}$ alkyl, halogen, CN, $C_{1-4}$ alkoxy and halo $C_{1-4}$ alkoxy,

provided that in -M-$(R_L)$n-, two identical -$NR_N$-, -O- or -S- are not attached;

$R_N$ is H or $C_{1-4}$ alkyl;

n is an integer of 0-10;

ULM is selected from U1, U2, U3, U4, U5, U6 or U7:

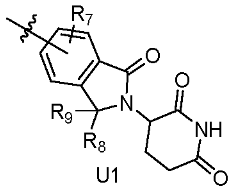
U1
,
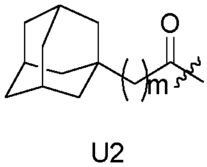
U2
,
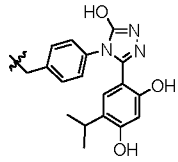
U3
,
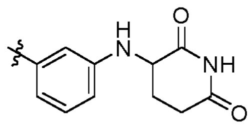
U4
,

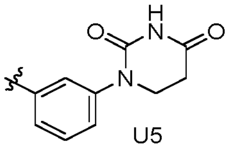
U5
,
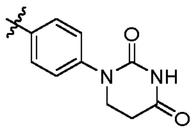
U6
,
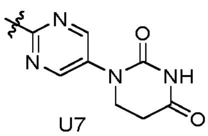
U7
;

m is 1, 2 or 3;

$R_7$ is H, halogen, CN, $NH_2$, OH, $C_{1-4}$ alkyl or $C_{1-4}$ alkoxy, wherein the alkyl or alkoxy is optionally substituted with 1-3 groups selected from halogen, CN, $NH_2$ and OH;

$R_8$ and $R_9$ are H or together form =O.

As a fifth technical solution of the present invention, provided is the PARP-1 degradation agent, or the stereoisomer, deuterated compound, solvate, pharmaceutically acceptable salt or co-crystal thereof according to the second or third technical solution, the PARP-1 degradation agent having a structure of formula (II-1) or (III-1):

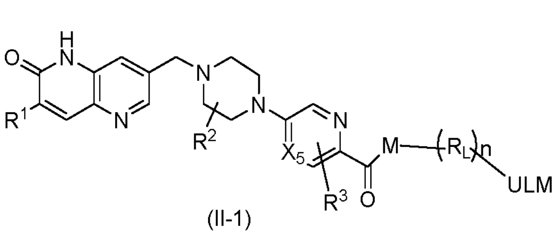
(II-1)
,
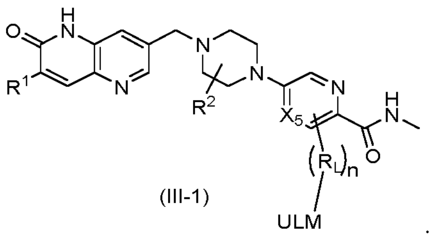
(III-1)
.

As a sixth technical solution of the present invention, provided is the PARP-1 degradation agent, or the stereoisomer, deuterated compound, solvate, pharmaceutically acceptable salt or co-crystal thereof according to the third technical solution, the PARP-1 degradation agent having a structure of formula (II-1), (III-1), (IV-1), (II-2), (III-2) or (IV-2):

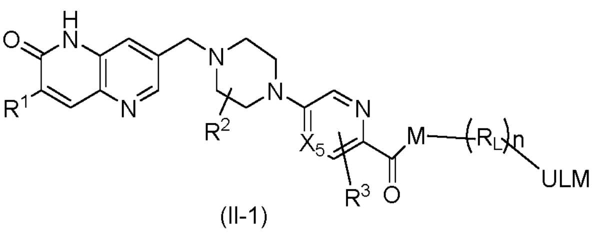
(II-1)
,
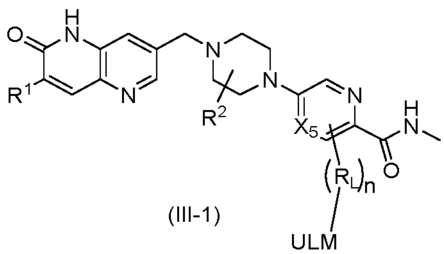
(III-1)

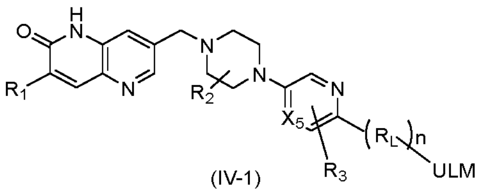
(IV-1)

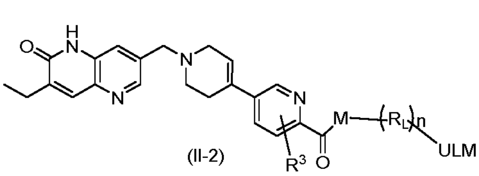
(II-2)

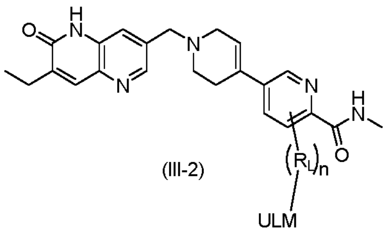
(III-2)

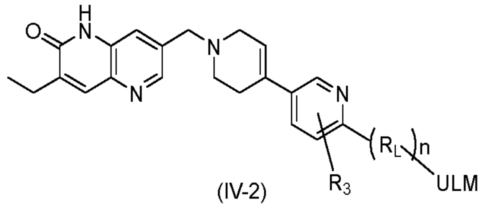
(IV-2).

As a seventh technical solution of the present invention, provided is the PARP-1 degradation agent, or the stereoisomer, deuterated compound, solvate, pharmaceutically acceptable salt or co-crystal thereof according to the third or fourth technical solution, the PARP-1 degradation agent having a structure of formula (II-1), (III-1), (IV-1), (II-2), (III-2), (IV-2), (V-1), (VI-1), (VII-1), (V-2), (VI-2) or (VII-2):

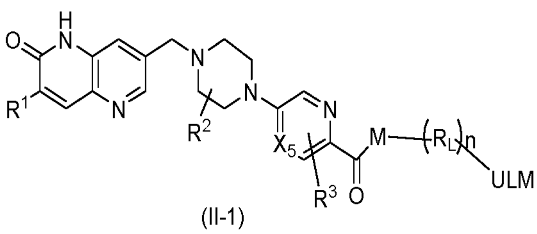
(II-1),

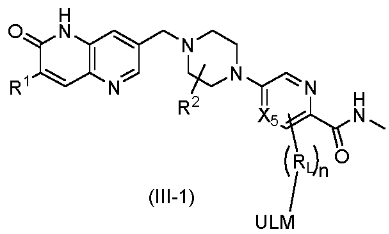
(III-1)

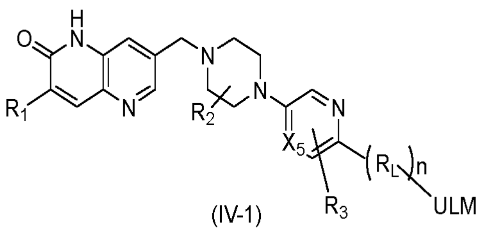
(IV-1),

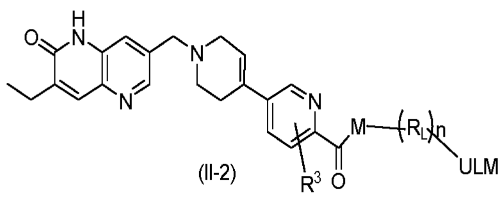
(II-2)

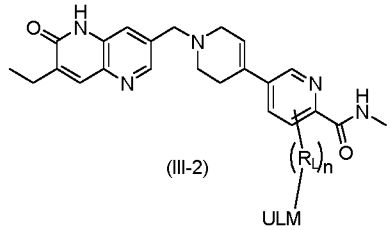
(III-2),

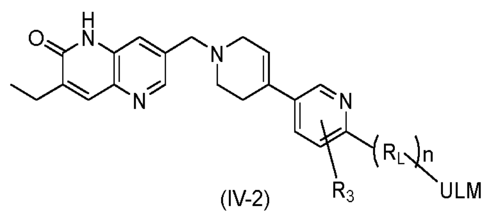
(IV-2)

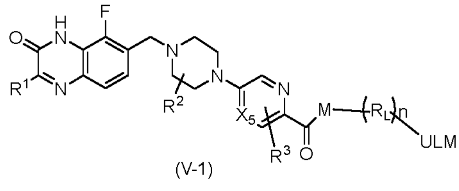
(V-1)

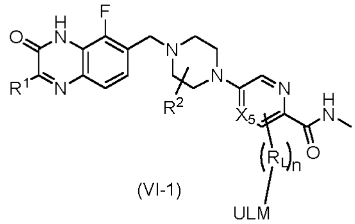
(VI-1)

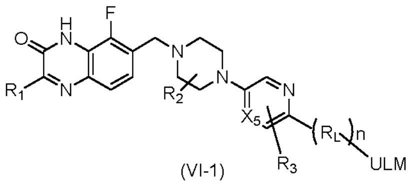
(VI-1)

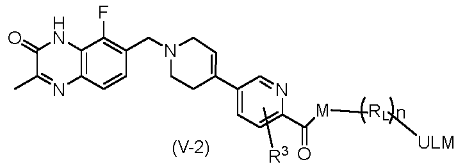
(V-2)

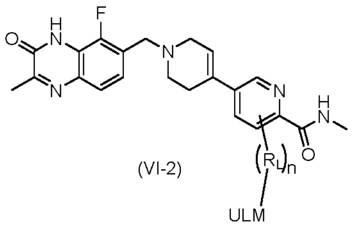
(VI-2)

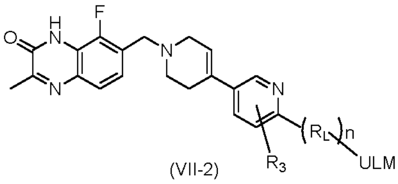
(VII-2)

.

As an eighth technical solution of the present invention, provided is the PARP-1 degradation agent, or the stereoisomer, deuterated compound, solvate, pharmaceutically acceptable salt or co-crystal thereof according to the first to seventh technical solutions,

wherein $X_5$ is CH or N;
$R_1$ is H or $C_{1-3}$ alkyl, wherein the alkyl is optionally substituted with 1-3 groups selected from F, Cl, Br, I, OH, $NH_2$ and COOH;
$R_2$ is H, $C_{1-3}$ alkyl, $C_{1-3}$ alkoxy, F, Cl, Br or I;
$R_3$ is H, $C_{1-3}$ alkyl, $C_{1-3}$ alkoxy, F, Cl, Br or I;
n is 0, 1, 2, 3, 4, 5, 6, 7 or 8; further, n is 2, 3, 4, 5, 6, 7 or 8; further, n is 1, 2, 3, 4, 5, 6 or 7; further, n is 2, 3, 4, 5, 6 or 7;
$R_5$ is H;
ring B is 4- to 10-membered monocyclic heterocycloalkyl or spiro heterocycloalkyl containing 1-2 N atoms and 0-2 heteroatoms selected from S and O;
each $R_L$ is independently -NH-, $C_{1-3}$ alkylene, -O-, $C_{3-6}$ cycloalkylene, phenyl, or 4- to 7-membered heterocycloalkylene containing 1-3 heteroatoms selected from N, S and O, wherein the alkylene, phenyl, cycloalkylene and heterocycloalkylene are optionally substituted with 1-3 groups selected from $C_{1-3}$ alkyl, F, Cl, Br, I, CN, $C_{1-3}$ alkoxy and halo $C_{1-3}$ alkoxy,
provided that in -M-$(R_L)$n-, two identical -NH- or -O- are not attached;
$R_7$ is H, $C_{1-3}$ alkyl, F, Cl, Br or I;
$R_8$ and $R_9$ are H or =O;
m is 1 or 2.

As a ninth technical solution of the present invention, provided is the PARP-1 degradation agent, or the stereoisomer, deuterated compound, solvate, pharmaceutically acceptable salt or co-crystal thereof according to the first to seventh technical solutions,

wherein $X_5$ is CH or N;
$R_1$ is H or $C_{1-3}$ alkyl, wherein the alkyl is optionally substituted with 1-3 groups selected from F, Cl, Br, I, OH, $NH_2$ and COOH;
$R_2$ is H, $C_{1-3}$ alkyl, $C_{1-3}$ alkoxy, F, Cl, Br or I;
$R_3$ is H, $C_{1-3}$ alkyl, $C_{1-3}$ alkoxy, F, Cl, Br or I;
n is 1, 2, 3, 4, 5, 6 or 7;
$R_5$ is H;
ring B is 4- to 10-membered monocyclic heterocycloalkyl or spiro heterocycloalkyl containing 1-2 N atoms and 0-2 heteroatoms selected from S and O;
each $R_L$ is independently -NH-, $C_{1-3}$ alkylene, -CO-, -O-, $C_{3-6}$ cycloalkylene, phenyl, 5- to 6-membered heteroaryl containing 1-3 heteroatoms selected from N, S and O, 4- to 10-membered heterocycloalkylene containing 1-3 heteroatoms selected from N, S and O, $C_{2-6}$ alkenylene, or $C_{2-6}$ alkynylene, wherein the alkylene, alkenylene, alkynylene, phenyl, heteroaryl, cycloalkylene and heterocycloalkylene are optionally substituted with 1-3 groups selected from $C_{1-3}$ alkyl, F, Cl, Br, I, CN, $C_{1-3}$ alkoxy and halo $C_{1-3}$ alkoxy,
provided that in -M-$(R_L)$n-, two identical -NH- or -O- are not attached;

$R_7$ is H, $C_{1-3}$ alkyl, F, Cl, Br or I;
$R_8$ and $R_9$ are H or =O;
m is 1 or 2.

As a tenth technical solution of the present invention, provided is the PARP-1 degradation agent, or the stereoisomer, deuterated compound, solvate, pharmaceutically acceptable salt or co-crystal thereof according to the first to seventh technical solutions,

wherein -M-($R_L$)n- or -($R_L$)n- in formula (II-1), (III-1), (IV-1), (II-2), (III-2) or (IV-2) is selected from one of the following structures, and * is the end connected to the group on the left side:

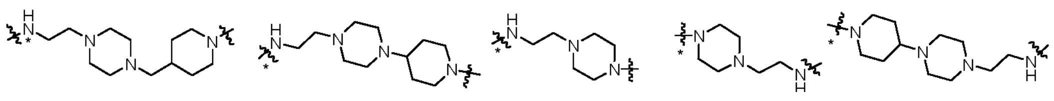

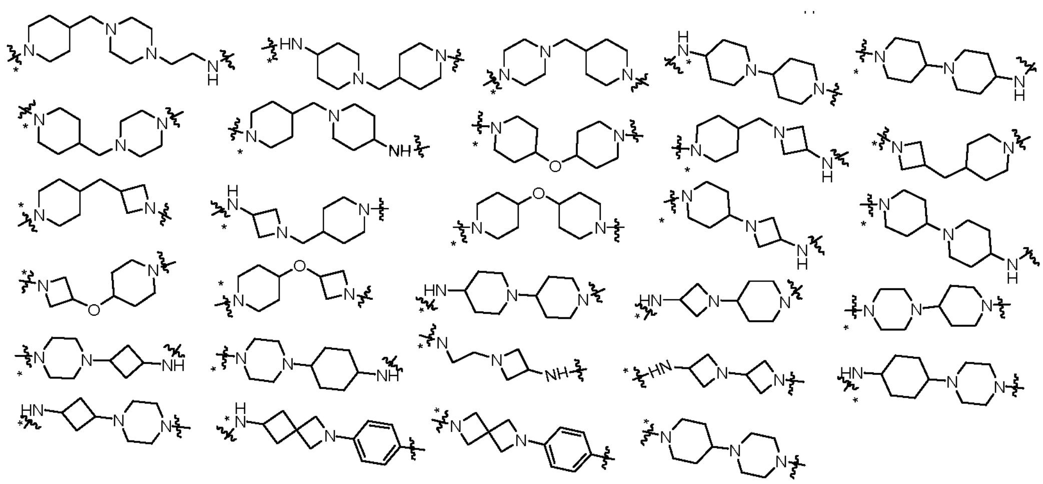

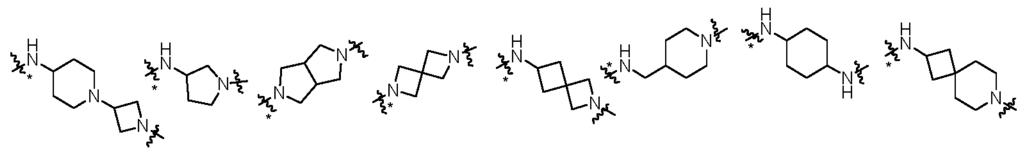

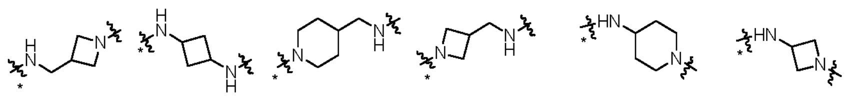

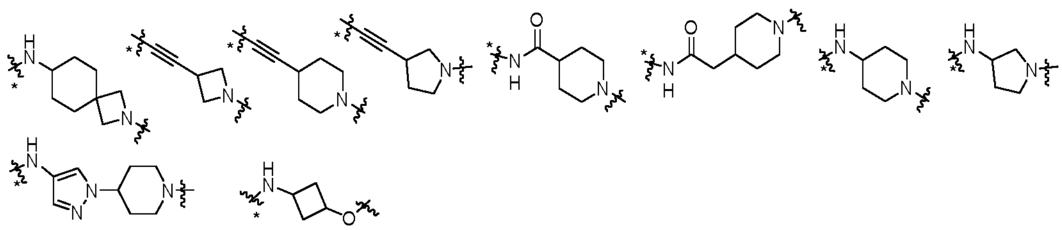

in some embodiments, -M-($R_L$)n- or -($R_L$)n- is selected from one of the following structures

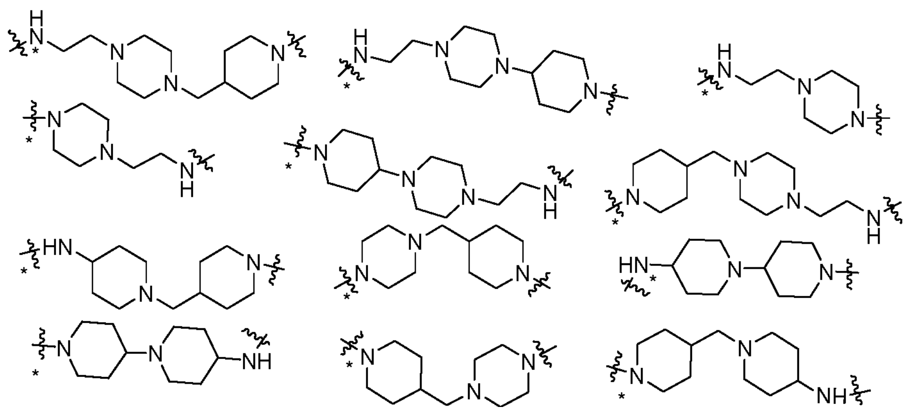

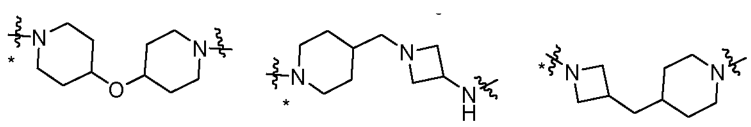

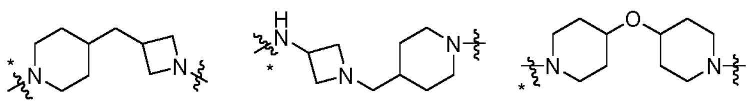

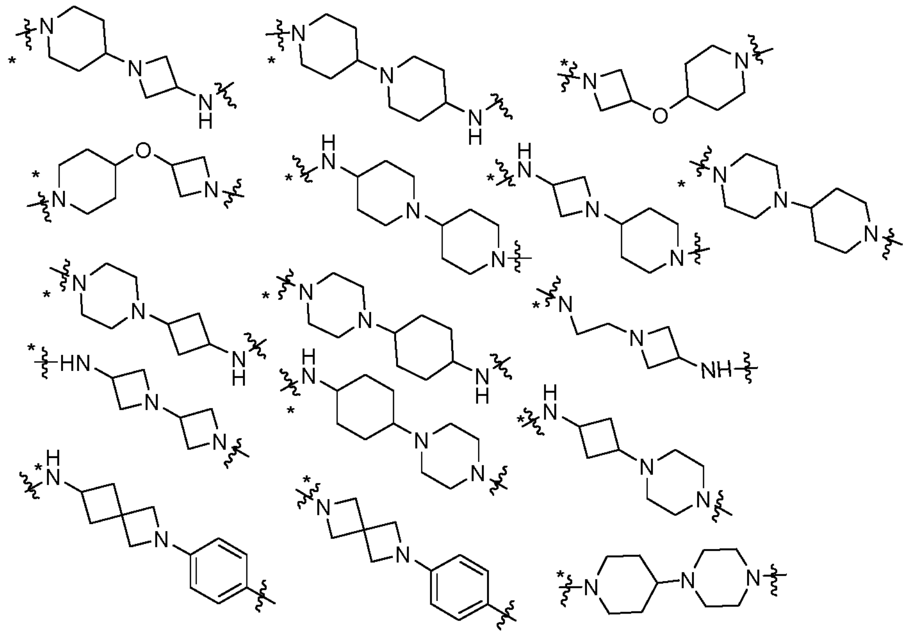

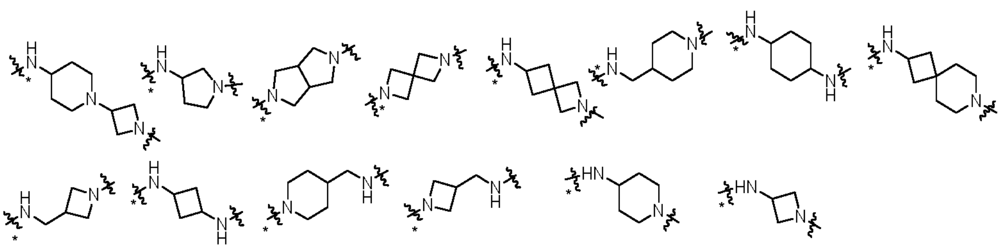

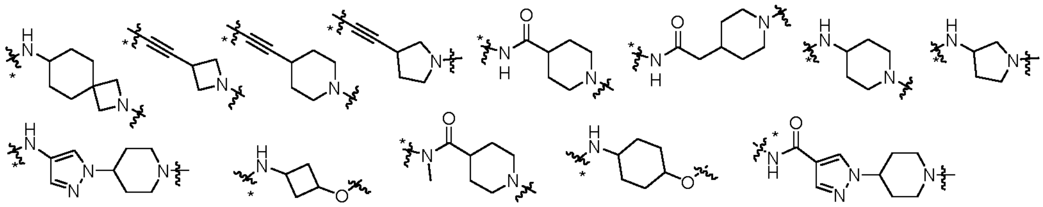

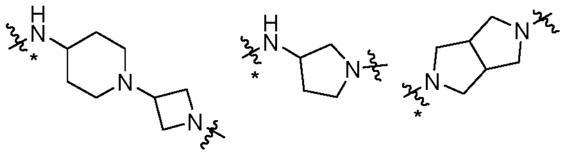

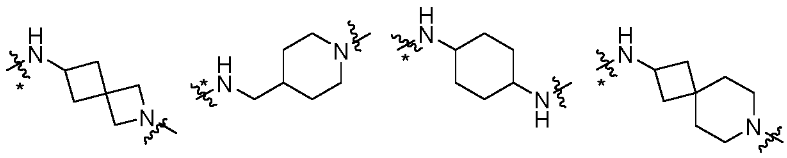

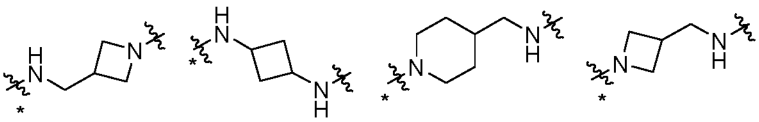

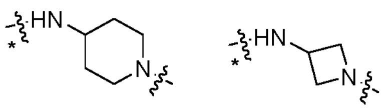

.

As an eleventh technical solution of the present invention, provided is the PARP-1 degradation agent, or the stereoisomer, deuterated compound, solvate, pharmaceutically acceptable salt or co-crystal thereof according to the first to tenth technical solutions, the degradation agent being selected from one of the following structures:

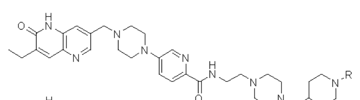
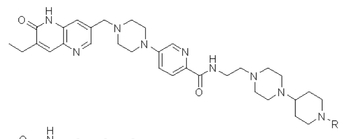
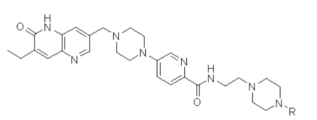
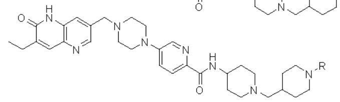
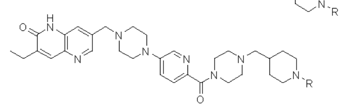
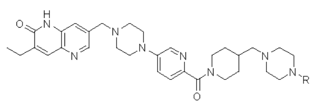
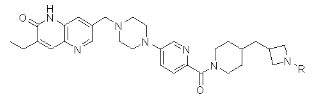
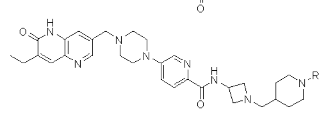
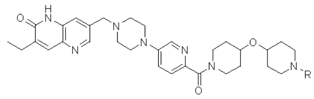
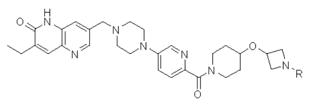
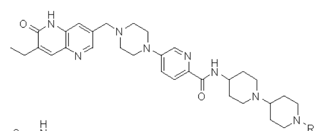
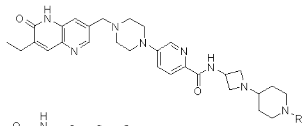
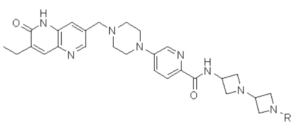
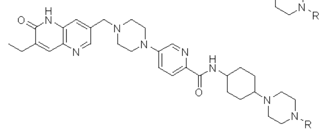
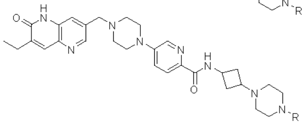
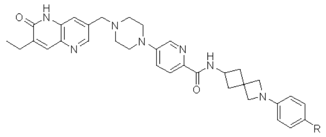
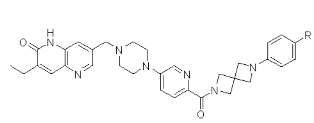
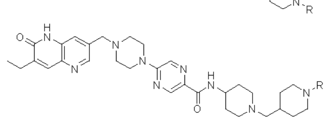

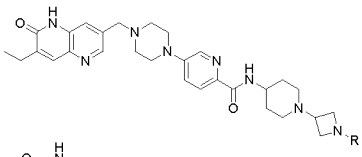
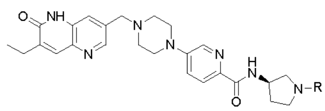
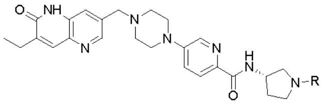
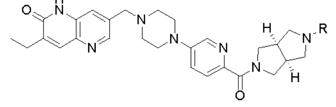
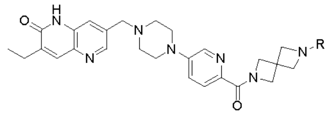
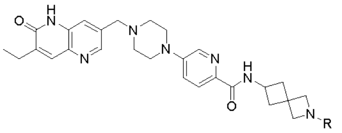
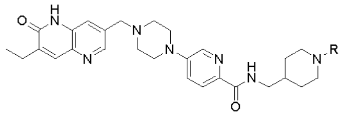
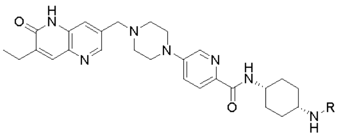
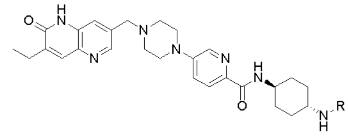
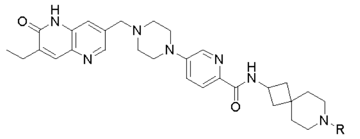
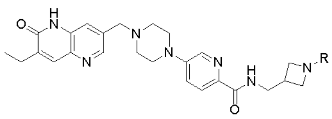
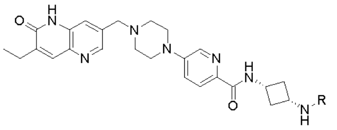
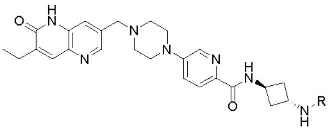
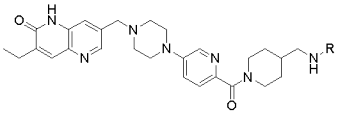
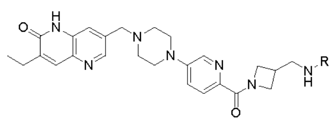
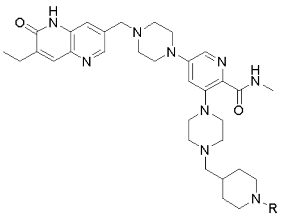
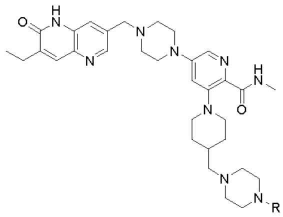
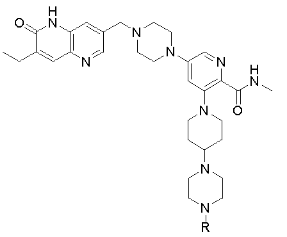
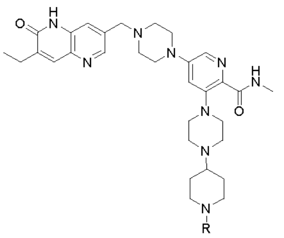
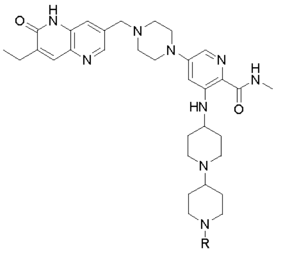
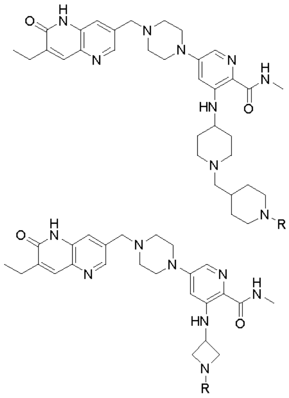
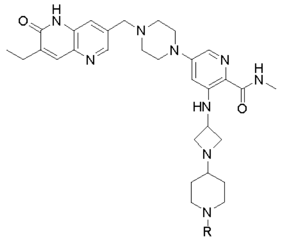
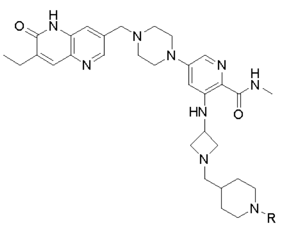
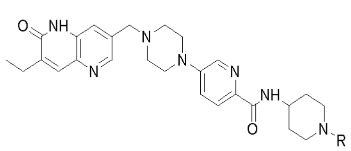
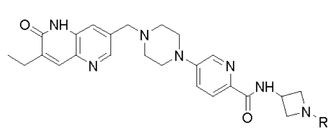

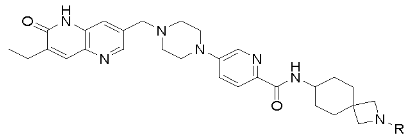

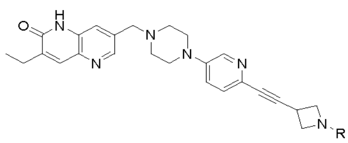
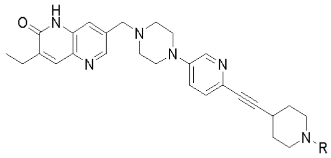
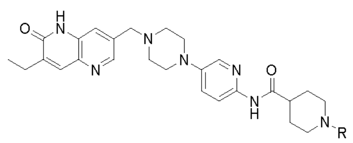

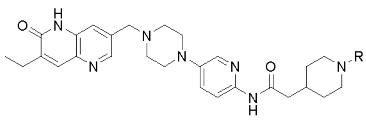
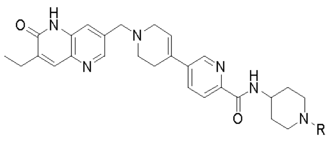
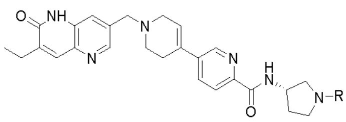

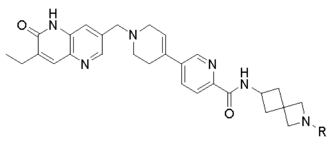
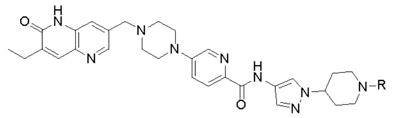

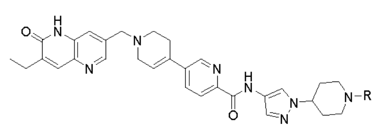

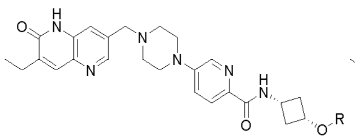
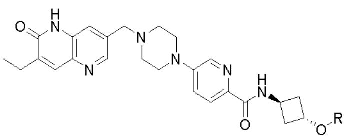
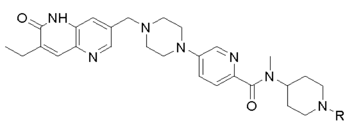

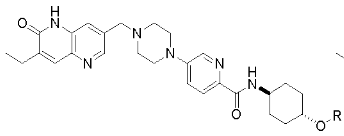
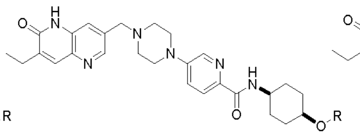
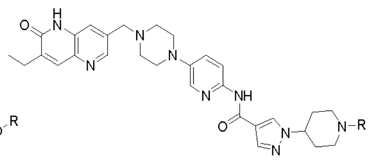

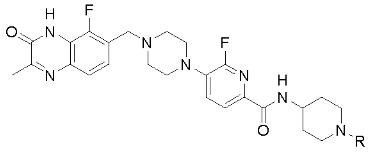,
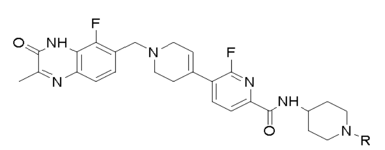

wherein in each of the structures above,

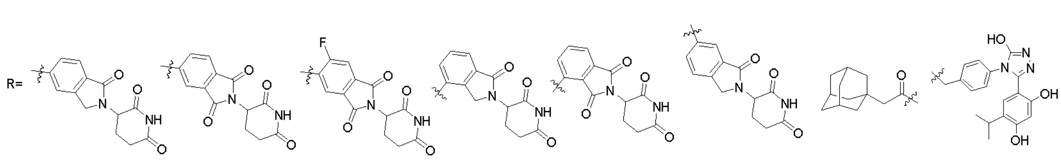

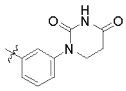 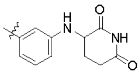 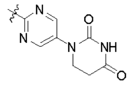 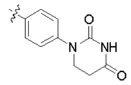

As a twelfth technical solution of the present invention, provided is the PARP-1 degradation agent, or the stereoisomer, deuterated compound, solvate, pharmaceutically acceptable salt or co-crystal thereof according to the first to tenth technical solutions, the degradation agent being selected from one of the structures in Table A:

Table A

| | | |
|---|---|---|
| 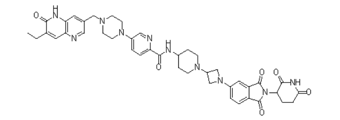 | 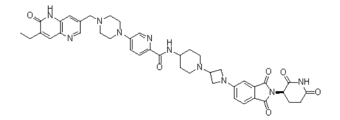 | 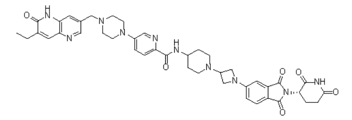 |
| 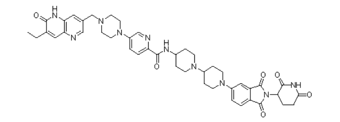 | 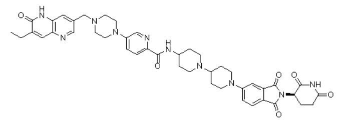 | 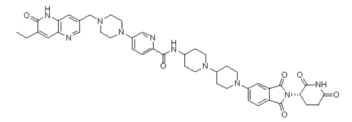 |
| 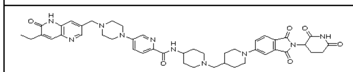 | 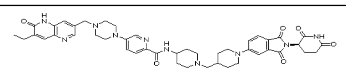 | 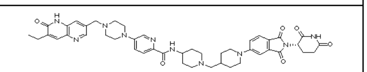 |
| 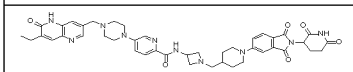 | 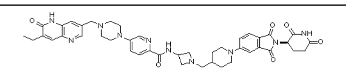 | 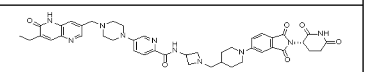 |
| 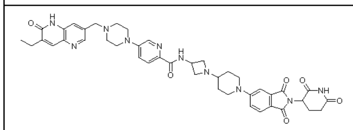 | 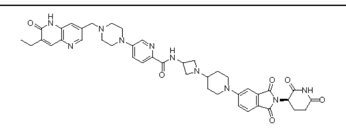 | 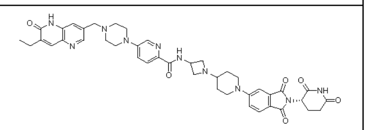 |
| 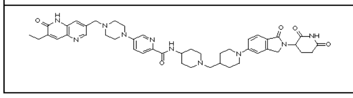 | 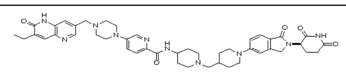 | 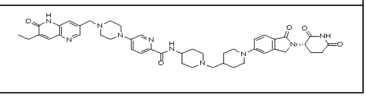 |

| | | |
|---|---|---|
| 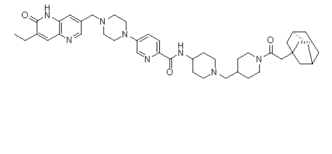 | 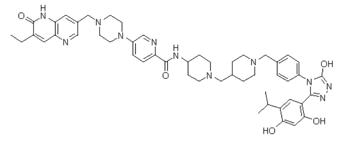 | 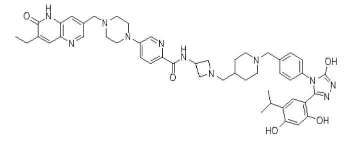 |
| 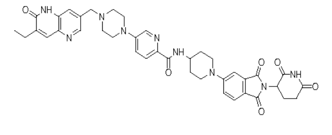 | 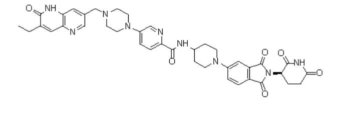 | 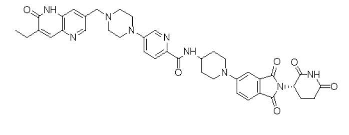 |
| 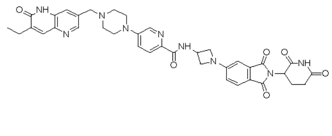 | 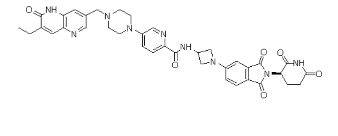 | 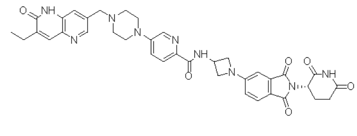 |
| 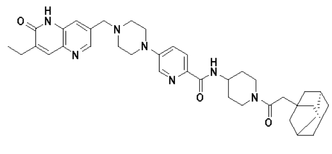 | 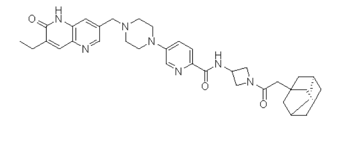 | |
| 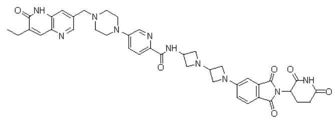 | 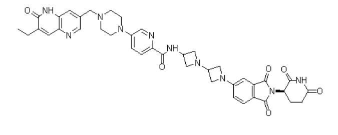 | 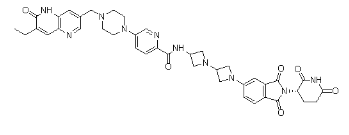 |
| 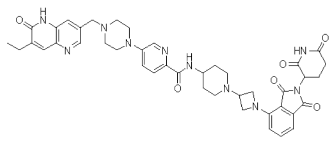 | 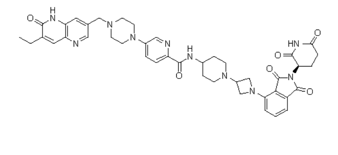 | 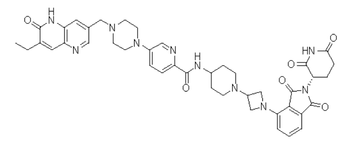 |
| 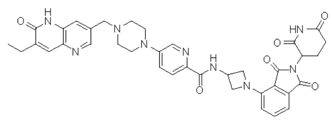 | 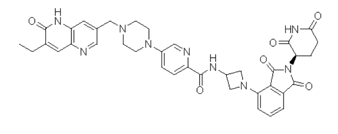 | 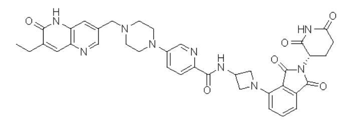 |
| 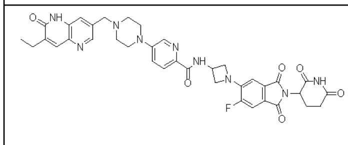 | 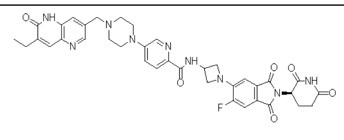 | 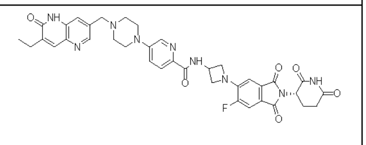 |
| 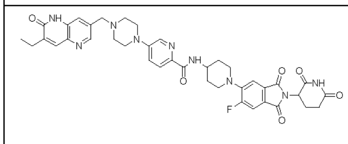 | 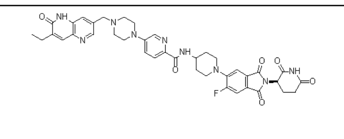 | 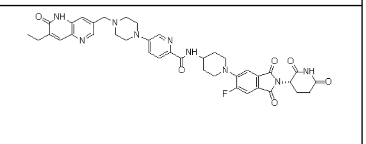 |
| 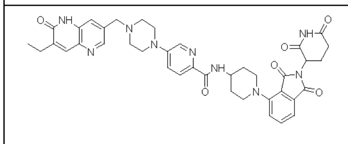 | 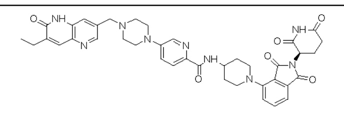 | 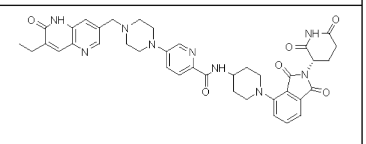 |
| 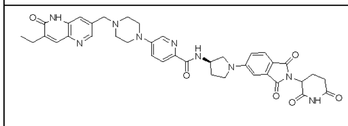 | 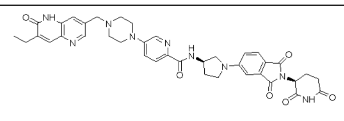 | 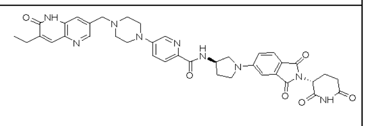 |
| 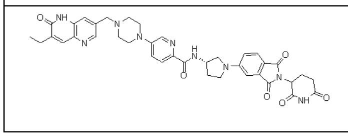 | 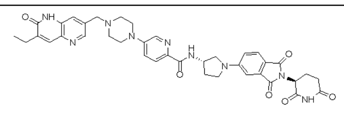 | 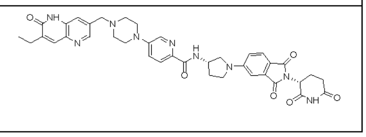 |

| | | |
|---|---|---|
| 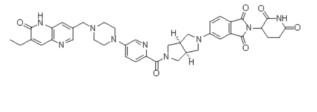 | 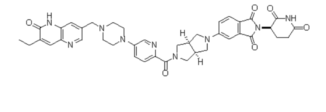 | 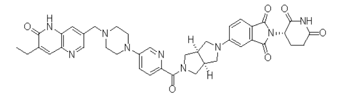 |
| 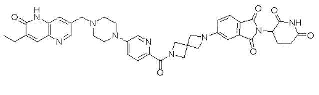 | 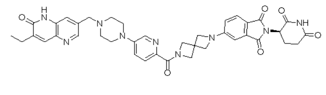 | 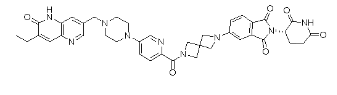 |
| 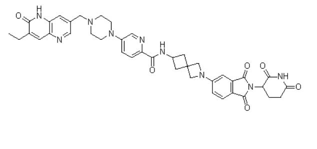 | 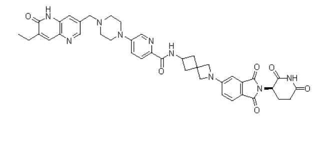 | 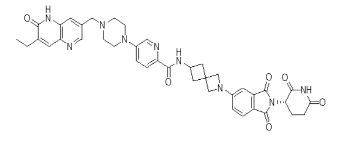 |
| 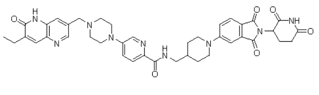 | 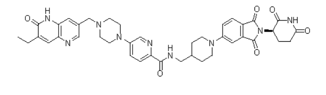 | 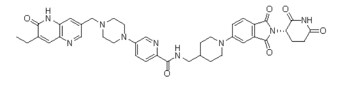 |
| 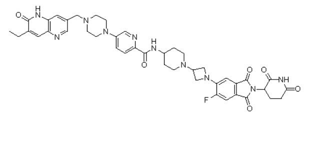 | 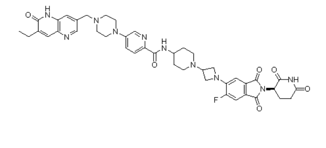 | 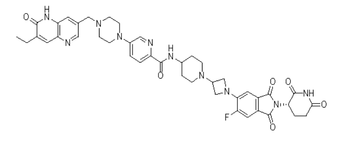 |
| 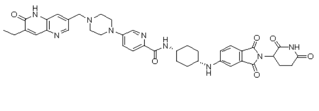 | 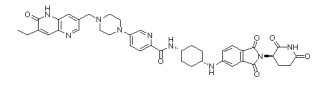 | 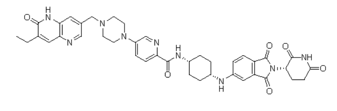 |
| 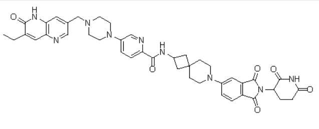 | 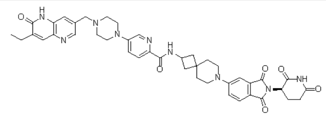 | 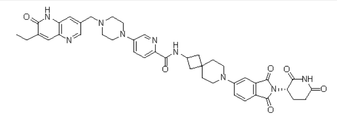 |
| 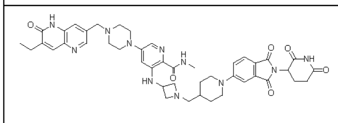 | 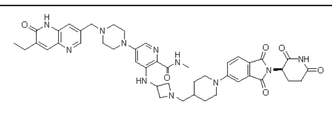 | 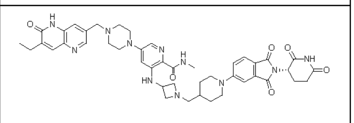 |
| 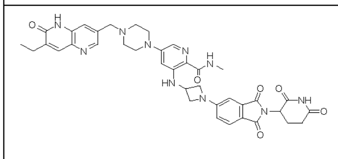 | 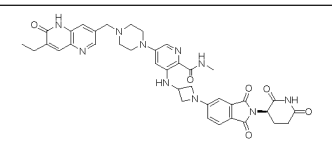 | 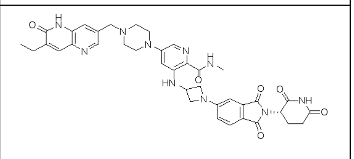 |
| 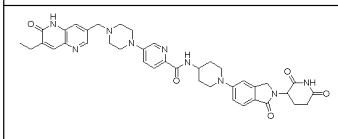 | 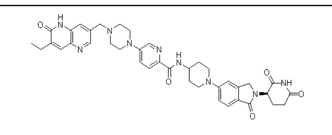 | 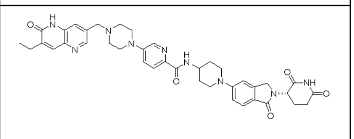 |
| 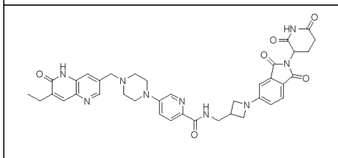 | 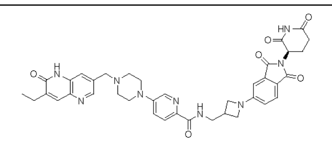 | 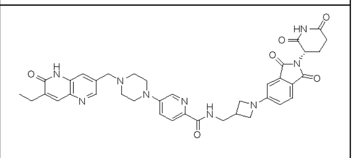 |
| 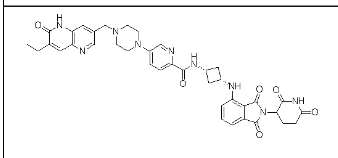 | 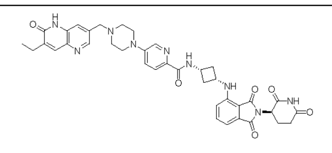 | 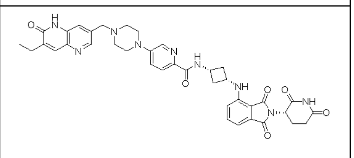 |
| 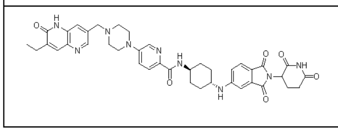 | 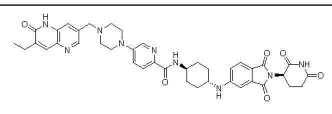 | 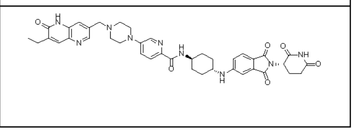 |

| | | |
|---|---|---|
| 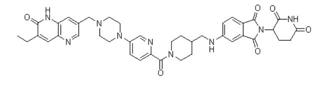 | 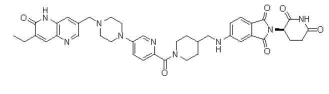 | 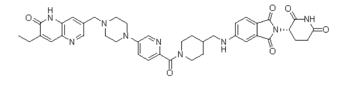 |
| 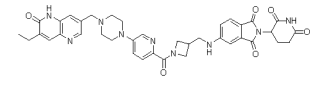 | 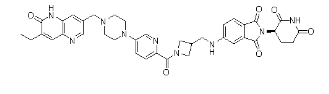 | 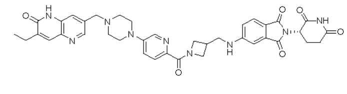 |
| 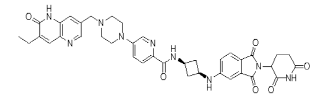 | 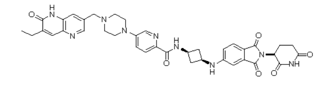 | 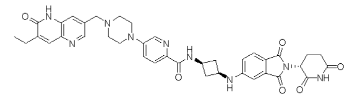 |
| 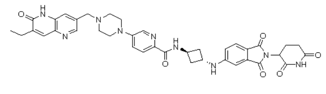 | 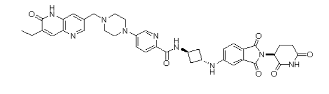 | 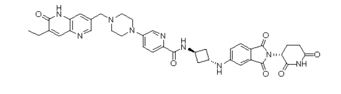 |
| 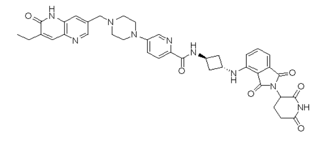 | 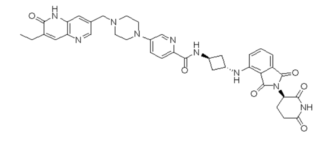 | 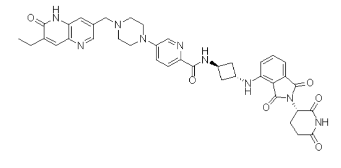 |
| 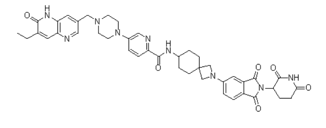 | 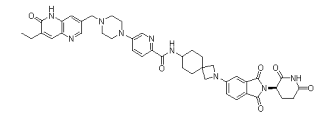 | 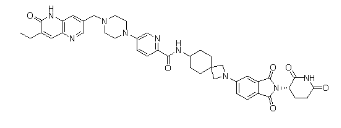 |
| 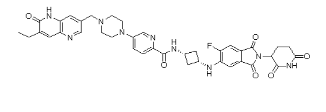 | 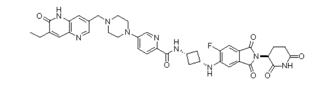 | 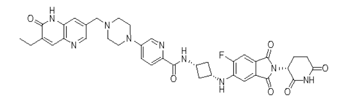 |
| 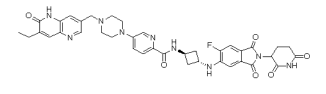 | 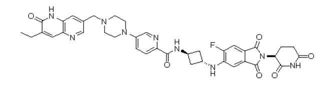 | 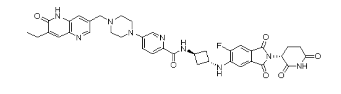 |
| 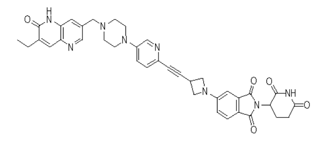 | 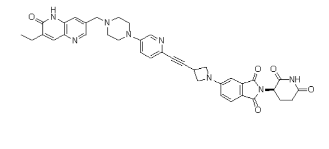 | 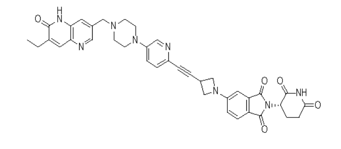 |
| 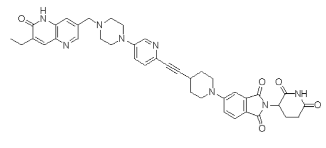 | 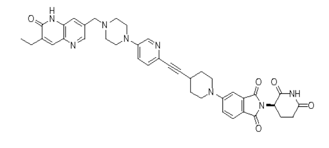 | 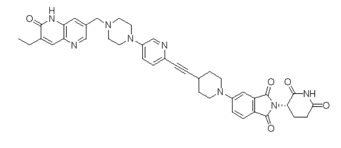 |
| 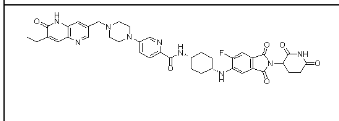 | 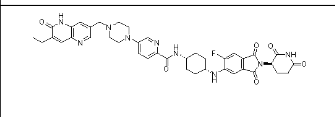 | 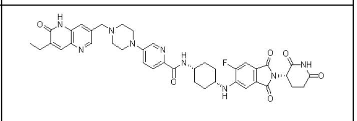 |
| 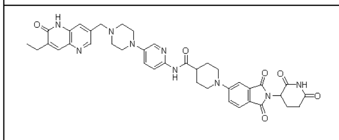 | 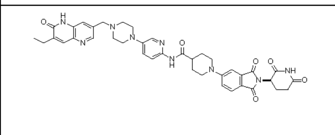 | 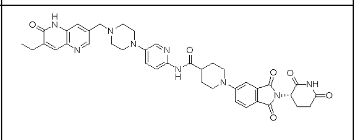 |
| 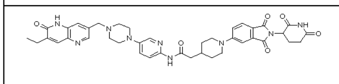 | 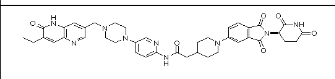 | 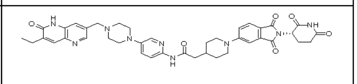 |
| 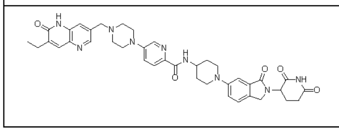 | | |

| | | |
|---|---|---|
| 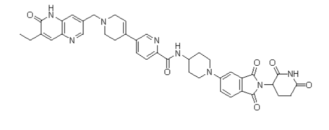 | 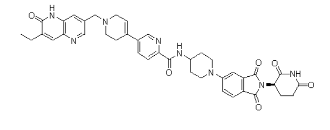 | 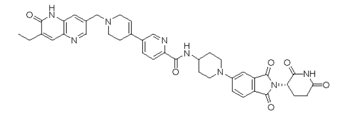 |
| 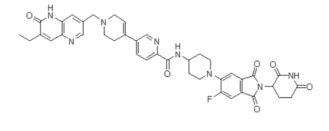 | 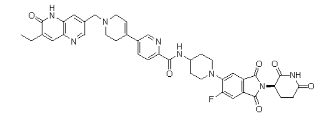 | 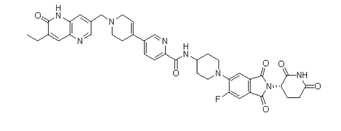 |
| 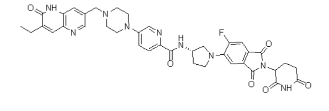 | 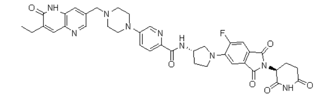 | 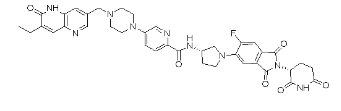 |
| 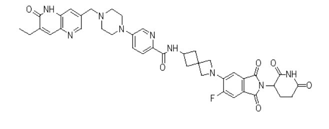 | 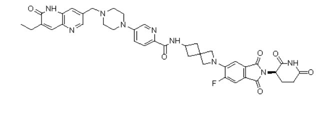 | 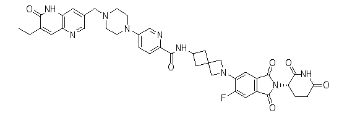 |
| 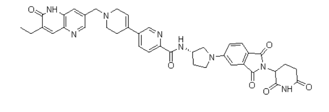 | 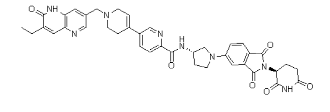 | 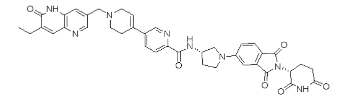 |
| 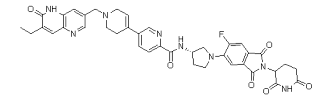 | 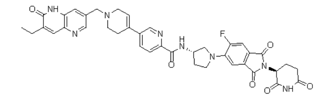 | 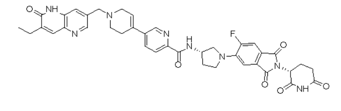 |
| 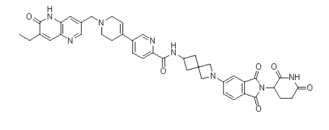 | 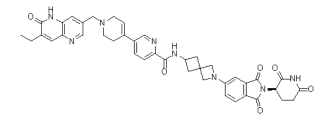 | 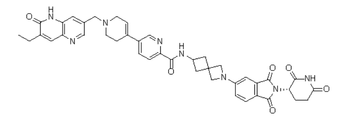 |
| 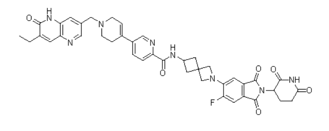 | 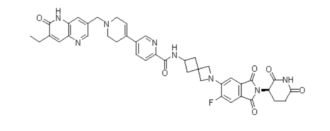 | 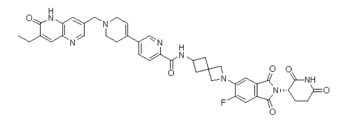 |
| 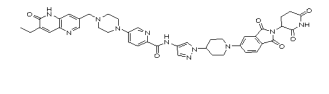 | 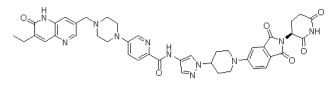 | 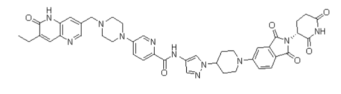 |
| 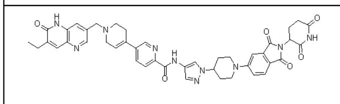 | 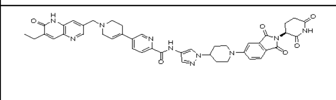 | 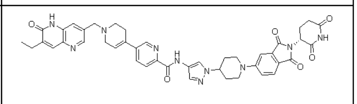 |
| 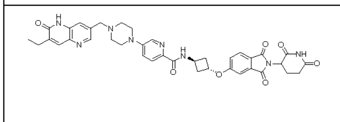 | 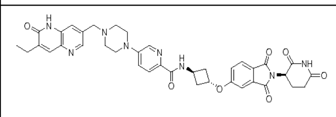 | 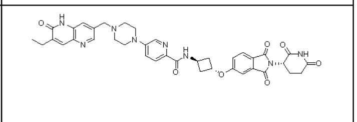 |
| 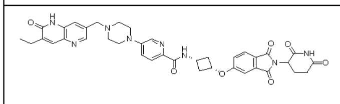 | 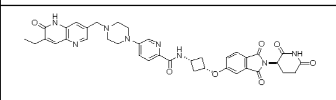 | 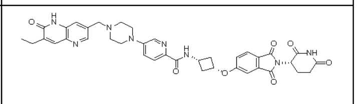 |
| 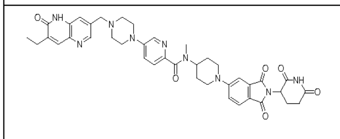 | 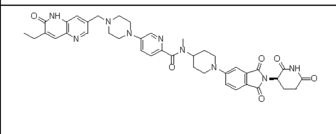 | 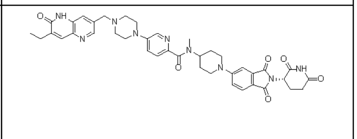 |
| 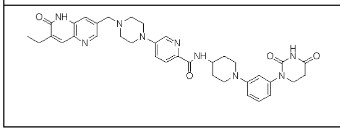 |  | |

| | | |
|---|---|---|
| 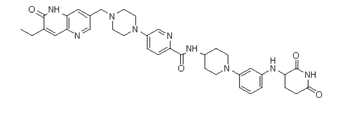 | 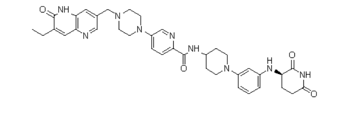 | 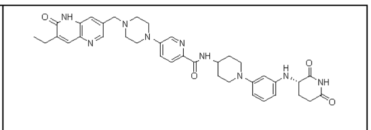 |
| 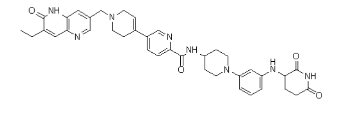 | 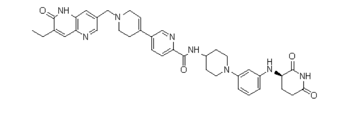 | 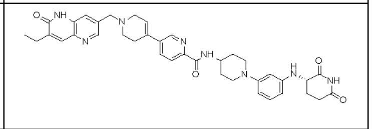 |
| 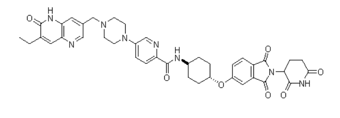 | 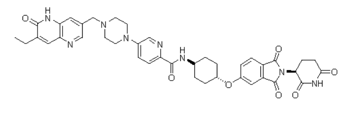 | 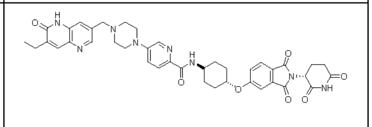 |
| 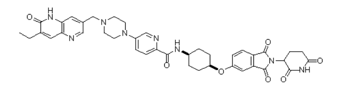 | 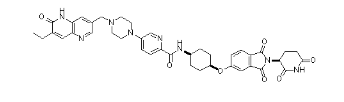 | 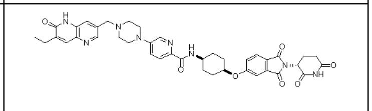 |
| 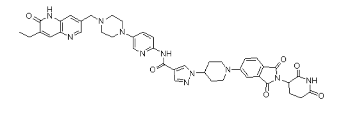 | 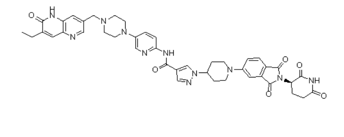 | 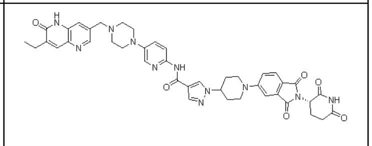 |
| 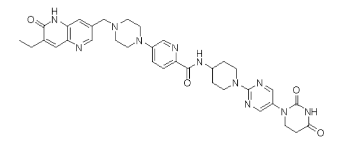 | 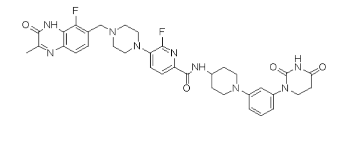 | 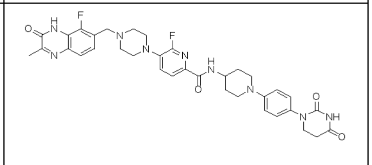 |
| 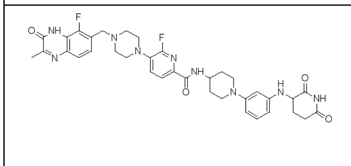 | 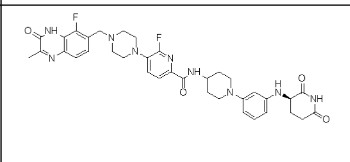 | 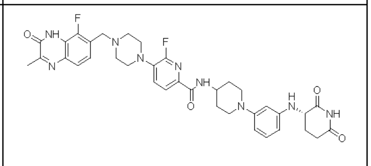 |
| 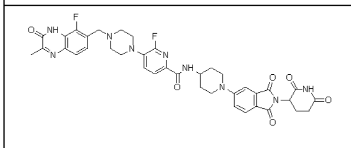 | 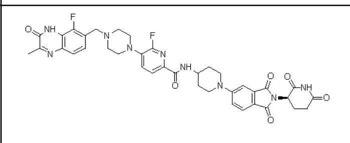 | 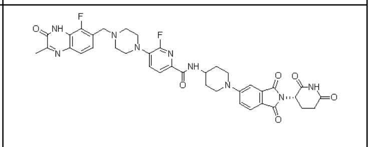 |
| 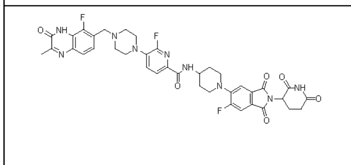 | 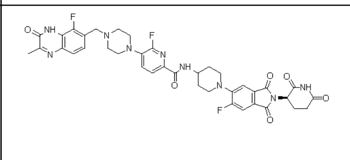 | 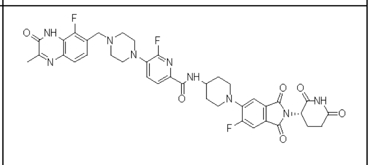 |
| 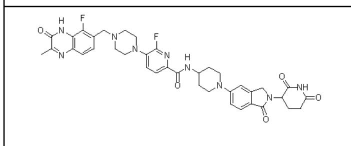 | 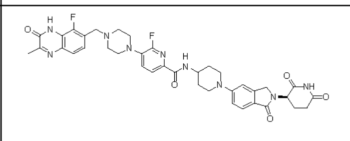 | 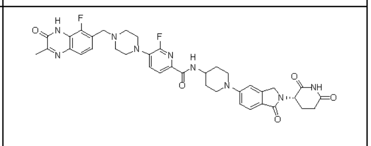 |
| 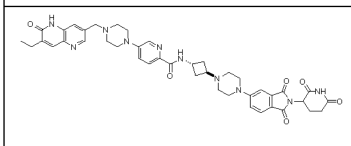 | 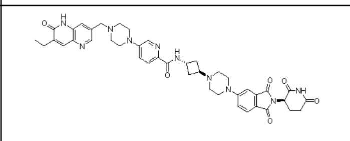 | 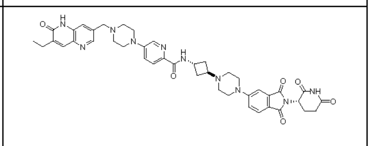 |
| 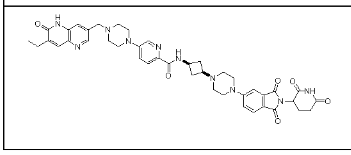 | 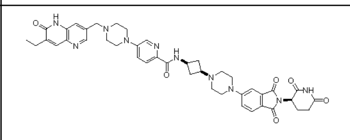 | 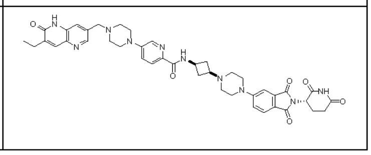 |

| | | |
|---|---|---|
| 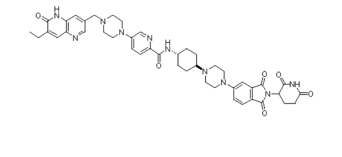 | 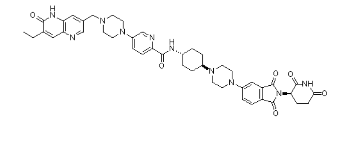 | 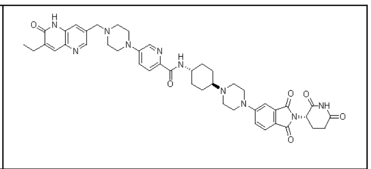 |
| 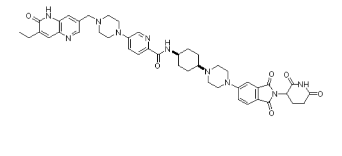 | 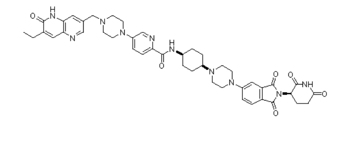 | 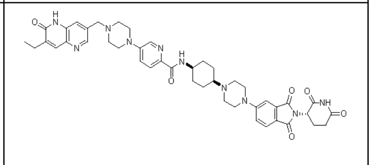 |
| 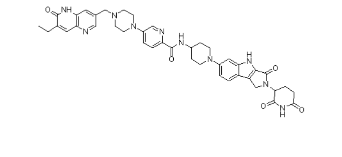 | 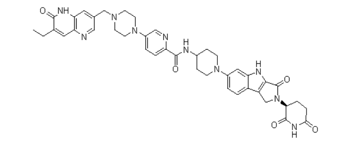 | 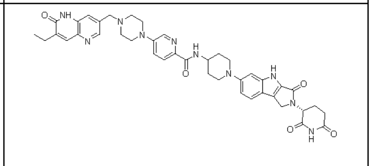 |
| 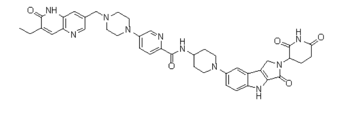 | 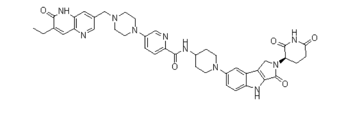 | 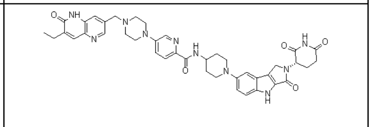 |
| 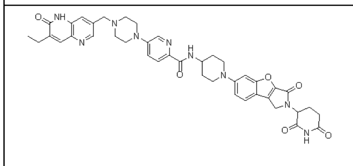 | 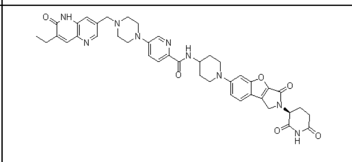 | 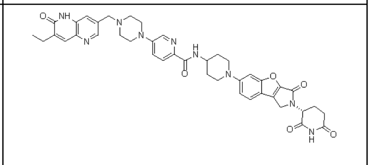 |
| 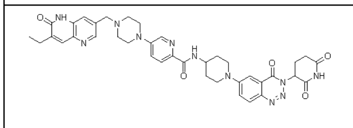 | 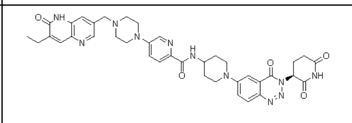 | 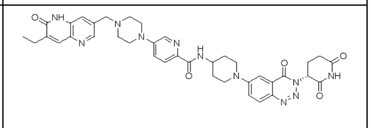 |
| 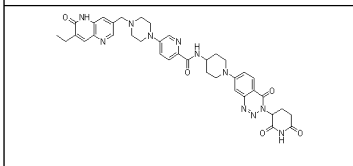 | 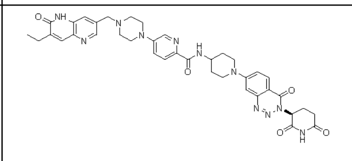 | 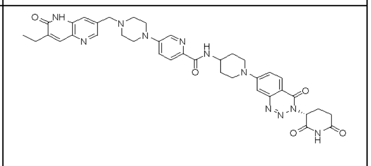 |
| 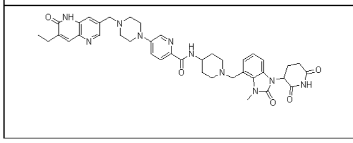 | 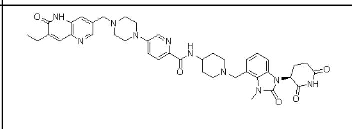 | 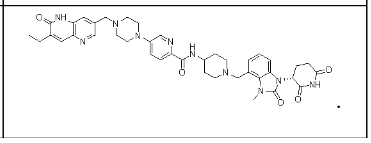 |

Secondly, the present invention further provides a pharmaceutical composition, comprising the PARP-1 degradation agent, or the stereoisomer, deuterated compound, solvate, pharmaceutically acceptable salt or co-crystal thereof according to the first to twelfth technical solutions, and a pharmaceutically acceptable carrier and/or excipient.

Further, the pharmaceutical composition or pharmaceutical preparation comprises 1-1500 mg of the compound, or the stereoisomer, deuterated compound, solvate, pharmaceutically acceptable salt or co-crystal thereof according to any one of the preceding technical solutions, and a pharmaceutically acceptable carrier and/or excipient.

Further, the present invention further provides the use of the compound or composition according to any one of the preceding embodiments in the preparation of a drug for treating/preventing a PARP-1-mediated disease.

The PARP-1-mediated disease includes, but is not limited to breast cancer, ovarian cancer, uterine cancer or cervical cancer.

The present invention further provides a method for treating a disease in a mammal, the method comprising administering to a subject a therapeutically effective amount of the compound, or the stereoisomer, deuterated compound, solvate, pharmaceutically acceptable salt or co-crystal thereof according to any one of the preceding technical solutions, and a pharmaceutically acceptable carrier and/or excipient, wherein the therapeutically effective amount is preferably 1-1500 mg, and the disease is preferably breast cancer, ovarian cancer, uterine cancer or cervical cancer.

The present invention further provides a method for treating a disease in a mammal, the method comprising administering to the mammal a therapeutically effective amount of the compound, or the stereoisomer, deuterated compound, solvate, pharmaceutically acceptable salt or co-crystal thereof or the pharmaceutical composition according

to the present invention. In some embodiments, the mammal according to the present invention comprises humans.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation comprising a therapeutically effective amount of the compound, or the stereoisomer, deuterated compound, solvate, pharmaceutically acceptable salt or co-crystal thereof according to the present invention, and a carrier and/or excipient. The pharmaceutical composition can be in a unit preparation form (the amount of the drug substance in the unit preparation is also referred to as the "preparation strength"). In some embodiments, the pharmaceutical composition comprises the compound, or the stereoisomer, deuterated compound, solvate, pharmaceutically acceptable salt, or co-crystal thereof according to the present invention in an amount including but not limited to 1-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

The present invention also provides a method for treating a disease in a mammal, the method comprising administering to a subject a therapeutically effective amount of the compound, or the stereoisomer, deuterated compound, solvate, pharmaceutically acceptable salt or co-crystal thereof according to the present invention, and a pharmaceutically acceptable carrier and/or excipient, wherein the therapeutically effective amount is preferably 1-1500 mg, and the disease is preferably breast cancer, ovarian cancer, uterine cancer or cervical cancer.

The present invention also provides a method for treating a disease in a mammal, comprising administering to a subject a drug, i.e., the compound, or the stereoisomer, deuterated compound, solvate, pharmaceutically acceptable salt or co-crystal thereof according to the present invention, and a pharmaceutically acceptable carrier and/or excipient in a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, wherein the kit can comprise a composition in the form of a single dose or multiple doses and comprises the compound, or the stereoisomer, deuterated compound, solvate, pharmaceutically acceptable salt or co-crystal thereof according to the present invention, and the amount of the compound, or the stereoisomer, deuterated compound, solvate, pharmaceutically acceptable salt or co-crystal thereof according to the present invention is identical to the amount of same in the above-mentioned pharmaceutical composition.

In the present invention, the amount of the compound, or the stereoisomer, deuterated compound, solvate, pharmaceutically acceptable salt or co-crystal thereof according to the present invention is calculated in the form of a free base in each case.

The term "preparation strength" refers to the weight of the drug substance contained in each vial, tablet or other

unit preparation.

**Synthetic route**

Patent document WO 2021013735 A1 introduces a method for preparing a PARP-1 inhibitor, and those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The PARP1-PROTAC of the present invention is prepared according to the following synthetic route:

1:

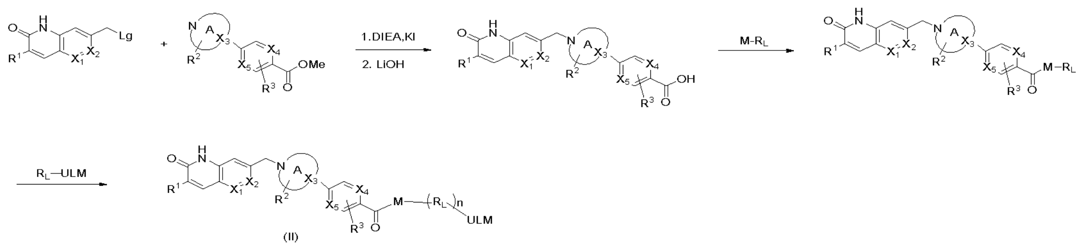


(II)

2:

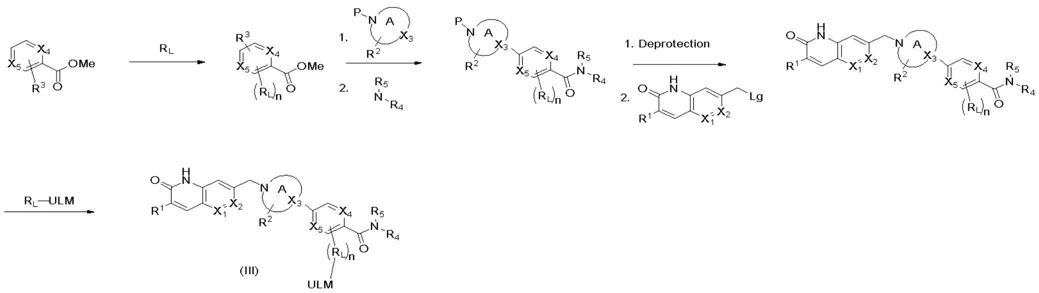


(III)

wherein the groups are as defined above.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalog are optionally

prepared by custom chemical synthesis plants, wherein many of standard chemical supply plants (such as those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

**Term**

Unless otherwise specified, the terms of the present invention have the following meanings.

The carbon, hydrogen, oxygen, sulphur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (D, also known as heavy hydrogen), and tritium (T, also known as superheavy hydrogen); the isotopes of oxygen comprise $^{16}O$, $^{17}O$ and $^{18}O$; the isotopes of sulphur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}Cl$ and $^{37}Cl$; and the isotopes of bromine comprise $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H).

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl, sec-butyl, neobutyl, tert-butyl, n-pentyl, isoamyl, neopentyl, n-hexyl, etc., and alkyl may be further substituted with a substituent.

The term "alkylene" refers to a bivalent straight or branched saturated alkyl. Examples of alkylene include, but are not limited to, methylene, ethylidene, *etc.*

The term "haloalkyl" refers to an alkyl group in which one or more hydrogens are substituted with one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to $-CF_3$, $-CH_2Cl$, $-CH_2CF_3$, $-CCl_2$, $CF_3$, etc.

The term "alkoxy" or "alkyloxy" refers to -O-alkyl, such as -O-$C_{1-8}$ alkyl, -O-$C_{1-6}$ alkyl, -O-$C_{1-4}$ alkyl or -O-$C_{1-2}$ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo $C_{1-8}$ alkyl, -O-halo $C_{1-6}$ alkyl, -O-halo $C_{1-4}$ alkyl or -O-halo $C_{1-2}$ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6

carbon atoms, and more further such as 2 to 4 carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a straight or branched divalent unsaturated hydrocarbon group containing at least one carbon-carbon double bond (C=C). Unless otherwise specified, the alkynylene contains 2-6 carbon atoms, preferably 2-4 carbon atoms. Non-limiting examples of alkynylene include ethynylene, and the alkenylene may be optionally substituted with a substituent.

The term "alkynyl" refers to a straight or branched hydrocarbon group containing at least one carbon-carbon triple bond (C=C) and generally comprises 2 to 18 carbon atoms; further, alkynyl comprises 2 to 8 carbon atoms; further, alkynyl comprises 2 to 6 carbon atoms, and more further, alkynyl comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a straight or branched, divalent unsaturated hydrocarbon group containing a carbon-carbon triple bond (C=C) and generally comprises 2-6 carbon atoms, and further comprises 2-4 carbon atoms. Non-limiting examples of alkynylene include ethynylene, propynylene, and butynylene, and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aryl rings, but the ring system is non-aromatic as a whole, and the connection site may be on an aryl ring or a non-aromatic ring. Generally, the cycloalkyl contains 3 to 20 carbon atoms; further, the cycloalkyl contains 3-8 carbon atoms; and still further, the cycloalkyl contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

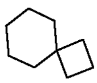, 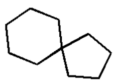, 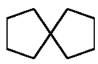,

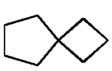, 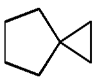, 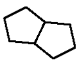, 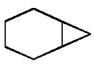, 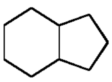, 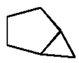, 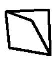, 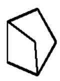, 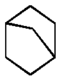, 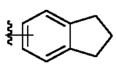,

etc., and the cycloalkyl may be optionally substituted with a substituent.

The term "cycloalkylene" refers to a divalent group of cycloalkyl.

"Aryl" refers to an aromatic carbocyclic ring that does not contain heteroatoms, including monocyclic aryl and fused aryl. Typically, it contains 6 to 13 carbon atoms, and further contains 6 to 9 carbon atoms, and it is further phenyl. The non-limiting examples thereof include phenyl, naphthyl, anthryl, and phenanthryl. The aryl may be optionally substituted with a substituent.

"Carbocyclic ring" or "carbocyclyl" refers to a saturated, partially unsaturated, or aromatic carbocyclic ring, and its meaning includes aryl and cycloalkyl. The carbocyclic ring can be a single ring, a bicyclic ring or a polycyclic ring, and the bicyclic ring or polycyclic ring includes bridged rings, fused rings, spiro rings and combinations thereof. Carbocyclic ring typically has 3 to 12 carbon atoms, or 3-10 carbon atoms, or 3-6 carbon atoms. In non-limiting embodiments, a monocyclic carbocyclic ring includes cyclopropyl, cyclobutyl, cyclopentyl, cyclohexyl, cycloheptyl or phenyl, *etc.*, a bicyclic bridged ring includes

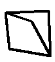, 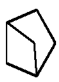, 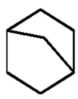,

*etc.,* a bicyclic fused ring includes

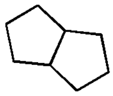, 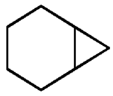, 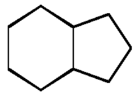, 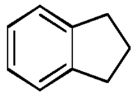,

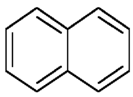 and 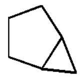,

*etc.*, and a bicyclic spiro ring includes

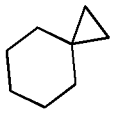, 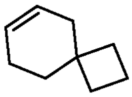,

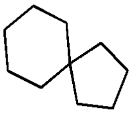, 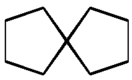, 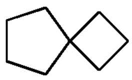 and 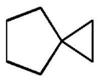,

*etc.* and carbocyclic ring may be optionally substituted with a substituent.

"Heterocycloalkyl" refers to a saturated or partially unsaturated non-aromatic carbocyclic ring containing 1, 2, 3, or 4 heteroatoms selected from N, S and O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aryl rings or heteroaromatic rings, but the ring system is non-aromatic as a whole, and the connection site may be on an aryl ring or a non-aromatic ring. Usually, the heterocycloalkyl is a 3-20-membered ring. When the heterocycloalkyl is monocyclic heterocycloalkyl, the heterocycloalkyl is usually a 3- to 15-membered ring, or a 3-to 10-membered ring, or a 3-8-membered ring, or a 3-6-membered ring; when the heterocycloalkyl is bicyclic or polycyclic heterocycloalkyl, the heterocycloalkyl is usually a 5- to 12-membered ring, or a 5- to 11-membered ring, or a 6- to 9-membered ring. The heteroatoms N and S include their oxidation states. Non-limiting examples of heterocycloalkyl include azetidinyl, morpholinyl, piperazinyl, tetrahydropyrazine, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which can be monocyclic, bicyclic, or polycyclic, wherein the bicyclic or polycyclic heteroaromatic ring or heteroaryl can be bridged rings, fused rings, spiro rings and combinations thereof. Bicyclic or polycyclic heteroaromatic ring or heteroaryl can be formed by fusion of heteroaryl to aryl, or of heteroaryl to heteroaryl, wherein the heteroaryl or aryl may be the connection site. Non-limiting examples of heteroaromatic ring or heteroaryl include furyl, thienyl, pyrrolyl, oxazolyl, thiazolyl, imidazolyl, pyrazolyl, pyridyl, pyrimidyl, pyridazinyl, pyrazinyl, indolyl, purinyl,

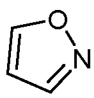, 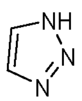, 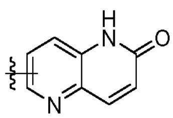, 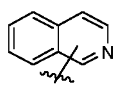, 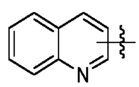,

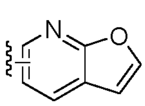, 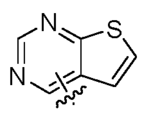, 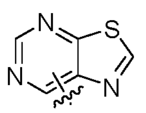, 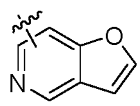,

etc. The heteroaryl may be optionally substituted with a substituent.

The term "heterocycle" or "heterocyclyl" refers to a saturated or unsaturated, aromatic or non-aromatic ring

containing 1 to 4 heteroatoms selected from N, O or S and their oxidation states, and its meaning includes heteroaryl and heterocycloalkyl. Heterocycles include monocyclic heterocycles, bicyclic bridged heterocycles, bicyclic fused heterocycles and bicyclic spiro heterocycles or combinations thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl,

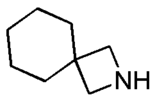, 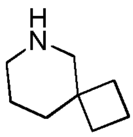, 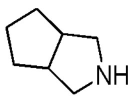, 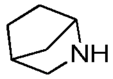, 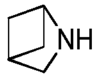,

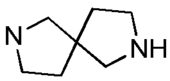, 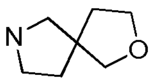, 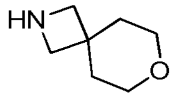, 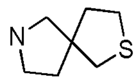,

etc., and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include

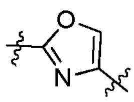, 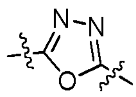, 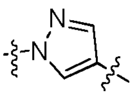, 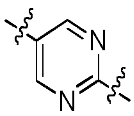,

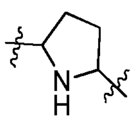,

*etc.*

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of spiro ring include:

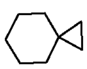 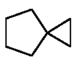 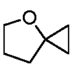 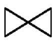 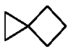 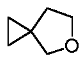 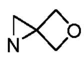 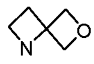 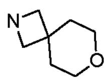

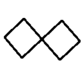 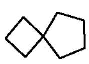 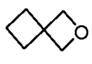 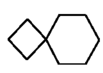 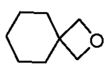 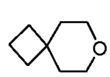 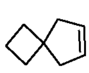 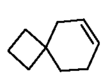

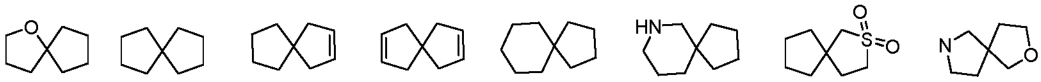

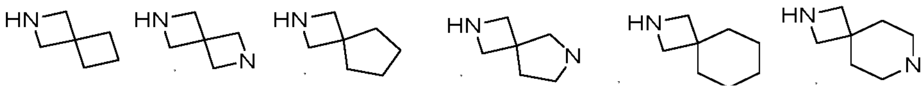

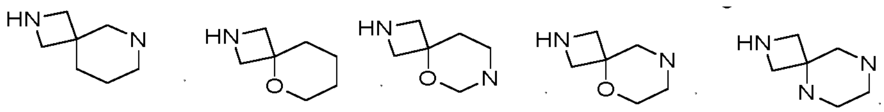

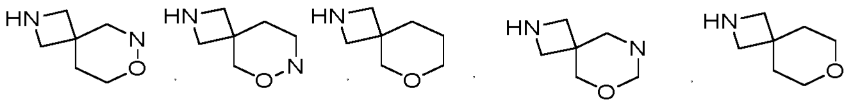

,

and the spiro ring may be optionally substituted with a substituent.

The term "fused ring" refers to a polycyclic group in which the rings share two adjacent ring atoms and one chemical bond, which may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of fused ring include purine, quinoline, isoquinoline, benzopyran, benzofuran, benzothiophene, and

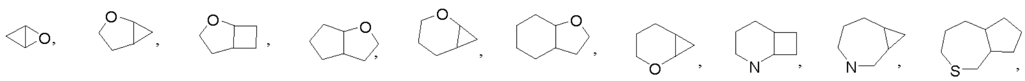

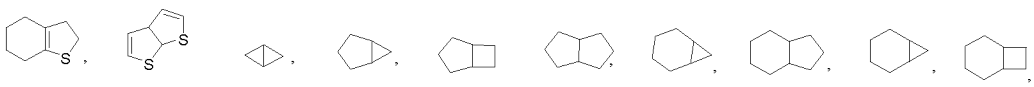

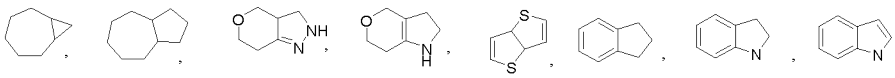

;

and the fused ring may be optionally substituted with a substituent.

The term "bridged ring" refers to a ring system in which two non-adjacent ring atoms are shared between two rings and a bridged ring may contain 1 or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of bridged ring include adamantane,

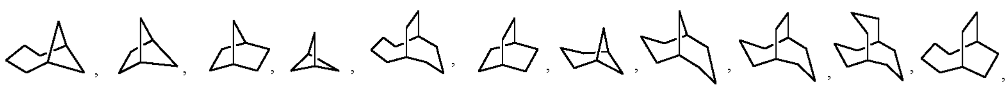

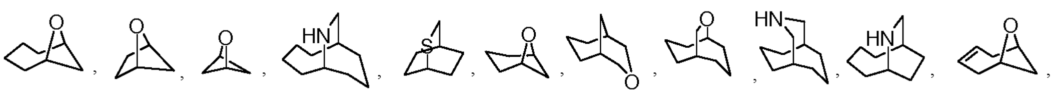

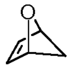, 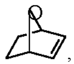, 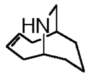, 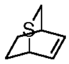, 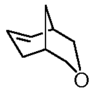, 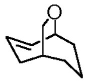, 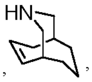, 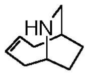, 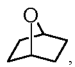, 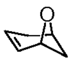

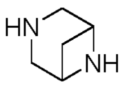, 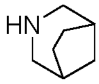, 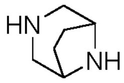, 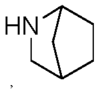, 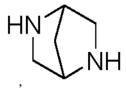, 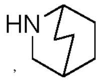, 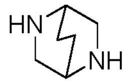,

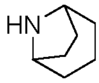, 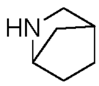, 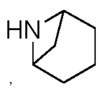, 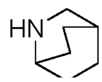, 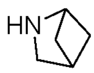, 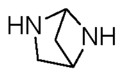, 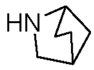,

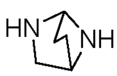, 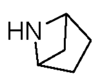, 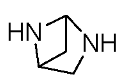, 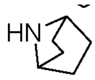

Unless otherwise specified, the term "substitute" or "substituent" refers to any substitution at a position allowed by chemical theory, and the number of substituents conforms to the rules of chemical bonding. Exemplary substituents include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulphinyl, sulphonyl, sulphoxide, halo $C_{1-6}$ alkyl, halo $C_{1-6}$ alkoxy, amino, phosphonic acid, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl$)C(=O)(C_{1-6}$ alkyl), $-NHCO_2(C_{1-6}$ alkyl), $-NHC(=O)N(C_{1-6}$ alkyl$)_2$, $-HC(=O)NH(C_{1-6}$ alkyl), $-NHC(=O)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, solvates, pharmaceutically acceptable salts or co-crystals thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and

(22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

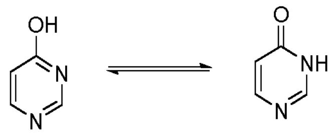

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio among various components. The co-crystal is a multi-component crystal, which includes both a binary co-crystal formed between two neutral solids and a multi-element co-crystal formed between a neutral solid and a salt or solvate.

**Brief Description of the Drawings**

Figure 1. Tumour growth curve of the mouse MDA-MB-436 subcutaneous in vivo transplanted tumour model;
Figure 2. Animal body weight change curve of the mouse MDA-MB-436 subcutaneous in vivo transplanted tumour model.

**Detailed Description of Embodiments**

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

**Detection method**

The structure of the compound is determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvents for determination are deuterated dimethyl sulphoxide (DMSO-d6), deuterated chloroform (CDCl3) and deuterated methanol (CD3OD); and the internal standard is tetramethylsilane (TMS);

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

Abbreviations

TBSCl: Tert-butyl dimethylsilyl chloride
HATU: 2-(7-azobenzotriazole)-N,N,N' ,N' -tetramethyluronium hexafluorophosphate
TBAF: Tetrabutylammonium fluoride
TsCl: P-toluenesulphonyl chloride
DIPEA: N,N-diisopropylethylamine

**Preparation of compounds**

Example 1:

N-(1'-(2-(2,6-dioxopiperidin-3-yl)-1,3-dioxoisoindolin-5-yl)-[1,4'-bipiperidin]-4-yl)-5-(4-((7-ethyl-6-oxo-5,6-dihydro-1,5-naphthyridin-3-yl)methyl)piperazin-1-yl)picolinamide (Compound 1)

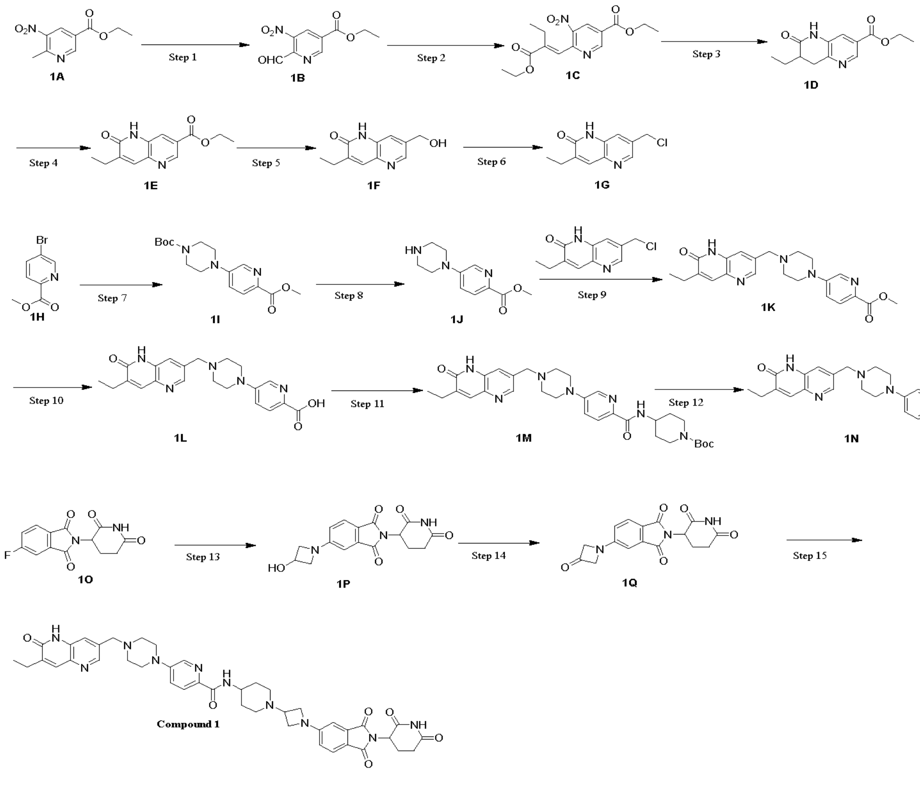


Step 1:

Ethyl 6-methyl-5-nitronicotinate **1A** (10 g, 47.6 mmol) and selenium dioxide (21.14 g, 190.5 mmol) were dissolved in 1,4-dioxane (100 ml), and the mixture was refluxed overnight at 100°C. After the reaction was completed, the reaction liquid was filtered through a funnel lined with diatomaceous earth, the diatomaceous earth was washed with ethyl acetate, the filtrate was concentrated, and the resulting residue was separated and purified by silica gel column chromatography (eluent: ethyl acetate :petroleum ether (v/v) = 0%-40%), to afford compound **1B** (10.104 g, 94.7%).
LCMS m/z =225.1 $[M+H]^+$

Step 2:

Sodium hydride (2.695 g, 112.3 mmol) was dissolved in anhydrous tetrahydrofuran (100 ml) and stirred at 0°C, and triethyl 2-phosphonobutyrate (28.3 g, 112.3 mmol) was added dropwise. After the dropwise addition was completed, the mixture was stirred at 0°C for 20 min, warmed to 40°C and stirred for 10 min, and then transferred to a dry ice ethanol bath. Compound **1B** (10.48 g, 46.8 mmol) was dissolved in anhydrous tetrahydrofuran (100 ml) and added dropwise to a reaction flask. The mixture was kept in the dry ice ethanol bath and stirred for 1 h. After the reaction was completed, the reaction liquid was quenched with saturated ammonium chloride solution (100 ml) and extracted with ethyl acetate

(200 ml). The organic phase was separated, and the aqueous phase was extracted with ethyl acetate (200 ml × 2). The organic phases were combined, dried over anhydrous sodium sulphate and concentrated, and the resulting residue was purified by silica gel column chromatography (eluent: ethyl acetate : petroleum ether (v/v) = 0-10%), to afford compound 1C (11.57 g, 76.8%), a mixture of two isomers.
LCMS m/z =323.1 $[M+H]^+$

Step 3:

Compound 1C (11.57 g, 35.9 mmol) was dissolved in ethanol (50 ml), and 10% palladium on carbon catalyst (1 g) was added. The mixture was subjected to hydrogen replacement three times, stirred overnight at room temperature, and filtered through a funnel lined with diatomaceous earth, and the diatomaceous earth was washed with anhydrous ethanol. The filtrate was concentrated, to the resulting residue was added a solution of hydrogen chloride in dioxane (60 ml, 4M), and the mixture was stirred at room temperature for 1 h and concentrated. To the resulting residue was added ethyl acetate (50 ml), the mixture was stirred and filtered, and the filter cake was washed with ethyl acetate and dried, to afford compound **1D** (4.28 g, 42.0%).

$^1$H NMR (400 MHz, DMSO-$d_6$) δ 10.39 (s, 1H), 8.62 (d, 1H), 7.75 (s, 1H), 4.38 - 4.29 (m, 2H), 3.24 (dd, 1H), 2.97 (dd, 1H), 2.62 - 2.53 (m, 1H), 1.83 - 1.64 (m, 1H), 1.45 (tt, 1H), 1.33 (dd, 3H), 0.94 (t, 3H).

Step 4:

Compound **1D** (4.28 g, 17.3 mmol) and 2,3-dichloro-5,6-dicyanobenzoquinone (4.309 g, 19.0 mmol) were dissolved in dioxane (86 ml), and the mixture was reacted at 100°C for 3.5 h at reflux. After the reaction was completed, saturated sodium bicarbonate solution (40 ml) and ethyl acetate (120 ml) were added. The organic phase was separated, and the aqueous phase was extracted with ethyl acetate (120 ml × 2). The organic phases were combined, dried over anhydrous sodium sulphate and concentrated, and the resulting residue was purified by silica gel column chromatography (eluent: ethyl acetate : petroleum ether (v/v) = 0-50%), to afford compound **1E** (3.375 g, 79.5%).
LCMS m/z = 247.1 $[M+H]^+$

Step 5:

Compound **1E** (3.375 g, 13.72 mmol) was dissolved in anhydrous tetrahydrofuran (150 ml), and the mixture was stirred at -78°C. Lithium aluminium hydride (1.564 g, 41.16 mmol) was added portionwise, and the mixture was stirred at -78°C for 20 min and then warmed to -40°C and stirred for 20 min. After the reaction was completed, 1M hydrochloric acid was added to adjust the system to a neutral pH, the solvent was removed by distillation under reduced pressure, to the resulting residue was added methanol/dichloromethane (1 : 10) (100 ml) for dissolution, and the mixture was subjected to ultrasonic vibration for 10 min and filtered. The filtrate was collected, the filter cake was redissolved in methanol/dichloromethane (1 : 10) (100 ml), and this process was repeated 8 times. The filtrate was combined and concentrated, to afford compound **1F** (2.8 g, 100.0%).

$^1$H NMR (400 MHz, DMSO-$d_6$) δ 11.86 (s, 1H), 8.37 (d, 1H), 7.72 (d, 1H), 7.62 (d, 1H), 5.44 (t, 1H), 4.61 (d, 2H), 2.57 - 2.51 (m, 2H), 1.18 (t, 3H).

Step 6:

Compound **1F** (2.94 g, 14.4 mmol) was dissolved in dichloromethane (140 ml), N,N-dimethylformamide (1 ml) was added, and the mixture was stirred at 0°C. Thionyl chloride (6.29 ml, 86.4 mmol) was added dropwise to the reaction liquid and after the dropwise addition, the reaction liquid was transferred to room temperature and stirred for 2 h. After the reaction was completed, the reaction liquid was concentrated, to afford compound **1G** (3.20 g, 100.0%).
LCMS m/z = 223.1 $[M+H]^+$

Step 7:

Compound **1H** (10 g, 46 mmol), N-Boc-piperazine (10.33 g, 56 mmol), methanesulphonato(2-dicyclohexylphosphino-2',6'-di-isopropoxy-1,1'-biphenyl)(2-amino-1,1'-biphenyl-2-yl)palladium (Ruphos Pd G 3, 1.55 g, 1.85 mmol) and caesium carbonate (30.19 g, 93 mmol) were dissolved in dioxane (100 ml) and the mixture was subjected to nitrogen replacement three times and refluxed overnight at 100°C. After the reaction was completed, the reaction liquid was filtered through a funnel lined with diatomaceous earth, the diatomaceous earth was washed with ethyl acetate, and the filtrate was washed with water (100 ml), dried over anhydrous sodium sulphate and concentrated. The resulting residue was separated and purified by silica gel column chromatography (eluent: ethyl acetate : petroleum ether (v/v) = 0%-

50%), to afford compound **11** (9.53 g, 64.1%).
LCMS m/z = 322.2 $[M+H]^+$

Step 8:

Compound **11** (9.53 g, 29.7 mmol) was dissolved in methanol (100 ml) and 4M hydrogen chloride-dioxane solution (100 ml) was added. The mixture was stirred at room temperature for 1 h. After the reaction was completed, the reaction liquid was concentrated to afford compound **1J** (8.70 g, 100.0%).
LCMS m/z = 222.2 $[M+H]^+$

Step 9:

Compound **1G** (3.20 g, 14.4 mmol), compound **1J** (4.23 g, 14.4 mmol), potassium iodide (478 mg, 2.88 mmol) and N,N-diisopropylethylamine (12.5 ml, 71.96 mmol) were dissolved in acetonitrile (150 ml) and the mixture was reacted at 80°C for 2 h at reflux. After the reaction was completed, the reaction liquid was concentrated, and extracted with water (80 ml), dichloromethane (150 ml) and methanol (15 ml). The organic phase was separated, and the aqueous phase was extracted with dichloromethane (150 ml × 3). The organic phases were combined and concentrated, and the resulting residue was purified by silica gel column chromatography (eluent: methanol : dichloromethane (v/v) = 0%-15%), to afford compound **1K** (4.24 g, 72.1%).
LCMS m/z = 408.2 $[M+H]^+$

Step 10:

Compound **1K** (4.24 g, 10.4 mmol) was dissolved in tetrahydrofuran (80 ml) and water (80 ml), lithium hydroxide (750 mg, 31.2 mmol) was added, and the mixture was stirred and reacted at room temperature for 2 h. After the reaction was completed, 1M hydrochloric acid was added to adjust the system to pH 4-5, the mixture was concentrated, and the resulting residue was purified by silica gel column chromatography (eluent: methanol : dichloromethane (v/v) = 0%-15%), to afford compound **1L** (4.01 g, 100.0%).
LCMS m/z = 394.2 $[M+H]^+$

Step 11:

Compound **1L** (1 g, 2.54 mmol) and tert-butyl 4-aminopiperidine-1-carboxylate (609 mg, 3.05 mmol) were dissolved in DMF (5 ml) and benzotriazole-N,N,N',N'-tetramethyluronium hexafluorophosphate (962 mg, 2.54 mmol) was added. The mixture was subjected to nitrogen replacement three times and stirred at room temperature for 20 min. N,N-diisopropylethylamine (1.31 ml, 7.54 mmol) was added and the reaction liquid was stirred and reacted at room temperature for 2 h. After the reaction was completed, water (10 ml) and ethyl acetate (12 ml) were added. The organic phase was separated, and the aqueous phase was extracted with ethyl acetate (10 ml × 2). The organic phases were combined, dried over anhydrous sodium sulphate and concentrated, and the resulting residue was separated and purified by silica gel column chromatography (eluent: methanol : dichloromethane (v/v) = 0%-15%), to afford compound **1M** (563 mg, 38.5%).
LCMS m/z = 576.3 $[M+H]^+$

Step 12:

Compound **1M** (563 mg, 0.98 mmol) was dissolved in dichloromethane (5 ml) and trifluoroacetic acid (1 ml) was added. The mixture was reacted at room temperature for 30 min. After the reaction was completed, the reaction liquid was concentrated and the resulting residue was purified by silica gel column chromatography (eluent: methanol : dichloromethane (v/v) = 0-15%), to afford compound **1N** (351 mg, 75.5%).
LCMS m/z = 476.3 $[M+H]^+$

Step 13:

Compound **1O** (5.0 g, 18.1 mmol) was dissolved in N-methylpyrrolidone (50 mL) and azetidin-3-ol (1.8 g, 18.1 mmol) and N,N-diisopropylethylamine (4.7 g, 45.0 mmol) were added. The mixture was reacted at 90°C for 2 h. The reaction liquid was cooled to room temperature and water (50 mL) was added. The resulting mixture was extracted with ethyl acetate (50 mL × 3), washed with water (50 mL × 2) and saturated brine (50 mL × 1), dried over anhydrous sodium sulphate and concentrated to afford crude product **1P** (5.36 g, yield 90.0%).

LCMS m/z = 330.1[M+H]$^+$

Step 14:

Compound **1P** (5.36 g, 16.9 mmol) was dissolved in dichloromethane (120 mL) and Dess-Martin periodinane (10.6 g, 25.2mmol) was added. The mixture was reacted at room temperature for 16 hours, washed with saturated sodium thiosulphate (50 mL × 1), saturated sodium bicarbonate (50 mL × 1) and saturated brine (50 mL × 1), dried over anhydrous sodium sulphate and concentrated to afford crude product **1Q** (5.33 g, yield 100.0%).
LCMS m/z = 328.1 [M+H]$^+$

Step 15:

Compound **1N** (50 mg, 0.105 mmol) and compound **1Q** (41 mg, 0.126 mmol) were dissolved in dry dichloroethane (5 ml). Titanium tetraisopropanolate (0.33 ml, 1.05 mmol) was added dropwise and the mixture was stirred and reacted at room temperature for 24 h. The reaction liquid was diluted with methanol (10 ml) and sodium cyanoborohydride (35 mg, 0.55 mmol) was added. The resulting mixture was stirred for 15 min and transferred to an ice water bath and stirred. Water (2 ml) was added dropwise and the resulting mixture was stirred for 10 min, adjusted with saturated sodium bicarbonate solution to pH 7-8 and filtered through a funnel lined with diatomaceous earth. The filtrate was adjusted with 1 M hydrochloric acid to pH 6-7 and concentrated and the resulting residue was separated and purified by liquid phase preparative column with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution: mobile phase A: 5%-50%; c. flow rate: 12 mL/min; d. elution time: 20 min, retention time: 10.0 min, to afford the target compound (16 mg, 15.0%).

LCMS m/z = 787.3 [M+H]$^+$
$^1$H NMR (400 MHz, DMSO-$d_6$) δ 12.17 (s, 1H), 11.07 (s, 1H), 8.62 (s, 1H), 8.54 (s, 1H), 8.34 (s, 1H), 7.91 (d, 1H), 7.81 (s, 1H), 7.79 - 7.71 (m, 2H), 7.49 (d, 1H), 6.91 (s, 1H), 6.76 (d, 1H), 5.08 (dd, 1H), 4.50 (s, 2H), 4.36 (d, 2H), 4.27 (d, 3H), 4.10 (s, 2H), 3.53 (s, 2H), 3.29 (s, 4H), 3.09 (s, 3H), 2.99 - 2.80 (m, 2H), 2.65 - 2.53 (m, 4H), 1.99 (d, 4H), 1.92 (s, 2H), 1.24 (s, 3H).

Example 2:

N-(1'-(2-(2,6-dioxopiperidin-3-yl)-1,3-dioxoisoindolin-5-yl)-[1,4'-bipiperidin]-4-yl)-5-(4-((7-ethyl-6-oxo-5,6-dihydro-1,5-naphthyridin-3-yl)methyl)piperazin-1-yl)picolinamide (Compound 2)

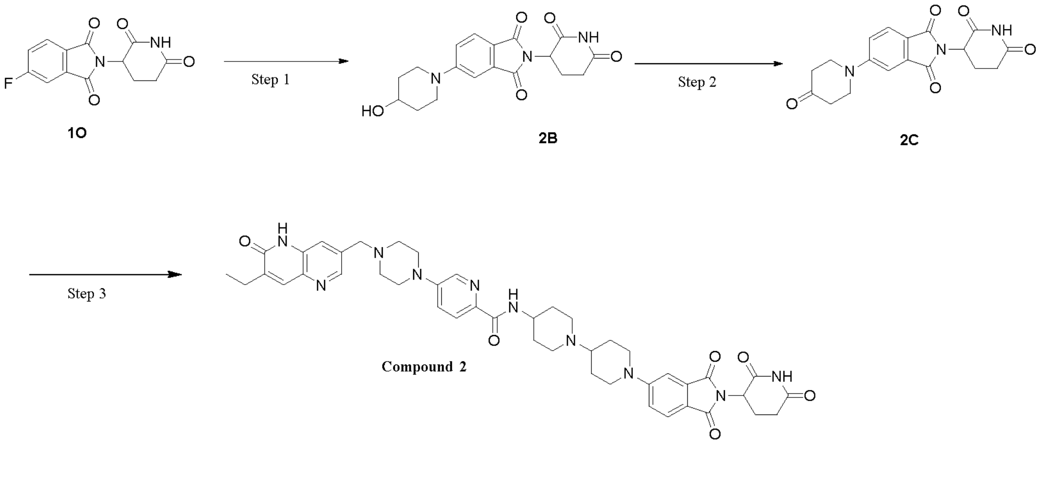


1O 2B 2C Compound 2

Step 1:

Compound **1O** (5.0 g, 18.1 mmol) was dissolved in N-methylpyrrolidone (50 mL) and piperidin-4-ylmethanol

(1.8 g, 18.1 mmol) and N,N-diisopropylethylamine (4.7 g, 45.0 mmol) were added. The mixture was reacted at 90°C for 2 h. The reaction liquid was cooled to room temperature and water (50 mL) was added. The resulting mixture was extracted with ethyl acetate (50 mL × 3), washed with water (50 mL × 2) and saturated brine (50 mL × 1), dried over anhydrous sodium sulphate and concentrated to afford crude product **2B** (6 g, yield 92%).
LCMS m/z = 358.1[M+H]$^+$

Step 2:

Compound **2B** (6 g, 16.8 mmol) was dissolved in dichloromethane (120 mL) and Dess-Martin periodinane (10.6 g, 25.2mmol) was added. The mixture was reacted at room temperature for 16 hours, washed with saturated sodium thiosulphate (50 mL × 1), saturated sodium bicarbonate (50 mL × 1) and saturated brine (50 mL × 1), dried over anhydrous sodium sulphate and concentrated to afford crude product **2C** (6 g, yield 100%).
LCMS m/z = 356.1 [M+H]$^+$

Step 3:

Compound **2C** (39 mg, 0.11 mmol) and **1N** (50 mg, 0.105 mmol) were dissolved in dry dichloroethane (5 ml). Titanium tetraisopropanolate (0.33 ml, 1.05 mmol) was added dropwise and the mixture was stirred and reacted at room temperature for 24 h. The reaction liquid was diluted with methanol (10 ml) and sodium cyanoborohydride (35 mg, 0.55 mmol) was added. The resulting mixture was stirred for 15 min and transferred to an ice water bath and stirred. Water (2 ml) was added dropwise and the resulting mixture was stirred for 10 min, adjusted with saturated sodium bicarbonate solution to pH 7-8 and filtered through a funnel lined with diatomaceous earth. The filtrate was adjusted with 1 M hydrochloric acid to pH 6-7 and concentrated and the resulting residue was separated and purified by liquid phase preparative column with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution: mobile phase A: 5%-50%; c. flow rate: 12 mL/min; d. elution time: 20 min, retention time: 9.0 min, to afford target compound **2** (21 mg, 22.4%).

LCMS m/z = 815.3 [M+H]$^+$
$^1$H NMR (400 MHz, DMSO-$d_6$) δ 12.23 - 12.02 (m, 1H), 11.06 (s, 1H), 9.12 (s, 1H), 8.52 (s, 2H), 8.32 (s, 1H), 7.90 (s, 1H), 7.80 (s, 1H), 7.71 (d, 1H), 7.48 (s, 1H), 7.41 (s, 1H), 7.33 (s, 1H), 5.32 (s, 1H), 5.07 (s, 1H), 4.23 (s, 2H), 4.07 (s, 2H), 3.50 (s, 4H), 3.22 - 3.09 (m, 4H), 2.95 (d, 5H), 2.59 (dd, 5H), 2.33 (s, 1H), 2.06 (d, 8H), 1.71 (s, 2H), 1.46 (s, 1H), 1.20 (t, 3H).

Example 3:

N-(1-((1-(2-(2,6-dioxopiperidin-3-yl)-1,3-dioxoisoindolin-5-yl)piperidin-4-yl)methyl)piperidin-4-yl)-5-(4-((7-ethyl-6-oxo-5,6-dihydro-1,5-naphthyridin-3-yl)methyl)piperazin-1-yl)picolinamide (Compound 3)

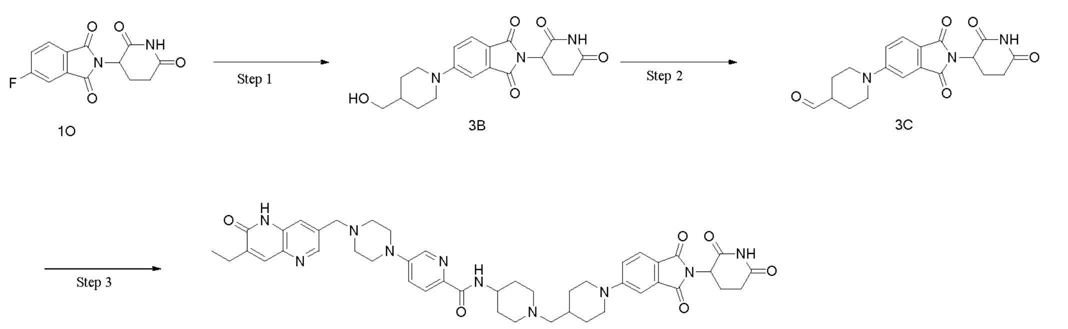

1O
3B
3C
Compound 3

Step 1:

Compound **1O** (5.0 g, 18.1 mmol) was dissolved in N-methylpyrrolidone (50 mL) and piperidin-4-ylmethanol (2.1 g, 18.1 mmol) and N,N-diisopropylethylamine (4.7 g, 45.0 mmol) were added. The mixture was reacted at 90°C for 2 h. The reaction liquid was cooled to room temperature and water (50 mL) was added. The resulting mixture was extracted with ethyl acetate (50 mL × 3), washed with water (50 mL × 2) and saturated brine (50 mL × 1), dried over anhydrous sodium sulphate and concentrated to afford crude product **3B** (6 g, yield 89.6%).
LCMS m/z = 372.1[M+H]$^+$

Step 2:

Compound **3B** (6 g, 16.2 mmol) was dissolved in dichloromethane (120 mL) and Dess-Martin periodinane (13.7 g, 32.4mmol) was added. The mixture was reacted at room temperature for 16 hours, washed with saturated sodium thiosulphate (50 mL × 1), saturated sodium bicarbonate (50 mL × 1) and saturated brine (50 mL × 1), dried over anhydrous sodium sulphate and concentrated to afford crude product **3C** (6 g, yield 100.0%).
LCMS m/z = 370.1 [M+H]$^+$

Step 3:

Compound **3C** (81 mg, 0.22 mmol) and **1N** (50 mg, 0.11 mmol) were added to ethanol (10 mL) and then 1,2-dichloroethane (5 mL) was added. Six drops of glacial acetic acid were added dropwise. The mixture was reacted at 80°C for 12 hours and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reversed phase preparative column, mobile phase: deionized water containing 0.1% trifluoroacetic acid (A) and acetonitrile (B), gradient elution, mobile phase B = 5%-50%, elution time: 15 min, flow rate: 12 mL/min, and column temperature: 30°C), to afford title compound 3 (30 mg, yield 33%, retention time: about 6.0 min).

MS M/Z (ESI): m/z = 829.4 [M+H]$^+$
$^1$H NMR (400 MHz, $CD_3OD$) δ 8.60 - 8.52 (m, 1H), 8.36 (d, 1H), 7.99 (d, 1H), 7.90 (d, 2H), 7.71 - 7.65 (m, 1H), 7.53 - 7.46 (m, 1H), 7.36 (d, 1H), 7.27 - 7.19 (m, 1H), 5.09 - 5.05 (m, 1H), 4.55 (s, 2H), 4.19 - 4.05 (m, 3H), 3.76 - 3.62 (m, 5H), 3.51 - 3.43 (m, 4H), 3.21 - 3.00 (m, 6H), 2.91 - 2.66 (m, 6H), 2.27 - 2.16 (m, 3H), 2.14 - 1.99 (m, 3H), 1.94 (d, 2H), 1.50 - 1.38 (m, 2H), 1.31 - 1.26 (m, 3H).

Example 4:

N-(1-((1-(2-(2,6-dioxopiperidin-3-yl)-1,3-dioxoisoindolin-5-yl)piperidin-4-yl)methyl)azetidin-3-yl)-5-(4-((7-ethyl-6-oxo-5,6-dihydro-1,5-naphthyridin-3-yl)methyl)piperazin-1-yl)picolinamide (Compound 4)

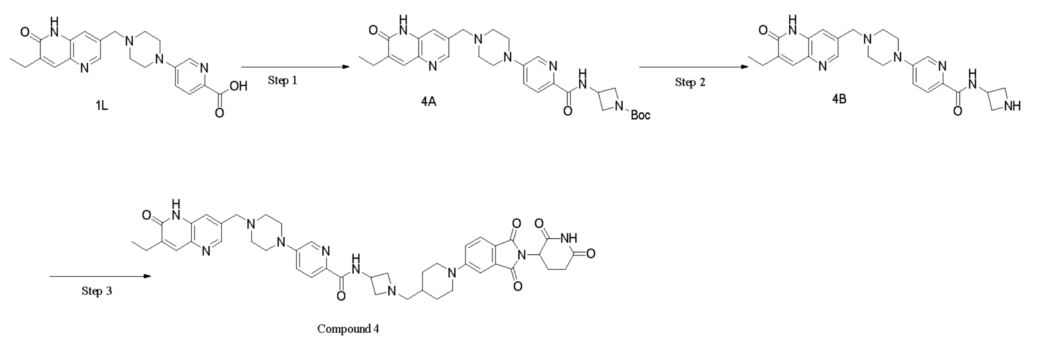


Step 1:

Compound **1L** (500 mg, 1.26 mmol) and tert-butyl 3-aminoazetidine-1-carboxylate (259 mg, 1.51 mmol) were dissolved in DMF (5 ml) and benzotriazole-N,N,N',N'-tetramethyluronium hexafluorophosphate (476 mg, 1.26 mmol) was added. The mixture was subjected to nitrogen replacement three times and stirred at room temperature for 20 min.

N,N-diisopropylethylamine (486 mg, 3.77 mmol) was added and the reaction liquid was stirred and reacted at room temperature for 2 h. After the reaction was completed, water (5 ml) and ethyl acetate (6 ml) were added. The organic phase was separated, and the aqueous phase was extracted with ethyl acetate (6 ml × 2). The organic phases were combined, dried over anhydrous sodium sulphate and concentrated, and the resulting residue was separated and purified by silica gel column chromatography (eluent: methanol : dichloromethane (v/v) = 0%-15%), to afford compound **4A** (313 mg, 45.0%).
LCMS m/z = 548.3 $[M+H]^+$

Step 2:

Compound **4A** (313 mg, 0.57 mmol) was dissolved in dichloromethane (5 ml) and trifluoroacetic acid (1 ml) was added. The mixture was reacted at room temperature for 30 min. After the reaction was completed, the reaction liquid was concentrated, adjusted to pH > 7 with triethylamine, and then purified by C18 column chromatography (acetonitrile:0.1% aqueous ammonia solution (v/v) = 50%) to afford compound **4B** (155 mg, 60.5%).
LCMS m/z = 448.3 $[M+H]^+$

Step 3:

Compound **3C** (81 mg, 0.22 mmol) and compound **4B** (50 mg, 0.11 mmol) were added to ethanol (10 mL) and then 1,2-dichloroethane (5 mL) was added. Three drops of glacial acetic acid were added dropwise. The mixture was reacted at room temperature for 1 hour and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **4** (30 mg, yield 34.1%, retention time: about 6.3 min).

MS M/Z (ESI): m/z = 801.3 $[M+H]^+$
$^1$H NMR (400 MHz, $CD_3OD$) δ 8.61 - 8.54 (m, 1H), 8.38 (d, 1H), 7.99 (d, 1H), 7.87 (s, 2H), 7.68 (d, 1H), 7.50 - 7.46 (m, 1H), 7.36 (d, 1H), 7.27 - 7.20 (m, 1H), 5.10 - 5.03 (m, 1H), 4.94 - 4.86 (m, 1H), 4.64 - 4.52 (m, 2H), 4.50 - 4.34 (m, 4H), 4.13 - 4.04 (m, 2H), 3.74 - 3.56 (m, 4H), 3.38 - 3.34 (m, 3H), 3.06 - 2.66 (m, 9H), 2.15 - 1.78 (m, 5H), 1.47 - 1.38 (m, 2H), 1.30 - 1.28 (m, 3H).

Example 5:

N-(1-(1-(2-(2,6-dioxopiperidin-3-yl)-1,3-dioxoisoindolin-5-yl)piperidin-4-yl)azetidin-3-yl)-5-(4-((7-ethyl-6-oxo-5,6-dihydro-1,5-naphthyridin-3-yl)methyl)piperazin-1-yl)picolinamide (Compound 5)

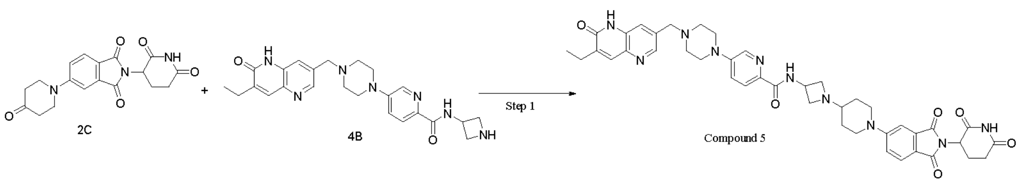


Step 1:

Compound **2C** (81 mg, 0.22 mmol) and compound **4B** (50 mg, 0.11 mmol) were added to ethanol (10 mL) and then 1,2-dichloroethane (5 mL) was added. Three drops of glacial acetic acid were added dropwise. The mixture was reacted at room temperature for 1 hour and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **5** (30 mg, yield 34.7%, retention time: about 6.2 min).

MS M/Z (ESI): m/z = 787.4 $[M+1]^+$

$^1H$ NMR (400 MHz, $CD_3OD$) $\delta$ 8.59 (s, 1H), 8.34 (d, 1H), 7.99 (s, 1H), 7.87 (s, 2H), 7.71 (d, 1H), 7.50 - 7.44 (m, 1H), 7.41 (d, 1H), 7.31 - 7.23 (m, 1H), 5.10 - 5.05 (m, 1H), 4.56 - 4.33 (m, 6H), 4.22 - 4.14 (m, 2H), 3.78 - 3.47 (m, 6H), 3.15 - 2.98 (m, 3H), 2.94 - 2.52 (m, 7H), 2.24 - 1.98 (m, 4H), 1.62 - 1.48 (m, 2H), 1.38 - 1.31 (m, 2H), 1.30 - 1.27 (m, 3H).

Example 6:

N-(1-((1-(2-(2,6-dioxopiperidin-3-yl)-1-oxoisoindolin-5-yl)piperidin-4-yl)methyl)piperidin-4-yl)-5-(4-((7-ethyl-6-oxo-5,6-dihydro-1,5-naphthyridin-3-yl)methyl)piperazin-1-yl)picolinamide (Compound 6)

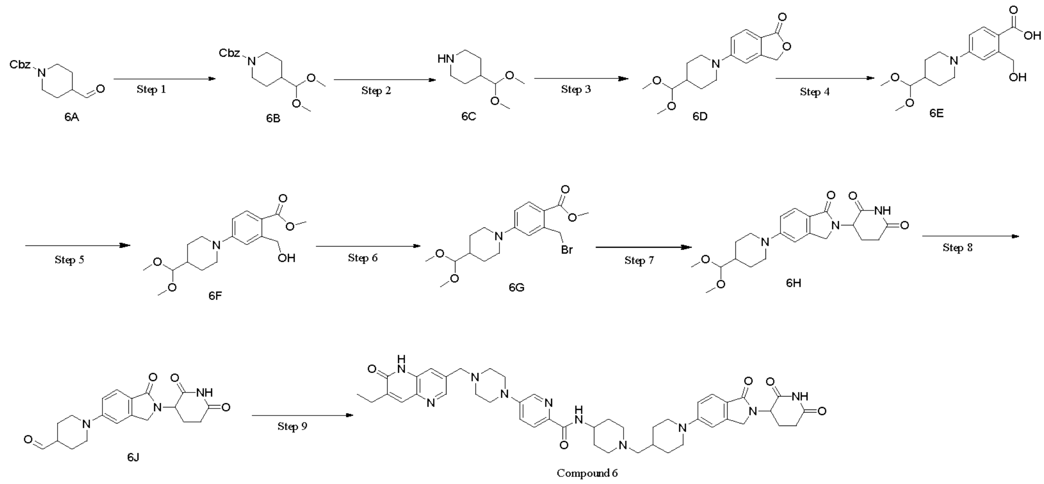

6A 6B 6C 6D 6E 6F 6G 6H 6J Compound 6

Step 1:

Compound **6A** benzyl 4-formylpiperidine-1-carboxylate (5.0 g, 20.2 mmol) was dissolved in methanol (60 mL) and then trimethyl orthoformate (20 mL) and p-toluenesulphonic acid (500 mg) were added. The mixture was reacted at room temperature for 4 h. After concentration, water (50 mL) was added and the resulting mixture was extracted with ethyl acetate (50 mL $\times$ 3), washed with saturated aqueous sodium bicarbonate solution (50 mL $\times$ 2) and saturated brine (50 mL $\times$ 1), dried over anhydrous sodium sulphate and concentrated to afford crude product **6B** (6 g, yield 100.0%).
LCMS m/z = 294.1$[M+H]^+$

Step 2:

Compound **6B** (6 g, 20.2 mmol) was dissolved in methanol (100 mL) and palladium on carbon (600 mg) was added. The mixture was subjected to hydrogenation at room temperature for 16 hours, filtered, dried and concentrated to afford crude product **6C** (3 g, yield 93.1%).
LCMS m/z = 160.1 $[M+H]^+$

Step 3:

Compound **6C** (2 g, 12.6 mmol) and 5-fluoro-1(3H)-isobenzofuranone (1.9 g, 12.6 mmol) were added to dimethyl sulphoxide (40 mL) and then N,N-diisopropylethylamine (3.3 g, 25.2 mmol) was added. The mixture was reacted at 120°C for 12 hours and cooled to room temperature. Water (50 mL) was added and the resulting mixture was extracted with ethyl acetate (50 mL $\times$ 3), washed with water (50 mL $\times$ 2) and saturated brine (50 mL $\times$ 1), dried over anhydrous sodium sulphate and concentrated. The residue was purified by silica gel column chromatography (petroleum ether : ethyl acetate (v/v) = 20 : 100) to afford product **6D** (3 g, yield 81.2%).
LCMS m/z = 292.2$[M+H]^+$

Step 4:

Compound **6D** (3 g, 10.3 mmol) was dissolved in methanol (30 mL), and water (30 mL) and sodium hydroxide (0.82 g, 20.6 mmol) were added. The mixture was reacted at room temperature for 2 hours, adjusted to pH = 5-6 with 1N hydrochloric acid, extracted with ethyl acetate (50 mL × 3), washed with water (50 mL × 1) and saturated brine (50 mL × 1), dried over anhydrous sodium sulphate and concentrated to afford product **6E** (2 g, yield 66.0%).
LCMS m/z = 310.1 $[M+H]^+$

Step 5:

Compound **6E** (2 g, 6.5 mmol) was dissolved in methanol (30 mL) and ethyl acetate (30 mL) was added. The mixture was cooled to 0°C and trimethylsilyldiazomethane (1.4 g, 12.1 mmol) was added dropwise. The mixture was reacted at room temperature for 2 hours, extracted with ethyl acetate (50 mL × 3), washed with water (50 mL × 1) and saturated brine (50 mL × 1), dried over anhydrous sodium sulphate and concentrated. The residue was purified by silica gel column chromatography (petroleum ether : ethyl acetate (v/v) = 20 : 100) to afford product **6F** (2 g, yield 95.2%).
LCMS m/z = 324.2$[M+H]^+$

Step 6:

Compound **6F** (0.9 g, 2.8 mmol) was dissolved in tetrahydrofuran (10 mL) and triphenylphosphine (0.73 g, 2.8 mmol) was added. The mixture was cooled to 0°C and carbon tetrabromide (0.93 g, 2.8 mmol) was added dropwise. The resulting mixture was reacted at room temperature for 1 hour and saturated ammonium chloride (20 mL) was added. The reaction liquid was then extracted with ethyl acetate (20 mL × 3), washed with saturated brine (50 mL × 1), dried over anhydrous sodium sulphate and concentrated. The residue was purified by silica gel column chromatography (petroleum ether : ethyl acetate (v/v) = 15 : 100) to afford product **6G** (0.4 g, yield 37.0%).
LCMS m/z = 386.1$[M+H]^+$

Step 7:

Compound **6G** (0.4 g, 1 mmol) was dissolved in acetonitrile (10 mL) and 3-amino-2,6-piperidinedione (153 mg, 1.2 mmol) and N,N-diisopropylethylamine (194 mg, 1.5 mmol) were added. The mixture was reacted at 80°C for 12 hours and concentrated, and the residue was purified by silica gel column chromatography (methanol : dichloromethane (v/v) = 5 : 100) to afford product **6H** (0.25 g, yield 60.0%).
LCMS m/z = 402.2$[M+H]^+$

Step 8:

Compound **6H** (0.25 g, 0.6 mmol) was dissolved in tetrahydrofuran (10 mL) and then water (2 mL) and pyridine p-toluenesulphonate (301 mg, 1.2 mmol) were added. The mixture was reacted at 80°C for 6 hours and water (20 mL) was added. The resulting mixture was extracted with dichloromethane (20 mL × 3), washed with saturated brine (50 mL × 1), dried over anhydrous sodium sulphate and concentrated. The residue was purified by silica gel column chromatography (methanol : dichloromethane (v/v) = 5 : 100) to afford product **6J** (0.21 g, yield 95.4%).
LCMS m/z = 356.2$[M+H]^+$

Step 9:

Compound **6J** (140 mg, 0.39 mmol) and compound 1N (50 mg, 0.11 mmol) were added to ethanol (10 mL) and then 1,2-dichloroethane (5 mL) was added. Six drops of glacial acetic acid were added dropwise. The mixture was reacted at 80°C for 12 hours and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **6** (30 mg, yield 33.5%, retention time: about 6.1 min)

MS M/Z (ESI): m/z = 815.4 $[M+H]^+$
$^1$H NMR (400 MHz, $CD_3OD$) δ 8.62 (d, 1H), 8.37 (d, 1H), 7.99 (d, 1H), 7.92 - 7.85 (m, 2H), 7.68 - 7.62 (m, 1H), 7.54 - 7.47 (m, 1H), 7.10 (d, 2H), 5.16 - 4.98 (m, 1H), 4.58 (s, 2H), 4.47 - 4.33 (m, 2H), 4.24 - 4.06 (m, 1H), 3.97 (d, 2H),

3.76 - 3.45 (m, 10H), 3.25 - 3.05 (m, 4H), 2.98 - 2.66 (m, 6H), 2.52 - 2.41 (m, 1H), 2.27 - 1.89 (m, 8H), 1.53 - 1.41 (m, 2H), 1.33 - 1.25 (m, 3H).

Example 7:

N-(1-((1-(2-((3R,SR)-adamantan-1-yl)acetyl)piperidin-4-yl)methyl)piperidin-4-yl)-5-(4-((7-ethyl-6-oxo-5,6-dihydro-1,5-naphthyridin-3-yl)methyl)piperazin-1-yl)picolinamide (Compound 7)

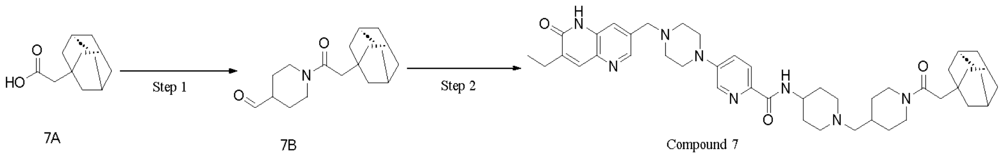


7A 7B Compound 7

Step 1:

Adamantaneacetic acid **7A** (5.0 g, 25.8 mmol) was dissolved in N,N-dimethylformamide (50 mL) and then N,N,N',N'-tetramethyl-O-(7-azabenzotriazol-1-yl)uronium hexafluorophosphate (14.7 g, 38.7 mmol), 4-formylpiperidine (3 g, 25.8 mmol) and N,N-diisopropylethylamine (16.6 g, 51.6 mmol) were added. The mixture was reacted at room temperature for 12 hours and water (50 mL) was added. The resulting mixture was extracted with ethyl acetate (50 mL × 3), washed with water (50 mL × 3) and saturated brine (50 mL × 1), dried over anhydrous sodium sulphate and concentrated. The residue was purified by silica gel column chromatography (petroleum ether : ethyl acetate (v/v) = 20 : 100) to afford product **7B** (2 g, yield 26.6%).
LCMS m/z = 290.2[M+H]$^+$

Step 2:

Compound **7B** (95 mg, 0.33 mmol) and compound **1N** (50 mg, 0.11 mmol) were added to ethanol (10 mL) and then 1,2-dichloroethane (5 mL) was added. Six drops of glacial acetic acid were added dropwise. The mixture was reacted at 80°C for 12 hours and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound 7 (28 mg, yield 34.1%, retention time: about 6.3 min).

MS M/Z (ESI): m/z = 749.5 [M+H]$^+$
$^1$H NMR (400 MHz, DMSO-$d_6$) δ 12.14 (s, 1H), 9.18 (d, 1H), 8.59 - 8.42 (m, 2H), 8.33 (s, 1H), 7.92 - 7.87 (m, 1H), 7.80 (s, 1H), 7.75 (s, 1H), 7.48 (d, 1H), 4.45 (d, 2H), 3.99 (d, 3H), 3.55 (d, 2H), 3.40 - 2.95 (m, 12H), 2.60 - 2.55 (m, 2H), 2.11 - 1.90 (m, 10H), 1.75 (d, 2H), 1.68 - 1.56 (m, 13H), 1.24 - 1.16 (m, 3H), 1.14 - 0.95 (m, 2H).

Example 8:

N-(1-((1-(4-(3-(2,4-dihydroxy-5-isopropylphenyl)-5-hydroxy-4H-1,2,4-triazol-4-yl)benzyl)piperidin-4-yl)methyl)piperidin-4-yl)-5-(4-((7-ethyl-6-oxo-5,6-dihydro-1,5-naphthyridin-3-yl)methyl)piperazin-1-yl)picolinamide (Compound 8)

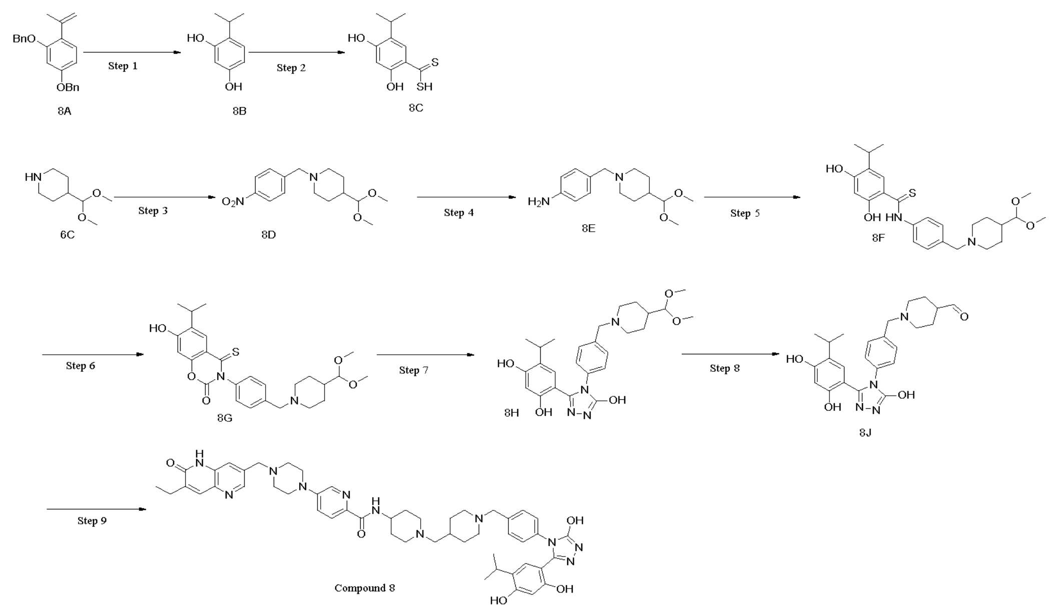


8A 8B 8C 6C 8D 8E 8F 8G 8H 8J Compound 8

Step 1:

Compound **8A** (20.0 g, 60.6 mmol) was dissolved in ethanol (400 mL) and then palladium on carbon (5 g) was added. The mixture was reacted under hydrogen at 60°C for 24 h. The reaction liquid was filtered and concentrated to afford crude product **8B** (9.5 g, yield 100.0%).
LCMS m/z = 153.1[M+H]$^+$

Step 2:

Compound **8B** (9.5 g, 60.6 mmol) was dissolved in DMF (200 mL) and then potassium O-ethyl dithiocarbonate (12.6 g, 78.78 mmol) was added. Under nitrogen protection, the mixture was reacted at 100°C for 12 hours and cold water (200 mL) was added under ice bath. The resulting mixture was adjusted to pH = 2-3 with 6 N hydrochloric acid and filtered, and the filter cake was washed with water and dried to afford crude product **8C** (7 g, yield 52.0%).
LCMS m/z = 229.0 [M+H]$^+$

Step 3:

Compound **6C** (9 g, 56.6 mmol) and p-nitrobenzyl bromide (12.2 g, 56.6 mmol) were added to dichloromethane (200 mL) and then triethylamine (8.6 g, 84.9 mmol) was added. The mixture was reacted at room temperature for 12 hours and water (200 mL) was added. The resulting mixture was extracted with dichloromethane (200 mL × 2), washed with saturated brine (200 mL × 1), dried over anhydrous sodium sulphate and concentrated. The residue was purified by silica gel column chromatography (petroleum ether : ethyl acetate (v/v) = 20 : 100) to afford product **8D** (7.5 g, yield 45.4%).
LCMS m/z = 295.2[M+H]$^+$

Step 4:

Compound **8D** (7.5 g, 25.5 mmol) was dissolved in methanol (200 mL) and then ammonium chloride (6.7 g, 127.5mmol) and reduced iron powder (7.1 g, 127.5 mmol) were added. The mixture was reacted at 60°C for 24 hours and filtered, and the filtrate was washed with saturated sodium bicarbonate solution (100 mL × 1), dried over anhydrous sodium sulphate and concentrated to afford crude product **8E** (6 g, yield 100.0%).
LCMS m/z = 265.2[M+H]$^+$

Step 5:

Compound **8C** (7 g, 30.7 mmol) was dissolved in DMF (100 mL) and then sodium 2-chloroacetate (5.4 g, 46.5 mmol) and sodium bicarbonate (5.2 g, 61.4 mmol) were added. Under nitrogen protection, the mixture was reacted at

room temperature for 4 hours and compound **8E** (6 g, 22.7 mmol, dissolved in DMF (10 mL)) was added. Under nitrogen protection, the mixture was reacted at 80°C for 5 hours. Subsequently, water (100 mL) was added at room temperature. The resulting mixture was extracted with ethyl acetate (200 mL × 3), washed with water (200 mL × 2) and saturated brine (200 mL × 1), dried over anhydrous sodium sulphate and concentrated to afford crude product **8F** (10 g, yield 97.6%).
LCMS m/z = 459.2$[M+H]^+$

Step 6:

Compound **8F** (10.0 g, 21.8 mmol) was dissolved in tetrahydrofuran (100 mL) and then N,N'-carbonyldiimidazole (4.24 g, 26.16 mmol) was added. The mixture was reacted at room temperature for 4 hours and then saturated ammonium chloride (100 mL) was added. The resulting mixture was extracted with ethyl acetate (200 mL × 3), washed with saturated sodium bicarbonate (100 mL × 1) and saturated brine (50 mL × 1), dried over anhydrous sodium sulphate and concentrated to afford crude product **8G** (11 g, yield 100.0%).
LCMS m/z = 485.2 $[M+H]^+$

Step 7:

Compound **8G** (11.0 g, 21.8 mmol) was dissolved in ethanol (100 mL) and hydrazine hydrate (3.3 g, 65.4 mmol) was added. The mixture was reacted at room temperature for 12 hours, concentrated and purified by silica gel column chromatography (methanol : dichloromethane (v/v) = 5 : 100) to afford product **8H** (4.0 g, yield 38.1%).
LCMS m/z = 483.2$[M+1]^+$

Step 8:

Compound **8H** (1.4 g, 2.9 mmol) was dissolved in trifluoroacetic acid (7 mL). The mixture was reacted at room temperature for 4 hours, adjusted to pH = 7-8 with saturated sodium bicarbonate and filtered. The filter cake was dried to afford crude product **8J** (1.2 g, yield 100.0%).
LCMS m/z = 437.2$[M+1]^+$

Step 9:

Compound **8J** (191 mg, 0.44 mmol) and compound **1N** (50 mg, 0.11 mmol) were added to ethanol (10 mL) and then 1,2-dichloroethane (5 mL) was added. Six drops of glacial acetic acid were added dropwise. The mixture was reacted at 80°C for 24 hours and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **8** (25 mg, yield 25.5%, retention time: about 6.2 min).

MS M/Z (ESI): m/z = 896.4 $[M+1]^+$
$^1$H NMR (400 MHz, DMSO-$d_6$) δ 12.04 (d, 2H), 9.62 (s, 1H), 9.36 (s, 1H), 8.52 (s, 1H), 8.32 (s, 1H), 7.89 (d, 1H), 7.80 (s, 1H), 7.75 (s, 1H), 7.52 - 7.47 (m, 3H), 7.31 - 7.23 (m, 3H), 6.84 (s, 1H), 6.26 (d, 1H), 4.43 - 4.24 (m, 4H), 4.06 - 3.99 (m, 2H), 3.59 - 3.55 (m, 2H), 3.42 - 3.31 (m, 4H), 3.20 - 2.87 (m, 11H), 2.60 - 2.55 (m, 2H), 2.02 - 1.75 (m, 8H), 1.45 - 1.33 (m, 2H), 1.22 - 1.17 (m, 3H), 0.99 (d, 6H).

Example 9:

N-(1-((1-(4-(3-(2,4-dihydroxy-5-isopropylphenyl)-5-hydroxy-4H-1,2,4-triazol-4-yl)benzyl)piperidin-4-yl)methyl)azetidin-3-yl)-5-(4-((7-ethyl-6-oxo-5,6-dihydro-1,5-naphthyridin-3-yl)methyl)piperazin-1-yl)picolinamide (Compound **9**)

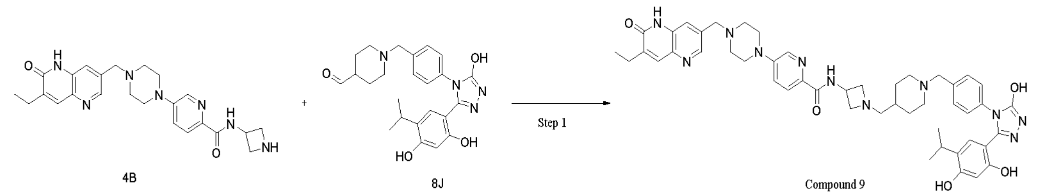


4B 8J Compound 9

Step 1:

Compound **8J** (191 mg, 0.44 mmol) and compound **4B** (50 mg, 0.11 mmol) were added to ethanol (10 mL) and then 1,2-dichloroethane (5 mL) was added. Three drops of glacial acetic acid were added dropwise. The mixture was reacted at room temperature for 1 hour and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **9** (5 mg, yield 5.3%, retention time: about 6.3 min)

MS M/Z (ESI): m/z = 868.4 $[M+H]^+$
$^1H$ NMR (400 MHz, DMSO-$d_6$) δ 11.97 (d, 2H), 9.63 (s, 1H), 9.37 (s, 1H), 9.12 (s, 1H), 8.65 (s, 1H), 8.46 (s, 1H), 8.24 (d, 1H), 8.06 (d, 1H), 7.77 (s, 1H), 7.66 (s, 1H), 7.50 (d, 2H), 7.28 (d, 2H), 6.83 (s, 1H), 6.27 (s, 1H), 5.08 - 4.91 (m, 1H), 4.84 - 4.70 (m, 1H), 4.42 - 4.23 (m, 4H), 3.95 (s, 2H), 3.38 (d, 2H), 3.16 (d, 4H), 3.03 - 2.73 (m, 10H), 2.59 - 2.55 (m, 2H), 2.00 - 1.82 (m, 3H), 1.47 - 1.32 (m, 2H), 1.21 -1.17 (m, 3H), 0.98 (d, 6H).

Example 10:

N-(1-(2-(2,6-dioxopiperidin-3-yl)-1,3-dioxoisoindolin-5-yl)piperidin-4-yl)-5-(4-((7-ethyl-6-oxo-5,6-dihydro-1,5-naphthyridin-3-yl)methyl)piperazin-1-yl)picolinamide (Compound **10**)

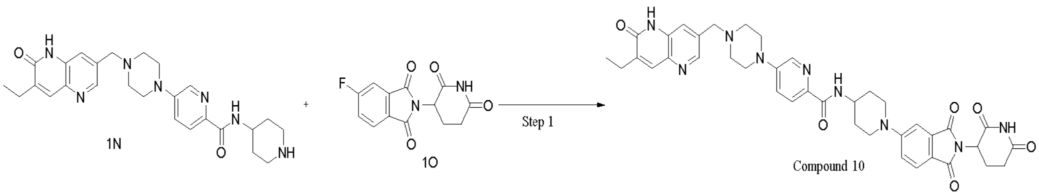


1N 1O Compound 10

Step 1:

**1N** (50 mg, 0.11 mmol) and compound **1O** (30 mg, 0.11 mmol) were added to dimethyl sulphoxide (2 mL) and then N,N-diisopropylethylamine (28 mg, 0.22 mmol) was added. The mixture was reacted at 100°C for 2 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **10** (30 mg, yield 37.3%, retention time: about 7.3 min).

$^1H$ NMR (400 MHz, $CD_3OD$) δ 8.61 (d, 1H), 8.34 (d, 1H), 7.99 (d, 1H), 7.88 (d, 2H), 7.67 (d, 1H), 7.49 (d, 1H), 7.37 (d, 1H), 7.24 (d, 1H), 5.06 (d, 1H), 4.56 (s, 2H), 4.23 - 4.12 (m, 1H), 4.09 - 4.00 (m, 2H), 3.84 - 3.35 (m, 8H), 3.24 - 3.11 (m, 2H), 2.92 - 2.60 (m, 5H), 2.15 - 2.00 (m, 3H), 1.83 - 1.66 (m, 2H), 1.35 - 1.23 (m, 3H).
MS M/Z (ESI): m/z = 732.3 $[M+H]^+$

Example 11:

N-(1-(2-(2,6-dioxopiperidin-3-yl)-1,3-dioxoisoindolin-5-yl)azetidin-3-yl)-5-(4-((7-ethyl-6-oxo-5,6-dihydro-1,5-naphthyridin-3-yl)methyl)piperazin-1-yl)picolinamide (Compound **11**)

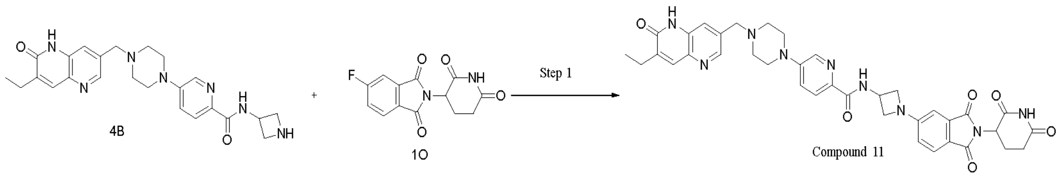


4B + 1O → Compound 11

Step 1:

**4B** (50 mg, 0.11 mmol) and compound **1O** (30 mg, 0.11 mmol) were added to dimethyl sulphoxide (2 mL) and then N,N-diisopropylethylamine (28 mg, 0.22 mmol) was added. The mixture was reacted at 100°C for 2 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **11** (5 mg, yield 6.5%, retention time: about 7.3 min).

$^1$H NMR (400 MHz, DMSO-$d_6$) $\delta$ 11.83 (s, 1H), 11.05 (s, 1H), 9.12 (d, 1H), 8.34 (d, 2H), 7.85 (d, 1H), 7.75 (s, 1H), 7.69 - 7.61 (m, 2H), 7.41 (d, 1H), 6.81 (s, 1H), 6.68 (d, 1H), 5.10 - 4.86 (m, 2H), 4.43 - 4.28 (m, 2H), 4.18 - 4.02 (m, 2H), 3.74 - 3.58 (m, 2H), 3.44 - 3.33 (m, 4H), 2.96 - 2.79 (m, 1H), 2.69 - 2.52 (m, 8H), 2.08 - 1.94 (m, 1H), 1.29 - 0.97 (m, 3H).

MS M/Z (ESI): m/z = 704.2 $[M+H]^+$

Example 12:

N-(1-(2-((3R,5R)-adamantan-1-yl)acetyl)piperidin-4-yl)-5-(4-((7-ethyl-6-oxo-5,6-dihydro-1,5-naphthyridin-3-yl)methyl)piperazin-1-yl)picolinamide (Compound **12**)

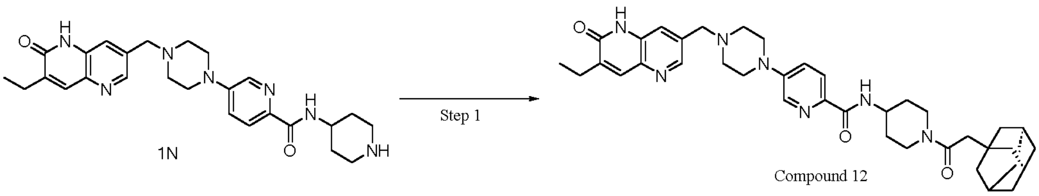


1N → Compound 12

Step 1:

**1N** (50 mg, 0.11mmol) and adamantaneacetic acid (42 mg, 0.22 mmol) were added to N,N-dimethylformamide (2 mL) and then HATU (84 mg, 0.22 mmol) and N,N-diisopropylethylamine (28 mg, 0.22 mmol) were added. The mixture was reacted at room temperature for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **12** (30 mg, yield 42.3%, retention time: about 7.1 min).

$^1$H NMR (400 MHz, $CD_3OD$) $\delta$ 8.62 (d, 1H), 8.36 (d, 1H), 8.00 (d, 1H), 7.92 (d, 1H), 7.88 (s, 1H), 7.51 (d, 1H), 4.65 - 4.51 (m, 3H), 4.18 - 4.00 (m, 2H), 3.88 - 3.40 (m, 8H), 3.29 - 3.19 (m, 1H), 2.88 - 2.74 (m, 1H), 2.72 - 2.60 (m, 2H), 2.31 - 2.13 (m, 2H), 2.05 - 1.90 (m, 5H), 1.78 - 1.64 (m, 12H), 1.63 - 1.49 (m, 2H), 1.33 - 1.23 (m, 3H).

MS M/Z (ESI): m/z = 652.3 $[M+H]^+$

Example 13:

N-(1-(2-((3R,5R)-adamantan-1-yl)acetyl)azetidin-3-yl)-5-(4-((7-ethyl-6-oxo-5,6-dihydro-1,5-naphthyridin-3-yl)methyl)piperazin-1-yl)picolinamide (Compound **13**)

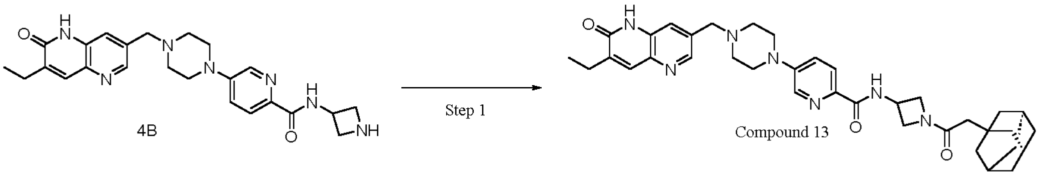


Step 1:

**4B** (50 mg, 0.11mmol) and adamantaneacetic acid (42 mg, 0.22 mmol) were added to N,N-dimethylformamide (2 mL) and then HATU (84 mg, 0.22 mmol) and N,N-diisopropylethylamine (28 mg, 0.22 mmol) were added. The mixture was reacted at room temperature for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **13** (30 mg, yield 42%, retention time: about 7.0 min).

$^1H$ NMR (400 MHz, $CD_3OD$) δ 8.62 (d, 1H), 8.38 (d, 1H), 7.97 (d, 1H), 7.90 (s, 1H), 7.87 (s, 1H), 7.49 (d, 1H), 4.79 - 4.72 (m, 1H), 4.61 - 4.50 (m, 3H), 4.35 - 4.21 (m, 2H), 4.07 - 3.98 (m, 1H), 3.88 - 3.34 (m, 8H), 2.77 - 2.61 (m, 2H), 2.06 - 1.85 (m, 5H), 1.79 - 1.56 (m, 12H), 1.38 - 1.17 (m, 3H).

MS M/Z (ESI): m/z = 624.3 $[M+H]^+$

Example 14:

N-(1'-(2-(2,6-dioxopiperidin-3-yl)-1,3-dioxoisoindolin-5-yl)-[1,3'-biazetidin]-3-yl)-5-(4-((7-ethyl-6-oxo-5,6-dihydro-1,5-naphthyridin-3-yl)methyl)piperazin-1-yl)picolinamide (Compound **14**)

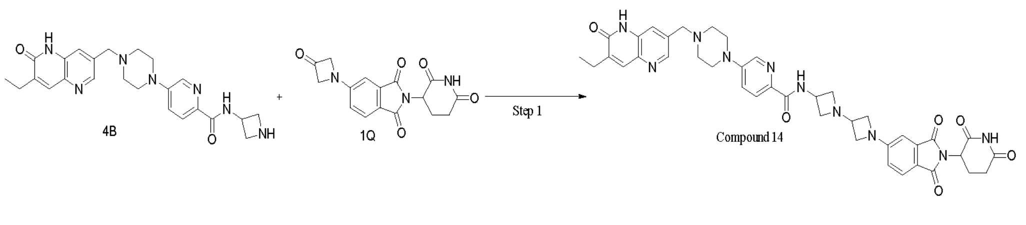


Step 1:

**4B** (50 mg, 0.11 mmol) and **1Q** (71 mg, 0.22 mmol) were added to ethanol (5 mL) and then 1,2-dichloroethane (5 mL) was added. Three drops of glacial acetic acid were added dropwise and the mixture was reacted at 60°C for 12 hours. Then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **14** (20 mg, yield 24.1%, retention time: about 6.5 min).

$^1H$ NMR (400 MHz, $CD_3OD$) δ 8.48 (d, 2H), 8.29 (d, 1H), 7.98 (d, 1H), 7.82 (s, 1H), 7.73 (s, 1H), 7.61 (d, 1H), 6.78 (s, 1H), 6.62 (d, 1H), 5.08 - 5.02 (m, 1H), 4.94 - 4.91 (m, 1H), 4.29 - 4.13 (m, 2H), 4.13 - 4.02 (m, 1H), 3.86 - 3.80 (m, 1H), 3.75 - 3.68 (m, 3H), 3.65 - 3.53 (m, 5H), 2.94 - 2.79 (m, 3H), 2.76 - 2.61 (m, 9H), 2.14 - 2.06 (m, 1H), 1.30 - 1.26 (m, 3H).

MS M/Z (ESI): m/z = 759.3 $[M+H]^+$

Example 15:

N-(1-(1-(2-(2,6-dioxopiperidin-3-yl)-1,3-dioxoisoindolin-4-yl)azetidin-3-yl)piperidin-4-yl)-5-(4-((7-ethyl-6-oxo-5,6-dihydro-1,5-naphthyridin-3-yl)methyl)piperazin-1-yl)picolinamide (Compound **15**)

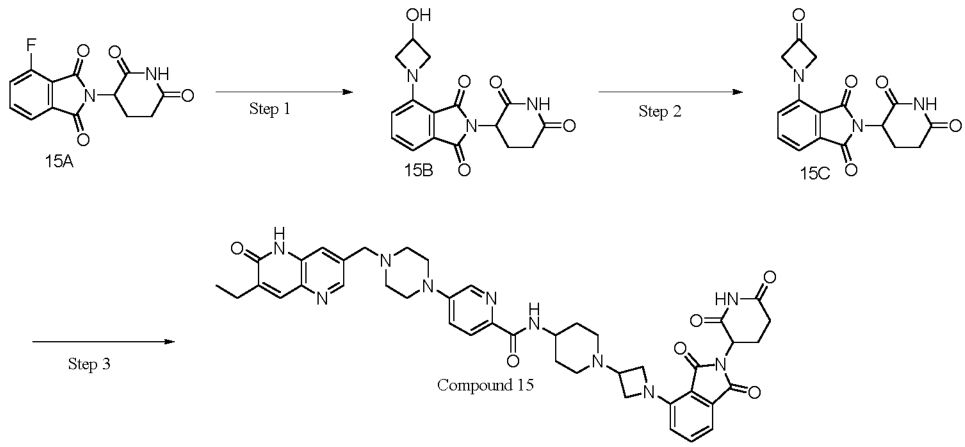


Step 1:

Compound **15A** (1.0 g, 3.6 mmol) was dissolved in dimethyl sulphoxide (10 mL) and then azetidin-3-ol hydrochloride (470 mg, 4.32 mmol) and N,N-diisopropylethylamine (936 mg, 7.2 mmol) were added. The mixture was reacted at 100°C for 2 h. The reaction liquid was cooled to room temperature and water (50 mL) was added. The resulting mixture was extracted with ethyl acetate (50 mL × 3), washed with water (50 mL × 2) and saturated brine (50 mL × 1), dried over anhydrous sodium sulphate and concentrated to afford crude product **15B** (1.2 g, yield 100.0%).
LCMS m/z = 330.1 $[M+H]^+$

Step 2:

Compound **15B** (1.2 g, 3.6 mmol) was dissolved in dichloromethane (30 mL) and then Dess-Martin periodinane (3.1 g, 7.2 mmol) was added. The mixture was reacted at room temperature for 16 hours, washed with saturated sodium thiosulphate (50 mL × 1), saturated sodium bicarbonate (50 mL × 1) and saturated brine (50 mL × 1), dried over anhydrous sodium sulphate and concentrated. The residue was separated and purified by silica gel column chromatography (methanol : dichloromethane (v/v) = 0 : 100-5 : 100) to afford title compound **15C** (600 mg, yield 50.1%).
LCMS m/z = 328.1 $[M+H]^+$

Step 3:

Compound **15C** (72 mg, 0.22 mmol) and **1N** (50 mg, 0.11 mmol) were added to ethanol (5 mL) and then 1,2-dichloroethane (5 mL) was added. Six drops of glacial acetic acid were added dropwise. The mixture was reacted at 80°C for 24 hours and then sodium cyanoborohydride (50 mg, 0.8 mmol) was added. The resulting mixture was reacted at room temperature for 1 h. The reaction liquid was spun to dryness, and then separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **15** (30 mg, yield 34.9%, retention time: about 6.3 min).

$^1H$ NMR (400 MHz, $CD_3OD$) δ8.63 (d, 1H), 8.37 (d, 1H), 8.00 (d, 1H), 7.94 (s, 1H), 7.87 (s, 1H), 7.63 - 7.58 (m, 1H), 7.54 - 7.49 (m, 1H), 7.26 (d, 1H), 6.86 (d, 1H), 5.12 - 5.04 (m, 1H), 4.66 - 4.56 (m, 4H), 4.52 - 4.42 (m, 2H), 4.28 - 4.15 (m, 2H), 3.80 - 3.49 (m, 10H), 3.29 - 3.05 (m, 2H), 2.91 - 2.62 (m, 5H), 2.34 - 1.86 (m, 5H), 1.31 - 1.27 (m, 3H).
MS M/Z (ESI): m/z = 787.3 $[M+H]^+$

Example 16:

N-(1-(2-(2,6-dioxopiperidin-3-yl)-1,3-dioxoisoindolin-4-yl)azetidin-3-yl)-5-(4-((7-ethyl-6-oxo-5,6-dihydro-1,5-naphthyridin-3-yl)methyl)piperazin-1-yl)picolinamide (Compound **16**)

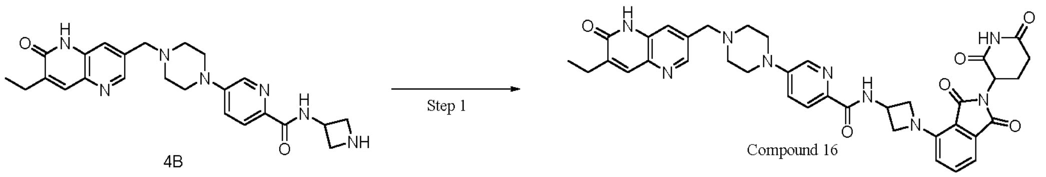


Step 1:

Compound **4B** (50 mg, 0.11 mmol) and 2-(2,6-dioxopiperidin-3-yl)-4-fluoroisoindoline-1,3-dione (36 mg, 0.13 mmol) were added to dimethyl sulphoxide (2 mL) and then N,N-diisopropylethylamine (28 mg, 0.22 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **16** (30 mg, yield 38.8%, retention time: about 7.0 min).

$^1$H NMR (400 MHz, DMSO-$d_6$) δ 11.82 (s, 1H), 11.04 (s, 1H), 9.07 (d, 1H), 8.40 (s, 1H), 8.27 (d, 1H), 7.84 (d, 1H), 7.75 (s, 1H), 7.63 (s, 1H), 7.61 - 7.54 (m, 1H), 7.40 (d, 1H), 7.13 (d, 1H), 6.81 (d, 1H), 5.12 - 4.97 (m, 1H), 4.90 - 4.76 (m, 1H), 4.62 - 4.45 (m, 2H), 4.33 - 4.13 (m, 2H), 3.65 (s, 2H), 3.40 - 3.32 (m, 4H), 3.31 - 3.30 (m, 2H), 2.97 - 2.76 (m, 1H), 2.59 - 2.53 (m, 6H), 2.00 (d, 1H), 1.22 - 1.12 (m, 3H).
MS M/Z (ESI): m/z = 704.3 [M+H]$^+$

Example 17:

N-(1-(2-(2,6-dioxopiperidin-3-yl)-6-fluoro-1,3-dioxoisoindolin-5-yl)azetidin-3-yl)-5-(4-((7-ethyl-6-oxo-5,6-dihydro-1,5-naphthyridin-3-yl)methyl)piperazin-1-yl)picolinamide (Compound **17**)

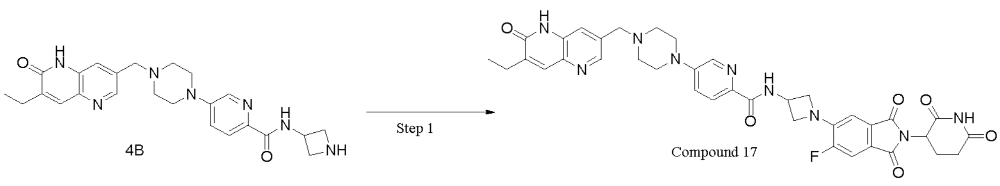


Step 1:

Compound **4B** (50 mg, 0.11 mmol) and 2-(2,6-dioxopiperidin-3-yl)-5,6-difluoroindoline-1,3-dione (38 mg, 0.13 mmol) were added to dimethyl sulphoxide (2 mL) and then N,N-diisopropylethylamine (28 mg, 0.22 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **17** (31 mg, yield 39.2%, retention time: about 7.1 min).

$^1$H NMR (400 MHz, DMSO-$d_6$) δ 11.83 (s, 1H), 11.07 (s, 1H), 9.08 (d, 1H), 8.40 (d, 1H), 8.24 (d, 1H), 7.85 (d, 1H), 7.75 (s, 1H), 7.67 - 7.52 (m, 2H), 7.40 (d, 1H), 6.96 (d, 1H), 5.11 - 5.03 (m, 1H), 4.97 - 4.85 (m, 1H), 4.52 - 4.41 (m, 2H), 4.28 - 4.19 (m, 2H), 3.65 (s, 2H), 3.45 - 3.32 (m, 4H), 3.31 - 3.31 (m, 2H), 2.95 - 2.80 (m, 1H), 2.60 - 2.53 (m, 6H), 2.08 - 1.94 (m, 1H), 1.20 - 1.16 (m, 3H).
MS M/Z (ESI): m/z = 722.2 [M+H]$^+$

Example 18:

N-(1-(2-(2,6-dioxopiperidin-3-yl)-6-fluoro-1,3-dioxoisoindolin-5-yl)piperidin-4-yl)-5-(4-((7-ethyl-6-oxo-5, 6-dihydro-1, 5-naphthyridin-3 - yl)methyl)piperazin-1-yl)picolinamide (Compound **18**)

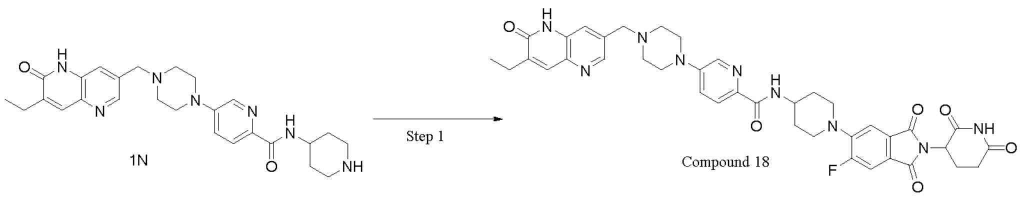


Step 1:

Compound **1N** (50 mg, 0.11 mmol) and 2-(2,6-dioxopiperidin-3-yl)-5,6-difluoroindoline-1,3-dione (38 mg, 0.13 mmol) were added to dimethyl sulphoxide (2 mL) and then N,N-diisopropylethylamine (28 mg, 0.22 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **18** (31 mg, yield 37.8%, retention time: about 7.1 min).

$^1$H NMR (400 MHz, DMSO-$d_6$) δ 12.18 (s, 1H), 11.09 (s, 1H), 8.54 (d, 1H), 8.41 - 8.30 (m, 2H), 7.92 (d, 1H), 7.79 (d, 2H), 7.72 (d, 1H), 7.55 - 7.45 (m, 2H), 5.14 - 5.08 (m, 1H), 4.53 (s, 2H), 4.24 - 3.83 (m, 3H), 3.68 - 3.59 (m, 2H), 3.57 - 3.10 (m, 6H), 3.09 - 3.00 (m, 2H), 2.94 - 2.83 (m, 1H), 2.72 - 2.52 (m, 4H), 2.09 - 2.00 (m, 1H), 1.94 - 1.76 (m, 4H), 1.23 - 1.16 (m, 3H).

MS M/Z (ESI): m/z = 750.3 $[M+H]^+$

Example 19:

N-(1-(2-(2,6-dioxopiperidin-3-yl)-1,3-dioxoisoindolin-4-yl)piperidin-4-yl)-5-(4-((7-ethyl-6-oxo-5,6-dihydro-1,5-naphthyridin-3-yl)methyl)piperazin-1-yl)picolinamide (Compound **19**)

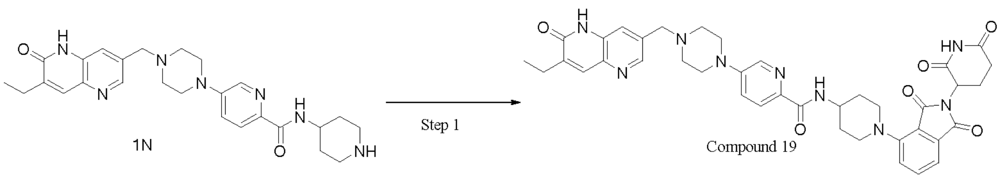


Step 1:

**1N** (50 mg, 0.11 mmol) and 2-(2,6-dioxopiperidin-3-yl)-4-fluoroisoindoline-1,3-dione (36 mg, 0.13 mmol) were added to dimethyl sulphoxide (2 mL) and then N,N-diisopropylethylamine (28 mg, 0.22 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **19** (30 mg, yield 37.3%, retention time: about 7.0 min).

$^1$H NMR (400 MHz, DMSO-$d_6$) δ 12.17 (s, 1H), 11.07 (s, 1H), 8.54 (s, 1H), 8.45 - 8.25 (m, 2H), 7.92 (d, 1H), 7.81 (s, 1H), 7.77 (s, 1H), 7.73 - 7.67 (m, 1H), 7.50 (d, 1H), 7.41 - 7.32 (m, 2H), 5.15 - 5.01 (m, 1H), 4.51 (s, 2H), 4.04 - 3.97 (m, 3H), 3.72 (d, 2H), 3.49 - 3.12 (m, 6H), 3.08 - 2.99 (m, 2H), 2.94 - 2.81 (m, 1H), 2.67 - 2.52 (m, 4H), 2.10 - 1.99 (m, 1H), 1.96 - 1.76 (m, 4H), 1.24 - 1.14 (m, 3H).

MS M/Z (ESI): m/z = 732.3 $[M+H]^+$

Example 20:

N-((3R)-1-(2-(2,6-dioxopiperidin-3-yl)-1,3-dioxoisoindolin-5-yl)pyrrolidin-3-yl)-5-(4-((7-ethyl-6-oxo-5, 6-dihydro-1, 5-naphthyridin-3-yl)methyl)piperazin-1-yl)picolinamide (Compound 20)

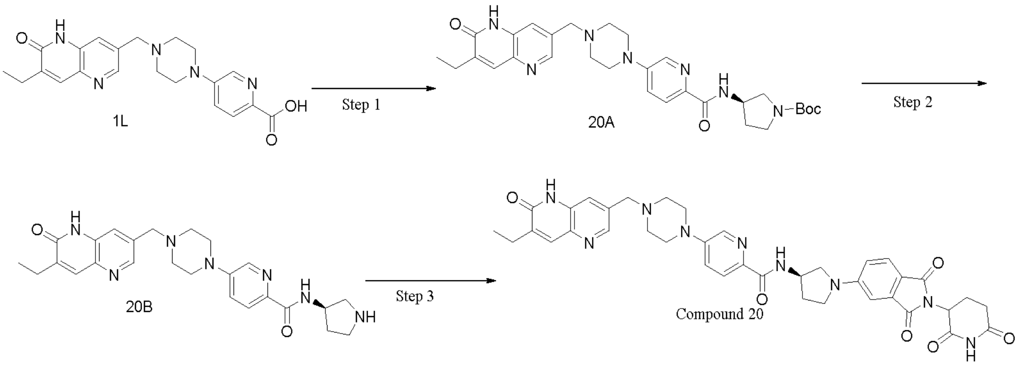


Step 1:

Compound **1L** (0.5 g, 1.27 mmol) and tert-butyl (R)-3-aminopyrrolidine-1-carboxylate (283 mg, 1.5 mmol) were dissolved in DMF (5 mL), benzotriazole-N,N,N',N'-tetramethyluronium hexafluorophosphate (570 mg, 1.5 mmol) and N,N-diisopropylethylamine (330 mg, 2.54 mmol) were added, and the mixture was stirred and reacted at room temperature for 1 h. After the reaction was completed, water (10 ml) and ethyl acetate (10 mL) were added. The organic phase was separated, and the aqueous phase was extracted with ethyl acetate (10 mL × 2). The organic phases were combined, dried over anhydrous sodium sulphate and concentrated, to afford crude product **20A** (700 mg, 98.0%).
LCMS m/z = 562.3 $[M+H]^+$

Step 2:

Compound **20A** (700 mg, 1.27 mmol) was dissolved in dichloromethane (6 mL) and trifluoroacetic acid (2 mL) was added. The mixture was reacted at room temperature for 30 min. After the reaction was completed, the reaction liquid was concentrated, adjusted to pH > 7 with triethylamine, and then purified by C18 column chromatography (acetonitrile:0.1% aqueous ammonia solution (v/v) = 50%) to afford product **20B** (400 mg, 68.2%).
LCMS m/z = 462.3 $[M+H]^+$

Step 3:

Compound **20B** (50 mg, 0.11 mmol) and 2-(2,6-dioxopiperidin-3-yl)-5-fluoroisoindoline-1,3-dione (36 mg, 0.13 mmol) were added to dimethyl sulphoxide (2 mL) and then N,N-diisopropylethylamine (28 mg, 0.22 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **20** (45 mg, yield 57.1%, retention time: about 7.0 min).

$^1$H NMR (400 MHz, DMSO-$d_6$) δ 11.81 (s, 1H), 11.03 (s, 1H), 8.61 (d, 1H), 8.40 (s, 1H), 8.26 (s, 1H), 7.86 (d, 1H), 7.75 (s, 1H), 7.67 - 7.60 (m, 2H), 7.41 (d, 1H), 6.93 (s, 1H), 6.84 (d, 1H), 5.12 - 4.99 (m, 1H), 4.79 - 4.54 (m, 1H), 3.76 - 3.69 (m, 1H), 3.65 (s, 2H), 3.62 - 3.57 (m, 1H), 3.54 - 3.41 (m, 2H), 3.39 - 3.33 (m, 4H), 2.94 - 2.81 (m, 1H), 2.66 - 2.52 (m, 8H), 2.33 - 2.24 (m, 1H), 2.24 - 2.15 (m, 1H), 2.06 - 1.96 (m, 1H), 1.22 - 1.13 (m, 3H).
MS M/Z (ESI): m/z = 718.2 $[M+H]^+$

Example 21:

N-((3S)-1-(2-(2,6-dioxopiperidin-3-yl)-1,3-dioxoisoindolin-5-yl)pyrrolidin-3-yl)-5-(4-((7-ethyl-6-oxo-5, 6-dihydro-1, 5-naphthyridin-3-yl)methyl)piperazin-1-yl)picolinamide (Compound 21)

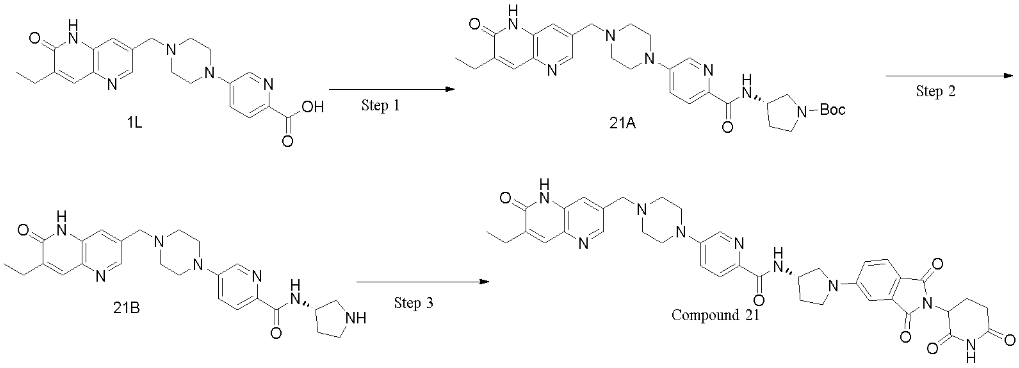


Step 1:

Compound **1L** (0.5 g, 1.27 mmol) and tert-butyl (S)-3-aminopyrrolidine-1-carboxylate (283 mg, 1.5 mmol) were dissolved in N,N-dimethylformamide (5 mL), benzotriazole-N,N,N',N'-tetramethyluronium hexafluorophosphate (570 mg, 1.5 mmol) and N,N-diisopropylethylamine (330 mg, 2.54 mmol) were added, and the mixture was stirred and reacted at room temperature for 1 hour. After the reaction was completed, water (10 ml) and ethyl acetate (10 mL) were added and the organic phase was separated. The aqueous phase was extracted with ethyl acetate (10 mL × 2). The organic phases were combined, dried over anhydrous sodium sulphate, filtered and concentrated to afford crude product **21A** (700 mg, 98.0 %), which was directly used in the next reaction without purification.
LCMS m/z = 562.3 $[M+H]^+$

Step 2:

Compound **21A** (700 mg, 1.27 mmol) was dissolved in dichloromethane (6 mL) and trifluoroacetic acid (2 mL) was added. The mixture was reacted at room temperature for 30 min. After the reaction was completed, the reaction liquid was concentrated, and the residue was adjusted to pH > 7 with triethylamine and then purified by C18 column chromatography (acetonitrile:0.1% aqueous ammonia solution = 1 : 20-1 : 1) to afford product **21B** (400 mg, 68.2%).
LCMS m/z = 462.3 $[M+H]^+$

Step 3:

Compound **21B** (50 mg, 0.11 mmol) and 2-(2,6-dioxopiperidin-3-yl)-5-fluoroisoindoline-1,3-dione (36 mg, 0.13 mmol) were added to dimethyl sulphoxide (2 mL) and then N,N-diisopropylethylamine (28 mg, 0.22 mmol) was added. The mixture was reacted at 100°C for 1 hour. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **21** (43 mg, yield 54.5%, retention time: about 7.0 min).

$^1H$ NMR (400 MHz, DMSO-$d_6$) δ 11.81 (s, 1H), 11.04 (s, 1H), 8.61 (d, 1H), 8.40 (s, 1H), 8.26 (s, 1H), 7.86 (d, 1H), 7.75 (s, 1H), 7.71 - 7.58 (m, 2H), 7.41 (d, 1H), 6.93 (s, 1H), 6.84 (d, 1H), 5.17 - 4.92 (m, 1H), 4.86 - 4.45 (m, 1H), 3.84 - 3.68 (m, 1H), 3.65 (s, 2H), 3.61 - 3.57 (m, 1H), 3.54 - 3.42 (m, 2H), 3.36 - 3.33 (m, 4H), 2.93 - 2.81 (m, 1H), 2.62 - 2.52 (m, 8H), 2.35 - 2.25 (m, 1H), 2.23 - 2.14 (m, 1H), 2.07 - 1.96 (m, 1H), 1.23 - 1.13 (m, 3H).
MS M/Z (ESI): m/z = 718.2 $[M+H]^+$

Example 22:

2-(2,6-dioxopiperidin-3-yl)-5-((3aR,6aS)-5-(5-(4-((7-ethyl-6-oxo-5,6-dihydro-1,5-naphthyridin-3-yl)methyl)piperazin-1-yl)picolinoyl)hexahydropyrrolo[3,4-c]pyrrol-2(1H)-yl)isoindoline-1,3-dione (Compound **22**)

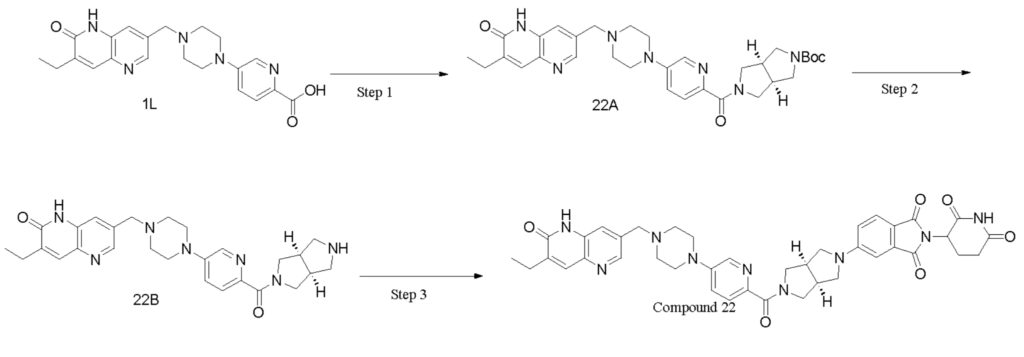


1L 22A 22B Compound 22

Step 1:

Compound **1L** (0.070 g, 0.18 mmol) and cis-2-BOC-hexahydropyrrolo[3,4-C]pyrrole (56.6 mg, 0.27 mmol) were dissolved in N,N-dimethylformamide (3 mL), benzotriazole-N,N,N',N'-tetramethyluronium hexafluorophosphate (82.1 mg, 0.22 mmol) and N,N-diisopropylethylamine (69.7 mg, 0.54 mmol) were added, and the mixture was stirred and reacted at room temperature for 1 hour. After the reaction was completed, water (10 ml) and ethyl acetate (10 mL) were added and the organic phase was separated. The aqueous phase was extracted with ethyl acetate (10 mL $\times$ 2) and the organic phases were combined, dried over anhydrous sodium sulphate and filtered. The filtrate was concentrated to afford crude product **22A** (100.0 mg, 95.1%), which was directly used in the next reaction without purification.
LCMS m/z = 588.3 $[M+H]^+$

Step 2:

Compound **22A** (100.0 mg, 0.17 mmol) was dissolved in dichloromethane (3 mL), trifluoroacetic acid (1 mL) was added, and the mixture was reacted at room temperature for 30 min. After the reaction was completed, the reaction liquid was directly concentrated to afford the trifluoroacetate of product **22B** (102.0 mg, 100%).
LCMS m/z = 488.3 $[M+1]^+$

Step 3:

Compound **22B** (102.0 mg, 0.17 mmol) and 2-(2,6-dioxopiperidin-3-yl)-5-fluoroisoindoline-1,3-dione (72.0 mg, 0.26 mmol) were added to dimethyl sulphoxide (2 mL) and then N,N-diisopropylethylamine (44.0 mg, 0.34 mmol) was added. The mixture was reacted at 100°C for 1 hour. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **22** (38.0 mg, yield 30.1%, retention time: about 6.9 min).

$^1$H NMR (400 MHz, DMSO-$d_6$) $\delta$ 11.81 (s, 1H), 11.04 (s, 1H), 8.40 (s, 1H), 8.26 (d, 1H), 7.86 (d, 1H), 7.75 (s, 1H), 7.69 - 7.72 (m, 3H), 7.37 (d, 1H), 6.93 (d, 1H), 6.84 (d, 1H), 5.07 - 5.02 (m, 1H), 4.09 - 4.06 (m, 1H), 3.84 - 3.62 (m, 6H), 3.53-3.48 (m, 1H), 3.41 - 3.39 (m, 1H), 3.15 - 3.09 (m, 2H), 2.92 - 2.83(m, 1H), 2.62 - 2.52 (m, 12H), 2.07 - 1.96 (m, 1H), 1.23 - 1.13 (m, 3H).
MS M/Z (ESI): m/z = 744.2 $[M+1]^+$

Example 23:

2-(2,6-dioxopiperidin-3-yl)-5-(6-(5-(4-((7-ethyl-6-oxo-5,6-dihydro-1,5-naphthyridin-3-yl)methyl)piperazin-1-yl)picolinoyl)-2,6-diazaspiro[3.3]heptan-2-yl)isoindoline-1,3-dione (Compound **23**)

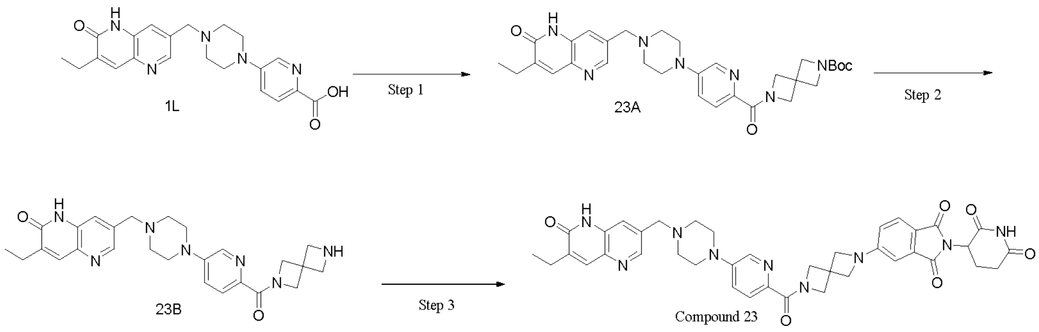


Step 1:

Compound **1L** (0.10 g, 0.25 mmol) and tert-butyl 2,6-diazaspiro[3.3]heptane-2-carboxylate oxalate (56.6 mg, 0.27 mmol) were dissolved in N,N-dimethylformamide (3 mL), benzotriazole-N,N,N',N'-tetramethyluronium hexafluorophosphate (82.1 mg, 0.22 mmol) and N,N-diisopropylethylamine (86.5 mg, 0.30 mmol) were added, and the mixture was stirred and reacted at room temperature for 1 hour. After the reaction was completed, water (10 ml) and ethyl acetate (10 mL) were added and the organic phase was separated. The aqueous phase was extracted with ethyl acetate (10 mL × 2) and the organic phases were combined, dried over anhydrous sodium sulphate and concentrated to afford crude product **23A** (130.0 mg, 90.1%), which was directly used in the next reaction without purification.
LCMS m/z = 574.3 $[M+H]^+$

Step 2:

Compound **23A** (130.0 mg, 0.22 mmol) was dissolved in dichloromethane (3 mL), trifluoroacetic acid (1 mL) was added, and the mixture was reacted at room temperature for 30 min. After the reaction was completed, the reaction liquid was directly concentrated to afford the trifluoroacetate of product **23B** (126.0 mg, 95%).
LCMS m/z = 474.3 $[M+H]^+$

Step 3:

Compound **23B** (126.0 mg, 0.21 mmol) and 2-(2,6-dioxopiperidin-3-yl)-5-fluoroisoindoline-1,3-dione (75.0 mg, 0.27 mmol) were added to dimethyl sulphoxide (2 mL) and then N,N-diisopropylethylamine (81.0 mg, 0.63 mmol) was added. The mixture was reacted at 100°C for 1 hour. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **23** (42.0 mg, yield 27.4%, retention time: about 6.7 min).

$^1$H NMR (400 MHz, DMSO-$d_6$) δ 11.81 (s, 1H), 11.04 (s, 1H), 8.40 (s, 1H), 8.27 (d, 1H), 7.80 (d, 1H), 7.75 (s, 1H), 7.65 (d, 1H), 7.62 (s, 1H), 7.38 (dd, 1H), 6.79 (d, 1H), 6.67 (dd, 1H), 5.08 - 5.03 (m, 1H), 4.77 (s, 2H), 4.26(s, 2H), 4.22(s, 4H), 3.65 (s, 2H), 3.36 - 3.34(m, 4H), 2.92 - 2.83(m, 1H), 2.62 - 2.52 (m, 8H), 2.02 - 1.99 (m, 1H), 1.18 - 1.16 (m, 3H).
MS M/Z (ESI): m/z = 730.3 $[M+H]^+$

Example 24:

N-(2-(2-(2,6-dioxopiperidin-3-yl)-1,3-dioxoisoindolin-5-yl)-2-azaspiro[3.3]heptan-6-yl)-5-(4-((7-ethyl-6-oxo-5,6-dihydro-1,5-naphthyridin-3-yl)methyl)piperazin-1-yl)picolinamide (Compound 24)

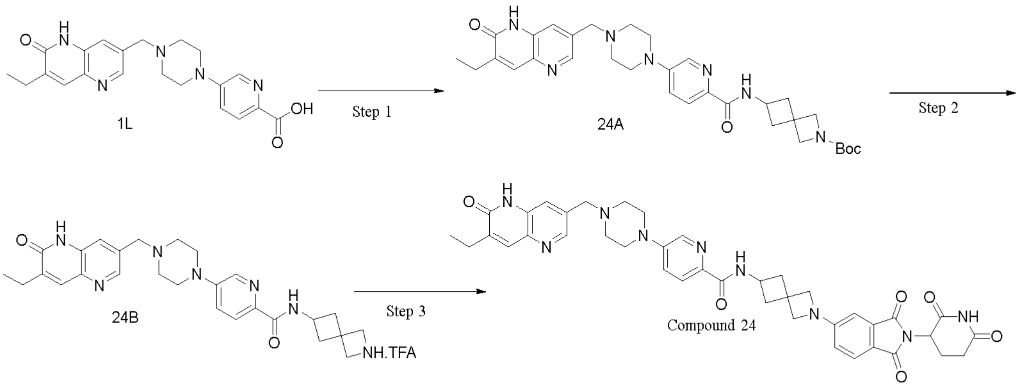


Step 1:

Compound **1L** (0.2 g, 0.51 mmol) and tert-butyl 6-amino-2-azaspiro[3.3]heptane-2-carboxylate (108 mg, 0.51 mmol) were dissolved in DMF (5 mL), benzotriazole-N,N,N',N'-tetramethyluronium hexafluorophosphate (232 mg, 0.61 mmol) and N,N-diisopropylethylamine (99 mg, 0.77 mmol) were added, and the mixture was stirred and reacted at room temperature for 1 h. After the reaction was completed, water (10 ml) and ethyl acetate (10 mL) were added. The organic phase was separated, and the aqueous phase was extracted with ethyl acetate (10 mL $\times$ 2). The organic phases were combined, dried over anhydrous sodium sulphate and concentrated, to afford crude product **24A** (300 mg, 100.0%).
LCMS m/z = 588.3 $[M+H]^+$

Step 2:

Crude compound **24A** (300 mg, 0.51 mmol) was dissolved in dichloromethane (6 mL), trifluoroacetic acid (2 mL) was added, and the mixture was reacted at room temperature for 30 min. After the reaction was completed, the reaction liquid was concentrated to afford crude product **24B** (300 mg, 100.0%).
LCMS m/z = 488.2 $[M+H]^+$

Step 3:

Crude compound **24B** (300 mg, 0.51 mmol) and 2-(2,6-dioxopiperidin-3-yl)-5-fluoroisoindoline-1,3-dione (140 mg, 0.51 mmol) were added to dimethyl sulphoxide (3 mL) and then N,N-diisopropylethylamine (133 mg, 1.02 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **24** (46 mg, yield 12.1%, retention time: about 7.5 min).
$^1$H NMR (400 MHz, DMSO-$d_6$) $\delta$ 11.83 (s, 1H), 11.04 (s, 1H), 8.63 (d, 1H), 8.41 (s, 1H), 8.28 (s, 1H), 7.83 (d, 1H), 7.75 (s, 1H), 7.70 - 7.59 (m, 2H), 7.40 (d, 1H), 6.77 (s, 1H), 6.64 (d, 1H), 5.11 - 4.98 (m, 1H), 4.45 - 4.35 (m, 1H), 4.12 (s, 2H), 3.99 (s, 2H), 3.66 (s, 2H), 3.39 - 3.32 (m, 4H), 2.97 - 2.80 (m, 1H), 2.63 - 2.53 (m, 10H), 2.48 - 2.42 (m, 2H), 2.10 - 1.96 (m, 1H), 1.21 - 1.16 (m, 3H).
MS M/Z (ESI): m/z = 744.3 $[M+H]^+$

Example 25:

N-((1-(2-(2,6-dioxopiperidin-3-yl)-1,3-dioxoisoindolin-5-yl)piperidin-4-yl)methyl)-5-(4-((7-ethyl-6-oxo-5,6-dihydro-1,5-naphthyridin-3-yl)methyl)piperazin-1-yl)picolinamide (Compound **25**)

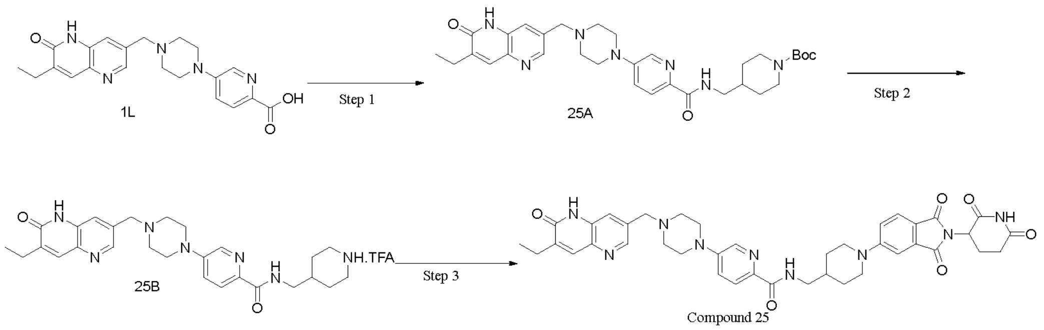

1L 25A 25B Compound 25

Step 1:

Compound **1L** (0.1 g, 0.25 mmol) and tert-butyl 4-(aminomethyl)piperidine-1-carboxylate (53 mg, 0.25 mmol) were dissolved in DMF(5 mL), benzotriazole-N,N,N',N'-tetramethyluronium hexafluorophosphate (114 mg, 0.3 mmol) and N,N-diisopropylethylamine (65 mg, 0.5 mmol) were added, and the mixture was stirred and reacted at room temperature for 1 h. After the reaction was completed, water (10 ml) and ethyl acetate (10 mL) were added. The organic phase was separated, and the aqueous phase was extracted with ethyl acetate (10 mL $\times$ 2). The organic phases were combined, dried over anhydrous sodium sulphate and concentrated, to afford crude product **25A** (150 mg, 100%).
LCMS m/z = 590.3 $[M+H]^+$

Step 2:

Crude compound **25A** (150 mg, 0.25 mmol) was dissolved in dichloromethane (3 mL), trifluoroacetic acid (1 mL) was added, and the mixture was reacted at room temperature for 30 min. After the reaction was completed, the reaction liquid was concentrated to afford crude product **25B** (160 mg, 100%).
LCMS m/z = 490.2 $[M+H]^+$

Step 3:

Crude compound **25B** (160 mg, 0.25 mmol) and 2-(2,6-dioxopiperidin-3-yl)-5-fluoroisoindoline-1,3-dione (70 mg, 0.25 mmol) were added to dimethyl sulphoxide (3 mL) and then N,N-diisopropylethylamine (65 mg, 0.5 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **25** (40 mg, yield 21.5%, retention time: about 7.2 min)

$^1$H NMR (400 MHz, DMSO-$d_6$) $\delta$ 11.83 (s, 1H), 11.05 (s, 1H), 8.48 (s, 1H), 8.41 (s, 1H), 8.28 (s, 1H), 7.84 (d, 1H), 7.75 (s, 1H), 7.71 - 7.56 (m, 2H), 7.40 (d, 1H), 7.30 (s, 1H), 7.22 (d, 1H), 5.12 - 4.97 (m, 1H), 4.04 (d, 2H), 3.66 (s, 2H), 3.40 - 3.32 (m, 4H), 3.31 - 3.30 (m, 2H), 3.23 - 3.16 (m, 2H), 3.00 - 2.81 (m, 3H), 2.63 - 2.52 (m, 8H), 2.06 - 1.97 (m, 1H), 1.94 - 1.80 (m, 1H), 1.80 - 1.67 (m, 2H), 1.20 - 1.16 (m, 3H).
MS M/Z (ESI): m/z = 746.3 $[M+H]^+$

Example 26:

N-(1-(1-(2-(2,6-dioxopiperidin-3-yl)-6-fluoro-1 ,3-dioxoisoindolin-5-yl)azetidin-3-yl)piperidin-4-yl)-5-(4-((7-ethyl-6-oxo-5,6-dihydro-1,5-naphthyridin-3-yl)methyl)piperazin-1-yl)picolinamide (Compound **26**)

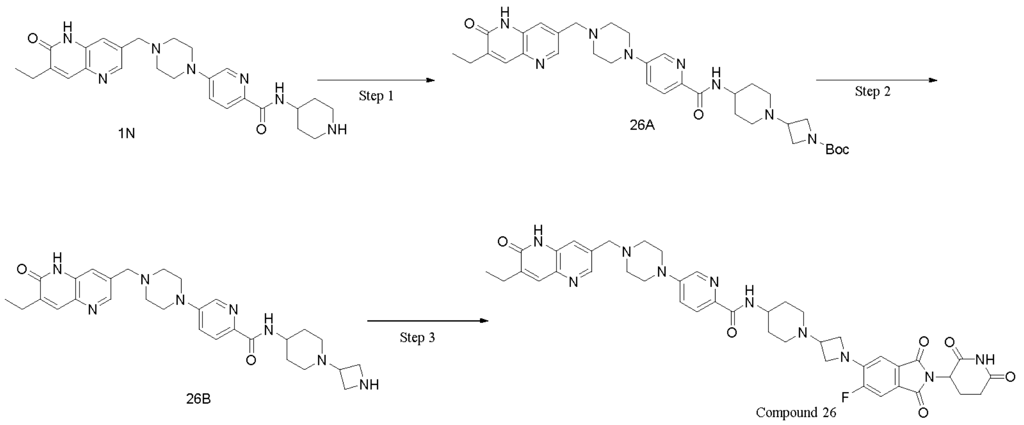


Step 1:

**1N** (0.3 g, 0.76 mmol) and tert-butyl 3-aminoazetidine-1-carboxylate (261 mg, 1.52 mmol) were dissolved in ethanol (5 mL) and then dichloroethane (5 mL) and glacial acetic acid (six drops) were added. The mixture was stirred and reacted at 80°C for 24 h and sodium cyanoborohydride (141 mg, 2.28 mmol) was added. The resulting mixture was reacted for 1 hour. After concentration, the residue was purified by C18 column chromatography (acetonitrile:0.1% trifluoroacetic acid solution (v/v) = 50%) to afford product **26A** (300 mg, 62.1%).
LCMS m/z = 631.4 $[M+H]^+$

Step 2:

Crude compound **26A** (150 mg, 0.253 mmol) was dissolved in dichloromethane (3 mL), trifluoroacetic acid (1 mL) was added, and the mixture was reacted at room temperature for 30 min. After the reaction was completed, the reaction liquid was concentrated to afford crude product **26B** (160 mg, 100.0%).
LCMS m/z = 531.3 $[M+H]^+$

Step 3:

Crude compound **26B** (160 mg, 0.23 mol) and 2-(2,6-dioxopiperidin-3-yl)-5-fluoroisoindoline-1,3-dione (70 mg, 0.25 mmol) were added to dimethyl sulphoxide (3 mL) and then N,N-diisopropylethylamine (65 mg, 0.5 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **26** (40 mg, yield 21.6%, retention time: about 6.8 min).

$^1$H NMR (400 MHz, DMSO-$d_6$) $\delta$ 11.83 (s, 1H), 11.07 (s, 1H), 8.40 (d, 1H), 8.27 (d, 1H), 8.20 (d, 1H), 7.83 (d, 1H), 7.75 (s, 1H), 7.66 - 7.55 (m, 2H), 7.42 - 7.37 (m, 1H), 6.92 (d, 1H), 5.24 - 4.95 (m, 1H), 4.32 - 4.18 (m, 2H), 4.06 - 3.92 (m, 2H), 3.88 - 3.74 (m, 1H), 3.65 (s, 2H), 3.37 - 3.31 (m, 4H), 2.92 - 2.75 (m, 3H), 2.58 - 2.53 (m, 6H), 2.08 - 1.90 (m, 3H), 1.84 - 1.74 (m, 2H), 1.69 - 1.57 (m, 2H), 1.20 - 1.16 (m, 3H).
MS M/Z (ESI): m/z = 805.4 $[M+H]^+$

Example 27:

N-((1s,4s)-4-((2-(-2,6-dioxopiperidin-3-yl)-1,3-dioxoisoindolin-5-yl)amino)cyclohexyl)-5-(4-((7-ethyl-6-oxo-5,6-dihydro-1,5-naphthyridin-3-yl)methyl)piperazin-1-yl)picolinamide (Compound **27**)

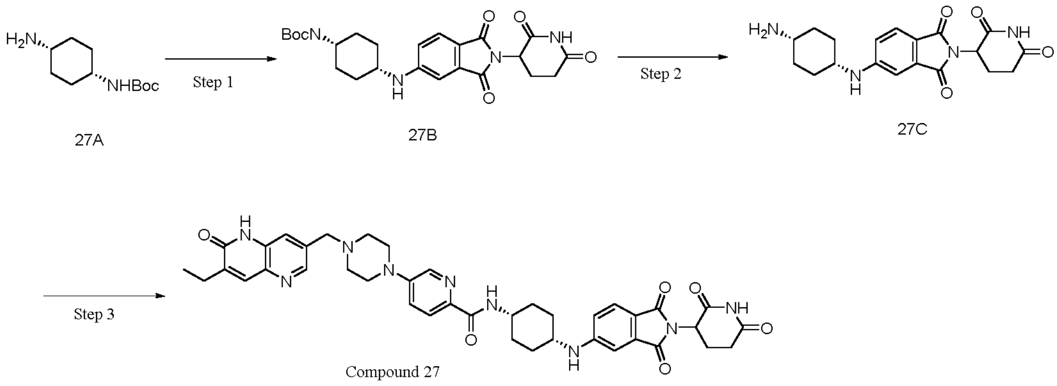


27A 27B 27C

Compound 27

Step 1:

**27A** (500 mg, 2.33 mmol), 2-(2,6-dioxopiperidin-3-yl)-5-fluoroisoindoline-1,3-dione (640 mg, 2.33 mmol) and N,N-diisopropylethylamine (900 mg, 6.99 mmol) were added to dimethyl sulphoxide (5 ml), and the mixture was reacted at 100°C for 1 h. The reaction liquid was cooled to room temperature and water (30 ml) was added. The mixture was extracted with ethyl acetate (15 ml × 2) and the organic phase was washed with saturated sodium chloride solution, dried over anhydrous sodium sulphate and filtered. The filtrate was concentrated under reduced pressure and the crude product was separated and purified by silica gel column chromatography (20 g silica gel column, eluent : EA/PE = 0-60%), to afford title compound **27B** (200 mg, 18%).

LC-MS (ESI): m/z = 470.2 $[M+H]^+$.

Step 2:

**27B** (200 mg, 0.43 mmol) was added to dichloromethane (9 ml) and then trifluoroacetic acid (3 ml) was added. The mixture was reacted at room temperature for 2 h. The reaction liquid was concentrated under reduced pressure to dryness to afford crude 27C (250 mg).

Step 3:

Intermediate **1L** (100 mg, 0.25 mmol), **27C** (250 mg, 0.43 mmol), 2-(7-azobenzotriazole)-N,N,N',N'-tetramethyluronium hexafluorophosphate (210 mg, 0.55 mmol) and N,N-diisopropylethylamine (194 mg, 1.5 mmol) were sequentially added to N,N-dimethylformamide (3 ml), and the mixture was reacted at room temperature for 5 h. The reaction liquid was directly separated and purified by HPLC. After purification, the product was concentrated under reduced pressure to remove acetonitrile and then extracted with (dichloromethane : methanol = 10 : 1), and the organic phase was concentrated under reduced pressure to afford title compound **27** (60 mg, 30%).

Preparative HPLC separation methods: 1. Instruments: waters 2767 (preparative liquid phase chromatographic instrument); chromatographic column: SunFire@ Prep C18 (19 mm × 250 mm) 2. The sample was dissolved in DMF and filtered with a 0.45 μm filter to prepare a sample solution. 3. Preparative chromatography conditions: a. composition of mobile phases A and B: mobile phase A: acetonitrile; mobile phase B: water (containing 0.1% ammonium acetate); b. gradient elution, mobile phase A: 5%-40%; c. flow rate: 12 mL/min; d. elution time: 20 min.

$^1H$ NMR (400 MHz, DMSO-$d_6$) δ 11.84 (s, 1H), 11.04 (s, 1H), 8.41 (d, 1H), 8.28 (d, 1H), 8.01 (d, 1H), 7.85 (d, 1H), 7.75 (s, 1H), 7.63 (s, 1H), 7.57 (d, 1H), 7.41 (dd, 1H), 7.03 (s, 1H), 6.98 - 6.89 (m, 2H), 5.03 (dd, 1H), 3.96 (s, 1H), 3.72-3.64 (m, 3H), 3.37-3.33 (m, 4H), 2.93 - 2.81 (m, 1H), 2.63 - 2.51 (m, 8H), 2.05 - 1.95 (m, 1H), 1.80-1.70 (m, 6H), 1.68 - 1.57 (m, 2H), 1.18 (t, 3H).

LC-MS (ESI): m/z = 746.3 $[M+H]^+$.

Example 28:

N-(7-(2-(2,6-dioxopiperidin-3-yl)-1,3-dioxoisoindolin-5-yl)-7-azaspiro[3.5]nonan-2-yl)-5-(4-((7-ethyl-6-oxo-5,6-dihydro-1,5-naphthyridin-3-yl)methyl)piperazin-1-yl)picolinamide (Compound **28**)

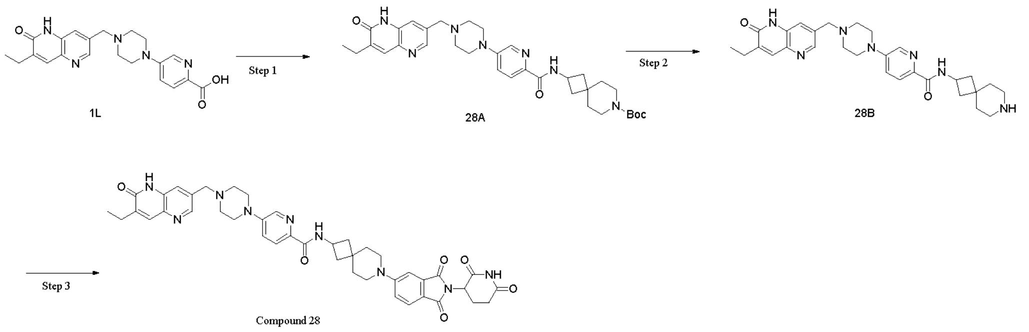


1L 28A 28B Compound 28

Step 1:

Compound **1L** (100 mg, 0.25 mmol) and tert-butyl 2-amino-7-azaspiro[3.5]nonane-7-carboxylate (73.2 mg, 0.3 mmol) were dissolved in DMF (5 mL), benzotriazole-N,N,N',N'-tetramethyluronium hexafluorophosphate (114 mg, 0.3 mmol) and N,N-diisopropylethylamine (64.5 mg, 0.5 mmol) were added, and the mixture was stirred and reacted at room temperature for 1 h. After the reaction was completed, water (10 ml) and ethyl acetate (10 mL) were added. The organic phase was separated, and the aqueous phase was extracted with ethyl acetate (10 mL $\times$ 2). The organic phases were combined, dried over anhydrous sodium sulphate and concentrated, to afford crude product **28A** (156 mg, 100.0%).
LCMS m/z = 616.3 $[M+1]^+$

Step 2:

Compound **28A** (156 mg, 0.25 mmol) was dissolved in dichloromethane (6 mL) and trifluoroacetic acid (2 mL) was added. The mixture was reacted at room temperature for 30 min. After the reaction was completed, the reaction liquid was concentrated, adjusted to pH > 7 with triethylamine, and then purified by C18 column chromatography (acetonitrile:0.1% aqueous ammonia solution (v/v) = 50%) to afford product **28B** (100 mg, 77.2%).
LCMS m/z = 516.3 $[M+1]^+$

Step 3:

Compound **28B** (100 mg, 0.19 mmol) and 2-(2,6-dioxopiperidin-3-yl)-5-fluoroisoindoline-1,3-dione (63 mg, 0.22 mmol) were added to dimethyl sulphoxide (2 mL) and then N,N-diisopropylethylamine (49 mg, 0.38 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **28** (60 mg, yield 41.0%, retention time: about 7.1 min).

$^1$H NMR (400 MHz, DMSO-$d_6$) $\delta$ 11.83 (s, 1H), 11.06 (s, 1H), 8.59-8.57 (d, 1H), 8.41 (s, 1H), 8.26 (s, 1H), 7.84-7.82 (d, 1H), 7.75 (s, 1H), 7.65 - 7.63 (m, 2H), 7.41-7.38 (d, 1H), 7.32 (s, 1H), 7.26-7.23 (d, 1H), 5.08 - 5.04 (m, 1H), 4.52 - 4.42 (m, 1H), 3.66 (s, 2H), 3.48-3.47 (m, 2H), 3.39 - 3.31 (m, 6H), 2.93 - 2.84 (m, 1H), 2.60 - 2.54 (m, 8H), 2.23 - 2.21 (t, 2H), 2.03 - 1.94 (m, 3H), 1.69 - 1.64 (m, 4H),1.20 - 1.17 (t, 3H).
MS M/Z (ESI): m/z = 772.4 $[M+1]^+$

Example 29

3-((1-((1-(2-(2,6-dioxopiperidin-3-yl)-1,3-dioxoisoindolin-5-yl)piperidin-4-yl)methyl)azetidin-3-yl)amino)-5-(4-((7-ethyl-6-oxo-5, 6-dihydro-1, 5-naphthyridin-3-yl)methyl)piperazin-1-yl)-N-methylpicolinamide (Compound **29**)

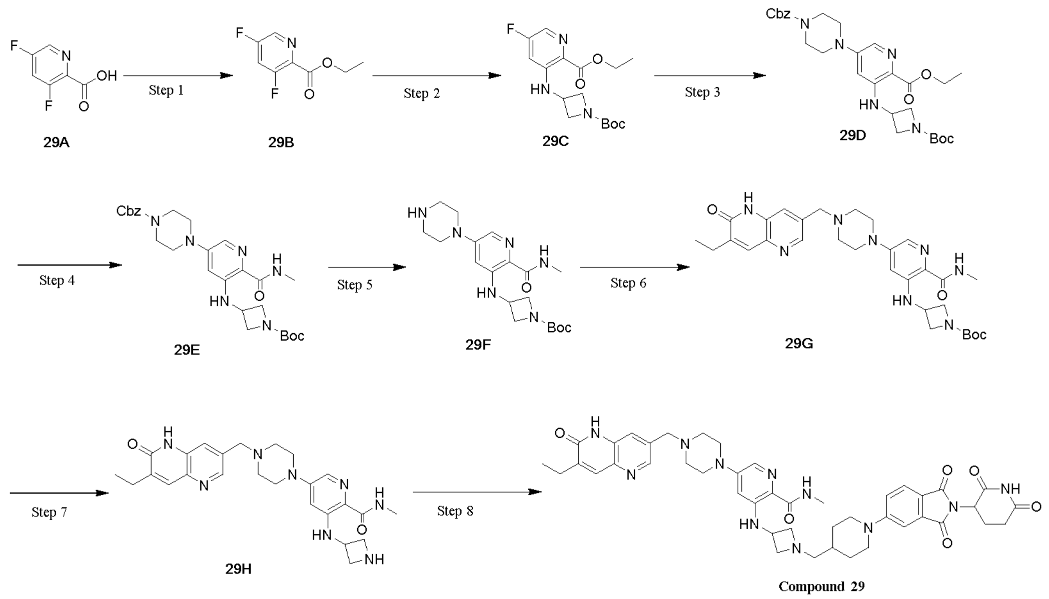


Step 1:

**29A** (9 g, 56.57 mmol) was added to ethanol (100 mL) and the mixture was stirred. Sulphuric acid (25 mL) was added and the resulting mixture was reacted at 70°C for 2 hours. Then the system was concentrated, diluted with ethyl acetate (200 mL) and washed with saturated brine, and the organic phase was concentrated and dried to afford title compound **29B** (10 g, 94.5%)
LC-MS(ESI): m/z = 188.1[M+H]$^+$

Step 2:

**29B** (10 g, 53.43 mmol) and 1-tertbutoxycarbonyl-3-aminocyclobutylamine (11 g, 64.12 mmol) were dissolved in DMF/DMSO (50 mL/50 mL), potassium carbonate (2.2 g, 160.3 mmol) was added, and the mixture was reacted at 100°C for 24 hours, then diluted with water (300 mL) and extracted with ethyl acetate (50 mL × 3). The organic phases were combined, washed with saturated brine, and dried over anhydrous sodium sulphate. The organic phase was concentrated and separated by silica gel chromatographic column (PE : EA = 1 : 0-1 : 1) to afford title compound **29C** (4.6 g, 25.4%).
LC-MS(ESI): m/z = 340.1[M+H]$^+$

Step 3:

**29C** (4.6 g, 13.55 mmol) and benzyl piperazine-1-carboxylate (3.6 g, 16.27 mmol) were dissolved in DMSO (50 mL), sodium carbonate (4.3 g, 40.66 mmol) was added, and the mixture was reacted overnight at 100°C, then quenched with water (200 mL), and extracted with ethyl acetate (50 mL × 3). The organic phases were combined, dried over anhydrous sodium sulphate, filtered and spun to dryness. The residue was separated by silica gel chromatographic column (PE : EA = 1 : 0-1 : 1), to afford title compound **29D** (4 g, 54.7%).

LC-MS (ESI): m/z = 540.2 [M+H]$^+$.

Step 4:

**29D** (2 g, 3.7 mmol) was dissolved in methanol (10 mL), methylamine aqueous solution (10 mL, 40%) was added, and the mixture was reacted at room temperature for 4 hours. The suspension was concentrated, saturated ammonium chloride solution was added, and the mixture was extracted with dichloromethane. The organic phases were combined, dried over anhydrous sodium sulphate, filtered and spun to dryness, to afford title compound **29E** (1.6 mg, 82.3%).

LC-MS (ESI): m/z = 525.2 [M+H]$^+$.

Step 5:

**29E** (500 mg, 0.95 mmol) was dissolved in methanol (5 mL), Pd/C (200 mg) was added, and the mixture was reacted in hydrogen environment for two hours, filtered and spun to dryness, to afford title compound **29F** (300 mg, 80.6%).
LC-MS (ESI): m/z = 391.2 $[M+H]^+$.

Step 6:

**1G** (142 mg, 0.64 mmol) and **29F** (300 mg, 0.77 mmol) were dissolved in anhydrous acetonitrile (10 mL), potassium iodide (11 mg, 0.06 mmol) and DIPEA (411 mg, 3.18 mmol) were added, and the mixture was subjected to nitrogen replacement and reacted at 80°C for 2 hours. Upon complete depletion of raw materials monitored by LCMS, the system was concentrated, saturated sodium bicarbonate solution (20 mL) was added, and the mixture was extracted with DCM (10 mL × 3). The organic phases were combined, dried over anhydrous sodium sulphate, concentrated, and passed through column (DCM : MeOH = 1 : 0-10 : 1) to afford title compound **29G** (180 mg, 50.1%).
LC-MS (ESI): m/z = 577.3 $[M+H]^+$.

Step 7:

**29G** (180 mg, 0.31 mmol) was added to dichloromethane (3 mL) and then trifluoroacetic acid (1 mL) was added dropwise. The mixture was stirred at room temperature for 2 hours and spun to dryness. The residue was purified by reverse phase chromatographic column (water (5%o $NH_3 \cdot H_2O$): acetonitrile), to afford title compound **29H** (140 mg, 95%).
LC-MS (ESI): m/z = 477.2 $[M+H]^+$.

Step 8:

**29H** (20 mg, 0.04 mmol) and **3C** (24 mg, 0.06 mmol) were added to methanol, a drop of acetic acid was added dropwise, and the mixture was refluxed overnight. Then sodium cyanoborohydride (34 mg, 0.21 mmol) was added. After the reaction was completed, the reaction liquid was concentrated and subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.5% aqueous ammonia); b. gradient elution, mobile phase A: 5%-50%; c. flow rate: 12 mL/min; d. elution time: 20 min. The preparative liquid was concentrated and lyophilized, to afford compound **29** (12 mg, 34.7%).
$^1H$ NMR (400 MHz, DMSO-$d_6$) δ 12.09 (s, 1H), 9.94 (s, 1H), 8.93 (dd, 1H), 8.51 (s, 1H), 8.42 - 8.32 (m, 1H), 7.85 - 7.63 (m, 4H), 7.38 - 7.31 (m, 1H), 7.14 - 6.95 (m, 1H), 6.35 - 6.18 (m, 1H), 5.13 - 5.00 (m, 1H), 4.71 - 4.34 (m, 4H), 4.19 - 3.98 (m, 5H), 3.33 - 3.09 (m, 6H), 3.04 - 2.82 (m, 5H), 2.76 (t, 3H), 2.65 - 2.51 (m, 6H), 2.09 - 1.83 (m, 2H), 1.82 - 1.67 (m, 2H), 1.35 - 1.14 (m, 5H).
LC-MS (ESI): m/z = 830.4 $[M+H]^+$.

Example 30

3-((1-(2-(2,6-dioxopiperidin-3-yl)-1,3-dioxoisoindolin-5-yl)azetidin-3-yl)amino)-5-(4-((7-ethyl-6-oxo-5,6-dihydro-1,5-naphthyridin-3-yl)methyl)piperazin-1-yl)-N-methylpicolinamide (Compound **30**)

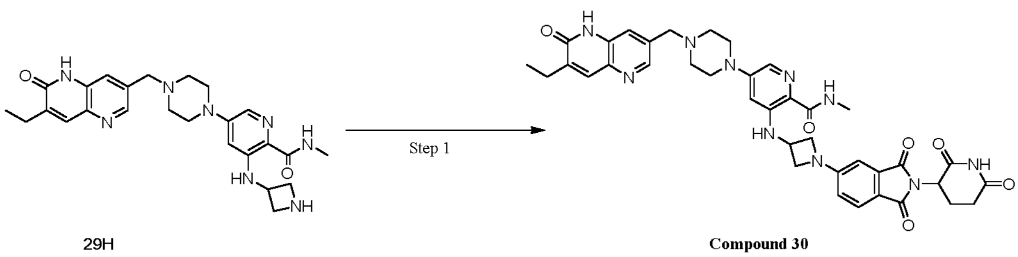


29H

Compound 30

Step 1:

**29H** (100 mg, 0.21 mmol) and 2-(2,6-dioxopiperidin-3-yl)-5-fluoroisoindoline-1,3-dione (58 mg, 0.21 mmol) were dissolved in dimethyl sulphoxide (3 mL) and then DIPEA (81 mg, 0.63 mmol) was added. The mixture was reacted at 100°C for 2 hours and directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.5% aqueous ammonia); b. gradient elution, mobile phase A: 5%-50%; c. flow rate: 12 mL/min; d. elution time: 20 min. The preparative liquid was concentrated and lyophilized, to afford compound **30** (30 mg, 19.5%).
$^1H$ NMR (400 MHz, DMSO-$d_6$) δ 11.83 (s, 1H), 11.04 (s, 1H), 8.88 (d, 1H), 8.41 (d, 1H), 8.34 - 8.27 (m, 1H), 7.75 (s, 1H), 7.70 - 7.60 (m, 3H), 6.86 (d, 1H), 6.72 (dd, 1H), 6.30 (d, 1H), 5.06 (dd, 1H), 4.64 - 4.53 (m, 1H), 4.53 - 4.45 (m, 2H), 3.89 - 3.77 (m, 2H), 3.66 (s, 2H), 3.37 - 3.32 (m, 4H), 2.94 - 2.81 (m, 1H), 2.73 (d, 3H), 2.62 - 2.52 (m, 8H), 2.07 - 1.97 (m, 1H), 1.19 (t, 3H).
LC-MS(ESI): m/z = 733.3[M+H]$^+$

Example 31

N-(1-(2-(2,6-dioxopiperidin-3-yl)-1-oxoisoindolin-5-yl)piperidin-4-yl)-5-(4-((7-ethyl-6-oxo-5,6-dihydro-1,5-naphthyridin-3-yl)methyl)piperazin-1-yl)picolinamide (Compound **31**)

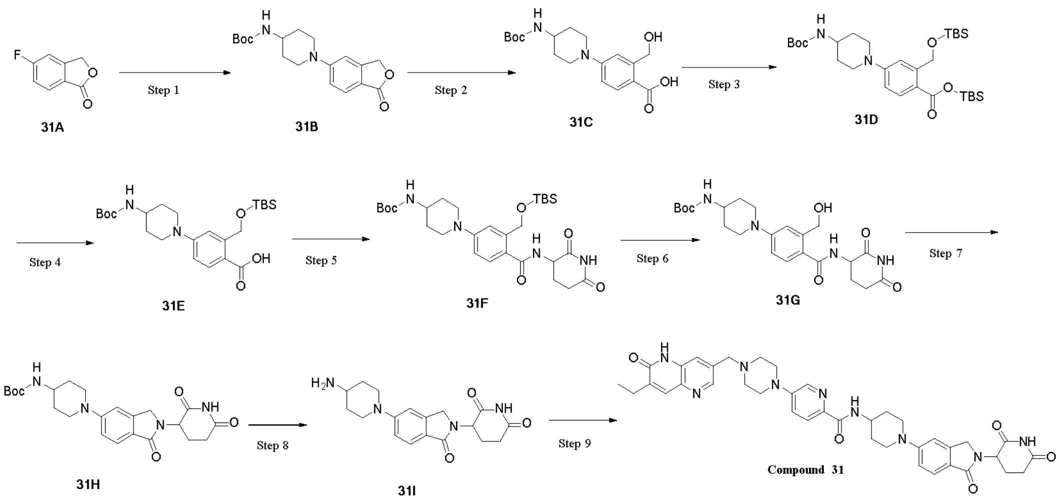


31A 31B 31C 31D 31E 31F 31G 31H 31I Compound 31

Step 1:

**31A** (2.8 g, 18.4 mmol) and tert-butyl azetidin-3-ylcarbamate (3.8 g, 22.1 mmol) were dissolved in dimethyl sulphoxide (40 mL), DIPEA(7.1 g, 55.2 mmol) was added, and the mixture was reacted at 100°C for 2 hours. After the reaction was completed, the reaction liquid was cooled and water (40 mL) was added to precipitate out a product, which was filtered, washed with water to remove dimethyl sulphoxide and dried to afford title compound **31B** (6.6 g, crude, containing DMSO).
LC-MS(ESI): m/z = 333.1[M+H]$^+$

Step 2:

**31B** (6.6 g, containing DMSO) was added to methanol (50 mL) and then aqueous sodium hydroxide solution (2.2 g, dissolved in 50 mL of water) was added. The mixture was reacted at 50°C for 2 hours. Upon completion of reaction as monitored by LCMS, the reaction liquid was concentrated to remove methanol and adjusted to pH 5 with 1 M aqueous hydrochloric acid solution. The product was filtered and dried to afford title compound **31C** (5.3 g, yield over two steps: 82.2%).
LC-MS(ESI): m/z = 351.1[M+H]$^+$

Step 3:

**31C** (5.3 g, 15.1 mmol) was added to dichloromethane (100 mL) and then tert-butyl dimethylsilyl chloride (4.6 g, 30.2 mmol) and imidazole (4.2 g, 61.0 mmol) were added. The mixture was reacted at room temperature for 16 hours and water (200 mL) was added. The resulting mixture was extracted with dichloromethane (50 mL × 3) and the organic phases were combined, dried over anhydrous sodium sulphate and concentrated. The residue was separated by silica gel chromatographic column (PE-PE : EA = 3 : 1) to afford title compound **31D** (7 g, 80.0%).
LC-MS(ESI): m/z = 579.3 $[M+H]^+$

Step 4:

**31D** (7 g, 12.1 mmol) was dissolved in ethanol (70 mL), potassium carbonate (5.0 g, 36.3 mmol) was added, and the mixture was reacted overnight at room temperature. After the reaction was completed, the reaction liquid was filtered to remove the solid and the filtrate was spun to dryness to afford title compound **31E** (5.6 g, 99.7%).
LC-MS(ESI): m/z = 465.2$[M+H]^+$

Step 5:

**31E** (2 g, 4.3 mmol), 3-aminopiperidine-2,6-dione hydrochloride (848 mg, 5.16 mmol), HATU (2.7 g, 8.6 mmol) and triethylamine (1.3 g, 13.0 mmol) were added to dichloromethane (20 mL), and the mixture was stirred overnight at room temperature, then diluted with water (100 mL) and extracted with dichloromethane (20 mL × 3). The organic phases were combined, dried over anhydrous sodium sulphate and concentrated. The residue was separated by silica gel chromatographic column (EA : PE = 0-100%) to afford title compound **31F** (1.48 g, 59.8%).
LC-MS(ESI): m/z = 575.3 $[M+H]^+$

Step 6:

**31F** (1.48 g, 2.57 mmol) was dissolved in dichloromethane (20 mL), tetrabutylammonium fluoride (2.57 mL, 1 mol/L of tetrahydrofuran solution) was added, and the mixture was stirred at room temperature for 2 hours, diluted with water (50 mL) and then extracted with dichloromethane (15 mL × 3). The organic phases were combined, dried over anhydrous sodium sulphate and spun to dryness to obtain title compound **31G** (680 mg, 57.3%).
LC-MS(ESI): m/z = 461.2 $[M+H]^+$

Step 7:

**31G** (680 mg, 1.48 mmol), p-methylbenzenesulphonyl chloride (422 mg, 2.21 mmol) and triethylamine (1.2 g, 11.8 mmol) were added to dichloromethane (10 mL), and the mixture was stirred at 40°C for 16 h, then diluted with water (50 mL) and extracted with dichloromethane (15 mL × 3). The organic phase was dried over anhydrous sodium sulphate and concentrated, and the residue was separated with silica gel chromatographic column to afford title compound **31H** (400 mg, 61.2%).
LC-MS(ESI): m/z = 443.2 $[M+H]^+$

Step 8:

**31H** (200 mg, 0.45 mmol) was added to dichloromethane (6 mL), trifluoroacetic acid (2 mL) was added dropwise, and the mixture was stirred at room temperature for 2 hours and directly spun to dryness to afford title compound **31I** (200 mg, containing TFA).
LC-MS (ESI): m/z = 343.1 $[M+H]^+$.

Step 9:

**31I** (200 mg, 0.45 mmol), **1L** (180 mg, 0.45 mmol), HATU (345 mg, 0.9 mmol) and triethylamine (230 mg, 2.27 mmol) were added to dichloromethane (5 mL), and the mixture was stirred overnight at room temperature and filtered. The filter cake was washed with dichloromethane and the organic phase was concentrated and then separated and purified by C18 reverse phase column (5‰ aqueous ammonia : acetonitrile) to afford compound **31** (40 mg, 12.2%).
$^1H$ NMR (400 MHz, DMSO-$d_6$) δ 8.40 (d, 1H), 8.30 - 8.23 (m, 2H), 7.85 (d, 1H), 7.75 (s, 1H), 7.62 (d, 1H), 7.51 (d, 1H), 7.40 (dd, 1H), 7.07 (dd, 1H), 7.03 - 6.99 (m, 1H), 5.30 (s, 2H), 4.56 (dd, 1H), 4.08 - 3.95 (m, 1H), 3.92 - 3.81 (m, 2H), 3.65 (s, 2H), 3.37 - 3.32 (m, 5H), 2.97 (t, 2H), 2.69 - 2.53 (m, 8H), 2.09 - 1.91 (m, 2H), 1.90 - 1.81 (m, 2H), 1.78

- 1.64 (m, 2H), 1.18 (t, 3H).

LC-MS (ESI): m/z = 718.3 $[M+H]^+$.

Example 32:

N-((1-(2-(2,6-dioxopiperidin-3-yl)-1,3-dioxoisoindolin-5-yl)azetidin-3-yl)methyl)-5-(4-((7-ethyl-6-oxo-5,6-dihydro-1,5-naphthyridin-3-yl)methyl)piperazin-1-yl)picolinamide (Compound **32**)

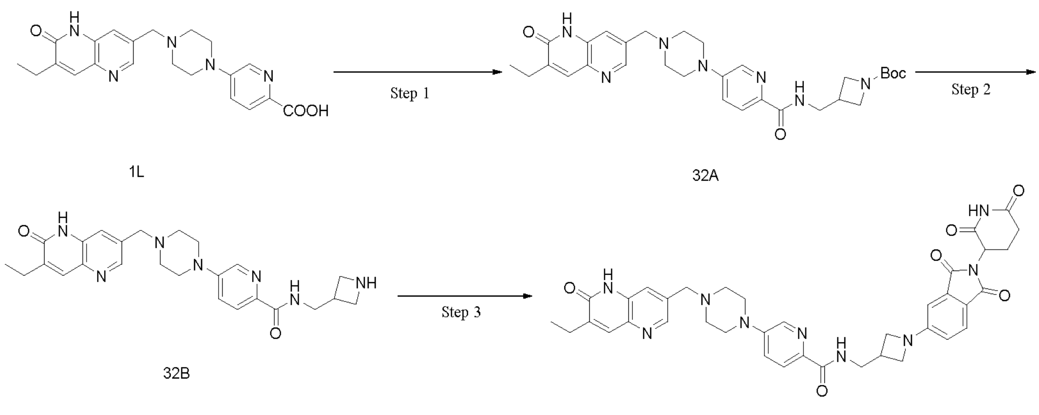


1L

32A

32B

Compound 32

Step 1:

**1L** (150 mg, 0.37 mmol), tert-butyl 3-(aminomethyl)azetidine-1-carboxylate (70 mg, 0.37 mmol), HATU (182 mg, 0.48 mmol), DIPEA (140 mg, 1.11 mmol) and DMF(3 mL) were added to a reaction flask and the mixture was stirred at 25°C for 5 h. After the reaction was completed, water (10 mL) was added and the reaction liquid was extracted with ethyl acetate (30 mL × 3), washed with saturated sodium chloride solution, dried over anhydrous sodium sulphate and concentrated under reduced pressure to afford crude **32A** (202 mg, 48%).
LCMS m/z = 562.3 $[M+H]^+$

Step 2:

Compound **32A** (202 mg, 0.36 mmol) was dissolved in dichloromethane (6 mL), trifluoroacetic acid (2 mL) was added, and the mixture was reacted at room temperature for 30 min. After the reaction was completed, the reaction liquid was concentrated under reduced pressure to afford crude **32B** (150 mg, 90%).
LCMS m/z = 462.2 $[M+H]^+$

Step 3:

Compound **32B** (143 mg, 0.31 mmol), 2-(2,6-dioxopiperidin-3-yl)-5-fluoroisoindoline-1,3-dione (86 mg, 0.31mmol), DIPEA (120 mg, 0.93 mmol) and dimethyl sulphoxide (3 mL) were added to a reaction flask and the mixture was stirred at 100°C for 3 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min to afford title compound **32** (40 mg, 20%).

LCMS m/z = 718.3 $[M+H]^+$
$^1H$ NMR (400 MHz, DMSO-$d_6$) δ 8.73 (t, 1H), 8.40 (d, 1H), 8.26 (d, 1H), 7.85 (d, 1H), 7.75 (s, 1H), 7.62 (d, 2H), 7.40 (dd, 1H), 6.75 (d, 1H), 6.62 (dd, 1H), 5.07 - 5.02 (m, 1H), 4.08 (t, 2H), 3.86 - 3.78 (m, 2H), 3.66 (s, 2H), 3.56 (t, 2H), 3.05 - 2.97 (m, 2H), 2.94 - 2.80 (m, 2H), 2.61 - 2.51 (m, 8H), 2.04 - 1.97 (m, 1H), 1.84 (s, 3H), 1.18 (t, 3H).

Example 33:

N-((1s,3s)-3-((2-(2,6-dioxopiperidin-3-yl)-1,3-dioxoisoindolin-4-yl)amino)cyclobutyl)-5-(4-((7-ethyl-6-oxo-5,6-dihydro-1,5-naphthyridin-3-yl)methyl)piperazin-1-yl)picolinamide (Compound **33**)

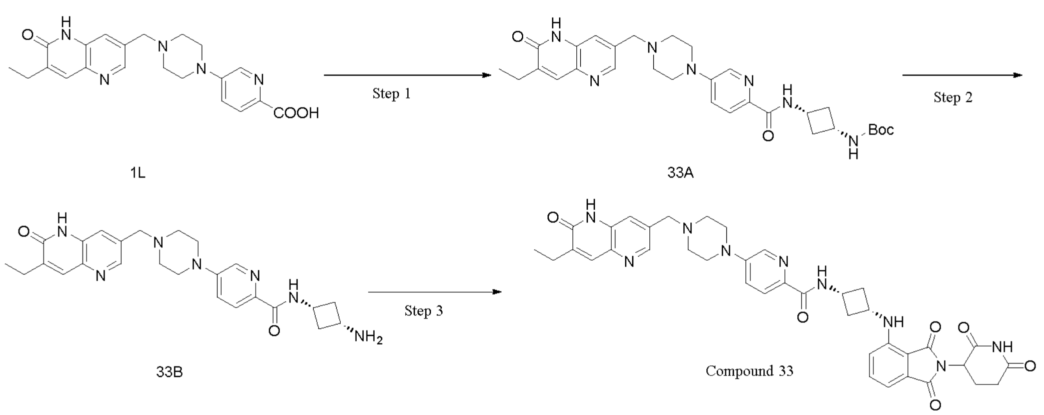


1L, 33A, 33B, Compound 33

Step 1:

**1L** (200 mg, 0.51 mmol), tert-butyl ((1s, 3s)-3-aminocyclobutyl)carbamate (94.99 mg, 0.51 mmol), HATU (252 mg, 0.66 mmol), DIPEA (198 mg, 1.53 mmol) and DMF(4 mL) were added to a reaction flask and the mixture was stirred at 25°C for 5 h. After the reaction was completed, water (12 mL) was added and the reaction liquid was extracted with ethyl acetate (30 mL × 3), washed with saturated sodium chloride solution, dried over anhydrous sodium sulphate and concentrated under reduced pressure to afford crude **33A** (153 mg, 53%).
LCMS m/z = 562.3 $[M+H]^+$

Step 2:

Compound **33A** (153 mg, 0.27 mmol) was dissolved in dichloromethane (6 mL), trifluoroacetic acid (2 mL) was added, and the mixture was reacted at room temperature for 30 min. After the reaction was completed, the reaction liquid was concentrated under reduced pressure to afford crude **33B** (120 mg, 100%).
LCMS m/z = 462.2 $[M+H]^+$

Step 3:

Compound **33B** (120 mg, 0.26 mmol), 2-(2,6-dioxopiperidin-3-yl)-4-fluoroisoindoline-1,3-dione (86 mg, 0.31mmol), DIPEA (120 mg, 0.93 mmol) and dimethyl sulphoxide (3 mL) were added to a reaction flask and the mixture was stirred at 100°C for 3 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min to afford title compound 33 (12 mg, 6%).

LCMS m/z = 718.2 $[M+H]^+$
$^1$H NMR (400 MHz, DMSO-$d_6$) δ 11.87 (s, 1H), 11.07 (s, 1H), 8.69 (d, 1H), 8.41 (d, 1H), 8.28 (d, 1H), 7.84 (d, 1H), 7.76 (s, 1H), 7.64 (s, 1H), 7.60 (t, 1H), 7.40 (dd, 1H), 7.13 - 7.01 (m, 2H), 6.54 (d, 1H), 5.07 (dd, 1H), 4.30 (dd, 1H), 3.94 (dd, 2H), 3.66 (s, 2H), 2.96 - 2.74 (m, 4H), 2.57 (dd, 9H), 2.22 (dd, 2H), 2.04 (dd, 2H), 1.19 (t, 3H).

Example 34:

N-((1r,4r)-4-((2-(2,6-dioxopiperidin-3-yl)-1,3-dioxoisoindolin-5-yl)amino)cyclohexyl)-5-(4-((7-ethyl-6-oxo-5,6-dihydro-1,5-naphthyridin-3-yl)methyl)piperazin-1-yl)picolinamide (Compound **34**)

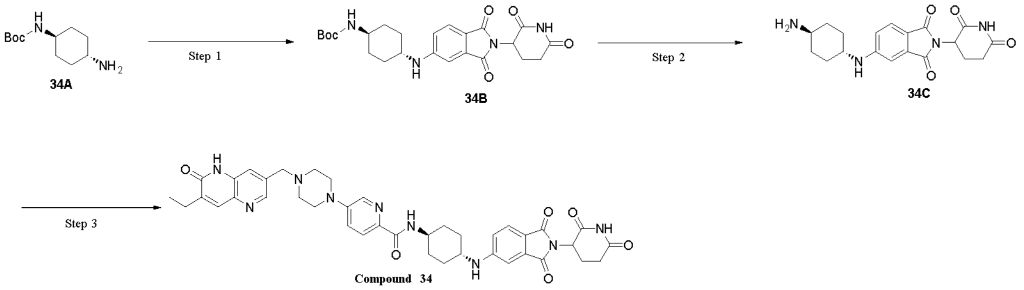


Step 1:

**34A** (517 mg, 2.41 mmol) was dissolved in dimethyl sulphoxide (10 ml) and 2-(2,6-dioxopiperidin-3-yl)-5-fluoroisoindoline-1,3-dione (1 g, 3.62 mmol) was added. The reaction system was placed in a 100°C oil bath pan and reacted for 1 h. After the reaction was completed, the reaction system was diluted with ethyl acetate (50 ml) and the organic phase was washed with water (10 ml), dried over anhydrous sodium sulphate, filtered and concentrated. The residue was separated and purified by flash column chromatography (eluent: MeOH : DCM = 0%-10%) to afford compound **34B** (0.4 g, 35%).
LC-MS (ESI): m/z = 415.1[M-56+H]$^+$

Step 2:

Compound **34B** (0.4 g, 0.85 mmol) was added to a reaction flask, dichloromethane (3 ml)/trifluoroacetic acid (1 ml) were added, and the mixture was reacted at room temperature for 2 h. After the reaction was completed, the reaction liquid was directly concentrated, to afford crude compound **34C** (0.3 g).
LC-MS (ESI): m/z = 371.1[M+H]$^+$

Step 3:

Intermediate **1L** (100 mg, 0.25 mmol) was dissolved in N,N-dimethylformamide (2 ml), compound **34C** (141 mg, 0.38 mmol), 2-(7-azobenzotriazole)-N,N,N',N'-tetramethyluronium hexafluorophosphate (190 mg, 0.5 mmol) and N,N-diisopropylethylamine (130 mg, 1 mmol) were added to the reaction system, and the mixture was reacted overnight at room temperature. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min to afford compound **34** (60 mg, 32%).

LC-MS (ESI): m/z = 746.3 [M+H]$^+$
$^1$H NMR (400 MHz, DMSO-$d_6$) $\delta$ = 11.83 (s, 1H), 11.04 (s, 1H), 8.41 (d, 1H), 8.28 (d, 1H), 8.12 (d, 1H), 7.83 (s, 1H), 7.75 (s, 1H), 7.63 (s, 1H), 7.56 (d, 1H), 7.40 (dd, 1H), 7.01 (d, 1H), 6.96 (s, 1H), 6.87 (dd, 1H), 5.03 (dd, 1H), 3.78 (dd, 1H), 3.66 (s, 2H), 3.46 - 3.37 (m, 1H), 3.34 (s, 4H), 2.94 - 2.79 (m, 1H), 2.56 (s, 4H), 2.01 (dd, 3H), 1.91 (s, 2H), 1.58 (dd, 2H), 1.34 (dd, 2H), 1.23 (s, 1H), 1.19 (t, 3H).

Example 35:

2-(2,6-dioxopiperidin-3-yl)-5-(((1-(5-(4-((7-ethyl-6-oxo-5,6-dihydro-1,5-naphthyridin-3-yl)methyl)piperazin-1-yl)picolinoyl)piperidin-4-yl)methyl)amino)isoindoline-1,3-dione (Compound **35**)

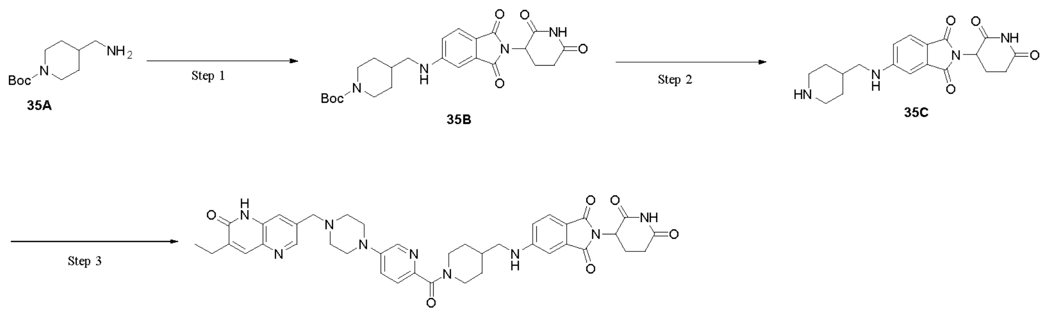


35A

35B

35C

Compound 35

Step 1:

**35A** (517 mg, 2.41 mmol) was dissolved in dimethyl sulphoxide (10 ml) and 2-(2,6-dioxopiperidin-3-yl)-5-fluoroisoindoline-1,3-dione (1 g, 3.62 mmol) was added. The reaction system was placed in a 100°C oil bath pan and reacted for 1 h. After the reaction was completed, the reaction system was diluted with ethyl acetate (50 ml) and the organic phase was washed with water (10 ml), dried over anhydrous sodium sulphate, filtered and concentrated. The residue was separated and purified by flash column chromatography (eluent: MeOH : DCM = 0%-10%) to afford compound **35B** (0.25 g, 22%).
LC-MS (ESI): m/z = 415.1[M-56+H]$^+$

Step 2:

Compound **35B** (0.25 g, 0.53 mmol) was added to a reaction flask, dichloromethane (3 ml)/trifluoroacetic acid (1 ml) were added, and the mixture was reacted at room temperature for 2 h. After the reaction was completed, the reaction liquid was directly concentrated, to afford crude compound **35C** (0.2 g).
LC-MS (ESI): m/z = 371.1[M+H]$^+$

Step 3:

Intermediate **1L** (100 mg, 0.25 mmol) was dissolved in N,N-dimethylformamide (2 ml), compound **35C** (141 mg, 0.38 mmol), 2-(7-azobenzotriazole)-N,N,N',N'-tetramethyluronium hexafluorophosphate (190 mg, 0.5 mmol) and N,N-diisopropylethylamine (130 mg, 1 mmol) were added to the reaction system, and the mixture was reacted overnight at room temperature. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%. c. flow rate: 12 mL/min; d. elution time: 20 min, retention time: 7.0 min, to afford compound **35** (50 mg, 27%).

LC-MS (ESI): m/z = 746.3 [M+H]$^+$
$^1$H NMR (400 MHz, DMSO-$d_6$) δ 11.83 (s, 1H), 11.04 (s, 1H), 8.41 (s, 1H), 8.24 (d, 1H), 7.75 (s, 1H), 7.63 (s, 1H), 7.55 (d, 1H), 7.49 - 7.41 (m, 1H), 7.41 - 7.32 (m, 1H), 7.19 (s, 1H), 6.98 (s, 1H), 6.89 (d, 1H), 5.02 (dd, 1H), 4.47 (s, 1H), 4.05 (s, 1H), 3.65 (s, 2H), 3.12 (s, 2H), 3.01 (s, 1H), 2.93 - 2.81 (m, 1H), 2.81 - 2.66 (m, 1H), 2.55 (s, 5H), 2.07 - 1.94 (m, 1H), 1.89 (d, 2H), 1.72 (s, 1H), 1.20 (dd, 6H).

Example 36:

2-(2,6-dioxopiperidin-3-yl)-5-(((1-(5-(4-((7-ethyl-6-oxo-5,6-dihydro-1,5-naphthyridin-3-yl)methyl)piperazin-1-yl)picolinoyl)azetidin-3-yl)methyl)amino)isoindoline-1,3-dione (Compound **36**)

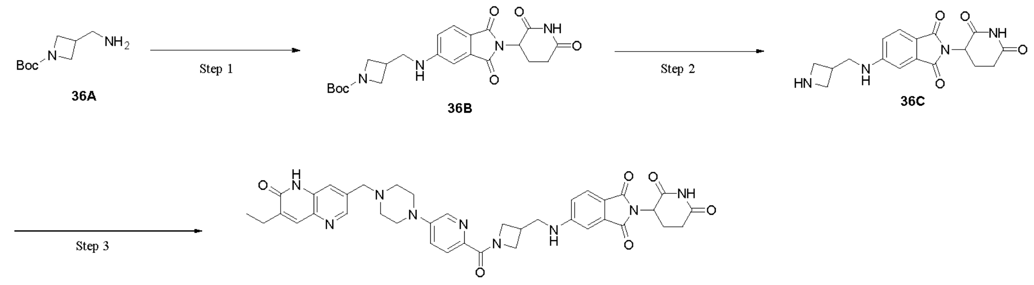


36A 36B 36C

Compound 36

Step 1:

**36A** (517 mg, 2.41 mmol) was dissolved in dimethyl sulphoxide (10 ml) and 2-(2,6-dioxopiperidin-3-yl)-5-fluoroisoindoline-1,3-dione (1 g, 3.62 mmol) was added. The reaction system was placed in a 100°C oil bath pan and reacted for 1 h. After the reaction was completed, the reaction system was diluted with ethyl acetate (50 ml) and the organic phase was washed with water (10 ml), dried over anhydrous sodium sulphate, filtered and concentrated. The residue was separated and purified by flash column chromatography (eluent: MeOH : DCM = 0%-10%) to afford compound **36B** (0.3 g, 26%).
LC-MS (ESI): m/z = 387.1[M-56+H]$^+$

Step 2:

Compound **36B** (0.25 g, 0.53 mmol) was added to 50 mL of reaction flask, dichloromethane (3 ml)/trifluoroacetic acid (1 ml) were added, and the mixture was reacted at room temperature for 2 h. After the reaction was completed, the reaction liquid was directly concentrated, to afford crude compound **36C** (0.25 g).
LC-MS (ESI): m/z = 343.1[M+H]$^+$

Step 3:

Intermediate **1L** (100 mg, 0.25 mmol) was dissolved in N,N-dimethylformamide (2 ml), compound **36C** (147 mg, 0.38 mmol), 2-(7-azobenzotriazole)-N,N,N',N'-tetramethyluronium hexafluorophosphate (190 mg, 0.5 mmol) and N,N-diisopropylethylamine (130 mg, 1 mmol) were added to the reaction system, and the mixture was reacted overnight at room temperature. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; d. elution time: 20 min, retention time: 7.0 min, to afford compound **36** (78 mg, 42%).

LC-MS (ESI): m/z = 718.3[M+H]$^+$
$^1$H NMR (400 MHz, DMSO-$d_6$) δ = 11.83 (s, 1H), 11.04 (s, 1H), 8.40 (d, 1H), 8.26 (d, 1H), 7.79 (d, 1H), 7.75 (s, 1H), 7.62 (s, 1H), 7.57 (d, 1H), 7.37 (dd, 1H), 7.25 (t, 1H), 7.01 (d, 1H), 6.90 (dd, 1H), 5.03 (dd, 1H), 4.64 (t, 1H), 4.30 (dd, 1H), 4.12 (t, 1H), 3.76 (dd, 1H), 3.65 (s, 2H), 3.45 (t, 2H), 3.33 (d, 4H), 2.96 - 2.83 (m, 2H), 2.54 (d, 6H), 2.05 - 1.93 (m, 1H), 1.91 (s, 1H), 1.23 (s, 1H), 1.18 (t, 3H).

Example 37:

N-((1s,3s)-3-((2-(2,6-dioxopiperidin-3-yl)-1,3-dioxoisoindolin-5-yl)amino) cyclobutyl)-5-(4-((7-ethyl-6-oxo-5, 6-dihydro-1, 5-naphthyridin-3-yl)methyl)piperazin-1-yl)picolinamide (Compound **37**)

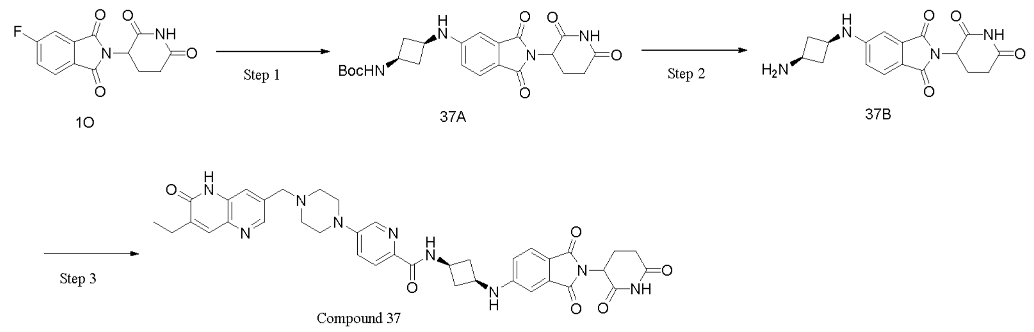


Step 1:

Tert-butyl cis-3-amino-1-cyclobutylcarbamate (500 mg, 2.68 mmol), 1O (1.11 g, 4.03 mmol) and N,N-diisopropylethylamine (1.04 g, 8.05 mmol) were added to a reaction flask, dimethyl sulphoxide (15 mL) was added, and the mixture was warmed to 100°C and reacted for about 4 hours. Upon completion of reaction as monitored by TLC, the reaction liquid was cooled to room temperature, and saturated brine (80 mL) was added. The aqueous phase was extracted with ethyl acetate (20 mL × 6) and the organic phase was dried over anhydrous sodium sulphate, filtered and concentrated. The resulting residue was separated and purified by silica gel column chromatography (dichloromethane : methanol (v/v) = 100 : 5) to afford **37A** (300 mg, 25.3%) as a yellow solid.
LC-MS (ESI): 441.1 $[M-H]^-$

Step 2:

Compound **37A** (150 mg, 0.339 mmol) was dissolved in dichloromethane (6 mL) and at room temperature, trifluoroacetic acid (2 mL) was added. After the addition, the mixture was reacted at room temperature for 30 minutes. Upon completion of reaction as monitored by TLC, the reaction liquid was directly concentrated under reduced pressure and dichloromethane (10 mL × 4) was added. The mixture was dried with suction again to remove excess trifluoroacetic acid. The resulting foamy yellow solid **37B** was directly used in the next reaction without purification.
LC-MS (ESI): 343.1 $[M+H]^+$.

Step 3:

Compound **1L** (60 mg, 0.152 mmol), **37B** (68 mg, 0.198 mmol), N,N-diisopropylethylamine (98.5 mg, 0.764 mmol) and 2-(7-azobenzotriazole)-N,N,N',N'-tetramethyluronium hexafluorophosphate (116 mg, 0.305 mmol) were added to a reaction flask and dry N,N-dimethylformamide (5 mL) was added. The mixture was reacted at room temperature for 4 hours. Upon completion of reaction as monitored by TLC, the reaction liquid was directly purified by preparative liquid phase chromatography. The crude was separated and purified by preparative HPLC with methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.5% ammonium acetate); b. gradient elution, mobile phase A: 5%-50%; c. flow rate: 12 mL/min; d. elution time: 10 min, to afford compound **37** (38 mg, 34.7%).

LC-MS (ESI): 718.2 $[M+H]^+$,
$^1$H NMR (400 MHz, DMSO-$d_6$) δ 11.83 (s, 1H), 11.04 (s, 1H), 8.61 (d, 1H), 8.41 (d, 1H), 8.27 (d, 1H), 7.83 (d, 1H), 7.75 (d, 1H), 7.66 - 7.62 (m, 1H), 7.58 (d, 1H), 7.40 (dd, 1H), 7.34 (d, 1H), 6.92 (d, 1H), 6.83 (dd, 1H), 5.03 (dd, 1H), 4.24 (h, 1H), 3.74 (p, 1H), 3.66 (s, 2H), 3.45 - 3.22 (m, 3H, overlapped), 2.93 - 2.86 (m, 1H), 2.78 (d, 2H), 2.61 - 2.47 (m, 8H), 2.13 - 1.93 (m, 4H), 1.18 (t, 3H).

Example 38:

N-((1r,3r)-3-((2-(2,6-dioxopiperidin-3-yl)-1,3-dioxoisoindolin-5-yl)amino)cyclobutyl)-5-(4-((7-ethyl-6-oxo-5,6-dihydro-1,5-naphthyridin-3-yl)methyl)piperazin-1-yl)picolinamide (Compound **38**)

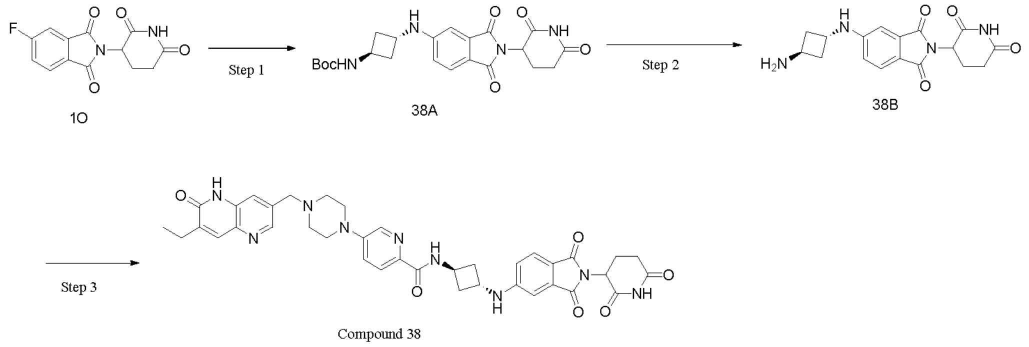


1O 38A 38B Compound 38

Step 1:

Tert-butyl trans-3-amino-1-cyclobutylcarbamate (500 mg, 2.68 mmol), 1O (1.11 g, 4.03 mmol) and N,N-diisopropylethylamine (1.04 g, 8.05 mmol) were added to a reaction flask, dimethyl sulphoxide (15 mL) was added, and the mixture was warmed to 100°C and reacted for about 4 hours. Upon completion of reaction as monitored by TLC, the reaction liquid was cooled to room temperature, and saturated brine (80 mL) was added. The aqueous phase was extracted with ethyl acetate (20 mL × 6) and the organic phase was dried over anhydrous sodium sulphate, filtered and concentrated. The resulting residue was separated and purified by silica gel column chromatography (dichloromethane : methanol (v/v) = 100 : 5) to afford **38A** (260 mg, 21.9 %) as a yellow solid.
LC-MS (ESI): 441.1 $[M-H]^-$

Step 2:

Compound **38A** (150 mg, 0.339 mmol) was dissolved in dichloromethane (6 mL) and at room temperature, trifluoroacetic acid (2 mL) was added. After the addition, the mixture was reacted at room temperature for 30 minutes. Upon completion of reaction as monitored by TLC, the reaction liquid was directly concentrated under reduced pressure and dichloromethane (10 mL × 4) was added. The mixture was dried with suction again to remove excess trifluoroacetic acid. The resulting foamy yellow solid **38B** was directly used in the next reaction without purification.
LC-MS (ESI): 343.1 $[M+H]^+$

Step 3:

Compound **1L** (50 mg, 0.127 mmol), **38B** (56.5 mg, 0.165 mmol), N,N-diisopropylethylamine (82.0 mg, 0.635 mmol) and 2-(7-azobenzotriazole)-N,N,N',N'-tetramethyluronium hexafluorophosphate (96.6 mg, 0.254 mmol) were added to a reaction flask and dry N,N-dimethylformamide (5 mL) was added. The mixture was reacted at room temperature for 4 hours. Upon completion of reaction as monitored by TLC, the reaction liquid was directly purified by preparative liquid phase chromatography. The crude was separated and purified by preparative HPLC with methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.5% ammonium acetate); b. gradient elution, mobile phase A: 5%-50%; c. flow rate: 12 mL/min; d. elution time: 10 min, to afford compound **38** (35 mg, 38.4%).

LC-MS (ESI):718.3 $[M+H]^+$;
$^1H$ NMR (400 MHz, DMSO-$d_6$) δ 11.84 (s, 1H), 11.05 (s, 1H), 8.82 (d, 1H), 8.41 (d, 1H), 8.30 (d, 1H), 7.84 (d, 1H), 7.75 (s, 1H), 7.63 (d, 1H), 7.59 (d, 1H), 7.53 (d, 1H), 7.40 (dd, 1H), 6.88 (s, 1H), 6.80 (d, 1H), 5.03 (dd, 1H), 4.61 (p, 1H), 4.11 (s, 1H), 3.66 (s, 2H), 3.45 - 3.22 (m, 3H, overlapped), 2.94 - 2.79 (m, 2H), 2.63 - 2.52 (m, 9H), 2.32 - 2.17 (m, 2H), 2.00 (d, 2H), 1.19 (t, 3H).

Example 39:

N-((1r,3r)-3-((2-(2,6-dioxopiperidin-3-yl)-1,3-dioxoisoindolin-4-yl)amino)cyclobutyl)-5-(4-((7-ethyl-6-oxo-5,6-dihydro-1,5-naphthyridin-3-yl)methyl)piperazin-1-yl)picolinamide (Compound **39**)

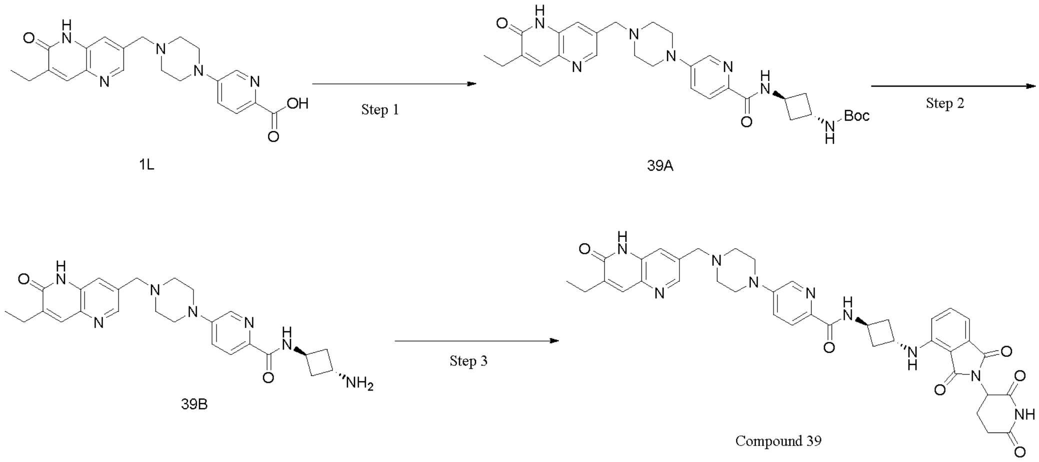


1L 39A 39B Compound 39

Step 1:

**1L** (150 mg, 0.38 mmol), tert-butyl ((1r, 3r)-3-aminocyclobutyl)carbamate (71 mg, 0.38 mmol), HATU (187 mg, 0.49 mmol), DIPEA (150 mg, 1.16 mmol) and DMF(3 mL) were added to a reaction flask and the mixture was stirred at 25°C for 5 h. After the reaction was completed, water (12 mL) was added and the reaction liquid was extracted with ethyl acetate (30 ml × 3), washed with saturated sodium chloride solution, dried over anhydrous sodium sulphate and concentrated under reduced pressure to afford crude **39A** (150 mg, 70%).
LCMS m/z = 562.3 $[M+H]^+$

Step 2:

Compound **39A** (150 mg, 0.27 mmol) was dissolved in dichloromethane (6 mL), trifluoroacetic acid (2 mL) was added, and the mixture was reacted at room temperature for 30 min. After the reaction was completed, the reaction liquid was concentrated under reduced pressure to afford crude **39B** (98 mg, 79%).
LCMS m/z = 462.2 $[M+H]^+$

Step 3:

Compound **39B** (98 mg, 0.21 mmol), 2-(2,6-dioxopiperidin-3-yl)-4-fluoroisoindoline-1,3-dione (58.43 mg, 0.21mmol), DIPEA (81 mg, 0.63 mmol) and dimethyl sulphoxide (3 mL) were added to a reaction flask and the mixture was stirred at 100°C for 3 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford title compound **39** (6 mg, 4%).

LCMS m/z = 718.2 $[M+H]^+$
$^1$H NMR (400 MHz, DMSO-$d_6$) δ 11.83 (s, 1H), 11.08 (s, 1H), 8.79 (d, 1H), 8.41 (s, 1H), 8.29 (s, 1H), 7.84 (d, 1H), 7.75 (s, 1H), 7.66 - 7.56 (m, 2H), 7.41 (d, 1H), 7.09 (d, 1H), 6.96 (d, 1H), 6.57 (d, 1H), 5.07 (dd, 1H), 4.55 (d, 1H), 4.28 (s, 1H), 3.66 (s, 2H), 3.36 (s, 4H), 2.97 - 2.80 (m, 2H), 2.65 - 2.52 (m, 8H), 2.34 (s, 2H), 2.09 - 1.94 (m, 2H), 1.19 (t, 3H).

Example 40:

N-(2-(2-(2,6-dioxopiperidin-3-yl)-1,3-dioxoisoindolin-5-yl)-2-azaspiro[3.5]nonan-7-yl)-5-(4-((7-ethyl-6-oxo-5,6-dihydro-1,5-naphthyridin-3-yl)methyl)piperazin-1-yl)picolinamide (Compound **40**)

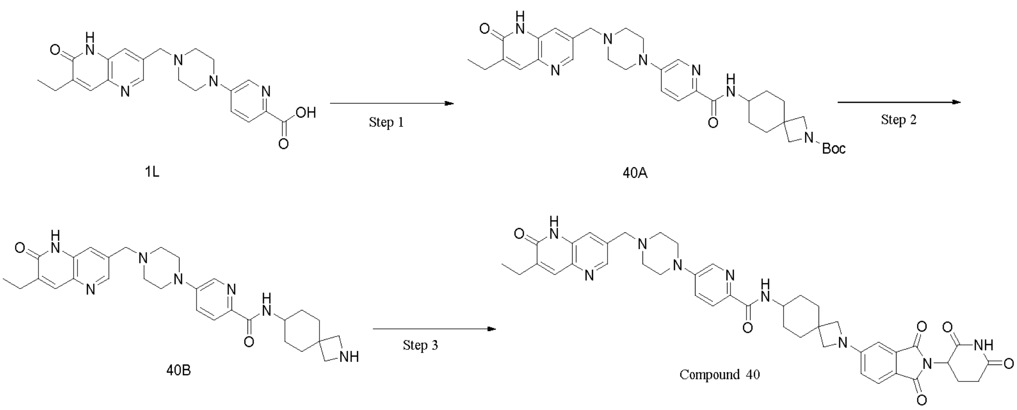


Step 1:

**1L** (200 mg, 0.51 mmol), 2-BOC-7-amino-2-azaspiro[3.5]nonane (120 mg, 0.51 mmol), HATU (250 mg, 0.66 mmol), DIPEA (200 mg, 1.53 mmol) and DMF (4 mL) were added to a reaction flask and the mixture was stirred at 25°C for 5 h. After the reaction was completed, water (12 mL) was added and the reaction liquid was extracted with ethyl acetate (30 ml × 3), washed with saturated sodium chloride solution, dried over anhydrous sodium sulphate and concentrated under reduced pressure to afford crude **40A** (300 mg, 95%).
LCMS m/z = 616.3 $[M+H]^+$

Step 2:

Compound **40A** (155 mg, 0.25 mmol) was dissolved in dichloromethane (6 mL), trifluoroacetic acid (2 mL) was added, and the mixture was reacted at room temperature for 30 min. After the reaction was completed, the reaction liquid was concentrated under reduced pressure to afford crude **40B** (110 mg, 85%).
LCMS m/z = 516.3 $[M+H]^+$

Step 3:

Compound **40B** (170 mg, 0.33 mmol), 2-(2,6-dioxopiperidin-3-yl)-5-fluoroisoindoline-1,3-dione (100 mg, 0.36mmol), DIPEA (120 mg, 0.93 mmol) and DMF (3 mL) were added to a reaction flask and the mixture was stirred at 100°C for 3 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford title compound **40** (20 mg, 8%).

LCMS m/z = 772.1 $[M+H]^+$
$^1$H NMR (400 MHz, DMSO-$d_6$) δ 11.84 (s, 1H), 11.06 (s, 1H), 8.41 (s, 1H), 8.28 (d, 1H), 8.11 (d, 1H), 7.84 (d, 1H), 7.76 (s, 1H), 7.64 (d, 2H), 7.40 (dd, 1H), 6.77 (s, 1H), 6.65 (dd, 1H), 5.05 (dd, 1H), 3.76 (d, 5H), 3.66 (s, 2H), 2.94 - 2.83 (m, 1H), 2.63 - 2.52 (m, 9H), 2.06 - 1.89 (m, 4H), 1.63 (ddd, 8H), 1.19 (t, 3H).

Example 41:

N-((1 s,3 s)-3-((2-(2,6-dioxopiperidin-3-yl)-6-fluoro-1,3-dioxoisoindolin-5-yl)amino)cyclobutyl)-5-(4-((7-ethyl-6-oxo-5,6-dihydro-1,5-naphthyridin-3-yl)methyl)piperazin-1-yl)picolinamide (Compound **41**)

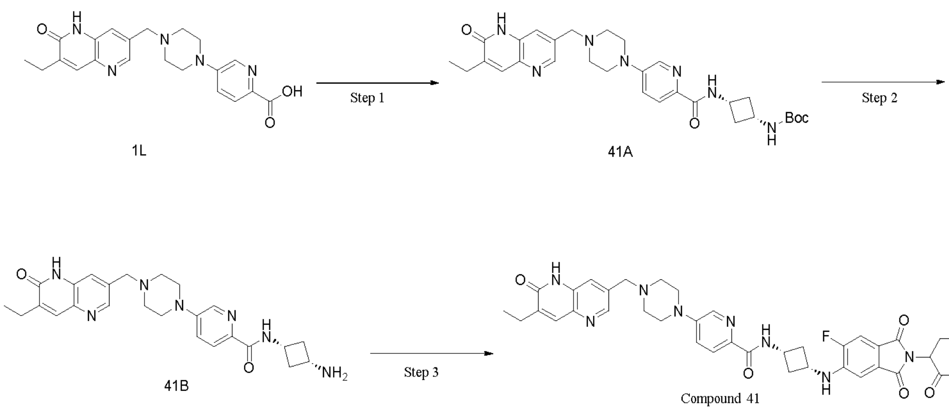

1L 41A 41B Compound 41

Step 1:

**1L** (300 mg, 0.76 mmol), tert-butyl ((1s, 3s)-3-aminocyclobutyl)carbamate (71 mg, 0.38 mmol), HATU (190 mg, 0.50 mmol), DIPEA (150 mg, 1.16 mmol) and DMF(4 mL) were added to a reaction flask and the mixture was stirred at 25°C for 5 h. After the reaction was completed, water (12 mL) was added and the reaction liquid was extracted with ethyl acetate (40 ml × 3), washed with saturated sodium chloride solution, dried over anhydrous sodium sulphate and concentrated under reduced pressure. The residue was separated and purified by a preparative thin layer chromatographic plate to afford pale yellow pure product **41A** (153 mg, 71.7%).
LCMS m/z = 562.2 $[M+H]^+$

Step 2:

Compound **41A** (153 mg, 0.27 mmol) was dissolved in dichloromethane (6 mL), trifluoroacetic acid (2 mL) was added, and the mixture was reacted at room temperature for 30 min. After the reaction was completed, the reaction liquid was concentrated under reduced pressure to afford crude **41B** (110 mg, 88%).
LCMS m/z = 462.2 $[M+H]^+$

Step 3:

Compound **41B** (110 mg, 0.24 mmol), 2-(2,6-dioxopiperidin-3-yl)-5,6-difluoroindoline-1,3-dione (92 mg, 0.31 mmol), DIPEA (93 mg, 0.72 mmol) and DMF (3 mL) were added to a reaction flask and the mixture was stirred at 100°C for 3 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford title compound **41** (39 mg, 22%).

LCMS m/z = 736.1 $[M+H]^+$
$^1$H NMR (400 MHz, DMSO-$d_6$) δ 11.83 (s, 1H), 11.06 (s, 1H), 8.50 (d, 1H), 8.41 (s, 1H), 8.29 (s, 1H), 7.84 (d, 1H), 7.75 (s, 1H), 7.71 - 7.55 (m, 2H), 7.41 (d, 1H), 7.07 (d, 1H), 6.98 (d, 1H), 5.05 (dd, 1H), 4.25 (dd, 1H), 3.92 (dd, 1H), 3.66 (s, 2H), 2.96 - 2.75 (m, 3H), 2.66 - 2.52 (m, 11H), 2.18 (d, 2H), 2.07 - 1.89 (m, 2H), 1.19 (t, 3H).

Example 42:

N-((1r, 3r)-3-((2-(2,6-dioxopiperidin-3-yl)-1,3-dioxoisoindolin-5-yl)amino) cyclobutyl)-5-(4-((7-ethyl-6-oxo-5,6-dihydro-1,5-naphthyridin-3-yl)methyl)piperazin-1-yl)picolinamide (Compound **42**)

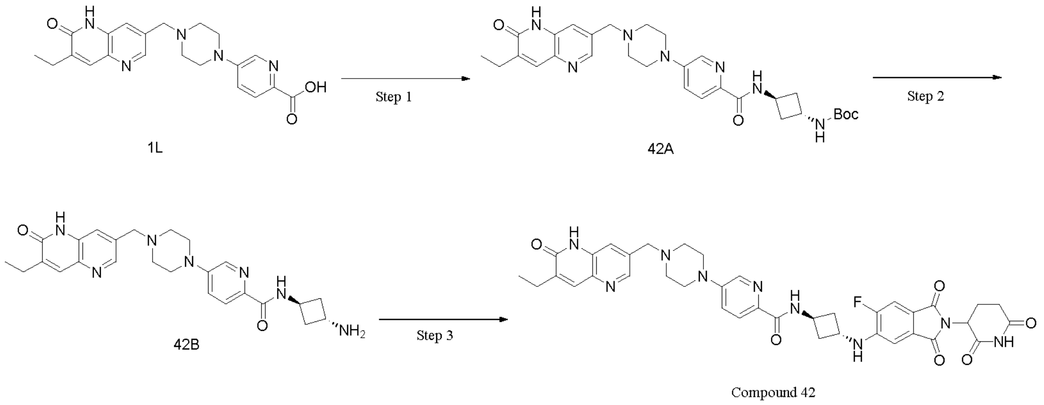


1L 42A 42B Compound 42

Step 1:

**1L** (500 mg, 1.27 mmol), tert-butyl ((1r, 3r)-3-aminocyclobutyl)carbamate (260 mg, 1.4 mmol), HATU (628 mg, 1.65 mmol), DIPEA (490 mg, 3.81 mmol) and DMF(4 mL) were added to a reaction flask and the mixture was stirred at 25°C for 5 h. After the reaction was completed, water (12 mL) was added and the reaction liquid was extracted with ethyl acetate (40 ml × 3), washed with saturated sodium chloride solution, dried over anhydrous sodium sulphate and concentrated under reduced pressure to afford crude **42A** (480 mg, 67.3%).
LCMS m/z = 562.2 $[M+H]^+$

Step 2:

Compound **42A** (480 mg, 0.85 mmol) was dissolved in dichloromethane (6 mL), trifluoroacetic acid (2 mL) was added, and the mixture was reacted at room temperature for 30 min. After the reaction was completed, the reaction liquid was concentrated under reduced pressure to afford crude **42B** (370 mg, 94%).
LCMS m/z = 462.2 $[M+H]^+$

Step 3:

Compound **42B** (185 mg, 0.4 mmol), 2-(2,6-dioxopiperidin-3-yl)-5,6-difluoroindoline-1,3-dione (130 mg, 0.44 mmol), DIPEA (155 mg, 1.2 mmol) and DMF (3 mL) were added to a reaction flask and the mixture was stirred at 100°C for 3 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford title compound **42** (42 mg, 12%).

LCMS m/z = 736.1 $[M+H]^+$
$^1H$ NMR (400 MHz, DMSO-$d_6$) δ 11.83 (s, 1H), 11.06 (s, 1H), 8.84 (d, 1H), 8.41 (d, 1H), 8.31 (d, 1H), 7.84 (d, 1H), 7.76 (s, 1H), 7.64 (s, 1H), 7.60 (d, 1H), 7.41 (dd, 1H), 7.23 (d, 1H), 6.98 (d, 1H), 5.05 (dd, 1H), 4.57 (dd, 1H), 4.27 (d, 1H), 3.67 (s, 2H), 2.94 - 2.80 (m, 1H), 2.63 - 2.50 (m, 12H), 2.49 - 2.32 (m, 3H), 2.12 - 1.91 (m, 2H), 1.19 (t, 3H).

Example 43:

2-(2,6-dioxopiperidin-3-yl)-5-(3-((5-(4-((7-ethyl-6-oxo-5,6-dihydro-1,5-naphthyridin-3-yl)methyl)piperazin-1-yl)pyridin-2-yl)ethynyl)azetidin-1-yl)isoindoline-1,3-dione (Compound **43**)

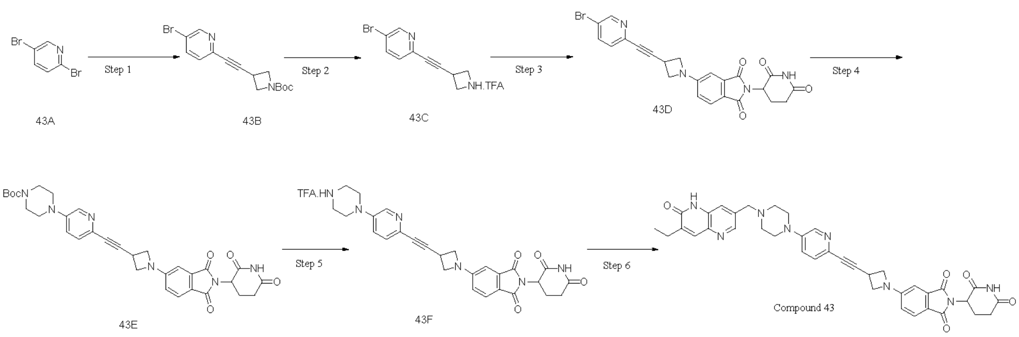


43A 43B 43C 43D 43E 43F Compound 43

Step 1:

**43A** (5 g, 21.11 mmol), tert-butyl 3-ethynylazetidine-1-carboxylate (4.02 g, 22.18 mmol), bistriphenylphosphine palladium dichloride (0.74 g, 1.06 mmol), cuprous iodide (0.4 g, 2.11 mmol) and triethylamine (6.41 g, 63.3 mmol) were added to tetrahydrofuran (50 ml). The mixture was subjected to $N_2$ replacement and reacted at room temperature for 16 h. Upon completion of reaction as monitored by TLC, water (100 ml) was added, and the mixture was extracted with ethyl acetate (100 ml × 2). The organic phase was washed with saturated sodium chloride solution, dried over anhydrous sodium sulphate, filtered and concentrated. The crude product was separated and purified by silica gel column chromatography (eluent: EA/PE = 0-25%), to afford title compound **43B** (5 g, 70.24%).
LC-MS (ESI): m/z = 337.1, 339.1$[M+H]^+$.

Step 2:

**43B** (5 g, 14.83 mmol) was added to dichloromethane (40 ml) and then trifluoroacetic acid (15 ml) was added. The mixture was reacted at room temperature for 2 h. After it was monitored that the reaction was completed, the reaction liquid was directly concentrated under reduced pressure to dryness to afford crude (8 g), which was directly used in the next reaction.

Step 3:

**43C** (crude, 8 g), 2-(2,6-dioxopiperidin-3-yl)-5-fluoroisoindoline-1,3-dione (7.63 g, 21.72 mmol) and N,N-diisopropylethylamine (11.23 g, 86.88 mmol) were added to dimethyl sulphoxide (50 ml) and the mixture was reacted at 100°C for 1 h. Upon completion of reaction as monitored by LCMS, the reaction liquid was cooled to room temperature and water (200 ml) was added. The mixture was extracted with dichloromethane (100 ml × 3), then washed with saturated sodium chloride solution, dried over anhydrous sodium sulphate and filtered. The filtrate was concentrated under reduced pressure and the crude product was separated and purified by silica gel column chromatography (eluent: EA/DCM = 0-50%) to afford the title compound (4 g).
LC-MS (ESI): m/z = 493.0, 495.0$[M+H]^+$.

Step 4:

**43D** (2 g, 4.05 mmol), tert-butyl piperazine-1-carboxylate (1.51 g, 8.07 mmol), methanesulphonato(2-dicyclohexylphosphino-2',6'-di-isopropoxy-1,1'-biphenyl)(2-amino-1,1'-biphenyl-2-yl)palladium (II) (0.17 g, 0.20 mmol) and caesium carbonate(2.64 g, 8.12 mmol) were added to dioxane (60 ml), and the mixture was subjected to $N_2$ replacement and reacted at 100°C for 16 h. The reaction was monitored by LC-MS. The reaction liquid was cooled to room temperature. The reaction liquid was directly concentrated under reduced pressure and DCM/MeOH = 10 : 1 (60 ml) was added. The mixture was stirred and filtered. The filtrate was concentrated under reduced pressure and the residue was separated and purified by silica gel column chromatography (eluent for purification: MeOH/DCM = 0-10%), to afford the title compound (0.35 g, 14%)

LC-MS (ESI): m/z = 599.3 $[M+H]^+$.

Step 5:

**43E** (0.35 g, 0.58 mmol) was added to dichloromethane (6 ml) and then trifluoroacetic acid (2 ml) was added. The mixture was reacted at room temperature for 2 h. After the reaction was completed, the reaction liquid was concentrated under reduced pressure to afford crude 43F (550 mg).

Step 6:

**1G** (0.1 g, 0.45 mmol) and **43F** (0.38 g, 0.45 mmol) were added to acetonitrile and then N,N-diisopropylethylamine (0.35 g, 2.71 mmol) and potassium iodide (0.015 g, 0.09 mmol) were added. The mixture was warmed to 80°C and reacted for 2 h. After it was monitored that the reaction was completed, the reaction liquid was directly separated and purified by HPLC. After purification, the product was concentrated under reduced pressure to remove acetonitrile and then extracted with (dichloromethane : methanol = 10 : 1), and the organic phase was concentrated under reduced pressure to afford the title compound (12 mg, 4%). Preparative HPLC separation methods: 1. Instruments: waters 2767 (preparative liquid phase chromatographic instrument); chromatographic column: SunFire@ Prep C18 (19 mm × 250 mm) 2. The sample was dissolved in DMF and filtered with a 0.45 μm filter to prepare a sample solution. 3. Preparative chromatography conditions: a. composition of mobile phases A and B: mobile phase A: acetonitrile; mobile phase B: water (containing 0.1% ammonium acetate); b. gradient elution, mobile phase A: 5%-40%; c. flow rate: 12 mL/min; d. elution time: 20 min.

$^1H$ NMR (400 MHz, DMSO-$d_6$) δ 11.82 (s, 1H), 11.05 (s, 1H), 8.40 (d, 1H), 8.25 (d, 1H), 7.74 (s, 1H), 7.68 (d, 1H), 7.62 (s, 1H), 7.33 (d, 1H), 7.26 (dd, 1H), 6.87 (d, 1H), 6.72 (dd, 1H), 5.06 (dd, 1H), 4.39 (t, 2H), 4.10 - 4.02 (m, 2H), 3.93-3.88 (m, 1H), 3.64 (s, 2H), 3.29 - 3.23 (m, 4H), 2.94 - 2.82 (m, 1H), 2.61-2.51 (m, 8H), 2.03-1.99 (m, 1H), 1.18 (t, 3H).

LC-MS (ESI): m/z = 685.3$[M+H]^+$.

Example 44:

2-(2,6-dioxopiperidin-3-yl)-5-(4-((5-(4-((7-ethyl-6-oxo-5,6-dihydro-1,5-naphthyridin-3-yl)methyl)piperazin-1-yl)pyridin-2-yl)ethynyl)piperidin-1-yl)isoindoline-1,3-dione (Compound **44**)

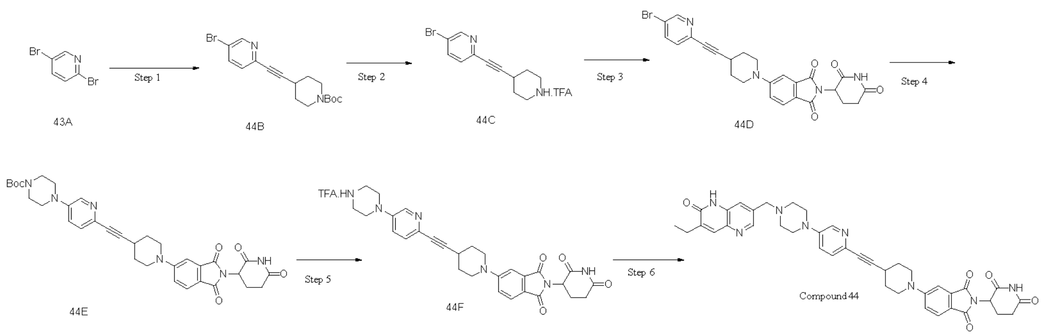


Step 1:

**43A** (5 g, 21.11 mmol), tert-butyl 4-ethynylpiperidine-1-carboxylate (4.64 g, 22.19 mmol), bistriphenylphosphine palladium dichloride (0.74 g, 1.06 mmol), cuprous iodide (0.4 g, 2.11 mmol) and triethylamine (6.41 g, 63.3 mmol) were added to tetrahydrofuran (50 ml). The mixture was subjected to $N_2$ replacement and reacted at room temperature for 16 h. Upon completion of reaction as monitored by TLC, water (100 ml) was added, and the mixture was extracted with ethyl acetate (100 ml × 2). The organic phase was washed with saturated sodium chloride solution, dried over anhydrous sodium sulphate, filtered and concentrated. The crude product was separated and purified by silica gel column chromatography (eluent: EA/PE = 0-25%), to afford the title compound (7 g, 90.78%).

Step 2:

**44B** (7 g, 19.16 mmol) was added to dichloromethane (45 ml) and then trifluoroacetic acid (15 ml) was added.

The mixture was reacted at room temperature for 2 h. After it was monitored that the reaction was completed, the reaction liquid was directly concentrated under reduced pressure to dryness to afford crude (10 g), which was directly used in the next reaction.

Step 3:

**44C** (crude, 7 g, 18.46 mmol), 2-(2,6-dioxopiperidin-3-yl)-5-fluoroisoindoline-1,3-dione (3.57 g, 12.92 mmol) and N,N-diisopropylethylamine (14.31 g, 110.76 mmol) were added to dimethyl sulphoxide (50 ml) and the mixture was reacted at 100°C for 1 h. Upon completion of reaction as monitored by LCMS, the reaction liquid was cooled to room temperature and water (200 ml) was added. The mixture was extracted with dichloromethane (100 ml × 3), then washed with saturated sodium chloride solution, dried over anhydrous sodium sulphate dried and filtered. The filtrate was concentrated under reduced pressure and the crude product was separated and purified by silica gel column chromatography (eluent: EA/DCM = 0-50%) to afford the title compound (3.5 g, 36%).

LC-MS (ESI): m/z = 521.1, 523.1$[M+H]^+$.

Step 4:

**44D** (2 g, 3.84 mmol), tert-butyl piperazine-1-carboxylate (1.07 g, 5.76 mmol), methanesulphonato(2-dicyclohexylphosphino-2',6'-di-isopropoxy-1,1'-biphenyl)(2-amino-1,1'-biphenyl-2-yl)palladium (II) (0.16 g, 0.19 mmol) and caesium carbonate(2.5 g, 7.67 mmol) were added to dioxane (60 ml), and the mixture was subjected to $N_2$ replacement and reacted at 100°C for 16 h. The reaction was monitored by LC-MS. The reaction liquid was cooled to room temperature. The reaction liquid was directly concentrated under reduced pressure and DCM/MeOH = 10 : 1 (60 ml) was added. The mixture was stirred and filtered. The filtrate was concentrated under reduced pressure and the residue was separated and purified by silica gel column chromatography (eluent for purification: MeOH/DCM = 0-10%), to afford the title compound (0.19 g, 7.9%)

LC-MS (ESI): m/z = 627.3 $[M+H]^+$.

Step 5:

**44E** (0.19 g, 0.3 mmol) was added to dichloromethane (6 ml), and then trifluoroacetic acid (2 ml) was added. The mixture was reacted at room temperature for 2 h, and the reaction solution was directly concentrated to dryness under reduced pressure to afford the title compound (0.24 g).

Step 6:

**1G** (0.1 g, 0.45 mmol) and **44F** (0.24 g) were added to acetonitrile and then N,N-diisopropylethylamine (0.35 g, 2.71 mmol) and potassium iodide (0.015 g, 0.09 mmol) were added. The mixture was warmed to 80°C and reacted for 2 h. After it was monitored that the reaction was completed, the reaction liquid was directly separated and purified by HPLC. After purification, the product was concentrated under reduced pressure to remove acetonitrile and then extracted with (dichloromethane : methanol = 10 : 1), and the organic phase was concentrated under reduced pressure to afford the title compound (30 mg, 9%). Preparative HPLC separation methods: 1. Instruments: waters 2767 (preparative liquid phase chromatographic instrument); chromatographic column: SunFire@ Prep C18 (19 mm × 250 mm) 2. The sample was dissolved in DMF and filtered with a 0.45 μm filter to prepare a sample solution. 3. Preparative chromatography conditions: a. composition of mobile phases A and B: mobile phase A: acetonitrile; mobile phase B: water (containing 0.1% ammonium acetate); b. gradient elution, mobile phase A: 5%-40%; c. flow rate: 12 mL/min. d. elution time: 20 min.

$^1$H NMR (400 MHz, DMSO-$d_6$) δ 11.81 (s, 1H), 11.05 (s, 1H), 8.39 (d, 1H), 8.21 (d, 1H), 7.74 (s, 1H), 7.67 (d, 1H), 7.62 (s, 1H), 7.36 (d, 1H), 7.30 - 7.21 (m, 3H), 5.06 (dd, 1H), 3.84-3.80(m, 2H), 3.64 (s, 2H), 3.37-3.30 (m, 2H), 3.26-3.22 (m, 4H), 2.99-2.93 (m, 1H), 2.92 - 2.82 (m, 1H), 2.63 - 2.51 (m, 8H), 2.04-1.94 (m, 3H), 1.74 - 1.62 (m, 2H), 1.18 (t, 3H).

LC-MS (ESI): m/z = 713.3$[M+H]^+$.

Example 45:

N-((1s,4s)-4-((2-(2,6-dioxopiperidin-3-yl)-6-fluoro-1,3-dioxoisoindolin-5-yl)amino)cyclohexyl)-5-(4-((7-ethyl-6-oxo-5,6-dihydro-1,5-naphthyridin-3-yl)methyl)piperazin-1-yl)picolinamide (Compound **45**)

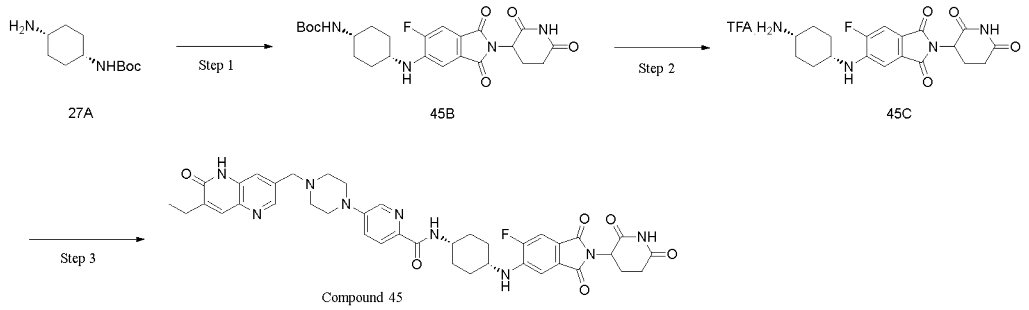


27A 45B 45C

Compound 45

Step 1:

**27A** (0.7 g, 3.27 mmol), 2-(2,6-dioxopiperidin-3-yl)-5,6-difluoroisoindoline-1,3-dione (1.06 g, 3.6 mmol) and N,N-diisopropylethylamine (1.27 g, 9.81 mmol) were added to dimethyl sulphoxide (10 ml), and the mixture was reacted at 100°C for 1 h. The reaction liquid was cooled to room temperature and water (40 ml) was added. The mixture was extracted with ethyl acetate (30 ml × 2) and the organic phase was washed with saturated sodium chloride solution, dried over anhydrous sodium sulphate and filtered. The filtrate was concentrated under reduced pressure and the crude product was separated and purified by silica gel column chromatography (20 g silica gel column, eluent: EA/PE = 0-60%), to afford the title compound (900 mg, 56%).

LC-MS (ESI): m/z = 489.2 $[M+H]^+$.

Step 2:

**45B** (300 mg, 0.61 mmol) was added to dichloromethane (6 ml) and then trifluoroacetic acid (2 ml) was added. The mixture was reacted at room temperature for 2 h. The reaction liquid was directly concentrated under reduced pressure to dryness to afford the title compound (0.35 g).

Step 3:

**1L** (145 mg, 0.37 mmol), **45C** (350 mg), 2-(7-azobenzotriazole)-N,N,N',N'-tetramethyluronium hexafluorophosphate (280 mg, 0.74 mmol) and N,N-diisopropylethylamine (200 mg, 1.51 mmol) were sequentially added to N,N-dimethylformamide (5 ml), and the mixture was reacted at room temperature for 5 h. The reaction liquid was directly separated and purified by HPLC. After purification, the product was concentrated under reduced pressure to remove acetonitrile and then extracted with (dichloromethane : methanol = 10 : 1), and the organic phase was concentrated under reduced pressure to afford the title compound (150 mg, 53%). Preparative HPLC separation methods: 1. Instruments: waters 2767 (preparative liquid phase chromatographic instrument); chromatographic column: SunFire@ Prep C18 (19 mm × 250 mm) 2. The sample was dissolved in DMF and filtered with a 0.45 μm filter to prepare a sample solution. 3. Preparative chromatography conditions: a. composition of mobile phases A and B: mobile phase A: acetonitrile; mobile phase B: water (containing 0.1% ammonium acetate); b. gradient elution, mobile phase A: 5%-40%; c. flow rate: 12 mL/min. d. elution time: 20 min.

$^1H$ NMR (400 MHz, DMSO-$d_6$) δ 11.84 (s, 1H), 11.06 (s, 1H), 8.41 (d, 1H), 8.29 (d, 1H), 8.10 (d, 1H), 7.86 (d, 1H), 7.75 (s, 1H), 7.63 (d, 1H), 7.58 (d, 1H), 7.42 (dd, 1H), 7.22 (d, 1H), 6.51-6.47 (m, 1H), 5.06 (dd, 1H), 4.07-4.03 (m, 1H), 3.80-3.74 (m, 1H), 3.66 (s, 2H), 3.37-3.33 (m, 4H), 2.95 - 2.82 (m, 1H), 2.63 - 2.51 (m, 8H), 2.06 - 1.96 (m, 1H), 1.89 - 1.63 (m, 8H), 1.18 (t, 3H).

LC-MS (ESI): m/z = 764.3 $[M+H]^+$.

Example 46:

1-(2-(2,6-dioxopiperidin-3-yl)-1,3-dioxoisoindolin-5-yl)-N-(5-(4-((7-ethyl-6-oxo-5,6-dihydro-1,5-naphthyridin-3 -yl)methyl)piperazin-1-yl)pyridin-2-yl)piperidine-4-carboxamide (Compound **46**)

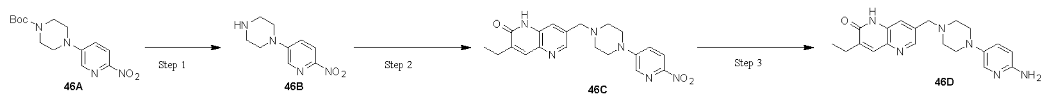


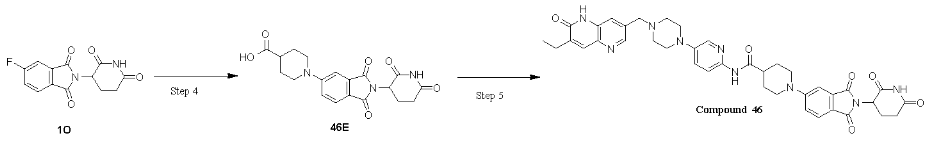


Step 1:

Compound **46A** (3 g, 9.73 mmol) was added to methanol (50 mL) and then 1,4-dioxane hydrochloride (6.06 g, 48.65 mmol) was added. The mixture was reacted overnight at room temperature. After the reaction was completed, the reaction liquid was filtered and washed. The filter cake was collected to afford compound **46B** (2.0 g, 99%).
LC-MS (ESI): m/z = 209.1[M+H]$^+$

Step 2:

Under nitrogen protection, compound **46B** (0.5 g, 2.4 mmol) was added to a flask, and dimethyl sulphoxide (2 mL) was added, followed by 7-(chloromethyl)-3-ethyl-1,2-dihydro-1,5-naphthyridin-2-one (0.53 g, 2.4 mmol), diisopropylethylamine (1.86 g, 14.4 mmol) and potassium iodide (0.08 g, 0.48 mmol). The mixture was warmed to 100°C and at this temperature, the mixture was reacted for 1 h. After the reaction was completed, the mixture was directly concentrated and the residue was separated and purified by flash column chromatography (eluent: MeOH : DCM = 0%-10%) to afford compound **46C** (0.6 g, 63%).
LC-MS (ESI): m/z = 395.1[M+H]$^+$

Step 3:

Compound **46C** (600 mg, 1.52 mmol) was dissolved in methanol (10 mL) and then palladium on carbon (0.16 g, 1.52 mmol) and hydrazine hydrate (0.076 g, 1.52 mmol) were added. The mixture was warmed to 75°C and reacted for 4 h. After the reaction was completed, the mixture was filtered and the organic phase was collected and concentrated. The residue was separated and purified by flash column chromatography (eluent: MeOH : DCM = 0%-10%) to afford compound **46D** (0.5 g, 90%).
LC-MS (ESI): m/z = 365.1[M+H]$^+$

Step 4:

Compound **1O** (500 mg, 1.81 mmol) and piperidine-4-carboxylic acid (0.23 g, 1.81 mmol) were added to dimethyl sulphoxide (10 mL) and then diisopropylethylamine (0.23 g, 1.81 mmol) was added. The mixture was warmed to 100°C and reacted overnight. After the reaction was completed, the reaction liquid was directly concentrated and the residue was separated and purified by flash column chromatography (eluent: MeOH : DCM = 0%-10%) to afford compound **46E** (0.49 g, 70%).
LC-MS (ESI): m/z = 386.1[M+H]$^+$

Step 5:

Compound **46D** (0.15 g, 0.41 mmol) and **46E** (0.16 g, 0.41 mmol) were dissolved in DMF (5 mL) and then 2-(7-aza-1H-benzotriazol-1-yl)-1,1,3,3-tetramethyluronium hexafluorophosphate (0.19 g, 0.49 mmol) and diisopropylethylamine (0.16 g, 1.23 mmol) were added. The mixture was reacted at room temperature for 1 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min. d. elution time: 20 min. retention time: 7.0 min, to afford compound **46** (50 mg, 17%).

LC-MS (ESI): m/z = 732.3 [M+H]$^+$

$^1$H NMR (400 MHz, DMSO-$d_6$) δ 11.82 (s, 1H), 11.05 (s, 1H), 10.23 (s, 1H), 8.40 (d, 1H), 7.99 (d, 1H), 7.91 (d, 1H), 7.75 (s, 1H), 7.66 (d, 1H), 7.62 (d, 1H), 7.40 - 7.33 (m, 2H), 7.26 (dd, 1H), 5.06 (dd, 1H), 4.10 (d, 2H), 3.64 (s, 2H), 3.14 (t, 4H), 3.05 - 2.97 (m, 2H), 2.63 - 2.52 (m, 8H), 2.05 - 1.97 (m, 1H), 1.86 (d, 2H), 1.74 - 1.63 (m, 2H), 1.24 (d, 2H), 1.18 (t, 3H).

Example 47:

2-(1-(2-(2,6-dioxopiperidin-3-yl)-1,3-dioxoisoindolin-5-yl)piperidin-4-yl)-N-(5-(4-((7-ethyl-6-oxo-5,6-dihydro-1,5-naphthyridin-3-yl)methyl)piperazin-1-yl)pyridin-2-yl)acetamide (Compound **47**)

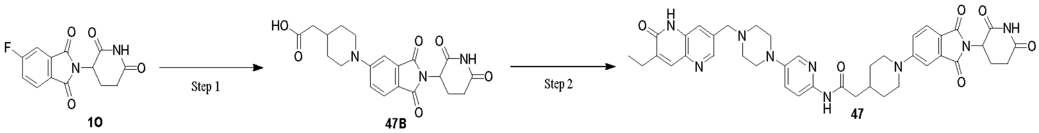


Step 1:

Compound **1O** (0.5 g, 1.81 mmol) and 4-piperidineacetic acid hydrochloride (0.33 g, 1.81 mmol) were added to dimethyl sulphoxide (10 mL) and then diisopropylethylamine (0.47 g, 3.62 mmol) was added. The mixture was warmed to 100°C and reacted overnight. After the reaction was completed, the reaction liquid was directly concentrated and the residue was separated and purified by flash column chromatography (eluent: MeOH : DCM = 0%-10%) to afford compound **47B** (0.5 g, 69%).
LC-MS (ESI): m/z = 400.2[M+H]$^+$

Step 2:

Compound **46D** (0.15 g, 0.41 mmol) and **47B** (0.16 g, 0.41 mmol) were dissolved in DMF (5 mL) and then 2-(7-aza-1H-benzotriazol-1-yl)-1,1,3,3-tetramethyluronium hexafluorophosphate (0.19 g, 0.49 mmol) and diisopropylethylamine (0.16 g, 1.23 mmol) were added. The mixture was reacted at room temperature for 1 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min. d. elution time: 20 min. retention time: 7.0 min, to afford compound **47** (60 mg, 20%).

LC-MS (ESI): m/z = 746.4[M+H]$^+$
$^1$H NMR (400 MHz, DMSO-$d_6$) δ 11.82 (s, 1H), 11.05 (s, 1H), 10.17 (s, 1H), 8.40 (d, 1H), 8.00 - 7.91 (m, 2H), 7.75 (s, 1H), 7.66 - 7.61 (m, 2H), 7.38 (dd, 1H), 7.30 (d, 1H), 7.23 (dd, 1H), 5.06 (dd, 1H), 4.03 (d, 2H), 3.64 (s, 2H), 3.14 (t, 4H), 2.98 (t, 2H), 2.62 - 2.52 (m, 8H), 2.30 (d, 2H), 2.09 - 1.97 (m, 2H), 1.75 (d, 2H), 1.25 (d, 3H), 1.18 (t, 3H).

Example 48:

N-(1-(2-(2,6-dioxopiperidin-3-yl)-3-oxoisoindolin-5-yl)piperidin-4-yl)-5-(4-((7-ethyl-6-oxo-5,6-dihydro-1,5-naphthyridin-3-yl)methyl)piperazin-1-yl)picolinamide (Compound **48**)

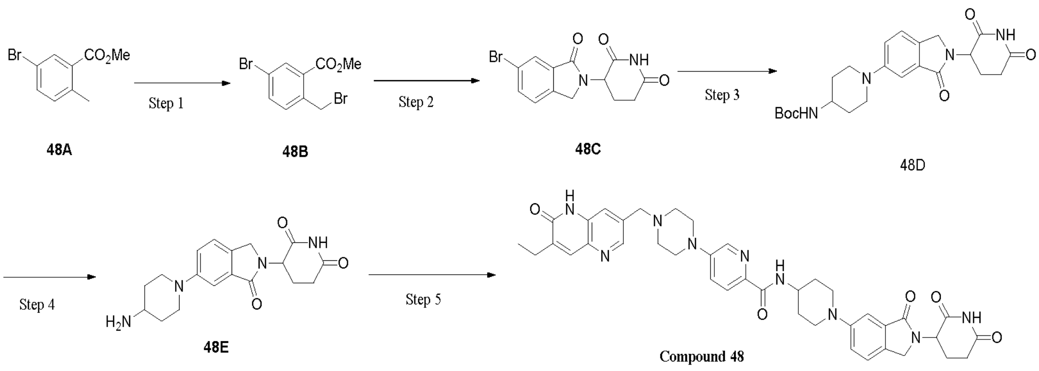


48A 48B 48C 48D 48E Compound 48

Step 1:

Methyl 5-bromo-2-methylbenzoate (5.00 g, 21.8 mmol), bromosuccinimide (4.28 g, 24.0 mmol) and dibenzoyl peroxide (1.06 g, 4.37 mmol) were added to a reaction flask and dry carbon tetrachloride (150 mL) was added. The mixture was warmed to 80°C and reacted overnight. Upon completion of reaction as monitored by TLC, water (200 mL) was added and the organic phase was separated. The aqueous phase was extracted with dichloromethane (50 mL × 3) and the organic phases were combined, dried over anhydrous sodium sulphate, filtered and concentrated. The resulting residue was separated and purified by silica gel column chromatography (petroleum ether: ethyl acetate (v/v) = 100 : 2) to afford compound **48B** (4.9 g, 72.9 %) as a colorless oil.

Step 2:

Compound **48B** (2.00 g, 6.49 mmol) was dissolved in N, N-dimethylformamide (50 mL) and then N,N-diisopropylethylamine (2.51 g, 19.5 mmol) and 3-amino-2,6-piperidinedione hydrochloride (1.28 g, 7.79 mmol) were sequentially added. The mixture was warmed to 80°C and reacted for 30 hours. Upon completion of reaction as monitored by TLC, water (50 mL) was added and the mixture was stirred for 5 minutes and then directly filtered. The solid was washed with dichloromethane solution (20 mL × 3, containing 5% methanol) and dried to afford silver grey crude compound **48C** (1.39 g, 66.2%).
LC-MS (ESI): 323.0 $[M+H]^+$;

Step 3:

Compound **48C** (100 mg, 0.310 mmol), Pd-G3-Ruphos (25.3 mg, 0.0310 mmol), 4-tertbutoxycarbonylaminopiperidine (80.6 mg, 0.403 mmol) and caesium carbonate (302 mg, 0.929 mmol) were added to a microwave reaction vial and then dry 1,4-dioxane (6 mL) was added. The mixture was heated to 120°C in a microwave reactor and reacted for 2 hours. Upon completion of reaction as monitored by TLC, the reaction liquid was directly dried with suction to remove the solvent. The solid residue was separated and purified by silica gel column chromatography (dichloromethane: methanol (v/v) = 10 : 1) to afford a brownish yellow mixture **48D** (110 mg).
LC-MS (ESI): 443.2 $[M+H]^+$,

Step 4:

Compound **48D** (110 mg), as a mixture, was dissolved in dichloromethane (3 mL) and trifluoroacetic acid (1 mL) was added at room temperature. The mixture was stirred at room temperature for another 30 minutes. Upon completion of reaction as monitored by TLC, the reaction liquid was directly dried with suction to remove the solvent. Ethyl acetate (10 mL) and water (5 mL) were added again. The aqueous phase and organic phase were separated. The organic phase was washed with water (2 mL × 3) and the aqueous phases were combined and back-flushed once with ethyl acetate (5 mL). The aqueous phase was lyophilized to afford a brownish yellow solid compound, i.e., the trifluoroacetate of compound **48E** (crude, 33 mg).
LC-MS (ESI): 343.2 $[M+H]^+$,

Step 5:

Compound **1L** (50 mg, 0.127 mmol), the trifluoroacetate of **48E** (69.7 mg, 0.153 mmol), 2-(7-azobenzotriazole)-N,N,N',N'-tetramethyluronium hexafluorophosphate (96.8 mg, 0.254 mmol) and N,N-diisopropylethylamine (82.1

mg, 0.636 mmol) were added to a round bottom flask and then N,N-dimethylformamide (5 mL) was added. The mixture was stirred and reacted at room temperature for about 2 hours. Upon completion of reaction as monitored by TLC, water (30 mL) was added and the aqueous phase was extracted with dichloromethane (5 mL × 8, containing 10% methanol). The organic phases were combined, dried over anhydrous sodium sulphate, filtered and concentrated. The resulting residue was separated and purified by a thick silica gel plate (dichloromethane : ethyl acetate : methanol (v/v/v) = 10 : 1 : 1) to afford compound **48** (14 mg, 15.4%).

LC-MS (ESI): 718.3 $[M+H]^+$;
$^1$H NMR (400 MHz, Methanol-*d4*) $\delta$ 8.49 (d, 1H), 8.23 (d, 1H), 7.98 (d, 1H), 7.83 (s, 1H), 7.76 (s, 1H), 7.61 (s, 3H), 7.44 - 7.39 (m, 2H), 7.30 (ddd, 2H), 5.14 (dd, 1H), 4.41 (d, 1H), 4.10 (d, 1H), 3.89 - 3.67 (m, 4H), 3.44 (s, 3H), 3.12 - 2.94 (m, 2H), 2.91 - 2.81 (m, 2H), 2.83 - 2.64 (m, 5H), 2.25 - 2.20 (m, 1H), 2.19 - 2.10 (m, 2H), 2.09 - 1.98 (m, 1H), 1.81 (q, 2H), 1.62 (t, 1H), 1.44 - 1.39 (m, 2H), 1.31(t, 3H).

Example 49:

N-(1-(2-(2,6-dioxopiperidin-3-yl)-1,3-dioxoisoindolin-5-yl)piperidin-4-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **49**)

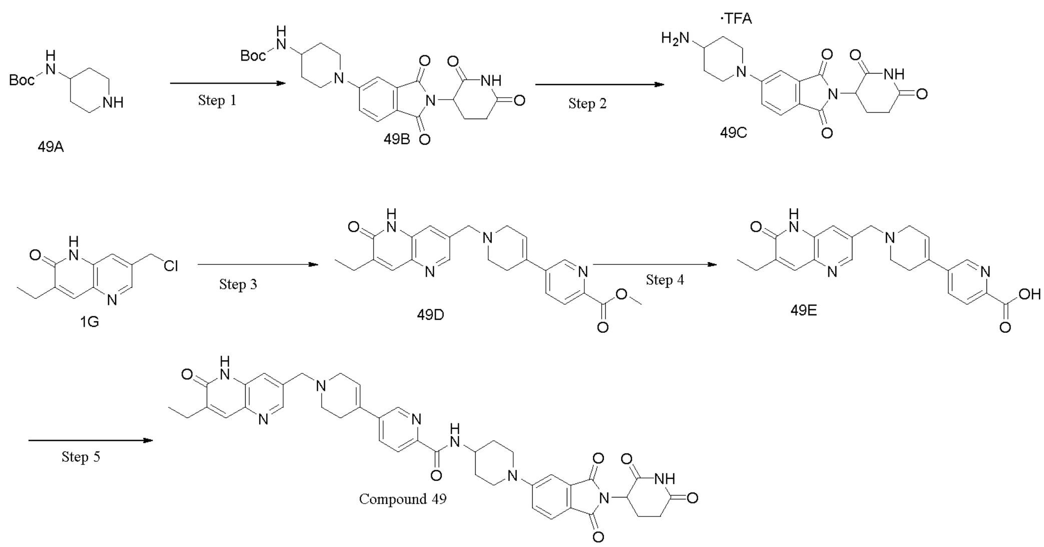


Step 1:

Compound **49A** (1 g, 5 mmol) and 2-(2,6-dioxopiperidin-3-yl)-5-fluoroisoindoline-1,3-dione (1.38 g, 5 mmol) were dissolved in dimethyl sulphoxide (20 mL) and then N,N-diisopropylethylamine (1.29 g, 10 mmol) was added. The mixture was reacted at 100°C for 1 h. After the reaction was completed, water (20 ml) and ethyl acetate (60 mL) were added and the organic phase was separated, washed with water (10 mL × 2) and saturated brine (10 mL × 1), dried over anhydrous sodium sulphate and concentrated. The residue was separated and purified by silica gel column chromatography (eluent: methanol : dichloromethane (v/v) = 0%-10%), to afford product **49B** (1.8 g, 78.1%).
LCMS m/z = 457.2 $[M+H]^+$

Step 2:

Compound **49B** (200 mg, 0.44 mmol) was dissolved in dichloromethane (6 mL), trifluoroacetic acid (2 mL) was added, and the mixture was reacted at room temperature for 30 min. After the reaction was completed, the reaction liquid was concentrated to afford crude product **49C** (200 mg, 100.0%).
LCMS m/z = 357.1 $[M+H]^+$

Step 3:

Compound **1G** (1.48 g, 6.66 mmol) and methyl 1',2',3',6'-tetrahydro-[3,4'-bipyridine]-6-carboxylic acid hydrochloride (1.69 g, 6.66 mmol) were dissolved in acetonitrile (30 mL) and then potassium iodide (150 mg) and N,N-diisopropylethylamine (8.6 g, 66.6 mmol) were added. The mixture was reacted overnight at 80°C. After the reaction was completed, the reaction liquid was concentrated and the residue was separated and purified by silica gel column chromatography (eluent: methanol : dichloromethane (v/v) = 0%-10%), to afford product **49D** (1.5 g, 55.3%).
LCMS m/z = 405.1 $[M+H]^+$

Step 4:

Compound **49D** (1.5 g, 3.7 mmol) and lithium hydroxide (0.444 g, 18.5 mmol) were dissolved in a mixed solvent of methanol (10 mL) and water (10 mL). The mixture was reacted at room temperature for 2 hours. After the reaction was completed, the reaction liquid was adjusted to pH = 3-4 with 4 N hydrochloric acid, and separated and purified by C18 column chromatography (eluent: acetonitrile : 0.5% aqueous trifluoroacetic acid solution (v/v) = 0%-50%), to afford product **49E** (1.3 g, 90.1%).
LCMS m/z = 391.1 $[M+H]^+$

Step 5:

Compound **49E** (100 mg, 0.26 mmol), benzotriazole-N,N,N',N'-tetramethyluronium hexafluorophosphate (114 mg, 0.3mmol), compound **49C** (200 mg, 0.44 mmol) and N,N-diisopropylethylamine (65 mg, 0.5 mmol) were dissolved in DMF (2 mL) and the mixture was reacted at room temperature for 1 hour. After the reaction was completed, the reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **49** (50 mg, yield 26.5%, retention time: about 7.5 min).

$^1$H NMR (400 MHz, DMSO-*d6*) δ11.83 (s, 1H), 11.05 (s, 1H), 8.68 (s, 1H), 8.61 (d, 1H), 8.41 (d, 1H), 8.04 - 7.97 (m, 2H), 7.75 (s, 1H), 7.67 (d, 2H), 7.35 (d, 1H), 7.30 - 7.25 (m, 1H), 6.41 (s, 1H), 5.18 - 4.94 (m, 1H), 4.21 - 4.01 (m, 3H), 3.72 (s, 2H), 3.22 - 3.03 (m, 4H), 2.96 - 2.82 (m, 1H), 2.74 - 2.67 (m, 2H), 2.63 - 2.51 (m, 6H), 2.05 - 1.97 (m, 1H), 1.91 - 1.84 (m, 2H), 1.80 - 1.68 (m, 2H), 1.20 - 1.17 (m, 3H).

MS M/Z (ESI): m/z = 729.3 $[M+1]^+$.

Example 50:

N-(1-(2-(2,6-dioxopiperidin-3-yl)-6-fluoro-1,3-dioxoisoindolin-5-yl)piperidin-4-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **50**)

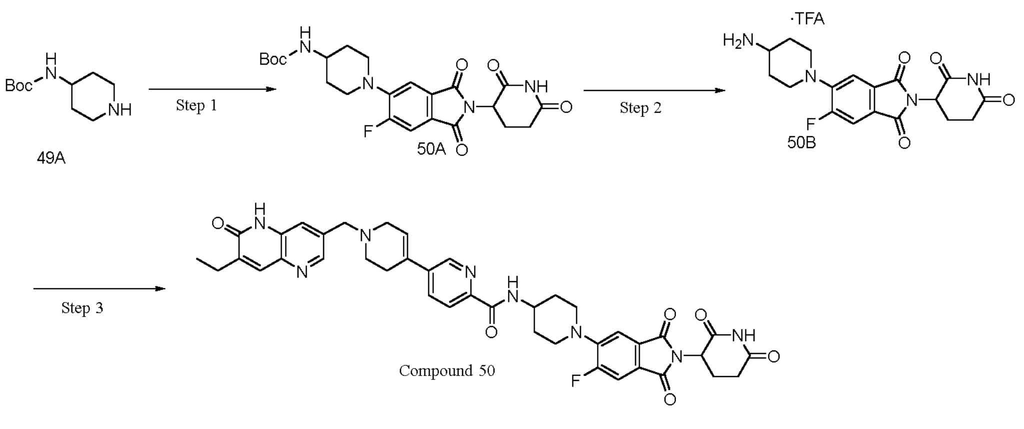


Step 1:

Compound **49A** (1 g, 5 mmol) and 2-(2,6-dioxopiperidin-3-yl)-5,6-fluoroisoindoline-1,3-dione (1.47 g, 5 mmol) were dissolved in dimethyl sulphoxide (20 mL) and then N,N-diisopropylethylamine (1.29 g, 10 mmol) was added. The

mixture was reacted at 100°C for 1 h. After the reaction was completed, water (20 ml) and ethyl acetate (60 mL) were added and the organic phase was separated, washed with water (10 mL × 2) and saturated brine (10 mL × 1), dried over anhydrous sodium sulphate and concentrated. The residue was separated and purified by silica gel column chromatography (eluent: methanol : dichloromethane (v/v) = 0%-10%), to afford product **50A** (1.7 g, 72.4%).
LCMS m/z = 475.2 $[M+H]^+$

Step 2:

Compound **50A** (200 mg, 0.42 mmol) was dissolved in dichloromethane (6 mL), trifluoroacetic acid (2 mL) was added, and the mixture was reacted at room temperature for 30 min. After the reaction was completed, the reaction liquid was concentrated to afford crude product **50B** (200 mg, 100.0%).
LCMS m/z = 375.1 $[M+H]^+$

Step 3:

Compound **49E** (100 mg, 0.26 mmol), benzotriazole-N,N,N',N'-tetramethyluronium hexafluorophosphate (114 mg, 0.3mmol), compound **50B** (200 mg, 0.42 mmol) and N,N-diisopropylethylamine (65 mg, 0.5 mmol) were dissolved in DMF (2 mL) and the mixture was reacted at room temperature for 1 hour. After the reaction was completed, the reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **50** (48 mg, yield 24.7%, retention time: about 7.4 min).

$^1H$ NMR (400 MHz, DMSO-$d_6$) δ11.82 (s, 1H), 11.08 (s, 1H), 8.71 (s, 1H), 8.65 (d, 1H), 8.41 (d, 1H), 8.04 - 7.97 (m, 2H), 7.76 - 7.69 (m, 2H), 7.65 (s, 1H), 7.49 (d, 1H), 6.42 (s, 1H), 5.21 - 4.91 (m, 1H), 4.11 - 3.96 (m, 1H), 3.73 (s, 2H), 3.68 - 3.59 (m, 2H), 3.22 - 3.14 (m, 2H), 3.10 - 3.00 (m, 2H), 2.95 - 2.83 (m, 1H), 2.75 - 2.67 (m, 2H), 2.62 - 2.52 (m, 6H), 2.06 - 2.01 (m, 1H), 1.93 - 1.85 (m, 4H), 1.19 - 1.18 (m, 3H).
MS M/Z (ESI): m/z = 747.3 $[M+H]^+$

Example 51:

N-((3S)-1-(2-(2,6-dioxopiperidin-3-yl)-6-fluoro-1,3-dioxoisoindolin-5-yl)pyrrolidin-3-yl)-5-(4-((7-ethyl-6-oxo-5,6-dihydro-1,5-naphthyridin-3-yl)methyl)piperazin-1-yl)picolinamide (Compound **51**)

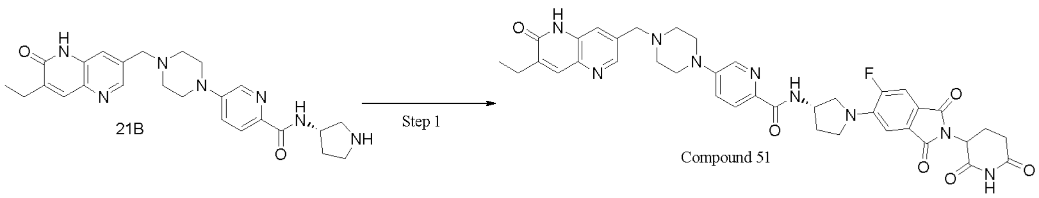


Step 1:

Compound **21B** (50 mg, 0.11 mmol) and 2-(2,6-dioxopiperidin-3-yl)-5,6-difluoroisoindoline-1,3-dione (38 mg, 0.13 mmol) were added to dimethyl sulphoxide (2 mL) and then N,N-diisopropylethylamine (28 mg, 0.22 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **51** (35 mg, yield 43.3%, retention time: about 7.0 min).

$^1H$ NMR (400 MHz, DMSO-$d_6$) δ11.84 (s, 1H), 11.06 (s, 1H), 8.61 (d, 1H), 8.40 (d, 1H), 8.26 (d, 1H), 7.86 (d, 1H), 7.75 (s, 1H), 7.66 - 7.56 (m, 2H), 7.45 - 7.36 (m, 1H), 7.07 (d, 1H), 5.13 - 5.01 (m, 1H), 4.68 - 4.50 (m, 1H), 3.93 - 3.79 (m, 1H), 3.75 - 3.57 (m, 5H), 3.41 - 3.32 (m, 4H), 2.96 - 2.80 (m, 1H), 2.63 - 2.52 (m, 8H), 2.26 - 1.98 (m, 3H), 1.22 - 1.13 (m, 3H).
MS M/Z (ESI): m/z = 736.2 $[M+H]^+$

Example 52:

N-(2-(2-(2,6-dioxopiperidin-3-yl)-6-fluoro-1,3-dioxoisoindolin-5-yl)-2-azaspiro[3.3]heptan-6-yl)-5-(4-((7-ethyl-6-oxo-5,6-dihydro-1,5-naphthyridin-3-yl)methyl)piperazin-1-yl)picolinamide (Compound **52**)

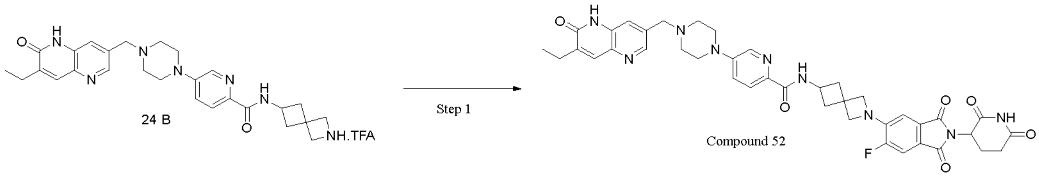


Step 1:

Crude compound **24B** (300 mg, 0.51 mmol) and 2-(2,6-dioxopiperidin-3-yl)-5,6-difluoroindoline-1,3-dione (150 mg, 0.51 mmol) were added to dimethyl sulphoxide (3 mL) and then N,N-diisopropylethylamine (133 mg, 1.02 mmol) was added. The mixture was reacted at 100°C for 1 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **52** (50 mg, yield 12.8%, retention time: about 7.4 min).

$^1$H NMR (400 MHz, DMSO-$d_6$) $\delta$ 11.84 (s, 1H), 11.07 (s, 1H), 8.62 (d, 1H), 8.41 (d, 1H), 8.27 (d, 1H), 7.82 (d, 1H), 7.75 (s, 1H), 7.65 - 7.55 (m, 2H), 7.39 (d, 1H), 6.89 (d, 1H), 5.13 - 4.98 (m, 1H), 4.42 - 4.30 (m, 1H), 4.25 (s, 2H), 4.12 (s, 2H), 3.66 (s, 2H), 3.38 - 3.32 (m, 4H), 2.93 - 2.82 (m, 1H), 2.63 - 2.51 (m, 10H), 2.46 - 2.40 (m, 2H), 2.05 - 1.95 (m, 1H), 1.20 - 1.17 (m, 3H).
MS M/Z (ESI): m/z = 762.3 $[M+H]^+$

Example 53:

N-((3S)-1-(2-(2,6-dioxopiperidin-3-yl)-1,3-dioxoisoindolin-5-yl)pyrrolidin-3-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **53**)

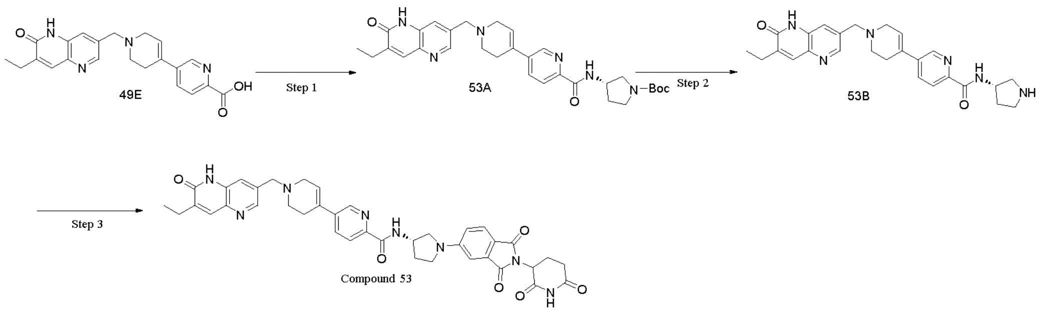


Step 1:

Compound **49E** (0.5 g, 1.28 mmol) and tert-butyl (S)-3-aminopyrrolidine-1-carboxylate (283 mg, 1.5 mmol) were dissolved in DMF (5 mL), benzotriazole-N,N,N',N'-tetramethyluronium hexafluorophosphate (570 mg, 1.5 mmol) and N,N-diisopropylethylamine (330 mg, 2.54 mmol) were added, and the mixture was stirred and reacted at room temperature for 1 h. After the reaction was completed, water (10 ml) and ethyl acetate (10 mL) were added. The organic phase was separated, and the aqueous phase was extracted with ethyl acetate (10 mL × 2). The organic phases were combined, dried over anhydrous sodium sulphate and concentrated, to afford crude product **53A** (750 mg, 100.0%).
LCMS m/z = 559.3 $[M+H]^+$

Step 2:

Compound **53A** (700 mg, 1.28 mmol) was dissolved in dichloromethane (6 mL) and trifluoroacetic acid (2 mL) was added. The mixture was reacted at room temperature for 30 min. After the reaction was completed, the reaction liquid was concentrated, adjusted to pH > 7 with triethylamine, and then purified by C18 column chromatography (acetonitrile:0.1% ammonia solution (v/v) = 50%) to afford product **53B** (400 mg, 68.2%).
LCMS m/z = 459.2 $[M+H]^+$

Step 3:

Compound **53B** (100 mg, 0.22 mmol) and 2-(2,6-dioxopiperidin-3-yl)-5-fluoroisoindoline-1,3-dione (61 mg, 0.22 mmol) were added to DMF (3 mL) and then N,N-diisopropylethylamine (57 mg, 0.44 mmol) was added. The mixture was reacted at 100°C for 2 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **53** (45 mg, yield 28.7%, retention time: about 7.0 min).

$^1H$ NMR (400 MHz, DMSO-$d_6$) δ 11.82 (s, 1H), 11.04 (s, 1H), 8.94 (d, 1H), 8.67 (s, 1H), 8.41 (d, 1H), 8.02 (s, 2H), 7.76 (s, 1H), 7.65 (d, 2H), 6.93 (d, 1H), 6.89 - 6.78 (m, 1H), 6.42 (s, 1H), 5.12 - 4.96 (m, 1H), 4.76 - 4.52 (m, 1H), 3.80 - 3.67 (m, 3H), 3.67 - 3.57 (m, 1H), 3.55 - 3.44 (m, 2H), 3.17 (s, 2H), 2.97 - 2.81 (m, 1H), 2.77 - 2.65 (m, 2H), 2.63 - 2.52 (m, 6H), 2.36 - 2.14 (m, 2H), 2.08 - 1.97 (m, 1H), 1.20 - 1.17 (m, 3H).
MS M/Z (ESI): m/z = 715.3 $[M+H]^+$

Example 54:

N-((3S)-1-(2-(2,6-dioxopiperidin-3-yl)-6-fluoro-1,3-dioxoisoindolin-5-yl)pyrrolidin-3-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **54**)

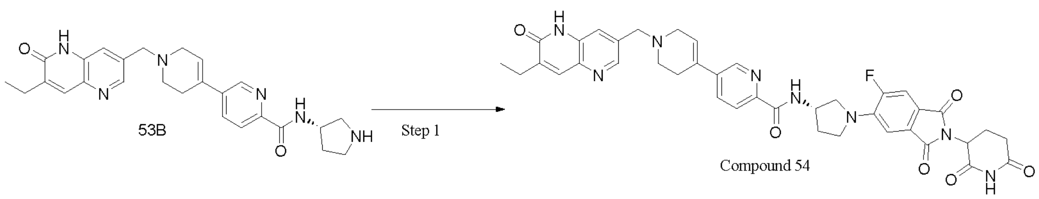


Step 1:

Compound **53B** (100 mg, 0.22 mmol) and 2-(2,6-dioxopiperidin-3-yl)-5,6-difluoroindoline-1,3-dione (65 mg, 0.22 mmol) were added to DMF (3 mL) and then N,N-diisopropylethylamine (57 mg, 0.44 mmol) was added. The mixture was reacted at 100°C for 2 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **54** (45 mg, yield 28.0%, retention time: about 7.0 min).

$^1H$ NMR (400 MHz, DMSO-$d_6$) δ 11.81 (s, 1H), 11.06 (s, 1H), 8.93 (d, 1H), 8.67 (s, 1H), 8.41 (d, 1H), 8.12 - 7.91 (m, 2H), 7.75 (s, 1H), 7.66 - 7.55 (m, 2H), 7.08 (d, 1H), 6.42 (s, 1H), 5.14 - 5.00 (m, 1H), 4.69 - 4.50 (m, 1H), 3.95 - 3.83 (m, 1H), 3.77 - 3.53 (m, 5H), 3.17 (s, 2H), 2.94 - 2.81 (m, 1H), 2.74 - 2.65 (m, 2H), 2.63 - 2.51 (m, 6H), 2.26 - 2.13 (m, 2H), 2.06 - 1.97 (m, 1H), 1.20 - 1.16 (m, 3H).
MS M/Z (ESI): m/z = 733.3 $[M+H]^+$

Example 55:

N-(2-(2-(2,6-dioxopiperidin-3-yl)-1,3-dioxoisoindolin-5-yl)-2-azaspiro[3.3]heptan-6-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **55**)

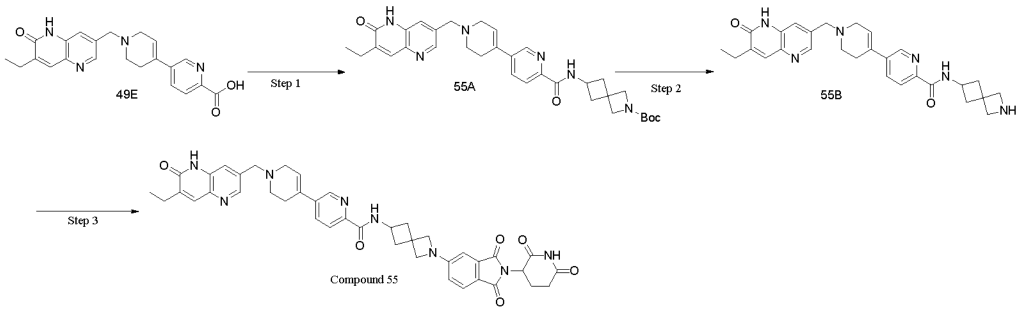

49E
55A
55B
Compound 55

Step 1:

Compound **49E** (0.5 g, 1.28 mmol) and tert-butyl 6-amino-2-azaspiro[3.3]heptane-2-carboxylate (318 mg, 1.5 mmol) were dissolved in DMF (5 mL), benzotriazole-N,N,N',N'-tetramethyluronium hexafluorophosphate (570 mg, 1.5 mmol) and N,N-diisopropylethylamine (330 mg, 2.54 mmol) were added, and the mixture was stirred and reacted at room temperature for 1 h. After the reaction was completed, water (10 ml) and ethyl acetate (10 mL) were added. The organic phase was separated, and the aqueous phase was extracted with ethyl acetate (10 mL × 2). The organic phases were combined, dried over anhydrous sodium sulphate and concentrated, to afford crude product **55A** (750 mg, 100.0%).
LCMS m/z = 585.3 $[M+H]^+$

Step 2:

Compound **55A** (750 mg, 1.28 mmol) was dissolved in dichloromethane (6 mL) and trifluoroacetic acid (2 mL) was added. The mixture was reacted at room temperature for 30 min. After the reaction was completed, the reaction liquid was concentrated, adjusted to pH > 7 with triethylamine, and then purified by C18 column chromatography (acetonitrile : 0.1% ammonia solution (v/v) = 50%) to afford product **55B** (300 mg, 48.3%).
LCMS m/z = 485.2 $[M+H]^+$

Step 3:

Compound **55B** (150 mg, 0.31 mmol) and 2-(2,6-dioxopiperidin-3-yl)-5-fluoroisoindoline-1,3-dione (86 mg, 0.31 mmol) were added to DMF (3 mL) and then N,N-diisopropylethylamine (80 mg, 0.62 mmol) was added. The mixture was reacted at 100°C for 2 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **55** (35 mg, yield 15.2%, retention time: about 7.0 min).

$^1H$ NMR (400 MHz, DMSO-$d_6$) δ 11.83 (s, 1H), 11.06 (s, 1H), 8.96 (d, 1H), 8.71 (d, 1H), 8.42 (d, 1H), 8.04 - 7.94 (m, 2H), 7.77 (s, 1H), 7.64 (d, 2H), 6.77 (d, 1H), 6.64 (d, 1H), 6.43 (s, 1H), 5.15 - 4.97 (m, 1H), 4.51 - 4.35 (m, 1H), 4.13 (s, 2H), 4.00 (s, 2H), 3.73 (s, 2H), 3.17 (s, 2H), 2.97 - 2.81 (m, 1H), 2.75 - 2.64 (m, 2H), 2.61 - 2.53 (m, 7H), 2.50 - 2.46 (m, 3H), 2.10 - 1.85 (m, 1H), 1.21 - 1.17 (m, 3H).
MS M/Z (ESI): m/z = 741.3 $[M+H]^+$

Example 56:

N-(2-(2-(2,6-dioxopiperidin-3-yl)-6-fluoro-1,3-dioxoisoindolin-5-yl)-2-azaspiro[3.3]heptan-6-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **56**)

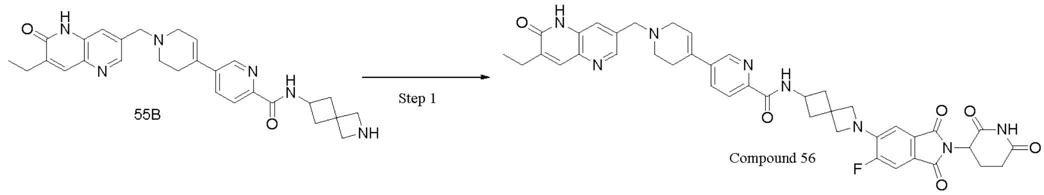


Step 1:

Compound **55B** (150 mg, 0.31 mmol) and 2-(2,6-dioxopiperidin-3-yl)-5,6-difluoroindoline-1,3-dione (91 mg, 0.31 mmol) were added to DMF (3 mL) and then N,N-diisopropylethylamine (80 mg, 0.62 mmol) was added. The mixture was reacted at 100°C for 2 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **56** (40 mg, yield: 17.0%, retention time: about 7.0 min).

$^1$H NMR (400 MHz, DMSO-$d_6$) δ 11.84 (s, 1H), 11.07 (s, 1H), 8.94 (d, 1H), 8.70 (s, 1H), 8.42 (d, 1H), 8.05 - 7.92 (m, 2H), 7.77 (s, 1H), 7.65 (s, 1H), 7.59 (d, 1H), 6.89 (d, 1H), 6.43 (s, 1H), 5.15 - 5.02 (m, 1H), 4.46 - 4.36 (m, 1H), 4.26 (s, 2H), 4.13 (s, 2H), 3.73 (s, 2H), 3.17 (s, 2H), 2.94 - 2.84 (m, 1H), 2.77 - 2.68 (m, 2H), 2.61 - 2.53 (m, 7H), 2.50 - 2.43 (m, 3H), 2.07 - 1.97 (m, 1H), 1.21 - 1.17 (m, 3H).
MS M/Z (ESI): m/z = 759.3 $[M+H]^+$

Example 57:

N-(1-(1-(2-(2,6-dioxopiperidin-3-yl)-1,3-dioxoisoindolin-5-yl)piperidin-4-yl)-1H-pyrazol-4-yl)-5-(4-((7-ethyl-6-oxo-5,6-dihydro-1,5-naphthyridin-3-yl)methyl)piperazin-1-yl)picolinamide (Compound **57**)

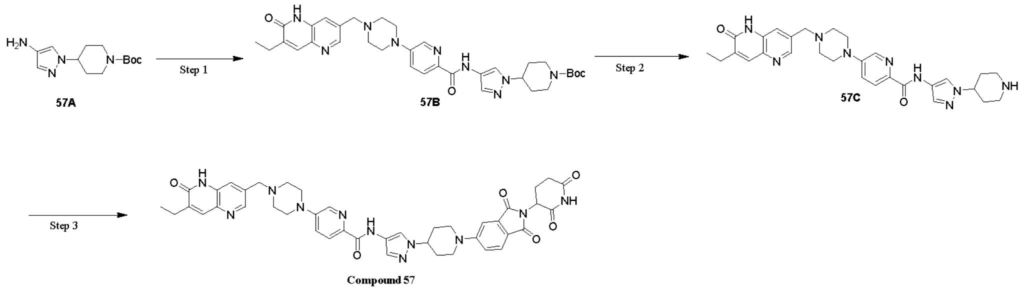


Step 1:

Intermediate **1L** (100 mg, 0.25 mmol) was dissolved in N,N-dimethylformamide (2 ml), compound **57A** (102 mg, 0.38 mmol), 2-(7-azobenzotriazole)-N,N,N',N'-tetramethyluronium hexafluorophosphate (190 mg, 0.5 mmol) and N,N-diisopropylethylamine (130 mg, 1 mmol) were added to the reaction system, and the mixture was reacted overnight at room temperature. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford compound **57B** (100 mg, 41%).
LC-MS (ESI): m/z = 642.3 $[M+H]^+$

Step 2:

Compound **57B** (0.1 g, 0.16 mmol) was added to a reaction flask, dichloromethane (3 ml)/trifluoroacetic acid

(1 ml) were added, and the mixture was reacted at room temperature for 2 h. After the reaction was completed, the reaction liquid was directly concentrated, to afford crude compound **57C** (0.1 g).
LC-MS (ESI): m/z = 542.3 [M+H]+

Step 3:

Crude **57C** (100 mg, 0.16 mmol, obtained in the step above) was dissolved in N-methylpyrrolidone (5 ml) and 2-(2,6-dioxopiperidin-3-yl)-5-fluoroisoindoline-1,3-dione (88 mg, 0.32 mmol) was added. The reaction system was placed under microwave and reacted at 150°C for 1 h. After the reaction was completed, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford compound **57** (50 mg, 40%).

LC-MS (ESI): m/z = 798.3 [M+H]+
$^1$H NMR (400 MHz, DMSO-$d_6$) δ = 11.83 (s, 1H), 11.06 (s, 1H), 10.49 (s, 1H), 8.41 (s, 1H), 8.32 (s, 1H), 8.10 (s, 1H), 7.91 (d, 1H), 7.74 (d, 2H), 7.69 (d, 1H), 7.63 (s, 1H), 7.44 (d, 1H), 7.40 (s, 1H), 7.32 (d, 1H), 5.07 (dd, 1H), 4.48 (t, 1H), 4.18 (d, 2H), 3.66 (s, 2H), 3.38 (s, 4H), 3.18 (t, 4H), 2.57 (d, 6H), 2.09 (d, 3H), 2.01 - 1.93 (m, 3H), 1.24 (s, 2H), 1.19 (t, 3H).

Example 58:

N-(1-(1-(2-(2,6-dioxopiperidin-3-yl)-1,3-dioxoisoindolin-5-yl)piperidin-4-yl)-1H-pyrazol-4-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **58**)

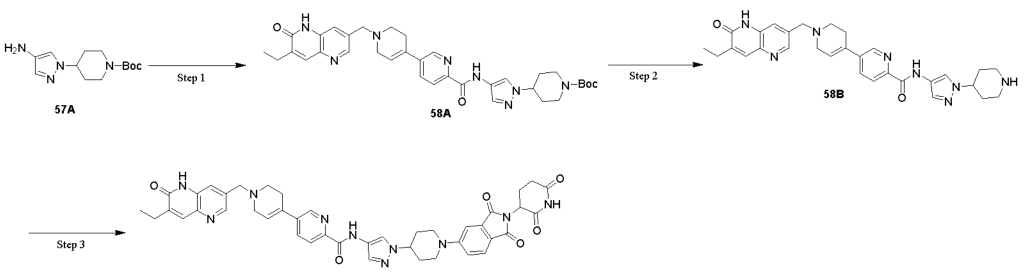

57A
58A
58B
Compound 58

Step 1:

Intermediate **49E** (100 mg, 0.26 mmol) was dissolved in N,N-dimethylformamide (2 ml), compound **57A** (104 mg, 0.38 mmol), 2-(7-azobenzotriazole)-N,N,N',N'-tetramethyluronium hexafluorophosphate (198 mg, 0.52 mmol) and N,N-diisopropylethylamine (135 mg, 1.04 mmol) were added to the reaction system, and the mixture was reacted overnight at room temperature. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford compound **58A** (100 mg, 60%).
LC-MS (ESI): m/z = 639.3[M+H]+

Step 2:

Compound **58A** (0.1 g, 0.16 mmol) was added to a reaction flask, dichloromethane (3 ml)/trifluoroacetic acid (1 ml) were added, and the mixture was reacted at room temperature for 2 h. After the reaction was completed, the

reaction liquid was directly concentrated, to afford crude compound **58B** (0.1 g).
LC-MS (ESI): m/z = 539.3[M+H]+

Step 3:

Crude **58B** (100 mg, 0.16 mmol, obtained in the step above) was dissolved in N-methylpyrrolidone (5 ml) and 2-(2,6-dioxopiperidin-3-yl)-5-fluoroisoindoline-1,3-dione (88 g, 0.32 mmol) was added. The reaction system was placed under microwave and reacted at 150°C for 1 h. After the reaction was completed, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford compound **58** (50 mg, 40%).

LC-MS (ESI): m/z = 795.3[M+H]+
$^1$H NMR (400 MHz, DMSO-$d_6$) δ11.83 (s, 1H), 11.07 (s, 1H), 10.83 (s, 1H), 8.75 (s, 1H), 8.42 (d, 1H), 8.15 (s, 1H), 8.05 (s, 2H), 7.77 (s, 1H), 7.76 (s, 1H), 7.69 (d, 1H), 7.65 (s, 1H), 7.41 (d, 1H), 7.35 - 7.28 (m, 1H), 6.45 (s, 1H), 5.08 (dd, 1H), 4.57 - 4.43 (m, 1H), 4.18 (d, 2H), 3.73 (s, 2H), 3.18 (s, 4H), 2.72 (t, 2H), 2.57 (d, 4H), 2.06 (dd, 3H), 1.98 (dd, 3H), 1.23 (s, 2H), 1.19 (t, 3H).

Example 59:

N-((1r,3r)-3-((2-(2,6-dioxopiperidin-3-yl)-1,3-dioxoisoindolin-5- yl)oxy)cyclobutyl)-5-(4-((7-ethyl-6-oxo-5,6-dihydro-1,5-naphthyridin-3-yl)methyl)piperazin-1-yl)picolinamide (Compound **59**)

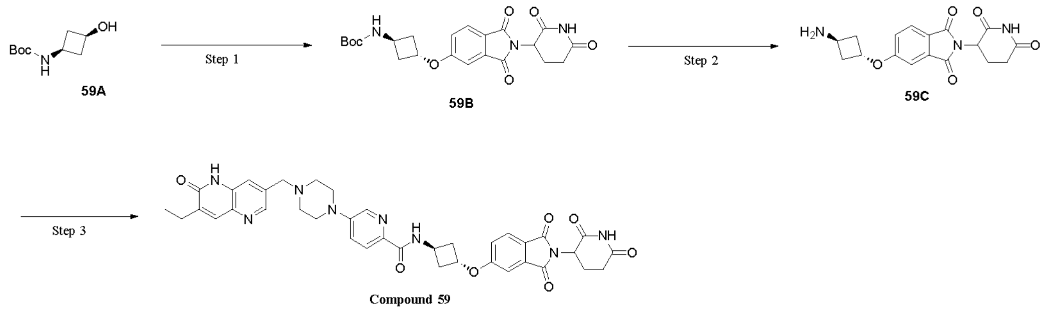


Step 1:

**59A** (500 mg, 2.67 mmol) was dissolved in anhydrous tetrahydrofuran (10 ml) and under nitrogen, 2-(2,6-dioxopiperidin-3-yl)-5-hydroxyisoindoline-1,3-dione (733 mg, 2.67 mmol) and triphenylphosphine (839 mg, 3.2 mmol) were added. The reaction system was placed in a 60°C oil bath pan, and diethyl azodicarboxylate (557 mg, 3.2 mmol) was added. The mixture was reacted at 60°C for another 3 h. After the reaction was completed, the reaction was quenched with water (20 ml), and extracted with ethyl acetate (20 ml × 3). The organic phases were combined, dried over anhydrous sodium sulphate, filtered and concentrated. The residue was separated and purified by flash column chromatography (eluent: ethyl acetate : petroleum ether = 0%-20%), to afford compound **59B** (0.3 g, 25%).
LC-MS (ESI): m/z = 442.1[M-H]+

Step 2:

Compound **59B** (0.3 g, 0.68 mmol) was added to a reaction flask, dichloromethane (3 ml)/trifluoroacetic acid (1 ml) were added, and the mixture was reacted at room temperature for 2 h. After the reaction was completed, the reaction liquid was directly concentrated, to afford crude compound **59C** (0.4 g).
LC-MS (ESI): m/z = 344.1[M+H]+

Step 3:

Intermediate **1L** (272 mg, 0.68 mmol) was dissolved in N,N-dimethylformamide (5 ml), crude compound **59C,** 2-(7-azobenzotriazole)-N,N,N',N'-tetramethyluronium hexafluorophosphate (517 mg, 1.36 mmol) and N,N-diisopropylethylamine (354 mg, 2.72 mmol) were added to the reaction system, and the mixture was reacted overnight at room temperature. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; d. elution time: 20 min, retention time: 7.0 min, to afford compound **59** (180 mg, 37%).

LC-MS (ESI): m/z = 719.3$[M+H]^+$

$^1$H NMR (400 MHz, DMSO-$d_6$) δ11.79 (s, 1H), 11.04 (s, 1H), 8.90 (d, 1H), 8.41 (d, 1H), 8.30 (d, 1H), 7.84 (d, 2H), 7.75 (s, 1H), 7.63 (s, 1H), 7.41 (dd, 1H), 7.33 (s, 1H), 7.30 (dd, 1H), 5.22 - 5.15 (m, 1H), 5.12 (dd, 1H), 4.65 - 4.53 (m, 1H), 3.66 (s, 2H), 3.36 (s, 5H), 2.96 - 2.82 (m, 1H), 2.74 - 2.64 (m, 2H), 2.57 (d, 6H), 2.54 (s, 1H), 2.04 (dd, 1H), 1.23 (s, 2H), 1.19 (t, 3H).

Example 60

N-((1s,3s)-3-((2-(2,6-dioxopiperidin-3-yl)-1,3-dioxoisoindolin-5-yl)oxy)cyclobutyl)-5-(4-((7-ethyl-6-oxo-5,6-dihydro-1,5-naphthyridin-3-yl)methyl)piperazin-1-yl)picolinamide (Compound **60**)

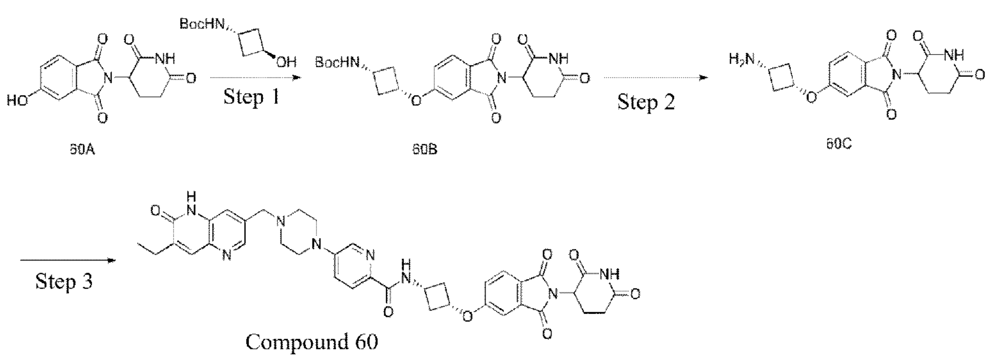


60A 60B 60C

Compound 60

Step 1:

**60A** (1.46 g, 5.34 mmol), tert-butyl ((1r,3r)-3-hydroxycyclobutyl)carbamate (1g, 5.34 mmol), triphenylphosphine (2.1 g, 8.01 mmol) and diethyl azodicarboxylate (1.4 g, 8.03 mmol) were added to tetrahydrofuran (20 ml). The mixture was subjected to $N_2$ replacement and reacted at 60°C for 16 h. The reaction liquid was cooled to room temperature and water (30 ml) was added. The mixture was extracted with ethyl acetate (30 ml × 2) and the organic phase was washed with saturated sodium chloride solution, dried over anhydrous sodium sulphate and filtered. The filtrate was concentrated under reduced pressure and the crude product was separated and purified by silica gel column chromatography (20 g silica gel column, eluent: EA/PE = 0-60%), to afford the title compound (500 mg, 21%).

LC-MS (ESI): m/z = 444.2 $[M+H]^+$.

Step 2:

**60B** (500 mg, 1.13 mmol) was added to dichloromethane (9 ml) and then trifluoroacetic acid (3 ml) was added. The mixture was reacted at room temperature for 2 h. After the reaction was completed, the mixture was directly concentrated to afford crude compound **60C** (0.65 g).

Step 3:

**1L** (130 mg, 0.33 mmol), **60C** (150 mg, 0.33 mmol), 2-(7-azobenzotriazole)-N,N,N',N'-tetramethyluronium hexafluorophosphate (190 mg, 0.5 mmol) and N,N-diisopropylethylamine (210 mg, 1.62 mmol) were sequentially added to N,N-dimethylformamide (5 ml), and the mixture was reacted at room temperature for 5 h. The reaction liquid was directly separated and purified by HPLC. After purification, the product was concentrated under reduced pressure to remove acetonitrile and then extracted with (dichloromethane : methanol = 10 : 1), and the organic phase was concentrated under reduced pressure to afford the title compound (30 mg, 12.65%). Preparative HPLC separation methods: 1. Instruments: waters 2767 (preparative liquid phase chromatographic instrument); chromatographic column: SunFire@ Prep C18 (19 mm × 250 mm) 2. The sample was dissolved in DMF and filtered with a 0.45 μm filter to prepare a sample solution. 3. Preparative chromatography conditions: a. composition of mobile phases A and B: mobile phase A: acetonitrile; mobile phase B: water (containing 0.1% trifluoroacetic acid); b. gradient elution, mobile phase A: 5%-40%; c. flow rate: 12 mL/min. d. elution time: 20 min.

$^1$H NMR (400 MHz, DMSO-$d_6$) δ 11.82 (s, 1H), 11.04 (s, 1H), 8.44 - 8.36 (m, 2H), 8.27 (d, 1H), 7.81 (d, 1H), 7.78 - 7.72 (m, 2H), 7.63 (d, 1H), 7.38 (dd, 1H), 7.21 - 7.14 (m, 2H), 5.11 (dd, 1H), 4.51-4.43(m, 1H), 4.27 - 4.12 (m, 1H), 3.65 (s, 2H), 3.34-3.30 (m, 4H), 2.99 - 2.86 (m, 1H), 2.79 - 2.64 (m, 3H), 2.57-2.54 (m, 8H), 2.49-2.45 (m, 1H), 2.06 - 1.94 (m, 1H), 1.19 (t, 3H).

LC-MS (ESI): m/z = 719.3[M+H]$^+$.

Example 61:

N-(1-(2-(2,6-dioxopiperidin-3-yl)-1,3-dioxoisoindolin-5-yl)piperidin-4-yl)-5-(4-((7-ethyl-6-oxo-5,6-dihydro-1,5-naphthyridin-3-yl)methyl)piperazin-1-yl)-N-methylpicolinamide (Compound **61**)

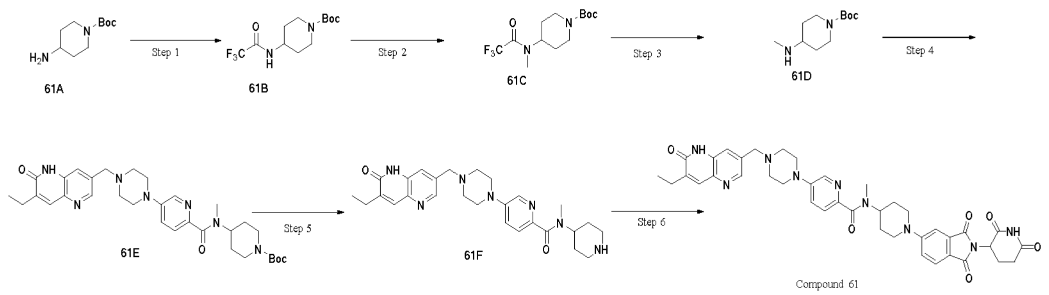


61A 61B 61C 61D 61E 61F Compound 61

Step 1:

Compound tert-butyl 4-aminopiperidine-1-carboxylate (1.0 g, 4.99 mmol) and ethyl trifluoroacetate (3.55 g, 24.95 mmol) were dissolved in methanol (10 mL) and the mixture was reacted overnight at 50°C and concentrated, to afford crude product **61B** (1.5 g, yield 100.0%).
LCMS m/z = 297.1[M+H]$^+$

Step 2:

Compound **61B** (1.5 g, 4.99 mmol) was dissolved in DMF (15 mL) and at 0°C, NaH (60%, 400 mg, 10.12 mmol) was added. The mixture was reacted for 10 min and then iodomethane (1.44 g, 10.12 mmol) was added. The resulting mixture was reacted at room temperature for 30 min. Ethyl acetate (50 mL) was added, and the reaction liquid was washed with water (30 mL × 2) and saturated brine (30 mL), dried over anhydrous sodium sulphate and concentrated, to afford crude product **61C** (1.6 g, yield 100.0%).
LCMS m/z = 311.3[M+H]$^+$

Step 3:

Compound **61C** (1.6 g, 4.99 mmol) was dissolved in methanol (10 mL), and then water (10 mL) was added, followed by sodium hydroxide (1.03 g, 25.8 mmol). The mixture was reacted at 50°C for 2 hours, extracted with dichloromethane (20 mL × 2), washed with saturated brine (20 mL), dried over anhydrous sodium sulphate and concentrated,

to afford crude product **61D** (0.5 g, yield 45.3%).
LCMS m/z = 215.3[M+H]$^+$

Step 4:

Compound **1L** (0.918 g, 2.33 mmol) and compound **61D** (500 mg, 2.33mmol) were dissolved in DMF (10 mL), benzotriazole-N,N,N',N'-tetramethyluronium hexafluorophosphate (0.89 g, 2.33 mmol) and N,N-diisopropylethylamine (0.45 mg, 3.47 mmol) were added, and the mixture was stirred and reacted at room temperature for 1 h. After the reaction was completed, water (20 ml) and ethyl acetate (50 mL) were added, and the organic phase was separated, washed with water (20 mL × 2) and saturated brine (20 mL), dried over anhydrous sodium sulphate and concentrated, to afford crude product **61E** (1.0 g, 72.5%).
LCMS m/z = 590.3 [M+H]$^+$

Step 5:

Compound **61E** (1.0 g, 1.7 mmol) was dissolved in dichloromethane (9 mL), trifluoroacetic acid (3 mL) was added, and the mixture was stirred and reacted at room temperature for 1 h. After the reaction was completed, the reaction liquid was adjusted to pH = 7-8 with triethylamine, and then purified by C18 column chromatography (acetonitrile:0.1% aqueous ammonia solution (v/v) = 40%) to afford product **61F** (300 mg, 36.1%).
LCMS m/z = 490.3 [M+H]$^+$

Step 6:

Compound **61F** (100 mg, 0.2 mmol) and 2-(2,6-dioxopiperidin-3-yl)-5-fluoroisoindoline-1,3-dione (55 mg, 0.2 mmol) were added to dimethyl sulphoxide (3 mL) and then N,N-diisopropylethylamine (52 mg, 0.4 mmol) was added. The mixture was reacted at 100°C for 2 h. The reaction liquid was directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **61** (40 mg, yield: 26.8%, retention time: about 7.5 min).

$^1$H NMR (400 MHz, DMSO-$d_6$) δ 11.84 (s, 1H), 11.06 (s, 1H), 8.40 (d, 1H), 8.26 (s, 1H), 7.75 (s, 1H), 7.72 - 7.61 (m, 2H), 7.50 - 7.42 (m, 1H), 7.42 - 7.21 (m, 3H), 5.10 - 5.03 (m, 1H), 4.26 - 3.96 (m, 3H), 3.65 (s, 2H), 3.33 - 3.31 (m, 4H), 3.17 - 3.04 (m, 1H), 2.94 - 2.76 (m, 5H), 2.63 - 2.51 (m, 8H), 2.06 - 1.98 (m, 1H), 1.89 - 1.70 (m, 4H), 1.20 -1.16 (m, 3H).
MS M/Z (ESI): m/z = 746.3 [M+H]$^+$

Example 62:

N-(1-(3-(2,4-dioxotetrahydropyrimidin-1(2H)-yl)phenyl)piperidin-4-yl)-5-(4-((7-ethyl-6-oxo-5,6-dihydro-1,5-naphthyridin-3-yl)methyl)piperazin-1-yl)picolinamide (Compound **62**)

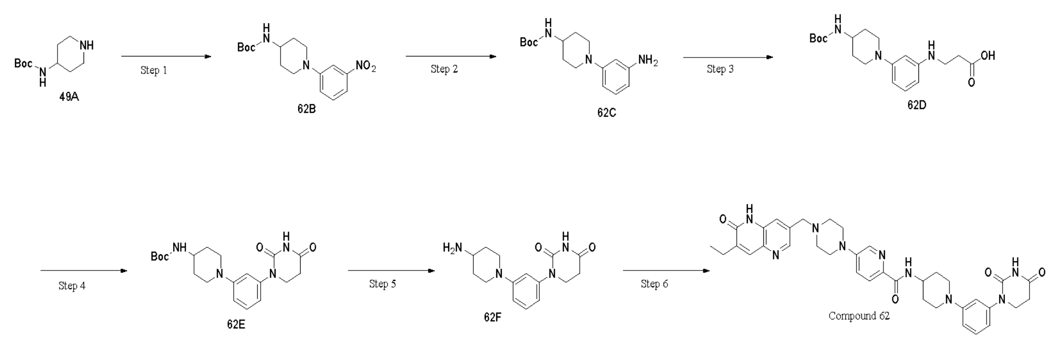


Step 1:

Compound tert-butyl 4-aminopiperidine-1-carboxylate (10.0 g, 50 mmol) and 1-fluoro-3-nitrobenzene (7.05 g,

50 mmol) were dissolved in dimethyl sulphoxide (100 mL), N,N-diisopropylethylamine (12.9 g, 100 mmol) was added, and the mixture was reacted at 150°C for 24 hours. Ethyl acetate (400 mL) was added, and the resulting mixture was washed with water (100 mL × 2) and saturated brine (100 mL), dried over anhydrous sodium sulphate and concentrated. The residue was purified by silica gel column chromatography (petroleum ether: ethyl acetate (v/v) = 100 : 30) to afford product **62B** (6 g, yield 37.5%).
LCMS m/z = 322.1[M+H]$^+$

Step 2:

Compound **62B** (6 g, 18.7 mmol) was dissolved in methanol (60 mL), palladium hydroxide on carbon (1.2 g) was added, and the mixture was subjected to hydrogenation overnight at 50°C, filtered and concentrated to afford crude product **62C** (5.8 g, yield 100.0%).
LCMS m/z = 292.2[M+H]$^+$

Step 3:

Compound **62C** (4 g, 13.7 mmol) was dissolved in toluene (40 mL), acrylic acid (1.48 g, 20.6 mmol) was added, and the mixture was reacted overnight at 100°C and then directly used in the next step.
LCMS m/z = 364.2[M+H]$^+$

Step 4:

To the reaction liquid obtained in the step above, acetic acid (40 mL) was added, followed by urea (1.2 g, 20.6 mmol), and the mixture was reacted overnight at 100°C, concentrated and purified by silica gel column chromatography (petroleum ether: ethyl acetate (v/v) = 100 : 20) to afford product **62E** (0.4 g, yield over two steps: 7.5%).
LCMS m/z = 389.2[M+H]$^+$

Step 5:

Compound **62E** (0.4 g, 1.0 mmol) was dissolved in dichloromethane (9 mL), trifluoroacetic acid (3 mL) was added, and the mixture was stirred and reacted at room temperature for 1 h and concentrated to afford product **62F** (410 mg, 100.0%).
LCMS m/z = 289.3 [M+H]$^+$

Step 6:

Compound **1L** (0.1 g, 0.25 mmol) and compound **62F** (101 mg, 0.25 mmol) were dissolved in DMF (3 mL), benzotriazole-N,N,N',N'-tetramethyluronium hexafluorophosphate (114 mg, 0.3mmol) and N,N-diisopropylethylamine (65 mg, 0.5 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **62** (30 mg, yield 18.1%, retention time: about 7.1 min).

$^1$H NMR (400 MHz, DMSO-$d_6$) δ11.84 (s, 1H), 10.28 (s, 1H), 8.41 (d, 1H), 8.36 - 8.23 (m, 2H), 7.85 (d, 1H), 7.75 (s, 1H), 7.63 (s, 1H), 7.47 - 7.37 (m, 1H), 7.25 - 7.12 (m, 1H), 6.91 (s, 1H), 6.84 (d, 1H), 6.71 (d, 1H), 4.01 - 3.85 (m, 1H), 3.78 - 3.72 (m, 2H), 3.71 - 3.64 (m, 4H), 3.39 - 3.38 (m, 4H), 2.89 - 2.76 (m, 2H), 2.74 - 2.64 (m, 2H), 2.59 - 2.52 (m, 6H), 1.92 - 1.81 (m, 2H), 1.80 - 1.66 (m, 2H), 1.22 - 1.14 (m, 3H).
MS M/Z (ESI): m/z = 664.3 [M+H]$^+$

Example 63:

N-(1-(3-(2,4-dioxotetrahydropyrimidin-1(2H)-yl)phenyl)piperidin-4-yl)-1'-((7-ethyl-6-oxo-5,6-dihydro-1,5-naphthyridin-3-yl)methyl)-1',2',3', 6'-tetrahydro-[3,4'-bipyridine]-6-carboxamide (Compound **63**)

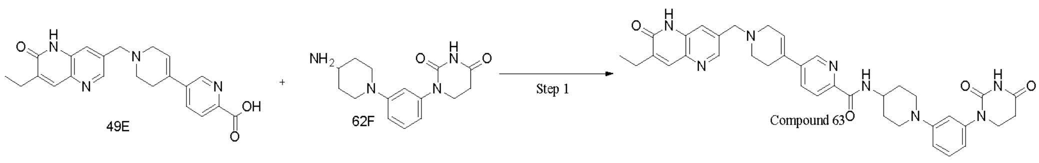

49E 62F Compound 63

Step 1:

Compound **49E** (0.1 g, 0.27 mmol) and **62F** (108 mg, 0.27 mmol) were dissolved in DMF (3 mL), benzotriazole-N,N,N',N'-tetramethyluronium hexafluorophosphate (103 mg, 0.27mmol) and N,N-diisopropylethylamine (70 mg, 0.54 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **63** (35 mg, yield 19.6%, retention time: about 7.3 min).

$^1$H NMR (400 MHz, DMSO-$d_6$) $\delta$12.17 (s, 1H), 10.28 (s, 1H), 8.74 (s, 1H), 8.64 - 8.54 (m, 2H), 8.10 - 8.02 (m, 2H), 7.81 (s, 2H), 7.26 - 7.21 (m, 1H), 6.94 (s, 1H), 6.87 (d, 1H), 6.74 (d, 1H), 6.41 (s, 1H), 4.59 (s, 2H), 4.05 - 3.97 (m, 1H), 3.91 (s, 2H), 3.79 - 3.65 (m, 5H), 3.43 - 3.26 (m, 1H), 2.92 - 2.79 (m, 4H), 2.72 - 2.66 (m, 2H), 2.61 - 2.55 (m, 2H), 1.93 - 1.74 (m, 4H), 1.24 - 1.15 (m, 3H).
MS M/Z (ESI): m/z = 661.3 $[M+H]^+$

Example 64:

N-(1-(3-(2,4-dioxotetrahydropyrimidin-1(2H)-yl)phenyl)piperidin-4-yl)-5-(4-((7-ethyl-6-oxo-5,6-dihydro-1,5-naphthyridin-3-yl)methyl)piperazin-1-yl)picolinamide (Compound **64**)

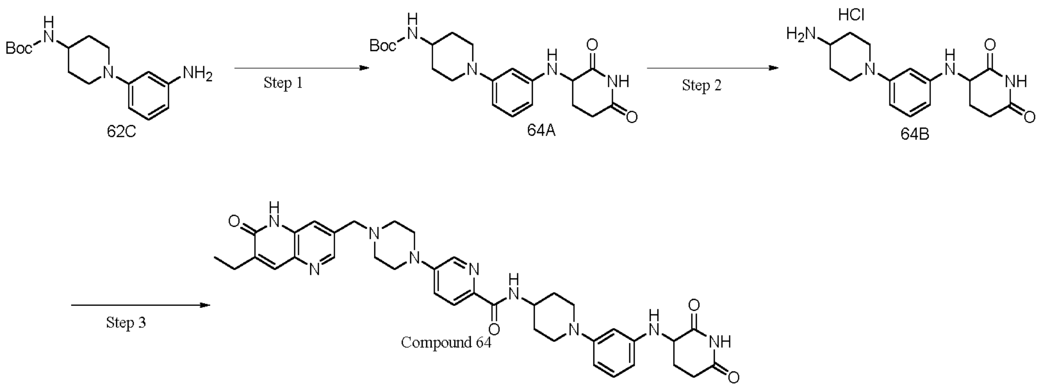

62C 64A 64B Compound 64

Step 1:

Compound **62C** (1.0 g, 3.44 mmol) and 3-bromopiperidine-2,6-dione (0.65 g, 3.44 mmol) were dissolved in DMF (10 mL), sodium bicarbonate (0.86 g, 10.32 mmol) was added, and the mixture was reacted at 80°C for 24 hours. Ethyl acetate (50 mL) was added, and the resulting mixture was washed with water (20 mL × 2) and saturated brine (10 mL), dried over anhydrous sodium sulphate and concentrated. The residue was purified by silica gel column chromatography (petroleum ether: ethyl acetate (v/v) = 100 : 20) to afford product **64A** (350 mg, yield 25.3%).
LCMS m/z = 403.2$[M+H]^+$

Step 2:

Compound **64A** (0.35 g, 0.9 mmol) was dissolved in hydrogen chloride-dioxane (3 mL) and the mixture was reacted at room temperature for 1 hour and concentrated to afford crude product **64B** (0.3 g, yield 100.0%).
LCMS m/z = 303.2$[M+H]^+$

Step 3:

Compound **1L** (0.1 g, 0.25 mmol) and compound **64B** (90 mg, 0.25 mmol) were dissolved in DMF (3 mL), benzotriazole-N,N,N',N'-tetramethyluronium hexafluorophosphate (114 mg, 0.3mmol) and N,N-diisopropylethylamine (65 mg, 0.5 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **64** (35 mg, yield 20.7%, retention time: about 7.3 min).

$^1$H NMR (400 MHz, DMSO-$d_6$) δ11.82 (s, 1H), 10.73 (s, 1H), 8.40 (d, 1H), 8.33 - 8.16 (m, 2H), 7.85 (d, 1H), 7.75 (s, 1H), 7.63 (s, 1H), 7.42 - 7.30 (m, 1H), 6.96 - 6.84 (m, 1H), 6.29 (s, 1H), 6.21 (d, 1H), 6.14 (d, 1H), 5.59 (d, 1H), 4.37 - 4.25 (m, 1H), 4.00 - 3.85 (m, 1H), 3.64 (s, 2H), 3.60 (d, 2H), 3.38 - 3.31 (m, 4H), 2.80 - 2.67 (m, 3H), 2.65 - 2.52 (m, 7H), 2.16 - 2.04 (m, 1H), 1.90 - 1.66 (m, 5H), 1.20 - 1.15 (m, 3H).
MS M/Z (ESI): m/z = 678.3 $[M+H]^+$

Example 65:

N-(1-(3-((2,6-dioxopiperidin-3-yl)amino)phenyl)piperidin-4-yl)-1'-((7-ethyl-6-oxo-5,6-dihydro-1,5-naphthyridin-3-yl)methyl)-1',2',3',6'-tetrahydro-[3,4'-bipyridine]-6-carboxamide (Compound **65**)

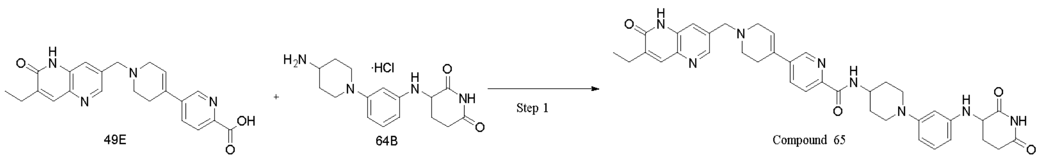


Step 1:

Compound **49E** (0.1 g, 0.27 mmol) and **64B** (91 mg, 0.27 mmol) were dissolved in DMF (3 mL), benzotriazole-N,N,N',N'-tetramethyluronium hexafluorophosphate (103 mg, 0.27mmol) and N,N-diisopropylethylamine (70 mg, 0.54 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **65** (35 mg, yield 19.2%, retention time: about 7.2 min).

$^1$H NMR (400 MHz, DMSO-*d6*) δ11.82 (s, 1H), 10.74 (s, 1H), 8.70 (s, 1H), 8.56 (d, 1H), 8.41 (d, 1H), 8.05 - 7.96 (m, 2H), 7.75 (s, 1H), 7.65 (s, 1H), 6.97 - 6.84 (m, 1H), 6.42 (s, 1H), 6.30 (s, 1H), 6.22 (d, 1H), 6.13 (d, 1H), 5.60 (d, 1H), 4.37 - 4.27 (m, 1H), 4.05 - 3.87 (m, 1H), 3.72 (s, 2H), 3.62 (d, 2H), 3.22 - 3.12 (m, 2H), 2.82 - 2.65 (m, 5H), 2.63 - 2.52 (m, 5H), 2.16 - 2.05 (m, 1H), 1.91 - 1.68 (m, 5H), 1.21 - 1.15 (m, 3H).
MS M/Z (ESI): m/z = 675.3$[M+H]^+$

Example 66:

N-((1r,4r)-4-((2-(2,6-dioxopiperidin-3-yl)-1,3-dioxoisoindolin-5-yl)oxy)cyclo hexyl)-5-(4-((7-ethyl-6-oxo-5, 6-dihydro-1, 5-naphthyridin-3-yl)methyl)piperazin-1-yl)picolinamide (Compound **66**)

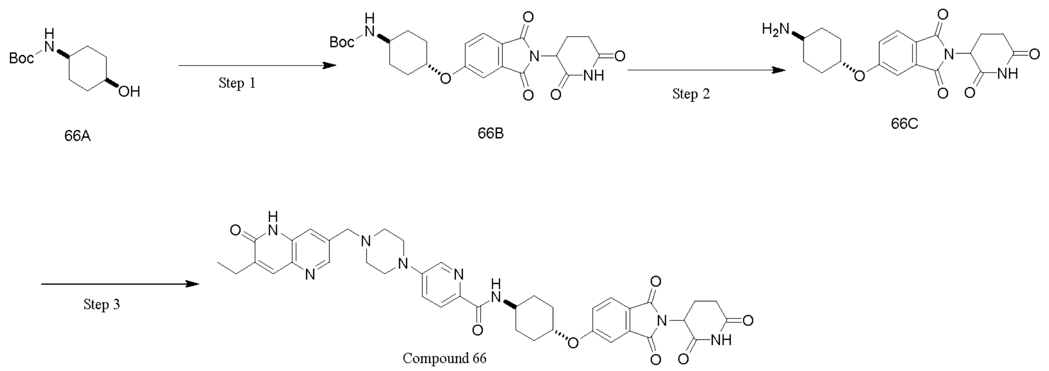


66A 66B 66C

Compound 66

Step 1:

Under nitrogen protection, compound **66A** (1.0 g, 4.64 mmol) and compound 4-hydroxythalidomide (1.27 g, 0.64 mmol) were dissolved in dry tetrahydrofuran (20 ml) and the mixture was cooled to 0°C. Triphenylphosphine (1.83 g, 6.98 mmol) was added. After the addition, the temperature was controlled at 0°C. Diethyl azodicarboxylate (1.29 g, 7.42 mmol) was added dropwise. After the addition, the mixture was warmed to room temperature and reacted for 30 min, then heated to 60°C and reacted for 15 hours. After the reaction was completed, the reaction was quenched with water (50 ml) and extracted with ethyl acetate (50 ml × 2). The organic phases were combined, washed with saturated brine, dried over anhydrous sodium sulphate and filtered. The filtrate was concentrated and the residue was separated and purified by silica gel column chromatography (petroleum ether: ethyl acetate (v/v) = 4 : 1) to afford title compound **66B** (0.82 g, 37.5%).

Step 2:

Compound **66B** (0.82 g, 1.74 mmol) was dissolved in dichloromethane (10 mL), trifluoroacetic acid (3 mL) was added, and the mixture was reacted at room temperature for 30 min. After the reaction was completed, the reaction liquid was directly concentrated to afford the trifluoroacetate of compound **66C** (0.84 g, 99%).

Step 3:

Under nitrogen protection, compound **66C** (0.13 g, 0.33 mmol) was dissolved in N,N-dimethylformamide (5 ml). The mixture was cooled to 0°C. Compound **1L** (0.16 g, 0.33 mmol), N,N-diisopropylethylamine (0.17 g, 1.32 mmol) and 2-(7-azobenzotriazole)-N,N,N',N'-tetramethyluronium hexafluorophosphate (0.15 g, 0.40 mmol) were added respectively. After the addition, the mixture was warmed to 25°C and reacted for 1 hour. After the reaction was completed, the reaction liquid was poured to water (20 ml). The resulting mixture was extracted with dichloromethane (50 mL × 3), and the organic phases were combined, washed with saturated brine, dried over anhydrous sodium sulphate and filtered. The filtrate was concentrated and the residue was subjected to preparative purification with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution: mobile phase A: 5%-50%; c. flow rate: 12 mL/min. d. elution time: 20 min, retention time: 8.8 min, to afford compound **66** (75 mg, 30.4%).

$^1$H NMR (400 MHz, DMSO-$d_6$) δ 11.83 (s, 1H), 11.09 (s, 1H), 8.40 (s, 1H), 8.28 (d, 1H), 8.16 (d, 1H), 7.84 (d, 2H), 7.75 (s, 1H), 7.63 (s, 1H), 7.49 (d, 1H), 7.43-7.39 (m, 2H), 5.13-5.09 (m, 1H), 4.90-4.88 (m, 1H), 3.93-3.89 (m, 1H), 3.66 (s, 2H), 3.46-3.42 (m, 4H), 2.92-2.86 (m, 1H), 2.62-2.53(m, 8H), 2.03-1.93 (m, 3H), 1.80-1.66 (m, 6H), 1.20-1.17 (m, 3H).

MS M/Z (ESI): m/z = 747.3 [M+H]$^+$

Example 67:

N-((1s,4s)-4-((2-(2,6-dioxopiperidin-3-yl)-1,3-dioxoisoindolin-5-yl)oxy)cyclo hexyl)-5-(4-((7-ethyl-6-oxo-5, 6-dihydro-1, 5-naphthyridin-3-yl)methyl)piperazin-1-yl)picolinamide (Compound **67**)

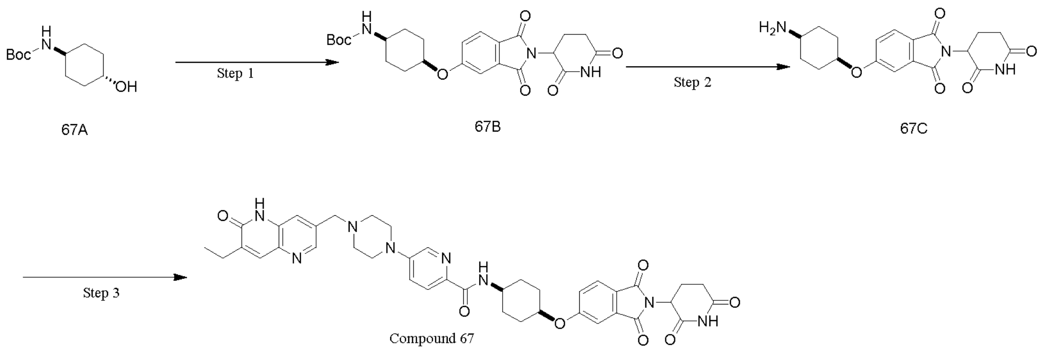


67A 67B 67C

Compound 67

Step 1:

Under nitrogen protection, compound **67A** (1.0 g, 4.64 mmol) and 4-hydroxythalidomide (1.27 g, 0.64 mmol) were dissolved in dry tetrahydrofuran (20 ml) and the mixture was cooled to 0°C. Triphenylphosphine (1.83 g, 6.98 mmol) was added. After the addition, the temperature was controlled at 0°C. Diethyl azodicarboxylate (1.29 g, 7.42 mmol) was added dropwise. After the addition, the mixture was warmed to room temperature and reacted for 30 min, then heated to 60°C and reacted for 15 hours. After the reaction was completed, the reaction was quenched with water (50 ml) and extracted with ethyl acetate (50 ml × 2). The organic phases were combined, washed with saturated brine, dried over anhydrous sodium sulphate and filtered. The filtrate was concentrated and the residue was separated and purified by silica gel column chromatography (petroleum ether: ethyl acetate (v/v) = 4 : 1) to afford title compound **67B** (0.52 g, 23.8%).

Step 2:

Compound **67B** (0.52 g, 1.1 mmol) was dissolved in dichloromethane (10 mL), trifluoroacetic acid (3 mL) was added, and the mixture was reacted at room temperature for 30 min. After the reaction was completed, the reaction liquid was directly concentrated to afford the trifluoroacetate of compound **67C** (0.52 g, 97%).

Step 3:

Under nitrogen protection, compound **67C** (0.13 g, 0.33 mmol) was dissolved in N,N-dimethylformamide (5 ml). The mixture was cooled to 0°C. Compound **1L** (0.16 g, 0.33 mmol), N,N-diisopropylethylamine (0.17 g, 1.32 mmol) and 2-(7-azobenzotriazole)-N,N,N',N'-tetramethyluronium hexafluorophosphate (0.15 g, 0.40 mmol) were added respectively. After the addition, the mixture was warmed to 25°C and reacted for 1 hour. After the reaction was completed, the reaction liquid was poured to water (20 ml). The resulting mixture was extracted with dichloromethane (50 mL × 3), and the organic phases were combined, washed with saturated brine, dried over anhydrous sodium sulphate and filtered. The filtrate was concentrated and the residue was subjected to preparative purification with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution: mobile phase A: 5%-50%; c. flow rate: 12 mL/min. d. elution time: 20 min, retention time: 8.6 min, to afford compound **67** (40 mg, 16.2%).

$^{1}$H NMR (400 MHz, DMSO-$d_6$) δ 11.83 (s, 1H), 11.09 (s, 1H), 8.40 (s, 1H), 8.28 (d, 1H), 8.14 (d, 1H), 7.86-7.81(m, 2H), 7.75 (s, 1H), 7.63 (s, 1H), 7.45 (d, 1H), 7.42-7.35 (m, 2H), 5.14-5.09 (m, 1H), 4.65-4.62 (m, 1H), 3.87-3.83 (m, 1H), 3.66 (s, 2H), 3.38-3.33(m, 4H), 2.94-2.86 (m, 1H), 2.62-2.50(m, 8H), 2.14-1.90 (m, 5H), 1.67-1.51 (m, 4H), 1.20-1.17 (m, 3H).

MS M/Z (ESI): m/z = 747.3 [M+H]$^+$

Example 68:

1-(1-(2-(2,6-dioxopiperidin-3-yl)-1,3-dioxoisoindolin-5-yl)piperidin-4-yl)-N-(5-(4-((7-ethyl-6-oxo-5,6-dihydro-1,5-naphthyridin-3-yl)methyl)piperazin-1-yl)pyridin-2-yl)-1H-pyrazole-4-carboxamide (Compound **68**)

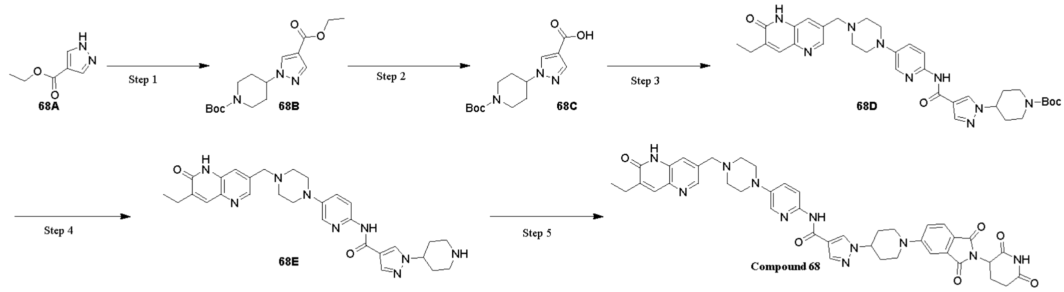


Step 1:

Compound **68A** (0.5 g, 3.54 mmol) and 1-Boc-4-methanesulphonyloxypiperidine (0.99 g, 3.54 mmol) were dissolved in DMF (10 mL) and then caesium carbonate (2.31 g, 7.08 mmol) was added. The mixture was warmed to 90°C and reacted overnight. After the reaction was completed, the reaction system was diluted with ethyl acetate (50 ml) and the organic phase was washed with water (10 ml), dried over anhydrous sodium sulphate, filtered and concentrated. The residue was separated and purified by flash column chromatography (eluent: MeOH : DCM = 0%-10%), to afford compound **68B** (0.9 g, 79%).
LC-MS (ESI): m/z = 268.2[M-56+H]$^+$

Step 2:

Compound **68B** (0.9 g, 2.78 mmol) was dissolved in methanol (3 mL) and tetrahydrofuran (3 mL), lithium hydroxide (0.27 g, 11.29 mmol) was added, and the mixture was warmed to 60°C and reacted for 2 h. The reaction liquid was cooled to room temperature and adjusted to pH 4 with 1M HCl. A white solid was precipitated out and filtered to afford **68C** (0.7 g, 85%) as a white solid.
LC-MS (ESI): m/z = 240.1[M-56+H]$^+$

Step 3:

Compound **68C** (0.4 g, 1.35 mmol) and **46D** (0.59 g, 1.62 mmol) were dissolved in DMF (5 mL), 2-(7-azobenzotriazole)-N,N,N',N'-tetramethyluronium hexafluorophosphate (0.62 g, 1.62 mmol) and N,N-diisopropylethylamine (0.52 g, 4.05 mmol) were added, and the mixture was reacted overnight at room temperature. After the reaction was completed, the reaction system was diluted with ethyl acetate (30 ml). The organic phase was washed with water (10 ml), dried over anhydrous sodium sulphate, filtered and concentrated, and the residue was separated and purified by flash column chromatography (eluent: MeOH : DCM = 0%-10%), to afford compound **68D** (0.2 g, 23%).
LC-MS (ESI): m/z = 642.4[M+H]$^+$

Step 4:

Compound **68D** (0.2 g, 0.31 mmol) was dissolved in trifluoroacetic acid (0.6 mL) and dichloromethane (1.8 mL) and the mixture was reacted at room temperature for 2 h. After the reaction was completed, the mixture was directly concentrated to afford crude compound **68E** (0.15 g).
LC-MS (ESI): m/z = 542.4[M+H]$^+$

Step 5:

Under nitrogen protection, compound **68E** (0.15 g, 0.28 mmol) and 2-(2,6-dioxo-piperidin-3-yl)-5-fluoro-isoindole-1,3-dione (0.077 g, 0.28 mmol) were dissolved in dimethyl sulphoxide (5 mL), and the mixture was warmed to 100°C and reacted for 2 h. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min. d. elution time: 20 min. retention time: 7.0 min, to afford compound **68** (50 mg, 22%).

LC-MS (ESI): m/z = 798.3 [M+H]$^+$
$^1$H NMR (400 MHz, DMSO-$d_6$) δ 11.80 (s, 1H), 11.08 (s, 1H), 10.18 (s, 1H), 8.55 (s, 1H), 8.40 (d, 1H), 8.09 (s, 1H), 8.02 (d, 1H), 7.99 (d, 1H), 7.75 (s, 1H), 7.69 (d, 1H), 7.63 (d, 1H), 7.41 (dd, 2H), 7.33 (dd, 1H), 5.07 (dd, 1H), 4.60 - 4.52 (m, 1H), 4.19 (d, 2H), 3.65 (s, 2H), 3.25 - 3.15 (m, 6H), 2.63 - 2.52 (m, 8H), 2.19 - 2.12 (m, 2H), 2.08 - 1.86 (m, 4H), 1.18 (t, 3H).

Example 69

N-(1-(5-(2,4-dioxotetrahydropyrimidin-1(2H)-yl)pyrimidin-2-yl)piperidin-4-yl)-5-(4-((7-ethyl-6-oxo-5, 6-dihydro-1, 5-naphthyridin-3-yl)methyl)piperazin-1-yl)picolinamide (Compound **69**)

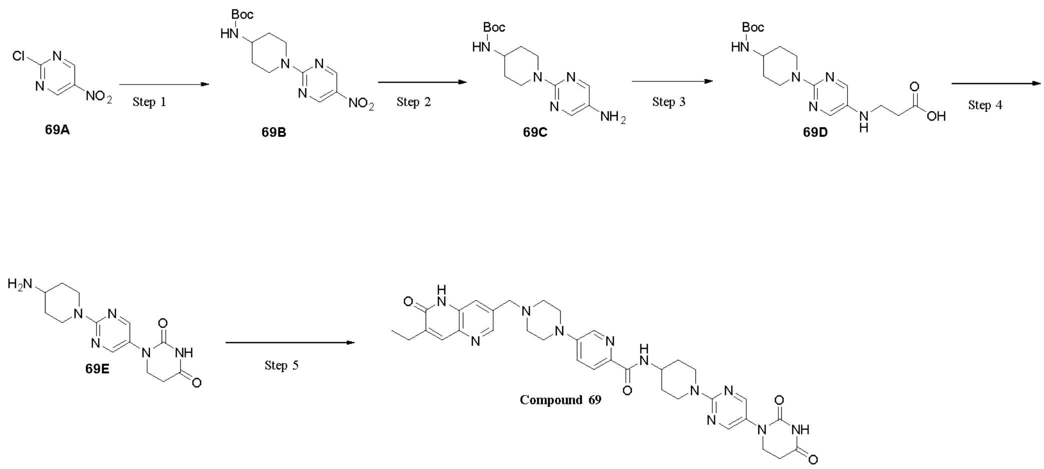


Step 1:

**69A** (5 g, 31.34 mmol) and tert-butyl piperidin-4-ylcarbamate (7.5 g, 37.5 mmol) were dissolved in DMF (50 mL), triethylamine (9.5 g, 93.88 mmol) was added, and the mixture was reacted overnight at room temperature. After the reaction was completed as monitored by TLC, water (200 mL) was added and the resulting mixture was filtered and dried to afford title compound **69B** (10 g, 98.7%).
LC-MS (ESI): m/z = 324.1 [M+H]$^+$.

Step 2:

**69B** (10 g, 0.25 mmol) was added to methanol (10 mL), palladium on carbon (5 g) was added, and the mixture was reacted and stirred in $H_2$ environment for 12 hours, then filtered and spun to dryness, to afford title compound **69C** (8 g, 88.2%).
LC-MS(ESI): m/z = 294.2 [M+H]$^+$.

Step 3:

**69C** (3 g, 10.23 mmol) and acrylic acid (1.47 g, 20.45 mmol) were added to toluene (30 mL). Under nitrogen protection, the mixture was reacted overnight at 100°C and the system was spun to dryness to afford crude **69D,** which was directly used in the next reaction.
LC-MS(ESI): m/z = 366.2 [M+H]$^+$.

Step 4:

**69D** and urea (614 mg, 10.24 mmol) were added to acetic acid (30 mL), and the reaction mixture was stirred overnight at 100°C and then concentrated. The residue was separated and purified by reverse phase column to afford title compound **69E** (180 mg, yield over two steps: 6.1%).
LC-MS(ESI): m/z = 291.1 [M+H]$^+$.

Step 5:

**69E** (180 mg, 0.62 mmol), **1L** (244 mg, 0.62 mmol), HATU (236 mg, 0.62 mmol) and DIPEA (400 mg, 3.10 mmol) were added to DMF (5 mL), and the mixture was stirred at room temperature for 2 h and then subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 12 mL/min; d. elution time: 20 min, and the preparative liquid was adjusted to pH 7-8 with sodium bicarbonate, and then the mixture was extracted with dichloromethane and concentrated, to afford compound **69** (130 mg, 31.5%).

$^1$H NMR (400 MHz, DMSO-$d_6$) δ 11.83 (s, 1H), 10.42 (s, 1H), 8.40 (d, 1H), 8.34 (s, 2H), 8.29 - 8.22 (m, 2H), 7.85 (d, 1H), 7.75 (s, 1H), 7.62 (s, 1H), 7.40 (dd, 1H), 4.70 - 4.56 (m, 2H), 4.15 - 3.96 (m, 1H), 3.72 (t, 2H), 3.65 (s, 2H), 3.37 - 3.32 (m, 4H), 3.05 (t, 2H), 2.71 (t, 2H), 2.60 - 2.52 (m, 6H), 1.89 - 1.77 (m, 2H), 1.65 - 1.49 (m, 2H), 1.18 (t, 3H).

LC-MS (ESI): m/z = 666.3 [M+H]$^+$.

Example 70

N-(1-(3-((2,6-dioxopiperidin-3-yl)amino)phenyl)piperidin-4-yl)-6-fluoro-5-(4-((5-fluoro-2-methyl-3-oxo-3,4-dihydroquinoxalin-6-yl)methyl)piperazin-1-yl)picolinamide (Compound **70**)

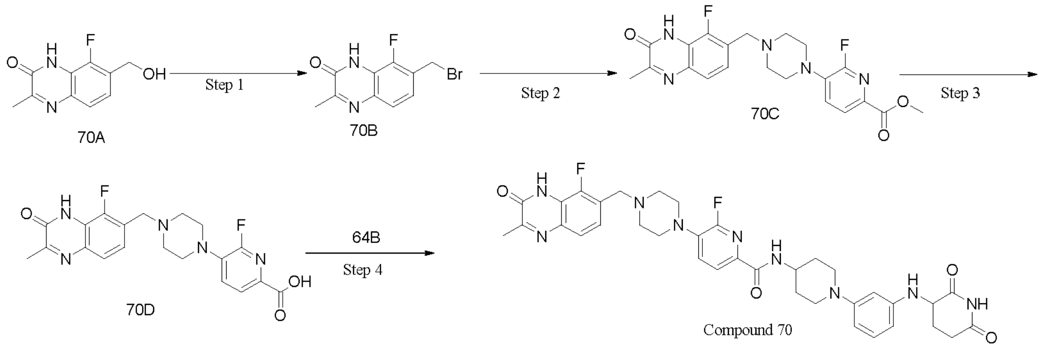


Step 1:

Compound **70A** (2.0 g, 9.6 mmol) was dissolved in anhydrous dichloromethane (10 mL), triphenylphosphine (7.5 g, 28.8 mmol) was added, and at 0°C, a solution of carbon tetrabromide (9.5 g, 28.8 mmol) in anhydrous dichloromethane (5 mL) was added. The mixture was reacted at room temperature for 30 min and filtered and the filter cake was dried to afford product **70B** (1.8 g, yield 69.2%).
LCMS m/z = 271.1[M+H]$^+$

Step 2:

Compound **70B** (1.8 g, 6.67 mmol) was dissolved in acetonitrile (15 mL), methyl 6-fluoro-5-(piperazin-1-yl)picolinate hydrochloride (1.83 g, 6.67 mmol), potassium iodide (180 mg) and N,N-diisopropylethylamine (4.3 g, 33.35 mmol) were added, and the mixture was reacted at 50°C for 3 hours and filtered. The filter cake was dried to afford crude product **70C** (2.4 g, yield 85.6%).
LCMS m/z = 430.2[M+H]$^+$

Step 3:

Compound **70C** (2.4 g, 5.6 mmol) was dissolved in methanol (24 mL), water (24 mL) and lithium hydroxide (1.2 g, 28 mmol) were added, and the mixture was reacted at room temperature for 1 hour, adjusted pH = 3-4 with 6 N hydrochloric acid and filtered. The filter cake was dried to afford crude product **70D** (2.1 g, yield 91.2%).
LCMS m/z = 416.4[M+H]$^+$

Step 4:

Compound **70D** (0.1 g, 0.24 mmol) and compound **64B** (81 mg, 0.24 mmol) were dissolved in DMF (3 mL), benzotriazole-N,N,N',N'-tetramethyluronium hexafluorophosphate (114 mg, 0.3mmol) and N,N-diisopropylethylamine (65 mg, 0.5 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **70** (35 mg, yield 20.8%, retention time: about 7.5 min).

$^1H$ NMR (400 MHz, DMSO-$d_6$) $\delta$12.42 (s, 1H), 10.74 (s, 1H), 8.23 (d, 1H), 7.85 (d, 1H), 7.59 - 7.41 (m, 2H), 7.34 - 7.25 (m, 1H), 6.93 - 6.86 (m, 1H), 6.29 (s, 1H), 6.21 (d, 1H), 6.14 - 6.08 (m, 1H), 5.60 (d, 1H), 4.40 - 4.22 (m, 1H), 3.95 - 3.81 (m, 1H), 3.70 (s, 2H), 3.62 (d, 2H), 3.23 - 3.10 (m, 4H), 2.78 - 2.67 (m, 3H), 2.63 - 2.53 (m, 5H), 2.41 (s, 3H), 2.14 - 2.06 (m, 1H), 1.90 - 1.68 (m, 5H).
MS M/Z (ESI): m/z = 700.3 $[M+H]^+$

Example 71

N-(1-(3-(2,4-dioxotetrahydropyrimidin-1(2H)-yl)phenyl)piperidin-4-yl)-6-fluoro-5-(4-((5-fluoro-2-methyl-3-oxo-3,4-dihydroquinoxalin-6-yl)methyl)piperazin-1-yl)picolinamide (Compound **71**)

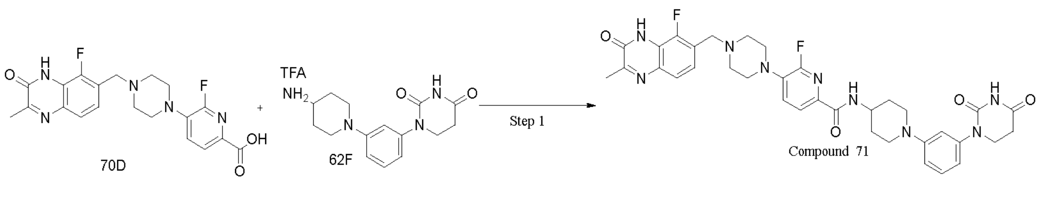


Step 1:

Compound **70D** (0.1 g, 0.24 mmol) and compound **62F** (92 mg, 0.24 mmol) were dissolved in DMF (3 mL), benzotriazole-N,N,N',N'-tetramethyluronium hexafluorophosphate (114 mg, 0.3mmol) and N,N-diisopropylethylamine (65 mg, 0.5 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **71** (35 mg, yield 21.2%, retention time: about 7.6 min).

$^1H$ NMR (400 MHz, DMSO-$d_6$) $\delta$12.34 (s, 1H), 10.26 (s, 1H), 8.24 (d, 1H), 8.00 - 7.79 (m, 1H), 7.68 - 7.48 (m, 2H), 7.38 - 7.15 (m, 2H), 6.90 (s, 1H), 6.87 - 6.78 (m, 1H), 6.75 - 6.62 (m, 1H), 4.02 - 3.87 (m, 1H), 3.79 - 3.66 (m, 6H), 3.21 - 3.10 (m, 4H), 2.85 - 2.72 (m, 2H), 2.71 - 2.65 (m, 2H), 2.64 - 2.55 (m, 4H), 2.41 (s, 3H), 1.86 - 1.71 (m, 4H).
MS M/Z (ESI): m/z = 686.3 $[M+H]^+$

Example 72

N-(1-(2-(2,6-dioxopiperidin-3-yl)-1,3-dioxoisoindolin-5-yl)piperidin-4-yl)-6-fluoro-5-(4-((5-fluoro-2-methyl-3-oxo-3,4-dihydroquinoxalin-6-yl)methyl)piperazin-1-yl)picolinamide (Compound **72**)

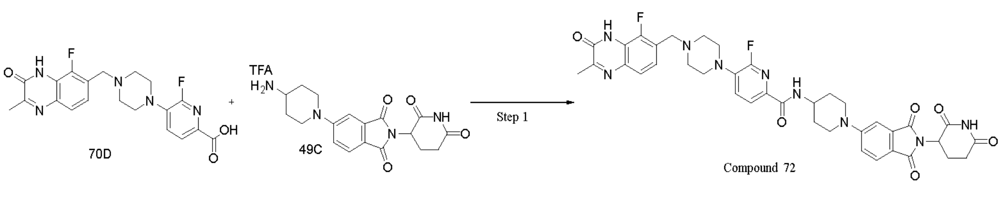

Step 1:

Compound **70D** (0.1 g, 0.24 mmol) and compound **49C** (109 mg, 0.24 mmol) were dissolved in DMF (3 mL), benzotriazole-N,N,N',N'-tetramethyluronium hexafluorophosphate (114 mg, 0.3mmol) and N,N-diisopropylethylamine (65 mg, 0.5 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **72** (40 mg, yield 22.2%, retention time: about 7.1 min).

$^1$H NMR (400 MHz, DMSO-$d_6$) δ 12.43 (s, 1H), 11.06 (s, 1H), 8.28 (d, 1H), 7.85 (d, 1H), 7.67 (d, 1H), 7.62 - 7.45 (m, 2H), 7.34 (s, 1H), 7.31 - 7.21 (m, 2H), 5.16 - 5.00 (m, 1H), 4.08 (d, 3H), 3.69 (s, 2H), 3.19 - 3.03 (m, 6H), 2.93 - 2.83 (m, 1H), 2.66 - 2.54 (m, 6H), 2.41 (s, 3H), 2.07 - 1.97 (m, 1H), 1.86 - 1.64 (m, 4H).
MS M/Z (ESI): m/z = 754.3 [M+H]$^+$

Example 73

N-(1-(2-(2,6-dioxopiperidin-3-yl)-6-fluoro-1,3-dioxoisoindolin-5-yl)piperidin-4-yl)-6-fluoro-5-(4-((5-fluoro-2-methyl-3-oxo-3,4-dihydroquinoxalin-6-yl)methyl)piperazin-1-yl)picolinamide (Compound **73**)

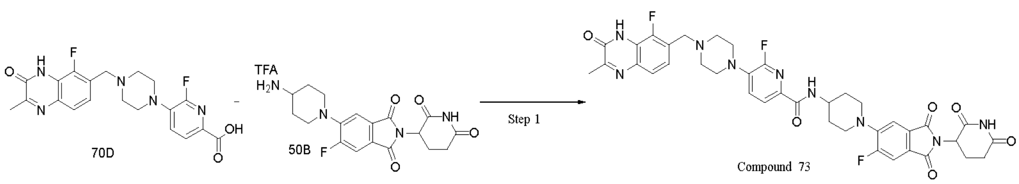


Step 1:

Compound **70D** (0.1 g, 0.24 mmol) and compound **50B** (113 mg, 0.24 mmol) were dissolved in DMF (3 mL), benzotriazole-N,N,N',N'-tetramethyluronium hexafluorophosphate (114 mg, 0.3mmol) and N,N-diisopropylethylamine (65 mg, 0.5 mmol) were added, and the mixture was stirred and reacted at room temperature for 1 h and directly separated and purified by liquid phase preparative column (liquid phase preparative conditions: C18 reverse-phase preparative column, mobile phase: deionized water containing 0.1% ammonium bicarbonate (A) and acetonitrile (B), gradient elution, mobile phase B = 5%-50%, elution time: 15 min, flow rate: 12 mL/min, and column temperature: 30°C), to afford title compound **73** (45 mg, yield 24.3%, retention time: about 7.2 min).

$^1$H NMR (400 MHz, DMSO-$d_6$) δ 12.41 (s, 1H), 11.08 (s, 1H), 8.31 (d, 1H), 7.86 (d, 1H), 7.71 (d, 1H), 7.60 - 7.52 (m, 1H), 7.48 (d, 2H), 7.28 - 7.17 (m, 1H), 5.14 - 5.07 (m, 1H), 4.06 - 3.94 (m, 1H), 3.74 - 3.55 (m, 4H), 3.23 - 3.11 (m, 4H), 3.10 - 2.96 (m, 2H), 2.94 - 2.82 (m, 1H), 2.67 - 2.52 (m, 6H), 2.40 (s, 3H), 2.10 - 1.98 (m, 1H), 1.94 - 1.75 (m, 4H).
MS M/Z (ESI): m/z = 772.6[M+H]$^+$

Example 74:

N-(1-(4-(2,4-dioxotetrahydropyrimidin-1(2H)-yl)phenyl)piperidin-4-yl)-6-fluoro-5-(4-((5-fluoro-2-methyl-3-oxo-3,4-dihydroquinoxalin-6-yl)methyl)piperazin-1-yl)picolinamide (Compound **74**)

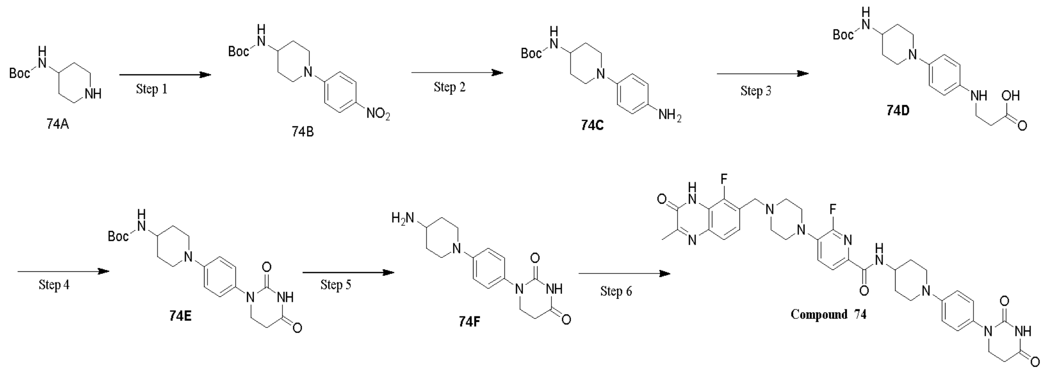

74A 74B 74C 74D 74E 74F Compound 74

Step 1:

**74A** (20 g, 0.1 mol) was dissolved in N-methylpyrrolidone (250 ml), and 1-fluoro-4-nitrobenzene (16.93 g, 0.2 mol) and potassium carbonate (13.82 g, 0.3 mol) were added. The mixture was reacted in an 80°C oil bath pan for 3 h. After the reaction was completed, the reaction liquid was cooled to room temperature. The reaction was quenched with water (700 ml). The aqueous phase was extracted with ethyl acetate (500 ml × 2) and the organic phases were combined, dried over anhydrous sodium sulphate and concentrated to afford crude compound **74B** (30 g).
LCMS (ESI): m/z = 266.2 $[M+H]^+$

Step 2:

To a solution of crude compound **74B** (30 g, obtained in the step above, dissolved in methanol (300 ml)) was added 10% Pd/C (3 g). The mixture was subjected to hydrogen replacement three times and reacted at 60°C under hydrogen atmosphere for 16 h. After the reaction was completed, the reaction liquid was cooled to room temperature and filtered. The filtrate was collected, spun to dryness and concentrated to afford crude compound **74 C** (25 g).
LCMS (ESI): m/z = 236.2 $[M+H]^+$

Step 3:

Compound **74C** (5 g, 17.18 mmol) was dissolved in toluene (70 ml) and then propenol (1.86 g, 25.77 mmol) was added. The mixture was reacted in a 110°C oil bath pan for 15 h. After the reaction was completed, the reaction liquid was directly spun to dryness and concentrated to afford crude compound **74D** (7 g).
LCMS (ESI): m/z = 364.3$[M+H]^+$

Step 4:

Crude compound **74D** (7 g, obtained in the step above) was dissolved in acetic acid/toluene (30 ml/30 ml) and then urea (5.16 g, 85.9 mmol) was added. The mixture was reacted in a 110°C oil bath pan for 15 h. After the reaction was completed, the reaction liquid was directly spun to dryness and concentrated, and the residue was separated and purified by flash column chromatography (eluent: EA/PE = 0%-20%), to afford compound **74E** (2.75 g, yield over two steps: 41%).
LCMS (ESI): m/z = 389.2$[M+H]^+$

Step 5:

Compound **74E** (2.75 g, 7.08 mmol) was placed in a flask, dichloromethane (15 ml)/trifluoroacetic acid (5 ml) was added, and the mixture was reacted at room temperature for 2 h. After the reaction was completed, the reaction liquid was adjusted to an alkaline pH with saturated aqueous sodium bicarbonate solution and the aqueous phase was extracted with dichloromethane /methanol = 10/1. The organic phases were combined, dried over anhydrous sodium sulphate and concentrated to afford crude compound **74F** (1.91 g).
LCMS (ESI): m/z = 289.2$[M+H]^+$

Step 6:

**70D** (150 mg, 0.36 mmol) was dissolved in N,N-dimethylformamide (2 ml), compound **74F** (134 mg, 0.36 mmol),

2-(7-azobenzotriazole)-N,N,N',N'-tetramethyluronium hexafluorophosphate (2744 mg, 0.72 mmol) and N,N-diisopropylethylamine (141 mg, 1.08 mmol) were added to the reaction system, and the mixture was reacted overnight at room temperature. After the reaction was completed as monitored by LCMS, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to afford compound **74** (95 mg, 39%).

LCMS (ESI): m/z = 686.5[M+H]$^+$
$^1$H NMR (400 MHz, DMSO) δ 12.45 (s, 1H), 10.25 (s, 1H), 8.27 (d, 1H), 7.86 (d, 1H), 7.55 (dd, 2H), 7.29 (t, 1H), 7.14 (d, 2H), 6.95 (d, 2H), 3.93 (d, 1H), 3.70 (t, 7H), 3.17 (s, 4H), 2.78 (t, 2H), 2.68 (t, 3H), 2.59 (s, 4H), 2.42 (s, 3H), 1.80-1.75 (m, 4H).

Example 75

N-(1-(2-(2,6-dioxopiperidin-3-yl)-1-oxoisoindolin-5-yl)piperidin-4-yl)-6-fluoro-5-(4-((5-fluoro-2-methyl-3-oxo-3,4-dihydroquinoxalin-6-yl)methyl)piperazin-1-yl)picolinamide (Compound **75**)

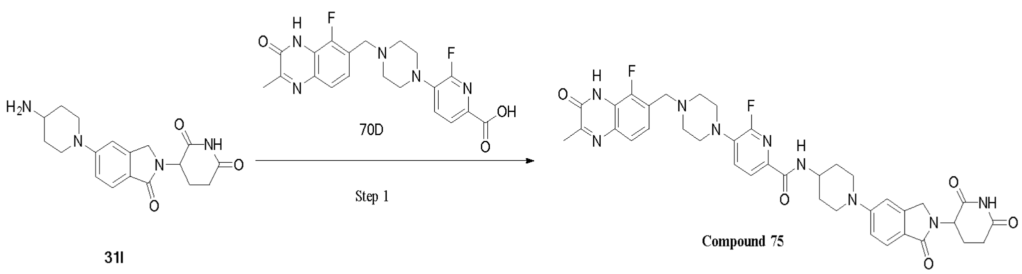


31I

70D

Compound 75

Step 1:

**31l** (180 mg, 0.53 mmol), **70D** (218 mg, 0.53 mmol), HATU (200 mg, 0.53 mmol) and DIPEA (339 mg, 2.63 mmol) were added to DMF (5 mL), and the mixture was stirred at room temperature for 2 h and then subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 12 mL/min; d. elution time: 20 min, and the preparative liquid was adjusted to pH 7-8 with sodium bicarbonate, and then the mixture was extracted with dichloromethane and concentrated, to afford compound **75** (70 mg, 18.0%).

$^1$H NMR (400 MHz, DMSO-$d_6$) δ 10.67 (s, 1H), 8.25 (d, 1H), 7.88 - 7.82 (m, 1H), 7.56 - 7.44 (m, 3H), 7.24 (t, 1H), 7.06 (d, 1H), 7.01 (s, 1H), 5.30 (s, 2H), 4.57 (dd, 1H), 4.09 - 3.96 (m, 1H), 3.69 (s, 2H), 3.25 - 3.13 (m, 6H), 2.95 (t, 2H), 2.65 - 2.56 (m, 4H), 2.41 (s, 3H), 2.11 - 1.90 (m, 4H), 1.89 - 1.66 (m, 4H).

LC-MS (ESI): m/z = 740.3 [M+H]$^+$.

**Biological test examples**

1. MDA-MB-436 cell activity test experiment

Breast tumour cells MDA-MB-436 were purchased from ATCC, the culture medium was Leibovitz's L-15 +10% FBS, and the cells were cultured in a $CO_2$-free incubator at 37°C. On day 1, the cells in the exponential growth phase were collected, and the cell suspension was adjusted to 4000 cells/135 μL with the culture medium. 135 μL of the cell suspension was added to each well of a 96-well cell culture plate and incubated overnight. On day 2, compounds at different concentrations were added, and the plate was placed in the incubator and incubated for 7 days. After the culture was completed, according to operation instructions for a CellTiter-Glo kit (Promega, G7573), 75 μL of CTG solution,

which was already pre-melted and equilibrated to room temperature, was added to each well, and the mixture was uniformly mixed for 2 minutes using a microplate shaker. The plate was placed at room temperature for 10 minutes, and then fluorescence signal values were determined using an Envision2104 plate reader (PerkinElmer). The inhibition rate was calculated using formula (1), where $RLU_{compound}$ was the readout of the drug treated group, $RLU_{control}$ was the average value of the solvent control group, and $RLU_{blank}$ was the average value of the cell-free well. The $IC_{50}$ value was calculated using GraphPad Prism software.

$$Inh.\% = (1-(RLU_{compound} - RLU_{blank}) / (RLU_{control} - RLU_{blank})) \times 100\% \text{ (formula 1)}$$

Results: the compound of the present invention had a significant inhibitory effect on breast tumour cells MDA-MB-436, with $IC_{50}$ value of less than 100 nM, further $IC_{50}$ value of less than 50 nM, furthermore $IC_{50}$ value of less than 20 nM, and the most excellent $IC_{50}$ value of less than 10 nM. At 10 μM, the maximum inhibition rate of the compound of the present invention on breast tumour cells MDA-MB-436 was up to 70% or more, further up to 80% or more, further up to 90%, with the optimal inhibition rate of 95% or more. The results of some examples were as shown in Table 1.

Table 1 MDA-MB-436 cell inhibitory activity

| Compound | $IC_{50}$ (nM) | Max inh.% 10 μM |
|---|---|---|
| 8 | 15 | 99.8 |
| 10 | 36 | 99.8 |
| 11 | 2 | 99.8 |
| 12 | 44 | 89.6 |
| 13 | 5 | 89.7 |
| 15 | 25 | 87.2 |
| 16 | 15 | 78.8 |
| 17 | 61 | 99.0 |
| 19 | 21 | 90.3 |
| 21 | 25 | 88.9 |
| 24 | 38 | 98.9 |
| 26 | 53 | 81.7 |
| 27 | 15 | 95.8 |
| 37 | 23 | 92.1 |
| 38 | 12 | 77.1 |
| 41 | 11 | 85.7 |
| 42 | 17 | 86.3 |
| 48 | 28 | 94.6 |
| 49 | 101 | 93.8 |
| 51 | 19 | 97.5 |
| 53 | 58 | 98.0 |
| 57 | 15.8 | 84.01 |
| 58 | 6.0 | 87.16 |
| 59 | 38 | 81.71 |
| 60 | 33 | 81.98 |
| 62 | 17.9 | 82.9 |
| 63 | 16.1 | 83.1 |

(continued)

| Compound | $IC_{50}$ (nM) | Max inh.% 10 μM |
|---|---|---|
| 64 | 27.9 | 84.9 |
| 65 | 39.5 | 76.8 |
| 66 | 97.8 | 93.2 |
| 67 | 100 | 96.2 |
| 68 | 12.3 | 88.6 |
| 69 | 15.3 | 88.4 |
| 71 | 73.4 | 99.0 |

Conclusion: the compound of the present invention has good inhibitory activity.

2. PARP1, PARP2 and PARP7 enzyme activity inhibition experiment

PARP1, PARP2 and PARP7 chemical fluorescence detection kits were all purchased from BPS Bioscience. The histone solution in the kit was diluted 5X with 1× PBS, and 25 μL of the diluted histone solution was added to a microwell plate and incubated overnight at 4°C. After the incubation was completed, the plate was washed three times with PBST (0.05% Tween-20). 25 μL of a blocking solution was added to the microwell plate and incubated at 25°C for 90 minutes. After the incubation was completed, the plate was washed three times with PBST. 2.5 μL of compounds at different concentrations diluted in test buffer and 5 μL of 2.5× substrate mixed solution were added to the microwell plate. PARP1, PARP2 and PARP7 enzymes were diluted to 0.33, 0.23 and 5.00 ng/μL, respectively, 5 μL of the diluent was added to the microwell plate, and the reaction system was incubated at 25°C for 60 minutes. After the incubation was completed, the plate was washed three times with PBST. Streptavidin-HRP was diluted 2000X with a blocking solution, and 25 μL of the diluent was added to the microwell plate and incubated at 25°C for 30 minutes. After the incubation was completed, the plate was washed three times with PBST. ELISA ECL substrate A and substrate B were mixed at a ratio of 1 : 1 (v/v), 25 μL of the mixture was added to the microwell plate, and the chemiluminescence value was read using a BMG microplate reader.

The inhibition rate was calculated according to formula $[(1-(RLU_{sample}-RLU_{min})/(RLU_{max}-RLU_{min})) \times 100\%]$, where $RLU_{sample}$ was the readout of the compound well, $RLU_{max}$ was the readout of the solvent control well, and $RLU_{min}$ was the readout of the control well without the PARP enzyme. Curve fitting was performed by four parameters (log(inhibitor) vs. response - Variable slope) using GraphPad Prism software, and the $IC_{50}$ value was calculated.

Table 2 PARP1, PARP2 and PARP7 enzyme activities

| Compound | PARP enzyme | $IC_{50}$ (nM) |
|---|---|---|
| 10 | PARP1 | 0.32 |
| 10 | PARP2 | 41 |
| 10 | PARP7 | 91 |

Conclusion: the inhibitory effect of compound 10 of the present invention on PARP2 and PARP7 enzyme activities in vitro is much weaker than the inhibitory effect on PARP1 enzyme activity, indicating that compound 10 has good PARP1 inhibitory selectivity.

3. Detection of PARP1 protein expression level in MDA-MB-231 cells

MDA-MB-231 cells were placed in a complete DMEM medium (supplemented with 10% FBS and 1% penicillin-streptomycin solution) and cultured at 37°C and 5% $CO_2$. The cells in the logarithmic growth phase were collected, the cell density was adjusted to $5 \times 10^5$ cells/well with the culture medium, and the cells were added to a 6-well cell culture plate at a volume of 1 mL/well. The compound to be tested was prepared to 2-fold the final concentration, 1 mL of the compounds at different concentrations were added to administration wells, a medium containing 0.2% DMSO was added to control wells, and the plate was incubated for 24 hours at 37°C and 5% $CO_2$. The cells were collected into a 1.5 mL centrifuge tube, 25 μL of RIPA lysis buffer (containing 1X protease inhibitor cocktail) was added, and the cells were

lysed on ice for 15 minutes and centrifuged at 12000 rpm/min at 4°C for 10 minutes. The supernatant was collected and the protein content was determined using the BCA method. The protein sample to be tested was diluted to 0.8 mg/mL, PARP1 was detected using a fully automated protein expression quantitative analyser (ProteinSimple), and the internal reference protein was β-actin (both antibodies were from CST). The software (Compass for SW) of the fully automated protein expression quantitative analyser was used to process the raw data and calculate the peak area and the PARP1 expression rate relative to the control group. $DC_{50}$ values were calculated using a four-parameter non-linear fitting model in Graphpad 8.3.0 software.

Results: the compound of the present invention had a significant inhibitory effect on MDA-MB-231 cells, with $DC_{50}$ value of less than 10 nM, further $DC_{50}$ value of less than 5 nM, furthermore $IC_{50}$ value of less than 2 nM, and the most excellent $IC_{50}$ value of less than 1 nM. The results of some examples were as shown in Table 3.

Table 3 PARP1 protein expression level in MDA-MB-231 cells

| Compound | $DC_{50}$ (nM) |
|---|---|
| 1 | 0.14 |
| 5 | 0.12 |
| 10 | 0.12 |

4. Detection of PARP2 protein expression level in MDA-MB-231 cells

MDA-MB-231 cells were placed in a complete DMEM medium (supplemented with 10% FBS and 1% penicillin-streptomycin solution) and cultured at 37°C and 5% $CO_2$. The cells in the logarithmic growth phase were collected, the cell density was adjusted to $5 \times 10^5$ cells/well with the culture medium, and the cells were added to a 6-well cell culture plate at a volume of 1 mL/well, and cultured overnight in a 37°C, 5% $CO_2$ incubator. On day 2, the compound to be tested was prepared to 2-fold the final concentration, 1 mL of the compounds at different concentrations were added to administration wells and a medium containing 0.2% DMSO was added to control wells, and the plate was incubated for 24 hours at 37°C and 5% $CO_2$. The cells were collected into a 1.5 mL centrifuge tube, 25 μL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 2 mg/mL, PARP2 was detected using a fully automated protein expression quantitative analyser (ProteinSimple), and the internal reference protein was β-actin (both antibodies were from CST). The software (Compass for SW) of the fully automated protein expression quantitative analyser was used to process the raw data and calculate the peak area and the PARP2 expression rate relative to the control group. $DC_{50}$ values were calculated using a four-parameter non-linear fitting model in Graphpad 8.3.0 software.

Table 4 PARP2 protein expression level in MDA-MB-231 cells

| Compound | $DC_{50}$ (nM) |
|---|---|
| 10 | > 5 |

Conclusion: the degradation activity of compound 10 of the present invention on PARP2 protein of MDA-MB-231 cells is much weaker than the degradation activity on PARP1 protein, indicating that compound 10 has good PARP1 degradation selectivity.

**5.** Detection of PARP1 protein expression level in MDA-MB-436 cells

MDA-MB-436 cells were placed in a complete DMEM medium (supplemented with 15% FBS; 10 μg/mL Insulin; 16 μg/mL Glutathione and 1% penicillin-streptomycin solution), and cultured at 37°C under $CO_2$-free conditions. The cells in the logarithmic growth phase were collected, the cell density was adjusted to $2 \times 10^5$ cells/well with the culture medium, and the cells were added to a 6-well cell culture plate at a volume of 1 mL/well. The compound to be tested was prepared to 2-fold the final concentration, 1 mL of the compounds at different concentrations were added to administration wells, a medium containing 0.2% DMSO was added to control wells, and the plate was incubated for 24 hours at 37°C. The cells were collected into a 1.5 mL centrifuge tube, 25 μL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 0.8 mg/mL, PARP1 was detected using a fully automated protein expression

quantitative analyser (ProteinSimple), and the internal reference protein was β-actin (both antibodies were from CST). The software (Compass for SW) of the fully automated protein expression quantitative analyser was used to process the raw data and calculate the peak area and the PARP1 expression rate relative to the control group.

MDA-MB-436 cells were placed in a complete L-15 medium (supplemented with 15% FBS; 10 μg/mL Insulin; 16 μg/mL Glutathione and 1% penicillin-streptomycin solution), and cultured at 37°C under $CO_2$-free conditions. The cells in the logarithmic growth phase were collected, the cell density was adjusted to $2 \times 10^5$ cells/well with the culture medium, and the cells were added to a 6-well cell culture plate at a volume of 1 mL/well and cultured overnight. On day 2, the compound to be tested was prepared to 2-fold the final concentration, 1 mL of the compounds at different concentrations were added to administration wells, a medium containing 0.2% DMSO was added to control wells, and the plate was incubated for 24 hours at 37°C. The cells were collected into a 1.5 mL centrifuge tube, 25 μL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 0.8 mg/mL, PARP1 was detected using a fully automated protein expression quantitative analyser (ProteinSimple), and the internal reference protein was β-actin (both antibodies were from CST). The software (Compass for SW) of the fully automated protein expression quantitative analyser was used to process the raw data and calculate the peak area and the PARP1 expression rate relative to the control group. $DC_{50}$ values were calculated using a four-parameter non-linear fitting model in Graphpad 8.3.0 software.

Results: the compound of the present invention had a significant inhibitory effect on MDA-MB-436 cells, with the maximum degradation rate on the PARP1 protein of breast tumour cells MDA-MB-436 at 100 nM of up to 70% or more, further the maximum degradation rate of up to 80% or more, and the optimal degradation rate of up to 90% or more. The $DC_{50}$ value of compound 10 was 0.014 nM. The results of some examples were as shown in Table 5.

Table 5 PARP1 protein expression level in MDA-MB-436 cells

| Compound | $DC_{50}$ (nM) | PARP1 degradation rate @ 100 nM |
|---|---|---|
| 10 | 0.014 | 79% |
| 24 | - | 76.2% |
| 26 | - | 83.5% |
| 31 | - | 92.1% |
| 57 | - | 80.6% |
| 58 | - | 79.6% |
| 66 | - | 76.4% |

6. Detection of PARP2 protein expression level in MDA-MB-436 cells

MDA-MB-436 cells were placed in a complete L-15 medium (supplemented with 15% FBS; 10 μg/mL Insulin; 16 μg/mL Glutathione and 1% penicillin-streptomycin solution), and cultured at 37°C under $CO_2$-free conditions. The cells in the logarithmic growth phase were collected, the cell density was adjusted to $2 \times 10^5$ cells/well with the culture medium, and the cells were added to a 6-well cell culture plate at a volume of 1 mL/well and cultured overnight. On day 2, the compound to be tested was prepared to 2-fold the final concentration, 1 mL of the compounds at different concentrations were added to administration wells, a medium containing 0.2% DMSO was added to control wells, and the plate was incubated for 24 hours at 37°C. The cells were collected into a 1.5 mL centrifuge tube, 25 μL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 2 mg/mL, PARP2 was detected using a fully automated protein expression quantitative analyser (ProteinSimple), and the internal reference protein was β-actin (both antibodies were from CST). The software (Compass for SW) of the fully automated protein expression quantitative analyser was used to process the raw data and calculate the peak area and the PARP2 expression rate relative to the control group. $DC_{50}$ values were calculated using a four-parameter non-linear fitting model in Graphpad 8.3.0 software.

Table 6 PARP2 protein expression level in MDA-MB-436 cells

| Compound | $DC_{50}$ (nM) |
|---|---|
| 10 | > 10000 |

Conclusion: the degradation activity of compound 10 of the present invention on PARP2 protein of MDA-MB-436 cells is much weaker than the degradation activity on PARP1 protein, indicating that compound 10 has good PARP1 degradation selectivity.

7. Pharmacokinetic test in rats

7.1 Experimental animals: male SD rats, about 220 g, 6-8 weeks old, 6 rats/compound, purchased from Chengdu Ddossy Experimental Animals Co., Ltd.

7.2 Experimental design: on the day of the experiment, 12 SD rats were randomly grouped according to their body weights. The animals were fasted with water available for 12 to 14 h one day before the administration, and were fed 4 h after the administration.

Table 7. Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Compound 10 | 2.5 | 0.5 | 5 | Plasma | Intravenously |
| G2 | 3 | | 10 | 1 | 10 | Plasma | Intragastrically |
| G3 | 3 | Compound 58 | 2.5 | 0.5 | 5 | Plasma | Intravenously |
| G4 | 3 | | 10 | 1 | 10 | Plasma | Intragastrically |
| Notes: solvent for intravenous administration: 10%DMA + 10% Solutol + 80% Saline; solvent for intragastric administration: 5% DMSO + 30% PEG400 + 65% (20% SBE-CD) (DMA: dimethylacetamide; Solutol: polyethylene glycol-15-hydroxystearate; Saline: physiological saline; DMSO: dimethyl sulphoxide; SBE-CD: β cyclodextrin) | | | | | | | |

Before and after the administration, 0.15 mL of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous administration group and intragastric administration group were: 0, 5 min, 15 min, 30 min, 1 h, 2 h, 4 h, 6 h, 8 h, and 24 h. Before analysis and detection, all samples were stored at -80°C. The samples were analysed quantitatively by LC-MS/MS. The results of some examples were as shown in Table 8.

Table 8 Pharmacokinetic parameters of test compounds in plasma of rats

| Test compound | Mode of administration | CL (mL/min/kg) | Vdss (L/kg) | AUC0-t (hr*ng/mL) | F (%) |
|---|---|---|---|---|---|
| Compound 10 | i.v. (2.5 mg/kg) | 1.42 | 0.405 | 31206 | - |
| | i.g. (10 mg/kg) | - | - | 42874 | 34.3 |
| Compound 58 | i.v. (2.5 mg/kg) | 0.713 | 0.366 | 51389 | - |
| | i.g. (10 mg/kg) | - | - | 12776 | 6.2 |
| -: not applicable. | | | | | |

Conclusion: compound 10 and compound 58 have good pharmacokinetic characteristics in rats.

8. Pharmacokinetic test in mice

8.1 Experimental animals: male ICR mice, 20 to 25 g, 18 mice/compound, purchased from Chengdu Ddossy Experimental Animals Co., Ltd.

8.2 Experimental design: on the day of the experiment, 36 ICR mice were randomly grouped according to their body weights. The animals were fasted with water available for 12 to 14 h one day before the administration, and were fed 4 h after the administration.

**Table 9. Administration information**

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 9 | Compound 10 | 2.5 | 0.5 | 5 | Plasma | Intravenously |
| G2 | 9 | | 10 | 1 | 10 | Plasma | Intragastrically |
| G3 | 9 | Compound 58 | 2.5 | 0.5 | 5 | Plasma | Intravenously |
| G4 | 9 | | 10 | 1 | 10 | Plasma | Intragastrically |
| Notes: solvent for intravenous administration: 5% DMA + 5% Solutol + 90% Saline; solvent for intragastric administration: 5% DMSO + 30% PEG400 + 65% (20% SBE-CD) (DMA: dimethylacetamide; Solutol: polyethylene glycol-15-hydroxystearate; Saline: physiological saline; DMSO: dimethyl sulphoxide; SBE-CD: β cyclodextrin) | | | | | | | |

Before and after the administration, 0.06 mL of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous group and intragastric administration group were: 0 min, 5 min, 15 min, 30 min, 1 h, 2 h, 4 h, 6 h, 8 h, and 24 h. Before analysis and detection, all samples were stored at -80°C. The samples were analysed quantitatively by LC-MS/MS.

**Table 10. Pharmacokinetic parameters of test compounds in plasma of mice**

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 10 | i.v. (2.5 mg/kg) | 2.02 | 0.358 | 20635 | - |
| | i.g. (10 mg/kg) | - | - | 69842 | 84.6 |
| Compound 58 | i.v. (2.5 mg/kg) | 0.0968 | 0.189 | 224429 | - |
| | i.g. (10 mg/kg) | - | - | 140413 | 15.6 |
| -: not applicable. | | | | | |

Conclusion: compound 10 and compound 58 have good pharmacokinetic characteristics in mice.

9. Mouse MDA-MB-436 subcutaneous in vivo transplanted tumour model

Human breast cancer MDA-MB-436 cells were placed in Leibovitz's L-15 medium (supplemented with 10 μg/mL insulin, 16 μg/mL glutathione, 10% foetal bovine serum and 1% penicillin-streptomycin solution) and cultured at 37°C. Conventional digestion treatment with trypsin was performed twice a week for passage. When the cell saturation was 80%-90%, and the number reached the requirement, the cells were harvested, counted, and inoculated. BALB/c nude mice (from Beijing Vital River Laboratory Animal Technology Co., Ltd.) were subcutaneously inoculated with 0.2 mL ($10 \times 10^6$) of MDA-MB-436 cells (plus matrigel, with the volume ratio of 1 : 1) on the right back. When the average tumour volume reached about 180 $mm^3$, grouping and administration were performed (marked as Day 0). The solvent group was given 5% DMSO, 30% PEG400 and 65% of 20% sulphobutyl-β-cyclodextrin solution, and the administration group was given 1 or 10 mg/kg compound 10. The administration frequency was once a day, the administration cycle was 29 days, and the drug withdrawal observation period was set to 14 days. After grouping, the tumour diameter was measured twice a week with a vernier calliper. The formula for calculating the tumour volume was: $V = 0.5 \times a \times b^2$, where a and b represented the long and short diameters of the tumour, respectively. The tumour inhibitory effect of compound 10 was evaluated by TGI (%) = [1-(average tumour volume at the end of administration in the treatment group - average tumour volume at the beginning of administration in the treatment group)/(average tumour volume at the end of treatment in the solvent control group - average tumour volume at the beginning of treatment in the solvent control group)] × 100%. The tumour growth curve and the animal body weight change curve were as shown in Figure 1 and Figure 2, respectively.

Test results: after 28 days of administration, the TGIs of the groups administered with 1 mg/kg compound 10 and 10 mg/kg compound 10 were 47.14% and 111%, respectively; after the drug withdrawal, the tumour of the animals in the group administered with 1 mg/kg compound 10 continued to grow, with the tumour volumes comparable to those in the solvent group on day 43 after administration; and after the drug withdrawal, the tumour volume of the animals in the group administered with 10 mg/kg compound 10 was consistently smaller than the tumour volume at the time of grouping, that is, the tumours grew very slowly. There was no significant decrease in the body weight of the animals in the group given compound 10.

Conclusion: in the mouse MDA-MB-436 subcutaneous in vivo transplanted tumour model, compound 10 of the present invention has good efficacy in inhibiting tumour growth and inducing tumour regression, and is well tolerated.

## Claims

1. A PARP-1 degradation agent represented by formula (I), or a stereoisomer, deuterated compound, solvate, pharmaceutically acceptable salt or co-crystal thereof,

PTM-L-ULM (I)

**characterised in that** ULM is an E3 ubiquitin ligase binding moiety;
L is a bond or chemically linking moiety that covalently couples the PTM and ULM;

the PTM is a PARP-1 binding moiety;

the PTM has a structure of formula (I-1), (I-2), (I-3) or (I-4), the L covalently binds to the PTM by replacing any H in formula (I-1), (I-2), (I-3) or (I-4) and thus forming a single bond with the PTM, or L covalently binds to PTM by forming, together with $R_4$ and $R_5$ and the N atom to which they are attached, 4- to 10-membered heterocycloalkyl containing 1-2 N atoms and 0-2 heteroatoms selected from S and O;

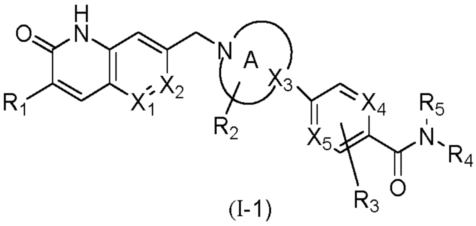
(I-1)

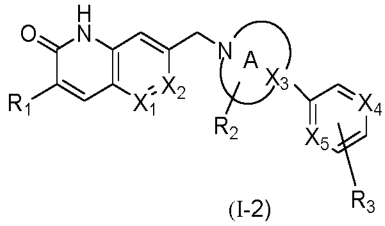
(I-2)

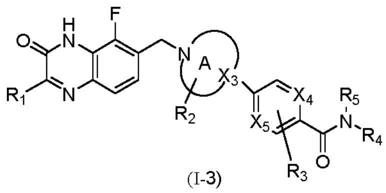
(I-3)

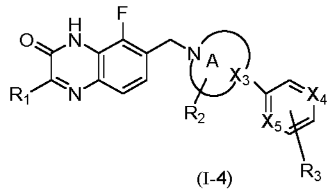
(I-4)

$X_1$, $X_2$, $X_3$, $X_4$ and $X_5$ are independently N or $CR_6$;

ring A is 4- to 12-membered heterocycloalkyl containing 1-2 N atoms and 0-2 heteroatoms selected from S and O;

$R_1$ is H, $C_{1-4}$ alkyl, halogen, CN or $C_{3-6}$ cycloalkyl, wherein the alkyl and cycloalkyl are optionally substituted with 1-3 groups selected from halogen, OH, $C_{1-4}$ alkoxy, $NH_2$ and COOH;

$R_2$ is H, $C_{1-4}$ alkyl, $C_{1-4}$ alkoxy, halogen, OH or $NH_2$, wherein the alkyl and alkoxy are optionally substituted with 1-3 groups selected from halogen, OH, $NH_2$, COOH and $C_{3-6}$ cycloalkyl;

$R_3$ is H, $C_{1-4}$ alkyl, halogen, $C_{1-4}$ alkoxy or $NH_2$, wherein the alkyl and alkoxy are optionally substituted with 1-3 groups selected from halogen, OH, $NH_2$, COOH and $C_{3-6}$ cycloalkyl;

$R_4$ is H, $C_{1-4}$ alkyl, $C_{3-6}$ cycloalkyl or 4- to 7-membered heterocycloalkyl containing 1-3 heteroatoms selected from N, S and O, wherein the alkyl, cycloalkyl and heterocycloalkyl are optionally substituted with 1-3 groups selected from halogen, OH, $NH_2$, COOH and $C_{3-6}$ cycloalkyl;

$R_5$ is H, $C_{1-4}$ alkyl or $C_{3-6}$ cycloalkyl, wherein the alkyl and cycloalkyl are optionally substituted with 1-3 groups selected from halogen, OH, $NH_2$ and COOH; or

optionally, $R_4$ and $R_5$ together with the N atom to which they are attached form 4- to 10-membered heterocycloalkyl containing 1-3 heteroatoms selected from N, S and O, wherein the heterocycloalkyl is optionally substituted with 1-3 groups selected from halogen, OH, $NH_2$, COOH and $C_{3-6}$ cycloalkyl;

each $R_6$ is independently H, $C_{1-4}$ alkyl, $C_{1-4}$ alkoxy, halogen, CN or $C_{3-6}$ cycloalkyl, wherein the alkyl or cycloalkyl is optionally substituted with 1-3 groups selected from halogen, OH, $NH_2$ and COOH.

**2.** The PARP-1 degradation agent, or the stereoisomer, deuterated compound, solvate, pharmaceutically acceptable salt or co-crystal thereof according to claim 1, **characterised in that** the PARP-1 degradation agent has a structure of formula (II), (III), (IV), (V), (VI) or (VII):

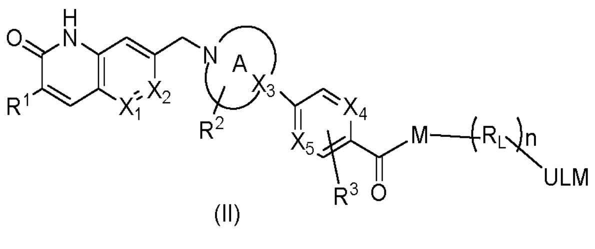
(II)

,

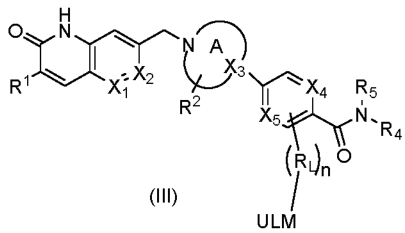
(III)

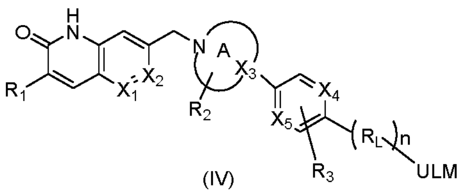
(IV)

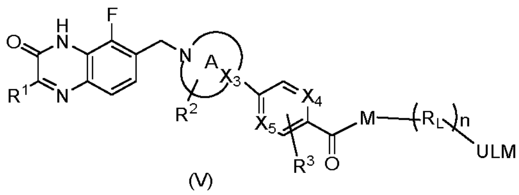
(V)

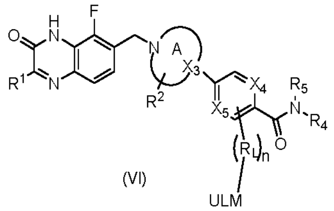
(VI)

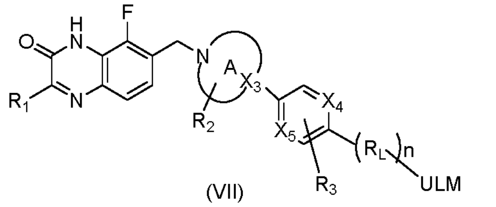
(VII)

wherein ring A is 4- to 12-membered monocyclic heterocycloalkyl, spiro heterocycloalkyl, bridged heterocycloalkyl or fused heterocycloalkyl containing 1-2 N atoms and 0-2 heteroatoms selected from S and O;
M is $-NR_5-$ or

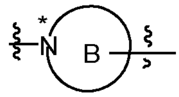
,

ring B is 4- to 10-membered heterocycloalkyl containing 1-2 N atoms and 0-2 heteroatoms selected from S and O, and * is the end connected to group on the left side;
each $R_L$ is independently $-NR_N-$, $C_{1-3}$ alkylene, -CO-, -O-, -S-, $C_{3-6}$ cycloalkylene, phenyl, 5- to 6-membered heteroaryl containing 1-3 heteroatoms selected from N, S and O, 4- to 10-membered heterocycloalkylene containing 1-3 heteroatoms selected from N, S and O, $C_{2-6}$ alkenylene, or $C_{2-6}$ alkynylene, wherein the alkylene, alkenylene, alkynylene, cycloalkylene, phenyl, heteroaryl and heterocycloalkylene are optionally substituted with 1-3 groups selected from $C_{1-4}$ alkyl, halogen, CN, $C_{1-4}$ alkoxy and halo $C_{1-4}$ alkoxy,
provided that in $-M-(R_L)n-$, two identical $-NR_N-$, -O- or -S- are not attached;
$R_N$ is H or $C_{1-4}$ alkyl;
n is an integer of 0-10;
ULM is selected from U1, U2, U3, U4, U5, U6 or U7:

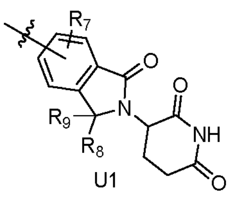
U1 ,

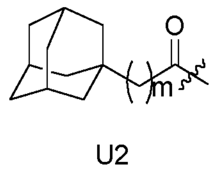
U2 ,

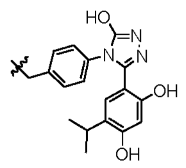
U3 ,

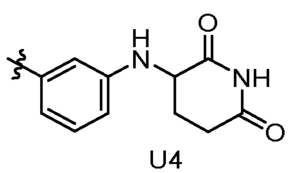
U4 ,

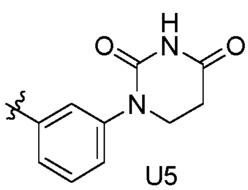
U5 ,

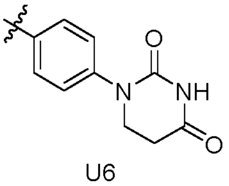
U6 ,

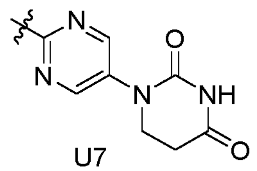
U7 ;

m is 1, 2 or 3;
$R_7$ is H, halogen, CN, $NH_2$, OH, $C_{1-4}$ alkyl or $C_{1-4}$ alkoxy, wherein the alkyl or alkoxy is optionally substituted with 1-3 groups selected from halogen, CN, $NH_2$ and OH;
$R_8$ and $R_9$ are H or together form =O.

**3.** The PARP-1 degradation agent, or the stereoisomer, deuterated compound, solvate, pharmaceutically acceptable salt or co-crystal thereof according to claim 2, **characterised in that** the PARP-1 degradation agent has a structure of formula (II-1), (III-1), (IV-1), (II-2), (III-2), (IV-2), (V-1), (VI-1), (VII-1), (V-2), (VI-2) or (VII-2):

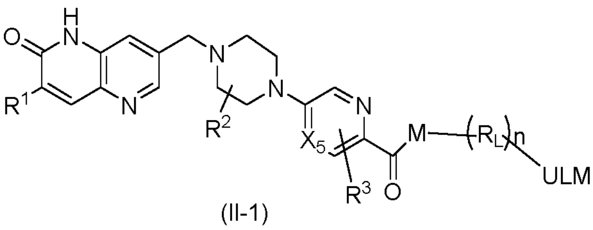
(II-1)

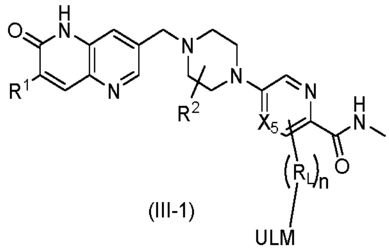
(III-1)
,

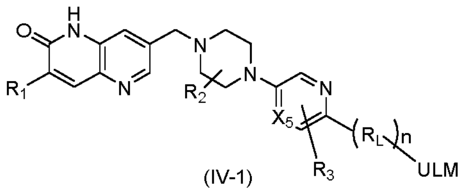
(IV-1)
,

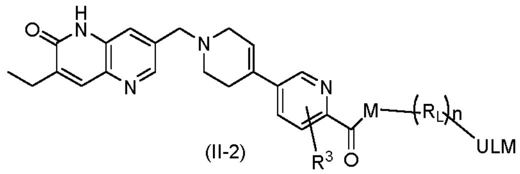
(II-2)

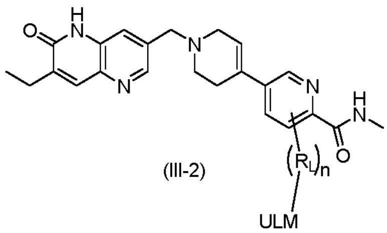
(III-2)
,

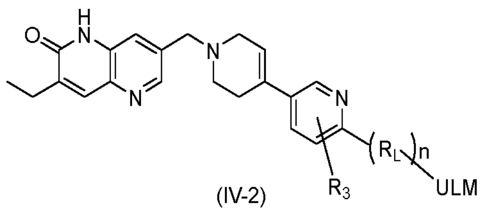
(IV-2)

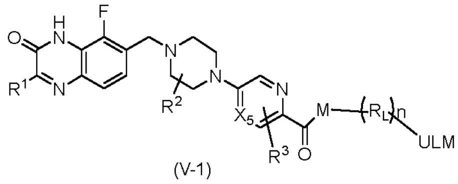
(V-1)

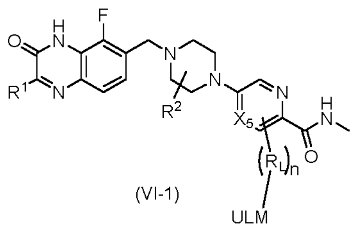
(VI-1)

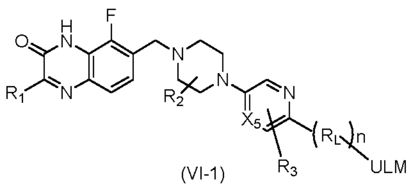
(VI-1)

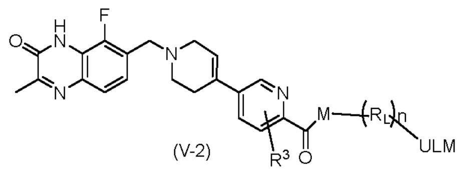
(V-2)

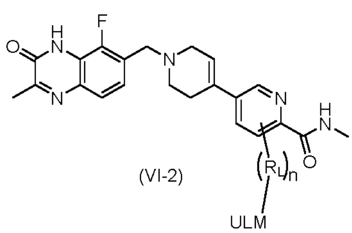
(VI-2)

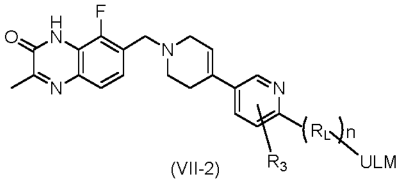
(VII-2)
.

**4.** The PARP-1 degradation agent, or the stereoisomer, deuterated compound, solvate, pharmaceutically acceptable

salt or co-crystal thereof according to claim 3,

**characterised in that** $X_5$ is CH or N;
$R_1$ is H or $C_{1-3}$ alkyl, wherein the alkyl is optionally substituted with 1-3 groups selected from F, Cl, Br, I, OH, $NH_2$ and COOH;
$R_2$ is H, $C_{1-3}$ alkyl, $C_{1-3}$ alkoxy, F, Cl, Br or I;
$R_3$ is H, $C_{1-3}$ alkyl, $C_{1-3}$ alkoxy, F, Cl, Br or I;
n is 1, 2, 3, 4, 5, 6 or 7;
$R_5$ is H;
ring B is 4- to 10-membered monocyclic heterocycloalkyl or spiro heterocycloalkyl containing 1-2 N atoms and 0-2 heteroatoms selected from S and O;
each $R_L$ is independently -NH-, $C_{1-3}$ alkylene, -CO-, -O-, $C_{3-6}$ cycloalkylene, phenyl, 5- to 6-membered heteroaryl containing 1-3 heteroatoms selected from N, S and O, 4- to 10-membered heterocycloalkylene containing 1-3 heteroatoms selected from N, S and O, $C_{2-6}$ alkenylene, or $C_{2-6}$ alkynylene, wherein the alkylene, alkenylene, alkynylene, phenyl, heteroaryl, cycloalkylene and heterocycloalkylene are optionally substituted with 1-3 groups selected from $C_{1-3}$ alkyl, F, Cl, Br, I, CN, $C_{1-3}$ alkoxy and halo $C_{1-3}$ alkoxy;
provided that in -M-$(R_L)$n-, two identical -NH- or -O- are not attached;
$R_7$ is H, $C_{1-3}$ alkyl, F, Cl, Br or I;
$R_8$ and $R_9$ are H or =O;
m is 1 or 2.

5. The PARP-1 degradation agent, or the stereoisomer, deuterated compound, solvate, pharmaceutically acceptable salt or co-crystal thereof according to claim 3, **characterised in that** -M-$(R_L)$n- or -$(R_L)$n- in formula (II-1), (III-1), (IV-1), (II-2), (III-2), (IV-2), (V-1), (VI-1), (VII-1), (V-2), (VI-2) or (VII-2) is selected from one of the following structures, and * is the end connected to the group on the left side:

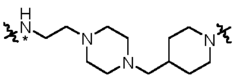 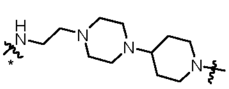 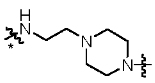 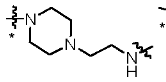 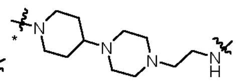

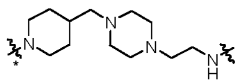 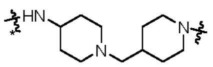 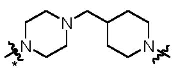 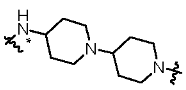 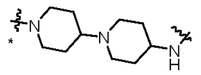

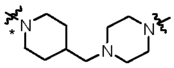 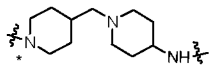 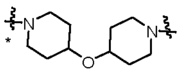 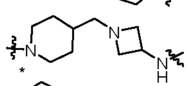 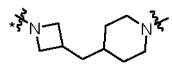

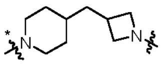 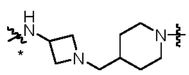 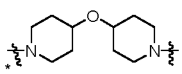 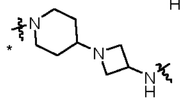 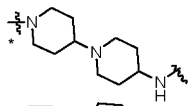

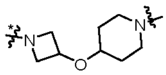 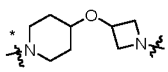 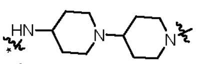 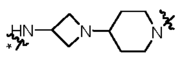 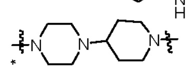

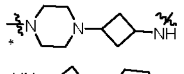 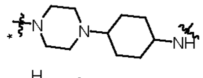 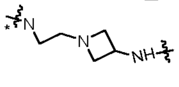 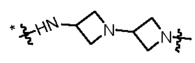 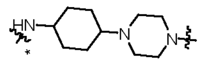

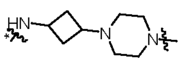 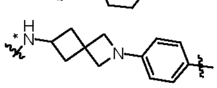 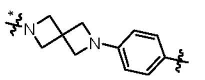 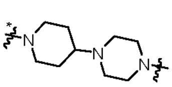

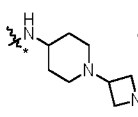 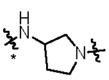 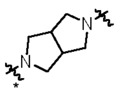 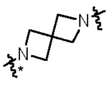 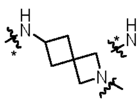 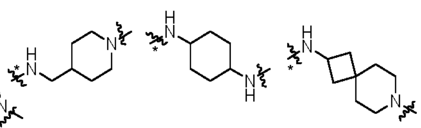

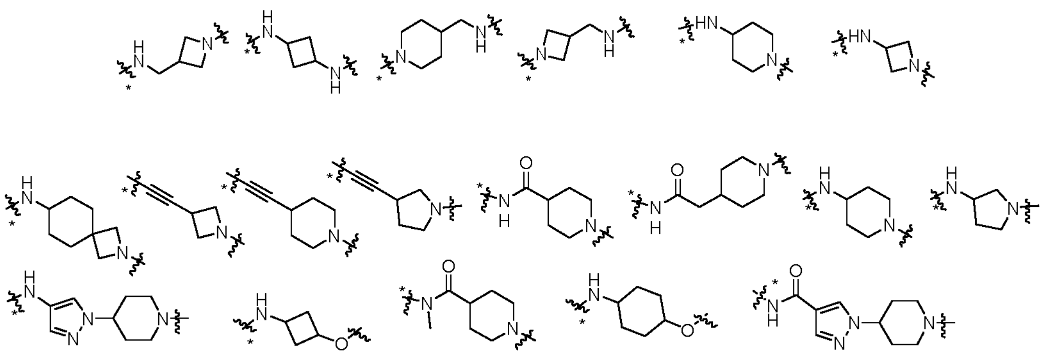

.

6. The PARP-1 degradation agent, or the stereoisomer, deuterated compound, solvate, pharmaceutically acceptable salt or co-crystal thereof according to claim 1, **characterised in that** the degradation agent is selected from one of the following structures:

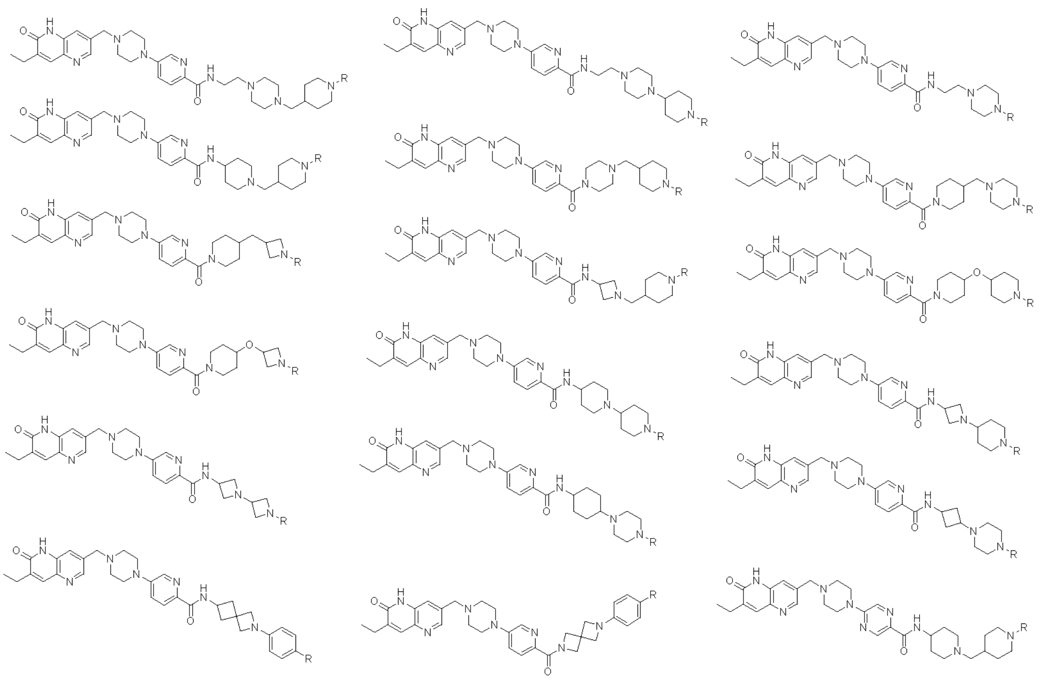

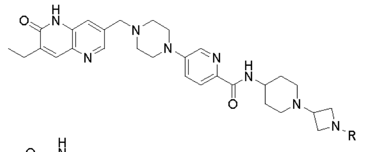
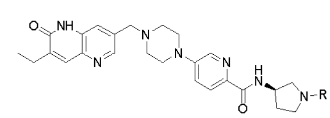
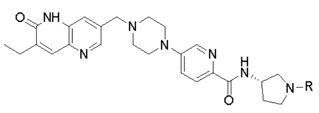
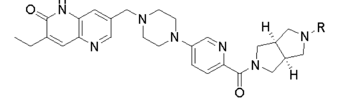
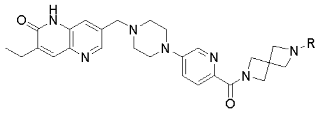
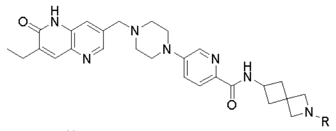
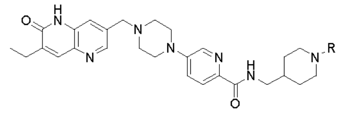
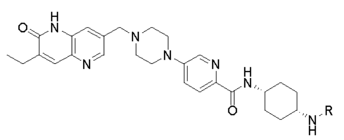
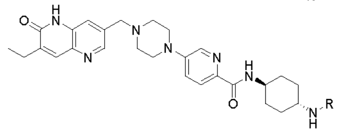
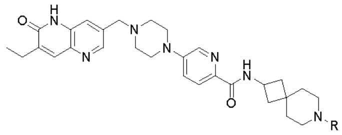
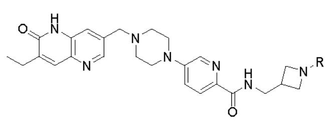
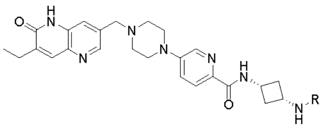
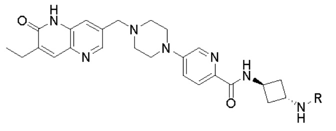
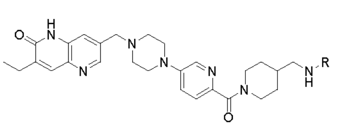
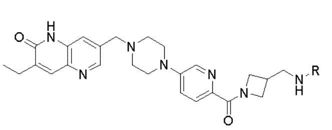
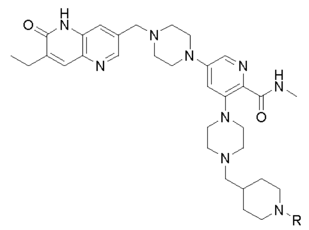
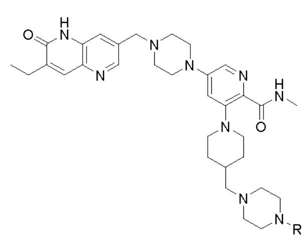
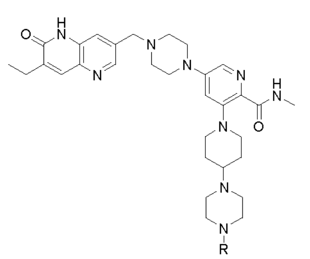
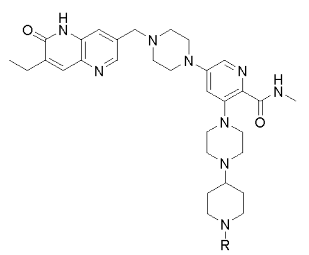
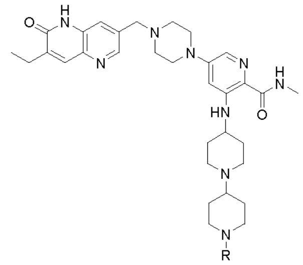
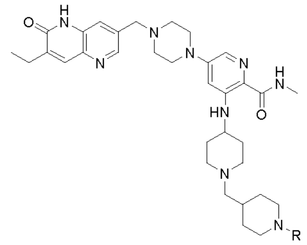
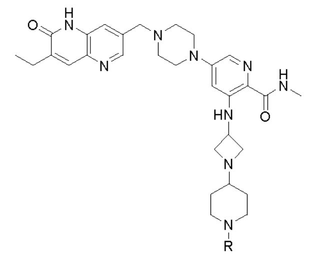
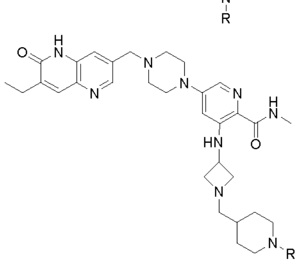
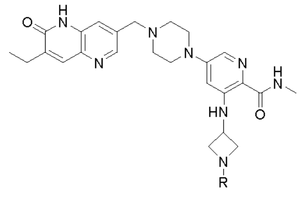
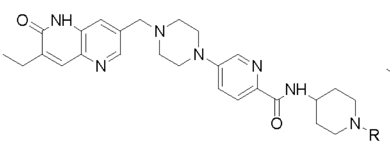
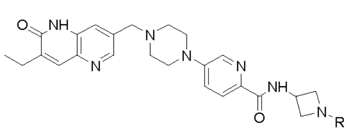
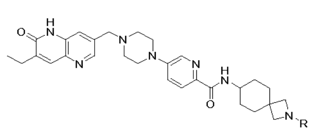

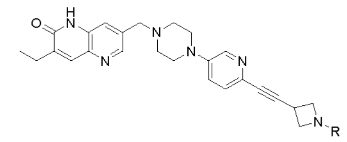 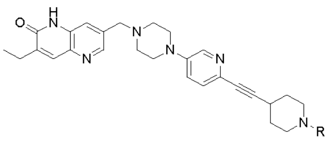 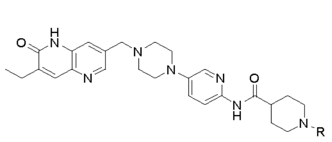

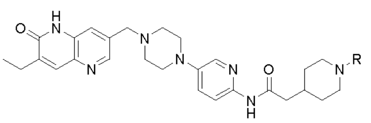 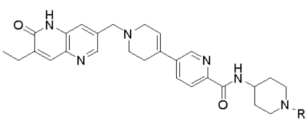 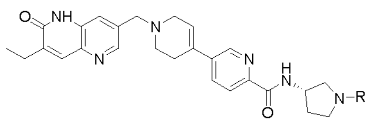

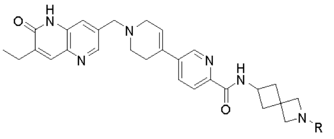 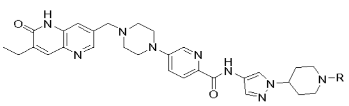 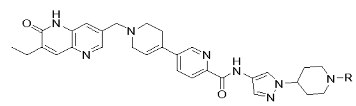

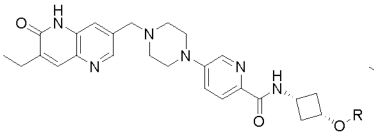 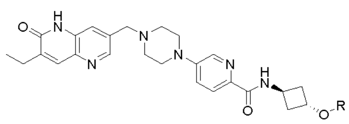 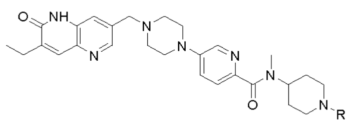

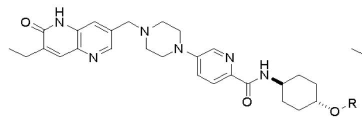 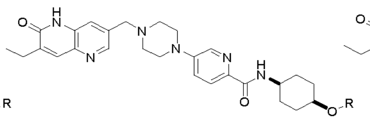 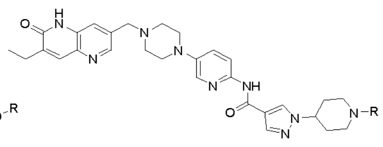

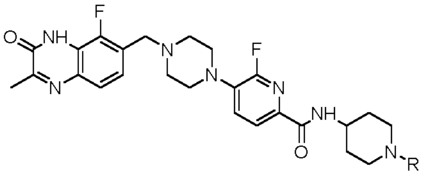, 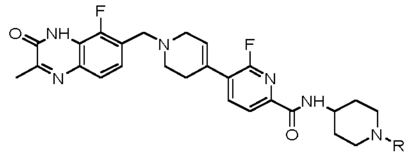;

wherein in each of the structures above,

R= 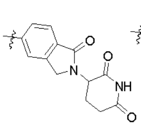 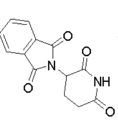 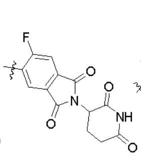 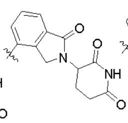 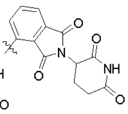 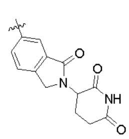 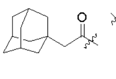 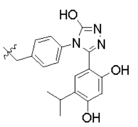

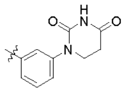 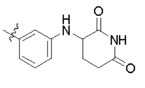 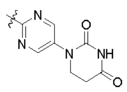 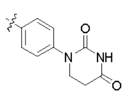

**7.** The PARP-1 degradation agent, or the stereoisomer, deuterated compound, solvate, pharmaceutically acceptable salt or co-crystal thereof according to claim 1, **characterised in that** the degradation agent is selected from one of the structures in Table A.

**8.** A pharmaceutical composition, **characterised in that** the pharmaceutical composition comprises the PARP-1 degradation agent, or the stereoisomer, deuterated compound, solvate, pharmaceutically acceptable salt or co-crystal thereof according to any one of claims 1-7, and a pharmaceutically acceptable carrier and/or excipient.

**9.** Use of the PARP-1 degradation agent, or the stereoisomer, deuterated compound, solvate, pharmaceutically acceptable salt or co-crystal thereof according to any one of claims 1-7, or the composition according to claim 8 in the preparation of a drug for treating/preventing a PARP-1-mediated disease.

**10.** The use according to claim 9, **characterised in that** the PARP-1-mediated disease is breast cancer, ovarian cancer, uterine cancer or cervical cancer.

**11.** A pharmaceutical composition or pharmaceutical preparation, **characterised in that** the pharmaceutical composition or pharmaceutical preparation comprises 1-1500 mg of the compound, or the stereoisomer, deuterated compound, solvate, or pharmaceutically acceptable salt thereof according to any one of claims 1-7, and a pharmaceutically acceptable carrier and/or adjuvant.

**12.** A method for treating a disease in a mammal, the method comprising administering to a subject a therapeutically effective amount of the compound, or the stereoisomer, deuterated compound, solvate, or pharmaceutically acceptable salt thereof according to any one of claims 1-7, **characterised in that** the therapeutically effective amount is preferably 1-1500 mg, and the disease is preferably breast cancer, ovarian cancer, uterine cancer or cervical cancer.

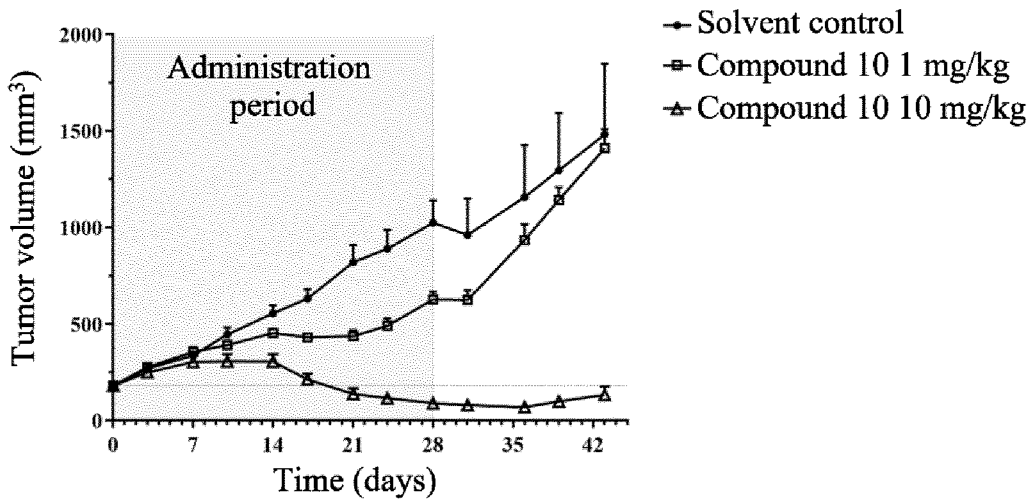


Figure 1

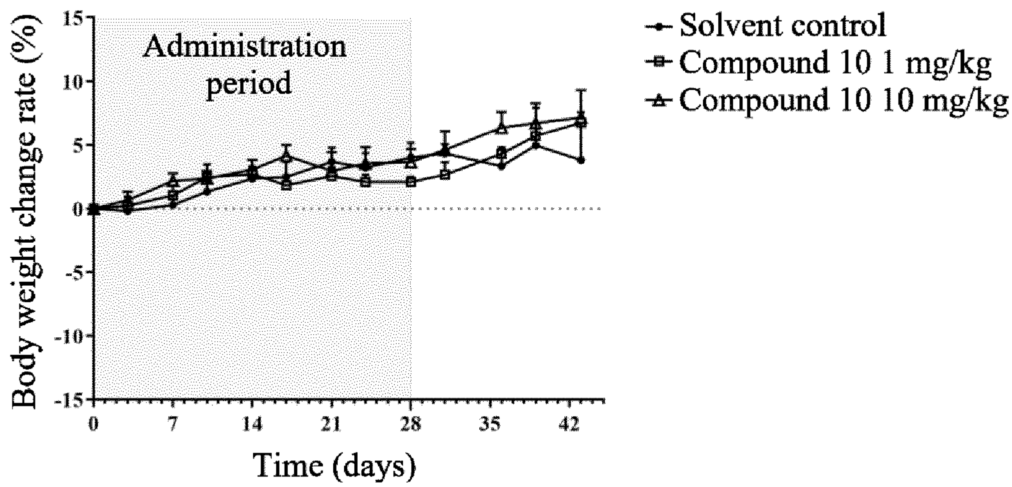


Figure 2

## INTERNATIONAL SEARCH REPORT

International application No.
**PCT/CN2022/126611**

**A. CLASSIFICATION OF SUBJECT MATTER**

C07D 471/04(2006.01)i; C07D 401/12(2006.01)i; A61K 31/498(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, VEN, WOTXT, USTXT, EPTXT, JPTXT, KRTXT, cnki, 读秀, DUXIU, Pubmed, Science Direct, ISI_Web of Science, stn: 海思科, 李瑶, 结构检索, 癌症, 乳腺癌, 卵巢癌, 子宫癌, 宫颈癌, PARP, structure search, cancer, breast cancer, ovarian cancer, uterine cancer, cervial cancer

**C. DOCUMENTS CONSIDERED TO BE RELEVANT**

| Category* | Citation of document, with indication, where appropriate, of the relevant passages | Relevant to claim No. |
|---|---|---|
| Y | CN 114144413 A (ASTRAZENECA AB) 04 March 2022 (2022-03-04)<br>abstract, description, embodiments 1-32, paragraphs 0023 and 0025, and claims 1-37 | 1-12 |
| Y | CN 111606969 A (SICHUAN UNIVERSITY) 01 September 2020 (2020-09-01)<br>description, paragraphs 0011-0015 | 1-12 |
| Y | CN 110563703 A (ZHEJIANG ACADEMY OF MEDICAL SCIENCES) 13 December 2019 (2019-12-13)<br>claims 1-10 | 1-12 |
| Y | CN 108601764 A (ARVINAS INC. et al.) 28 September 2018 (2018-09-28)<br>claims 1-26 | 1-12 |
| Y | WO 2009053373 A1 (JANSSEN PHARMACEUTICA NV.) 30 April 2009 (2009-04-30)<br>claims 1-13 | 1-12 |
| Y | JOHANNES, J. W. et al. "Discovery of 5-{4-[(7-Ethyl-6-oxo-5, 6-dihydro-1,5-naphthyridin-3-yl)methyl]piperazin-1-yl}-N-methylpyridine-2-carboxamide (AZD5305): A PARP1-DNA Trapper with High Selectivity for PARP1 over PARP2 and Other PARPs"<br>*Journal of Medicinal Chemistry,* Vol. 64, 27 September 2021 (2021-09-27),<br>pages 14498-14512 | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 December 2022** | **15 December 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/126611**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:

[1] Claim 12 sets forth a method for treating a disease in a mammal. The claim relates to a treatment method which uses a human or animal body as a direct object. Therefore, claim 12 does not comply with PCT Rule 39.1(iv). A search for claim 12 was made on the basis of a use of the PARP-1 degradation agent according to any one of claims 1-7, and a stereoisomer, deuterated substance, solvate, and pharmaceutically acceptable salt or eutectic crystal thereof in the preparation of a drug for treating breast cancer, ovarian cancer, uterine cancer or cervical cancer.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/126611**

| Patent document cited in search report | Publication date (day/month/year) | Patent family member(s) | Publication date (day/month/year) |
|---|---|---|---|
| CN 114144413 A | 04 March 2022 | AU 2020318599 A1 | 10 March 2022 |
| | | US 2021040084 A1 | 11 February 2021 |
| | | UY 38793 A | 26 February 2021 |
| | | CR 20220070 A | 21 March 2022 |
| | | EC SP22012826 A | 31 March 2022 |
| | | BR 112022000534 A2 | 10 May 2022 |
| | | AR 119424 A1 | 15 December 2021 |
| | | IL 289534 A | 01 March 2022 |
| | | KR 20220035941 A | 22 March 2022 |
| | | JP 2022541483 A | 26 September 2022 |
| | | CL 2022000110 A1 | 20 September 2022 |
| | | DO P2022000006 A | 15 March 2022 |
| | | CO 2022001590 A2 | 18 March 2022 |
| | | US 2022227768 A1 | 21 July 2022 |
| | | TW 202116750 A | 01 May 2021 |
| | | EP 3999506 A1 | 25 May 2022 |
| | | CA 3145644 A1 | 28 January 2021 |
| | | WO 2021013735 A1 | 28 January 2021 |
| CN 111606969 A | 01 September 2020 | None | |
| CN 110563703 A | 13 December 2019 | None | |
| CN 108601764 A | 28 September 2018 | CA 2979070 A1 | 22 September 2016 |
| | | AU 2020273338 A1 | 17 December 2020 |
| | | US 2020392131 A1 | 17 December 2020 |
| | | AU 2016232705 A1 | 21 September 2017 |
| | | EP 3270917 A1 | 24 January 2018 |
| | | MX 2017011919 A | 22 May 2018 |
| | | HK 1249058 A1 | 26 October 2018 |
| | | JP 2021073251 A | 13 May 2021 |
| | | RU 2017134730 A | 05 April 2019 |
| | | KR 20180011759 A | 02 February 2018 |
| | | BR 112017019751 A2 | 29 May 2018 |
| | | JP 2018512449 A | 17 May 2018 |
| | | WO 2016149668 A1 | 22 September 2016 |
| | | US 2016272639 A1 | 22 September 2016 |
| WO 2009053373 A1 | 30 April 2009 | PL 2215075 T3 | 30 April 2014 |
| | | US 2010222348 A1 | 02 September 2010 |
| | | ES 2448870 T3 | 17 March 2014 |
| | | EP 2215075 A1 | 11 August 2010 |
| | | JP 2011500758 A | 06 January 2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021013735 A1


### Non-patent literature cited in the description


- Synthetic Organic Chemistry. John Wiley & Sons, Inc
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopedia. John Wiley & Sons, 1999, vol. 8
- Organic Reactions. John Wiley & Sons, 1942, vol. 55
- Chemistry of Functional Groups. John Wiley & Sons, vol. 73
- **P. H. STAHL ; C. G. WERMUTH.** Handbook of Pharmaceutical Salts. Verlag Helvetica Chimica Acta, 2002